# EUROPEAN PATENT APPLICATION

(11) **EP 1 986 010 A1**
(43) Date of publication of application: **29.10.2008**
(21) Application number: 07105722.8
(22) Date of filing: 05.04.2007
(51) Int. Cl.: G01N 33/574

(54) **Methods and tools for discriminating colorectal adenomas and adenocarcinomas**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: van Wermeskerken, Stephanie Christine

(57) **Abstract**

The present invention relates to the differential expression of genes in colorectal adenoma and adenocarcinoma cells and their correlation with chromosomal aberrations. The present invention provides tools for detecting chromosomal aberrations linked to progression of adenomas into adenocarcinoma cells. The present invention discloses methods and tools for *in vivo* and *in vitro* diagnosis of colorectal tumours.

## Description

### FIELD OF THE INVENTION

The present invention relates to methods for tumour diagnosis, more particularly for the diagnosis of colorectal adenomas and adenocarcinomas. The present invention further provides marker genes, probes and arrays for performing these methods.

### BACKGROUND OF THE INVENTION

Cancer of the colorectal part of the gastrointestinal tract is a frequently occurring disorder. In a first stage a benign tumour (adenoma) occurs which can turn into a malignant cancer (adenocarcinoma). Not all adenomas progress to carcinomas. Indeed this progression into carcinomas occurs only in a small subset of tumours. Initiation of genomic instability is a crucial step and occurs in two ways in colorectal cancer (Lengauer et al. (1998) Nature, 396, 643-649). DNA mismatch repair deficiency leading to microsatellite instability (abbreviated as MSI or MIN), has been most extensively studied (di Pietro et al. (2005) Gastroenterology, 129, 1047-1059), but explains only about 15% of adenoma to carcinoma progression. In the other 85% of cases where colorectal adenomas progress to carcinomas, genomic instability occurs at the chromosomal level (CIN) giving rise to aneuploidy. While for a long time these chromosomal aberrations have been regarded as random noise, secondary to cancer development, it has now been well established that these DNA copy number changes occur in specific patterns and are associated with different clinical behaviour (Hermsen et al. (2002). Gastroenterology 123, 1109-1119). Chromosomal aberrations frequently reported in colorectal cancers are 7pq, 8q, 13q, 20q gains and 4pq, 5q, 8p, 15q, 17p, 18q losses. It was shown that 8q, 13q and 20q gains and 8p, 15q, 17p and 18q losses, are associated with progression of colorectal adenomas to carcinomas (Hermsen *et al.* (2002) cited above). Gain of chromosome arm 20q is the most frequent gain observed in colorectal cancer, being altered in more than 65% of the cases (Meijer et al. (1998) J. Clin. Pathol. 51, 901-909). Gains on 20q, in particular the region 20q12-q13, are also commonly described in other types of solid tumours and have been associated with poor outcome in both gastric and colorectal cancers.

It is of utmost clinical importance to identify the above described progression of adenomas into adenocarcinomas in as early a stage as possible, to allow early stage treatment of carcinomas while avoiding unnecessary surgical intervention of adenomas. Ideally, the adenocarcinomas can be identified at a stage where the presence of malignant cells is not yet detectable by classical microscopic analyses.

Different studies refer to individual or limited sets of genes which show a different expression level in adenomas compared to adenocarcinomas, mostly without correlation with the underlying chromosomal instability (Habermann et al. (2007) Genes Chromosomes Cancer. 46, 10-26, US20040258761).

### SUMMARY OF THE INVENTION

The present invention is based on the observation that there is a significant difference in the expression pattern of genes in colorectal adenocarcinoma cells when compared with the expression pattern of genes of colorectal adenoma cells and that these differences are correlated with the occurrence of chromosomal aberrations in adenocarcinomas. Accordingly adenocarcinomas can be classified according to their chromosomal aberration and marker genes for these chromosomal aberrations have been identified. The large number of samples investigated in the present analysis resulted in a set of marker genes for all occurring chromosomal aberrations.

The present invention thus relates to methods and tools for cancer diagnosis, more specifically for the discrimination between adenomas and carcinomas in the colon and/or the rectum. More particularly, the present invention relates to the detection of the progression of an adenoma into a adenocarcinoma. It is an advantage of the methods of the present invention that they allow detection of the presence of adenocarcinoma cells at a very early stage i.e. before their presence can be detected by echography, radiography or MRI (magnetic resonance imaging).

The present invention is based on the finding that the progression of a colorectal adenoma into an adenocarcinoma is often caused by the appearance of a chromosomal gain or loss in an adenoma cell. Accordingly the present invention provides methods and tools for detecting adenocarcinomas by detecting a chromosomal gain or loss by indirect analysis methods.

According to the present invention chromosomal gain or loss, correlated with the progression from colorectal adenoma into adenocarcinoma, is detected indirectly by detection of altered expression levels of markers genes for the chromosomal gain or loss. Marker genes of which the expression is linked to chromosomal gain or loss can be divided into two classes. The first class of genes are those genes which are located in the regions of the chromosomes which are gained or lost. While a gene which is located in a chromosomal region which is lost will not be expressed, regulatory mechanism in the cell may upregulate the expression of the corresponding gene on the other intact chromosome. Contrary to what may be expected, not all genes which are located on a region of chromosomal gain are overexpressed. Thus the mere knowledge of the location of a gene within a region of chromosomal gain or loss is not sufficient to predict whether, and if so how, the expression of the gene is affected. The second class of genes are those genes which are themselves not located in a region of chromosomal gain or loss, but of which the expression is influenced by one or more genes located on a chromosomal aberration.

The present invention provides combinations of marker genes which allow a reliable detection of the presence of adenocarcinoma cells in a sample.

In one aspect, the present invention discloses marker genes which have not previously been identified as marker genes for the progression of colorectal adenoma cells into carcinoma cells (Table 17).

Accordingly, the present invention provides *in vitro* methods for detecting the presence of colorectal adenocarcinoma cells in a patient, the method comprising the steps of (a) detecting in a test sample of that patient the expression level of one or more marker genes, selected from the group listed in Table 17, and (b) comparing the expression level of these one or more marker genes used in step (a) to that of a control. An elevated or decreased expression level of the marker genes in the test sample compared to the control sample, is indicative of the presence of colorectal adenocarcinoma cells in the patient. According to a particular embodiment, the methods of the invention comprise detecting the expression level of one or more marker genes, selected from the group listed in Table 17, whereby the marker genes are genes of which the expression is upregulated in adenocarcinoma cells compared to adenoma cells.

In another aspect, the present invention provides an extensive list of marker genes such that a representative number of marker genes can be used to reliably determine the progression of colorectal adenoma cells into carcinoma cells (Table 1).

Accordingly, the present invention provides *in vitro* methods for detecting the presence of colorectal adenocarcinoma cells in a patient, the method comprising the steps of (a) detecting in a test sample of said patient the expression level of at least 12 of the marker genes, selected from the group listed in Table 1, and (b) comparing the expression level of the marker genes used in step a) to that of a control sample. An elevated or decreased expression level of these marker genes in the test sample compared to the control sample, is indicative of the presence of colorectal adenocarcinoma cells in the patient.

In yet another aspect, the present invention discloses marker genes the expression level of which is correlated with the presence of a specific type of chromosomal aberration in a colorectal adenoma cell (Tables 2 to 9).

Accordingly, the present invention provides *in vitro* methods for detecting the presence of colorectal adenocarcinoma cells in a patient based on the detection of marker genes the expression of which is correlated with the presence of a chromosomal aberration. More specifically, the methods according to this aspect of the invention comprise the steps of (a) detecting in a test sample of the patient the expression level of one or more marker genes, the expression of which is altered upon the presence of a chromosomal aberration, and (b) comparing the expression level of the one or more marker genes used in step (a) to that of control sample(s). An elevated or decreased expression level of the one or more marker genes in the test sample compared to the control sample, is indicative of the presence of adenocarcinoma cells in the patient.

In particular embodiments of this aspect of the invention, the one or more marker genes comprise:
- two or more marker genes selected from the group of marker genes depicted in Table 2 of which the expression level is altered by chromosomal loss at chromosome 8p and/or,
- one or more marker genes selected from the group of marker genes depicted in Table 3 of which the expression level is altered by chromosomal gain at chromosome 8q and/or,
- three or more marker genes selected from the group of marker genes depicted in Table 4 of which the expression level is altered by chromosomal gain at chromosome 13q and/or
- one or more marker genes selected from the group of marker genes depicted in Table 5 of which the expression level is altered by chromosomal loss at chromosome 15q and/or
- one or more marker genes selected from the group of marker genes depicted in Table 6 of which the expression level is altered by chromosomal loss at chromosome 17p and/or,
- three or more marker genes selected from the group of marker genes depicted in Table 7 of which the expression level is altered by chromosomal loss at chromosome 18q and/or,
- nine or more marker genes selected from the group of marker genes depicted in Table 8 of which the expression level is altered by chromosomal gain at chromosome 20q and/or
- two or more marker genes selected from the group of marker genes depicted in Table 9 of which the expression level is altered by chromosomal gain at chromosome 20q.

In yet another aspect, the present invention discloses sets of marker genes which are representative for the different chromosomal aberrations linked to colorectal adenocarcinoma. These aberrations are known to occur in at least 85% of all occurring colorectal adenocarcinomas, thus representing a reliable screening assay for the detection of adenocarcinomas.

Accordingly, the present invention provides *in vitro* methods for detecting the presence of colorectal adenocarcinoma cells in a patient, the method comprising the steps of (a) detecting in a test sample of the patient the expression level of a plurality of marker genes representative of the occurrence of chromosomal aberrations linked to colorectal adenocarcinomas and (b) comparing the expression level of the plurality of marker genes used in step a) to that of a control sample. An elevated or decreased expression level of the plurality of markers in the test sample compared to the control sample, is indicative of the presence of adenocarcinoma cells in the patient. More particularly, the plurality of marker genes comprises:
- at least one marker gene of which the expression level is altered by chromosomal loss at chromosome 8p, selected from the group of marker genes depicted in Table 2, and
- at least one marker gene of which the expression level is altered by chromosomal gain at chromosome 8q, selected from the group of marker genes depicted in Table 3, and
- at least one marker gene of which the expression level is altered by chromosomal gain at chromosome 13q, selected from the group of marker genes depicted in Table 4, and
- at least one marker gene of which the expression level is altered by chromosomal loss at chromosome 15q, selected from the group of marker genes depicted in Table 5, and
- at least one marker gene of which the expression level is altered by chromosomal loss at chromosome 17p, selected from the group of marker genes depicted in Table 6, and
- at least one marker gene of which the expression level is altered by chromosomal loss at chromosome 18q, selected from the group of marker genes depicted in Table 7, and
- at least one marker gene of which the expression level is altered by chromosomal gain at chromosome 20q, selected from the group of marker genes depicted in Table 8 or in Table 9.

In yet another aspect, the present invention discloses marker genes the expression level of which is altered upon the presence a specific type of chromosomal aberration in a colorectal adenoma cell and which are located within that chromosomal aberration (Tables 10-16).

Accordingly, the present invention provides *in vitro* methods for detecting the presence of colorectal adenocarcinoma cells in a patient, the method comprising the steps of (a) detecting in a test sample of the patient the expression level of a plurality of marker genes representative of the occurrence of chromosomal aberrations linked to colorectal adenocarcinomas and (b) comparing the expression level of the plurality of marker genes used in step a) to that of a control sample. An elevated or decreased expression level of the plurality of markers in the test sample compared to the control sample, is indicative of the presence of adenocarcinoma cells in the patient. More particularly, the plurality of marker genes comprises:
- at least one marker gene located in the region of chromosomal loss at chromosome 8p, selected from the group of marker genes depicted in Table 10, and
- at least one marker gene located in the region of chromosomal gain at chromosome 8q, selected from the group of marker genes depicted in Table 11, and
- at least one marker gene located in the region of chromosomal gain at chromosome 13q, selected from the group of marker genes depicted in Table 12, and
- at least one marker gene located in the region of chromosomal loss at chromosome 15q, selected from the group of marker genes depicted in Table 13, and
- at least one marker gene located in the region of chromosomal loss at chromosome 17p, selected from the group of marker genes depicted in Table 14, and
- at least one marker gene located in the region of chromosomal loss at chromosome 18q, selected from the group of marker genes depicted in Table 15, and
- at least one marker gene located in the region of chromosomal gain at chromosome 20q, selected from the group of marker genes depicted in Table 16.
   In particular embodiments of the above methods of the invention, the marker genes used are selected such that
- the one or more marker genes, the expression level of which is altered by a chromosomal loss at chromosome 8p or which are located in a region of chromosomal loss at chromosome 8p are selected from the group of marker genes consisting of NM_020749, NM_004315, NM_003747, NM_016353, NM_152415, NM_006197, NM_000662, NM_000015, D31887, NM_017884, NM_004462, NM_006765, NM_001715, NM_012331, NM_139167, NM_013354 and NM_005144, depicted in Table 10, and/or
- the one or more marker genes, the expression level of which is altered by a chromosomal gain at chromosome 8q or which are located in a region of chromosomal gain at chromosome 8q, are selected from the group of marker genes consisting of NM_138455, NM_032611, NM_032862, AL713790, BC030520, NM_024035, NM_017767, NM_002346, AF289596, NM_012162 and AB051475, depicted in Table 11 and/or
- the one or more marker genes, the expression level of which is altered by a chromosomal gain at chromosome 13q or which are located in a region of chromosomal gain at chromosome 13q, are selected from the group of marker genes consisting of NM_145293, NM_005358, U50531, NM_012158, NM_017817, NM_003899, NM_003903, NM_018386, BC008975, NM_023011, NM_001260, NM_006646, U50524, NM_033111, NM_024808, NM_014832, NM_006002, NM_015057, NM_024546, BC026126, NM_006493, NM_018210 and NM_017664, depicted in Table 12 and/or
- the one or more marker genes, the expression level of which is altered by a chromosomal loss at chromosome 15q or which are located in a region of chromosomal loss at chromosome 15q, are selected from the group of marker genes consisting of NM_030574, NM_004255, NM_002573, AB033025, NM_033240, NM_000126, NM_015079, NM_015969 and NM_016073, depicted in Table 13 and/or
- the one or more marker genes, the expression level of which is altered by a chromosomal loss at chromosome 17p or which are located in a region of chromosomal loss at chromosome 17p are selected from the group of marker genes consisting of NM_130766, NM_015721 and NM_031430, depicted in Table 14 and/or
- the one or more marker genes, the expression level of which is altered by a chromosomal loss at chromosome 18q or which are located in a region of chromosomal loss at chromosome 18q are selected from the group of marker genes consisting of NM_004715, NM_006701 and NM_014913, depicted in Table 15 and/or
- the one or more marker genes, the expression level of which is altered by a chromosomal gain at chromosome 20q or which are located in a region of chromosomal gain at chromosome 20q are selected from the group of marker genes consisting of NM_016397, NM_018270, NM_006602, NM_080476, NM_017896, NM_006097, NM_021809, NM_018840, NM_003600, NM_017495, NM_007002, NM_016354, NM_014071, NM_002212, NM_003185, NM_152255, NM_022082, NM_018244, NM_014902, NM_032013, NM_020182, NM_006886, NM_020673, BC003122, NM_012325, NM_014183, NM_021100, NM_004738, NM_016045, NM_014054, NM_022105, NM_015666, NM_032527, BC025345, NM_033405, NM_006892, NM_005225, NM_000687, BC035639, NM_018677, NM_006047, NM_016436, NM_015511, NM_016082, NM_007238, NM_003908, NM_003610, NM_153360, NM_080425, NM_000114, NM_001853, NM_144498, NM_017798 and NM_012384, depicted in Table 16, more particularly are selected from the group consisting of The method according to claims 3 or 4, wherein the marker genes the expression level ofwhich is altered by a chromosomal gain at chromosome 20q are selected from the group of marker genes consisting ofNM_016397, NM_018270, NM_006602, NM_080476, NM_017896, NM_006097, NM_021809, NM_018840, NM_003600, NM_017495, NM_007002, NM_016354, NM_014071, NM_002212, NM_003185, NM_152255, NM_022082, NM_018244, NM_014902, NM_032013, NM_020182, NM_006886, NM_020673, BC003122, NM_012325, NM_014183, NM_0211 00, NM_004738, NM_016045, NM_014054, NM_022105, NM_015666, NM_032527, BC025345 and NM_033405, depicted in Table 16.

The present invention further discloses a set of marker genes of which a difference in expression level is correlated with the presence of three, four or even five different chromosomal aberrations (Table 18).

Accordingly, the present invention provides *in vitro* methods for detecting the presence of colorectal adenocarcinoma cells in a patient, the method comprising the steps of (a) detecting in a test sample of the patient the expression level of one or more marker genes selected from the marker genes depicted in Table 18 and (b) comparing the expression level of these marker genes used in step (a) to that of a control sample. An elevated or decreased expression level of these one or more marker genes in the test sample compared to the control sample, is indicative of the presence of adenocarcinoma cells in the patient.

Particular embodiments of the methods of the invention described above, relate to methods wherein the marker genes are further selected based on their p-value or FDR value. More specifically, the marker genes have a p or FDR value below 0,05 or more particularly below 0,01, as indicted in Tables 1 to 9.

Further particular embodiments of the methods of the invention described above, relate to methods wherein the marker genes are further selected based on the size of the difference in expression between the adenocarcinoma and adenoma cells. More particularly, those markers are selected which are indicated as having a difference in expression level of at least `2', more particularly, of at least '4', in accordance with Tables 2 to 9.

Typically, in the in vitro methods of the present invention, the test sample is a sample which comprises or is suspected to comprise cells of the colorectal lesion. In one embodiment, the test sample is a sample from a biopsy or ressection of the colorectal lesion. Alternatively, the test sample is selected from the group consisting of a sample of urine, blood, saliva, sweat and stool.

In particular embodiments of the methods of the present invention the control sample is a sample of the same tissue of a patient comprising colorectal adenoma cells and not colorectal carcinoma cells. In alternative embodiments the control sample is an adenoma standard.

In particular embodiments of the in vitro methods of the present invention, the expression level is determined at the DNA or RNA level. Alternative embodiments encompass the determination of marker gene expression at the protein level.

The methods of the present invention are particularly suitable for diagnosing the progression of a colorectal adenoma into a colorectal carcinoma.

A further aspect of the invention provides kits for detecting colorectal adenocarcinoma cells comprising agents for specifically detecting marker genes the expression of which is indicative of the presence of adenocarcinoma cells. The agents are optionally functionalised with a label, such as a label selected from a radioactive label, a magnetic label an MRI contrast label, a chromophoric label, and an ultrasound label. In a particular embodiment, the kits comprise agents for the specific detection of the expression of at least 12 of the marker genes depicted in Table 1.

Alternatively, kits are provided for detecting colorectal adenocarcinoma cells comprising agents for specifically detecting the expression of at least one of the marker genes depicted in Table 17.

Specific embodiments of the invention relate to kits for detecting colorectal adenocarcinoma cells comprising agents for specifically detecting the expression of one or more marker genes, said kit comprising:
- agents for specifically detecting three or more marker genes selected from the group depicted in Table 2 and/or,
- agents for specifically detecting one or more marker genes selected from the group depicted in Table 3 and/or,
- agents for specifically detecting two or more marker genes selected from the group depicted in Table 4 and/or,
- agents for specifically detecting one or more marker genes selected from the group depicted in Table 5 and/or,
- agents for specifically detecting one or more marker genes selected from the group depicted in Table 6 and/or,
- agents for specifically detecting three or more marker genes selected from the group depicted in Table 7 and/or,
- agents for specifically detecting seven or more marker genes selected from the group depicted in Table 8 and/or,
- agents for specifically detecting seven or more marker genes selected from the group depicted in Table 9.

Further embodiments of the kits of the present invention relate to kits for detecting colorectal adenocarcinoma cells comprising a set of agents for specifically detecting the expression of at least one marker gene of each of the following groups: the marker genes depicted in Table 2, the marker genes depicted in Table 3, the marker genes depicted in Table 4, The marker genes depicted in Table 5, the marker genes depicted in Table 6, the marker genes depicted in Table 7, the marker genes depicted in Table 8 and the marker genes depicted in Table 9.

Further specific embodiments of the kits of the present invention described above provide agents for the specific detection of marker genes wherein said marker genes have a p or FDR value below 0,05, more specifically below 0,01, according to Table 1 to 9.

Further specific embodiments of the kits of the present invention described above provide agents for the specific detection of marker genes wherein said marker genes have a difference in expression level between an adenoma and an adenocarcinoma cell of at least "2", more particularly at least "4", according to Table 2 to 9 or 17.

In further specific embodiments of the kits described herein, the agents are oligonucleotides or antibodies. The oligonucleotides are optionally arrayed on a support. Further specific embodiments of the kits described herein relate to kits comprising antibodies, wherein the antibodies bind to proteins encoded by marker genes. In a particular embodiment, the antibodies bind to proteins encoded by the marker genes whereby the proteins are secreted proteins or proteins on the cell surface.

Yet a further aspect of the invention relates to *in vivo* methods for the detection of adenocarcinoma cells in colorectal tissue of a patient.

More specifically, the present invention provides *in vivo* methods for detecting adenocarcinoma cells in a colorectal lesion said method comprising the steps of contacting the colorectal lesion with a labelled agent capable of specifically binding or interacting with a protein expressed by a marker gene selected from the gene depicted in Table 17 and (b) detecting the binding or interaction of the agent with said colorectal lesion. The detection of a difference in the binding or interaction of said agent within loci of the colorectal lesion is indicative for the presence of adenocarcinoma cells in the lesion.

In particular embodiments, the marker gene used in the in vivo methods of the present invention is a gene encoding a protein located at the cell membrane, a secreted protein or an enzyme.

The present invention thus provides agent binding or interacting specifically with a marker gene depicted in Table 17 or 18, for use as an in vivo diagnostic for detecting the presence of an adenocarcinoma cell in a colorectal adenoma tissue.

Accordingly, the present invention also relates to the use of an agent binding or interacting specifically with a marker gene depicted in Table 17 or 18, in the manufacture of a diagnostic for detecting the presence of adenocarcinoma cell in a colorectal tumour tissue.

The above and other characteristics, features and advantages of the present invention will become apparent from the following detailed description. This description is given for the sake of example only, without limiting the scope of the invention.

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### Definitions

"Tumour" or "neoplasm" as used herein refers to an abnormal tissue that grows by cellular proliferation more rapidly than normally, and continues to grow after the stimuli that initiated the new growth cease. The term "lesion", generally referring to an abnormality involving any tissue or organ due to any disease or any injury, is also used herein to refer to a neoplasm. Tumours, neoplasm or lesions can be either benign or malignant.

"Cancer" is a general term referring to any type of malignant neoplasm.

"Adenoma", (or non-progressed adenoma) as used herein, relates to a benign epithelial neoplasm. Adenomas are usually well circumscribed, they can be flat or polypoid and the neoplastic cells do not infiltrate or invade adjacent tissue.

"Adenocarcinoma", as used herein, relates to a malignant neoplasm of epithelial cells. Most commonly carcinoma's form a glandular or glandlike pattern. Synonyms are "glandular cancer" and "glandular carcinoma". Malignant cells are often characterized by characterized by progressive and uncontrolled growth. They can spread locally or though the blood stream and lymphatic system to other parts of the body.

"Progressed adenoma" refers to an adenoma that harbours a focus of a cancer. This is also called a "malignant polyp". Colorectal adenomas are common in the elderly population, but only a small proportion of these pre-malignant tumours (estimated approximately 5%) progresses to malignant tumours (i.e. colorectal adenocarcinoma).

"Colorectal" relates to of the colon and/or the rectum, i.e. the complete large intestine.

"Chromosomal aberration", as used herein refers to a chromosomal loss or gain, i.e. a region in the chromosome which has been deleted or duplicated.

"Marker gene", as used herein is a gene of which expression differs (decreased or increased) between adenoma and adenocarcinoma cells, and which can thus be used to differentiate between adenoma and carcinoma cells.

"Expression profile" refers to the expression level of a number of a marker genes in a cell or a sample.

The present invention provides diagnostic methods and tools for distinguishing between malignant and benign colorectal lesions and for diagnosing the presence of colorectal cancer in a subject. The diagnostic methods of the present invention allow a reliable detection of very early-stage colorectal cancers.

One aspect of the present invention relates to *in vitro* methods for detecting the presence of colorectal adenocarcinoma cells in a patient, the method comprising the steps of detecting in a test sample of that patient the expression level of one or more marker genes. The expression level of the marker gene(s) is compared to that of a control sample, whereby

a difference in expression level of the marker gene(s) in the test sample compared to the expression level in the control sample, is indicative of the presence of colorectal adenocarcinoma cells in the patient.

In general, the marker genes used in the methods of the invention are differentially expressed between adenoma and carcinoma cells. A further selection can be made based on the size of the difference, i.e. the extent to which up-or down-regulation of a marker gene occurs (e.g. two-fold, four-fold or eight-fold or even more) in a adenocarcinoma sample. The expression levels are indicated in the Tables under the header "effect" as log-2 values. Accordingly a value of 1 refers to a 2-fold higher expression of a gene in an adenocarcinoma compared to an adenoma, a value of 2 refers to a 4-fold higher expression, a value of 3 refers to an 8-fold higher expression, etc. Similarly, a value of -1 refers to a 2-fold lower expression of a gene in an adenocarcinoma compared to an adenoma, a value of -2 refers to a 4-fold lower expression, a value of-3 refers to an 8-fold lower expression, etc. Alternatively, a further selection of the marker genes may also be made based on a calculation of the statistical significance (using p-value or FDR (False Discovery Rate) value) of the differential expression in adenoma and carcinoma cells of the marker. In particular embodiments, both selection criteria are used.

In one embodiment of the present invention, the presence of adenocarcinoma cells in a patient is identified by determining the expression of one or more marker genes of which the expression is changed (increased or decreased) more than two-fold, four-fold or eight-fold when comparing adenoma and adenocarcinoma cells, and/or of which the p-value for the correlation between marker gene expression and occurrence of adenoma or adenocarcinoma cells is lower than 0,01 (is statistically significant).

The sample used for detection in the *in vitro* methods of the present invention may be collected in any clinically acceptable manner, but is collected such that nucleic acids (in particular RNA) or proteins are preserved. The samples which are analysed according to the present invention are generally colorectal biopsies or resections. Intact cells or lysed cells from tumour tissue may also detach from the colon without intervention and will end up in the faeces. Accordingly, stool samples are also considered as a suitable source for isolating RNA. Furthermore, colorectal adenocarcinoma cells may migrate into other tissues. Consequently, also blood and other types of sample can be used. As it is the aim to detect the progression from colorectal adenoma to adenocarcinoma as early as possible, a biopsy or resection may contain a majority of adenoma cells and only a minority of adenocarcinoma cells. To increase the signal/background ratio, a resection can be divided into different sub-samples prior to analysis. Even if the total number of carcinoma cells in the biopsy or resection is limited, it can be expected that at least one of the sub-samples will contain a increased ratio of adenocarcinoma versus adenoma cells.

In the *in vitro* methods of the present invention, the expression of one or more marker genes is determined, at RNA or protein level, in a sample of the patient and compared to the expression of these genes in a control sample. The control sample can be an adenoma sample from the same patient. Alternatively, the control sample is an adenoma sample obtained from another individual or obtained from pooled samples of colorectal adenomas from one or more other individuals. Controls can also be made artificially by pooling a set of nucleic acids or proteins to mimic the RNA/protein content of a colorectal adenoma tissue.

In a first embodiment, the marker genes characterised as being either up- or down-regulated in colorectal carcinoma cells compared to a control such as colorectal adenoma cells, are selected from Table 1. While any of the marker genes listed in Table 1 is suitable for use in the methods of the present invention for identifying the presence of colorectal adenocarcinoma cells in a sample, an important advantage of the present invention is the provision of an extensive list of suitable markers so as to allow an increased reliability of detection. Accordingly, particular embodiments of the invention relate to the use of at least 2, at least 5, at least 10, 12 or 15, at least 20, at least 50, at least 100, at least 200, at least 500 or all of the marker genes of Table 1. In a particular embodiment, a subset of marker genes of Table 1 is used, namely those marker genes of Table 1, which have a p value below 0,01.

In another embodiment, the marker genes characterised as being either up regulated or down-regulated in colorectal carcinoma cells compared to a control such as colorectal adenoma cells, are selected from Table 17. None of the marker genes of Table 17 have previously been identified as marker genes of colorectal adenocarcinomas and each of the marker genes of Table 17 is suitable for use in the methods of the present invention for identifying the presence of colorectal adenocarcinoma cells in a sample. In particular embodiments the expression of 1, 2, 3, 4, 5, 6, 7, 8, 9, 10 or more of the genes identified in Table 17 is determined in a sample of the patient and compared to a control to identify the presence of adenocarcinoma cells in the patient.

According to a further embodiment of the methods of the present invention, subsets of marker genes of Table 1 are use of which the expression level is directly correlated with a particular chromosomal aberration in colorectal tumour cells.

In one embodiment, the present invention provides a set of marker genes of which the expression level is altered when a chromosomal loss occurs in colorectal adenoma cells at chromosome 8p. These marker genes are listed in Table 2. Accordingly any of the marker genes listed in Table 2 can be used in the methods and tools of the present invention for identifying colorectal carcinoma cells. In the method and tools of the present invention at least 1, at least 2, at least 3, at least 5, at least 10, at least 20, at least 50, at least 100, at least 200 or all of the marker genes of Table 2 are used. Additionally or alternatively, the marker gene(s) used in the methods of the invention is/are selected from a subset of Table 2, namely those marker genes of Table 2, which have a FDR (False Discovery Rate) value below 0,05 or, more particularly below 0,01. Additionally or alternatively, the marker gene(s) used in the methods of the present invention is/are selected from a subset of Table 2 corresponding to those marker genes for which the expression level is increased in adenocarcinoma cells compared to adenocarcinoma cells. Additionally or alternatively, the marker gene(s) used in the methods of the present invention is/are selected from a subset of Table 2 corresponding to those marker genes for which the expression level in adenoma and adenocarcinoma cells differs with at least a factor 2, at least a factor 4 or at least a factor 8. Equally a subset can be created of marker genes having a difference in expression level (increase or decrease) between adenoma and adenocarcinoma of at least a factor 2, 4 or 8 and for which the FDR value is below 0,05 or below 0,01.

In another embodiment, the present invention provides a set of marker genes of which the expression level is altered when a chromosomal gain occurs in colorectal adenoma cells at chromosome 8q. These marker genes are listed in Table 3. Accordingly any of the marker genes listed in Table 3 can be used in the methods and tools of the present invention for identifying colorectal carcinoma cells. In the methods and tools of the present invention at least 1, at least 2, at least 3, at least 5, at least 10, at least 20, or all of the marker genes of Table 3 are used. Additionally or alternatively, the marker gene(s) used in the methods of the invention is/are selected from a subset of Table 3, namely those marker genes of Table 3, which have a FDR (False Discovery Rate) value below 0,05 or, more particularly below 0,01. Additionally or alternatively, the marker gene(s) used in the methods of the present invention is/are selected from a subset of Table 3 corresponding to those marker genes for which the expression level is increased in adenocarcinoma cells compared to adenocarcinoma cells. Additionally or alternatively, the marker gene(s) used in the methods of the present invention is/are selected from a subset of Table 3 corresponding to those marker genes for which the expression level in adenoma and adenocarcinoma cells differs with at least a factor 2, at least a factor 4 or at least a factor 8. Equally a subset can be created from Table 3 of marker genes having a difference in expression level (increase or decrease) between adenoma and adenocarcinoma of at least a factor 2, 4 or 8 and for which the FDR value is below 0,05 or below 0,01.

In another embodiment, the present invention provides a set of marker genes of which the expression level is altered when a chromosomal gain occurs in colorectal adenoma cells at chromosome 13q. These marker genes are listed in Table 4. Accordingly any of the marker genes listed in Table 4 can be used in the methods and tools of the present invention for identifying colorectal carcinoma cells. In one embodiment of the methods and tools of the present invention at least 1, at least 2, at least 3, at least 5, at least 10, at least 20, at least 50, at least 100 or all of the marker genes of Table 4 are used. Additionally or alternatively, the marker gene(s) used in the methods of the invention is/are selected from a subset of Table 4, namely those marker genes of Table 4, which have a FDR (False Discovery Rate) value below 0,05 or, more particularly below 0,01. Additionally or alternatively, the marker gene(s) used in the methods of the present invention is/are selected from a subset of Table 4 corresponding to those marker genes for which the expression level is increased in adenocarcinoma cells compared to adenocarcinoma cells. Additionally or alternatively, the marker gene(s) used in the methods of the present invention is/are selected from a subset of Table 4 corresponding to those marker genes for which the expression level in adenoma and adenocarcinoma cells differs with at least a factor 2, at least a factor 4 or at least a factor 8. Equally a subset can be created from Table 4 of marker genes having a difference in expression level (increase or decrease) between adenoma and adenocarcinoma of at least a factor 2, 4 or 8 and for which the FDR value is below 0,05 or below 0,01.

In another embodiment, the present invention provides a set of marker genes of which the expression level is altered when a chromosomal loss occurs in colorectal adenoma cells at chromosome 15q. These marker genes are listed in Table 5. Accordingly any of the marker genes listed in Table 5 can be used in the methods and tools of the present invention for identifying colorectal carcinoma cells. In a particular embodiment of the methods and tools of the present invention at least 1, at least 2, at least 3, at least 5, at least 10, at least 20, at least 30 or all of the marker genes of Table 5 are used. Additionally or alternatively, the marker gene(s) used in the methods of the invention is/are selected from a subset of Table 5, namely those marker genes of Table 5, which have a FDR (False Discovery Rate) value below 0,05 or, more particularly below 0,01. Additionally or alternatively, the marker gene(s) used in the methods of the present invention is/are selected from a subset of Table 5 corresponding to those marker genes for which the expression level is increased in adenocarcinoma cells compared to adenoma cells. Additionally or alternatively, the marker gene(s) used in the methods of the present invention is/are selected from a subset of Table 5 corresponding to those marker genes for which the expression level in adenoma and adenocarcinoma cells differs with at least a factor 2, at least a factor 4 or at least a factor 8. Equally a subset can be created of marker genes from Table 5 having a difference in expression level (increase or decrease) between adenoma and adenocarcinoma of at least a factor 2, 4 or 8 and for which the FDR value is below 0,05 or below 0,01.

In another embodiment, the present invention provides a set of marker genes of which the expression level is altered when a chromosomal loss occurs in colorectal adenoma cells at chromosome 17p. These marker genes are listed in Table 6. Accordingly, any of the marker genes listed in Table 6 can be used in the methods and tools of the present invention for identifying colorectal carcinoma cells. In a particular embodiment of the methods and tools of the present invention at least 1, at least 2, at least 3, at least 4, at least 6 or all of the marker genes of Table 6 are used. Additionally or alternatively, the marker gene(s) used in the methods of the invention is/are selected from a subset of Table 6, namely those marker genes of Table 6, which have a FDR (False Discovery Rate) value below 0,1. Additionally or alternatively, the marker gene(s) used in the methods of the present invention is/are selected from a subset of Table 6 corresponding to those marker genes for which the expression level is increased in adenocarcinoma cells compared to adenoma cells. Additionally or alternatively, the marker gene(s) used in the methods of the present invention is/are selected from a subset of Table 6 corresponding to those marker genes for which the expression level in adenoma and adenocarcinoma cells differs with at least a factor 2, at least a factor 4 or at least a factor 8. Equally a subset can be created of Table 6, of marker genes having a difference in expression level (increase or decrease) between adenoma and adenocarcinoma of at least a factor 2, 4 or 8 and for which the FDR value is below 0,1.

In another embodiment, the present invention provides a set of marker genes of which expression level is altered when a chromosomal loss occurs in colorectal adenoma cells at chromosome 18q. These marker genes are listed in Table 7. Accordingly any of the marker genes listed in Table 7 is used in the methods and tools of the present invention for identifying colorectal carcinoma cells. In a particular embodiment of the methods and tools of the present invention at least 1, at least 2, at least 3, at least 5, at least 10, at least 20, at least 50, at least 100, at least 200 or all of the marker genes of Table 7 are used. Additionally or alternatively, the marker gene(s) used in the methods of the invention is/are selected from a subset of Table 7, namely those marker genes of Table 7, which have a FDR (False Discovery Rate) value below 0,05 or, more particularly below 0,01. Additionally or alternatively, the marker gene(s) used in the methods of the present invention is/are selected from a subset of Table 7 corresponding to those marker genes for which the expression level is increased in adenocarcinoma cells compared to adenoma cells. Additionally or alternatively, the marker gene(s) used in the methods of the present invention is/are selected from a subset of Table 7 corresponding to those marker genes for which the expression level in adenoma and adenocarcinoma cells differs with at least a factor 2, at least a factor 4 or at least a factor 8. Equally a subset can be created of Table 7, of marker genes having a difference in expression level (increase or decrease) between adenoma and adenocarcinoma of at least a factor 2, 4 or 8 and for which the FDR value is below 0,05 or, more particularly below 0,01.

In another embodiment, the present invention provides a set of marker genes of which expression level is altered when a chromosomal gain occurs in colorectal adenoma cells at chromosome 20q. These marker genes are listed in Table 8 and 9, wherein the data in Table 8 are derived from a univariant analysis and the data in Table 9 from a multivariant analysis. Accordingly, any of the marker genes listed in Table 8 or 9 can be used in the methods and tools of the present invention for identifying colorectal carcinoma cells. In a particular embodiment of the methods and tools of the present invention at least 1, at least 2, at least 4, at least 6, at least 8, at least 10, at least 15, at least 20, at least 25, at least 50, at least 100, at least 200, at least 400, at least 600 or all of the marker genes of Table 8 or 9 are used. Additionally or alternatively, the marker gene(s) used is/are selected from a subset of Table 8 or 9, namely those marker genes of Table 8 or 9, which have a FDR value below 0,05 or more particularly below 0,01. Additionally or alternatively, the marker gene(s) used in the methods of the present invention is/are selected from a subset of Table 8 or 9 corresponding to those marker genes for which the expression level is increased in colorectal adenocarcinoma cells compared to adenoma cells. Additionally or alternatively, the marker gene(s) is/are selected from another subset of Table 8 or 9, namely those marker genes for which the expression level in adenoma and adenocarcinoma differs at least a factor 2, at least a factor 4 or at least a factor 8. Equally subsets of marker genes can be created of Table 8 or 9, wherein the marker genes have a difference in expression levels (increase or decrease) between adenoma and adenocarcinoma of at least a factor 2, 4 or 8 and an FDR value below 0,05 or below 0,01.

The above described sets of marker genes listed in Tables 2 to 9 comprise marker genes which are linked to a specific type of chromosomal aberration. It is however possible that a certain gene is upregulated by different chromosomal aberrations. Accordingly Tables 2 to 9 show a certain degree of redundancy. The marker genes which occur more than once in Tables 2 to 9 are particularly suitable for use in the methods of the present invention, as they are more generally characteristic of the occurrence of 'a' chromosomal aberration. Marker genes which expression level is altered by 3, 4 or 5 different chromosomal aberrations are presented in Table 18. Thus, a particular embodiment of the methods of the present invention relates to the use of at least 1, at least 2, at least 4, at least 6, at least 8, at least 10, at least 15, at least 20, at least 25, at least 50, at least 100, at least 200, at least 400, at least 600 or all of the marker genes of Table 18, for the identification of colorectal adenocarcinoma cells in a patient. Additionally or alternatively, the marker gene(s) used in the methods of the present invention is/are selected from a subset of Table 18 corresponding to those marker genes for which the expression level is increased in adenocarcinoma cells compared to adenoma cells.

The present invention not only provides marker genes differentially expressed between colorectal adenoma and adenocarcinoma cells, but further correlates this differential expression to the presence of a chromosomal aberration. This has allowed the development of a diagnostic tool using a set of marker genes representative for each of the chromosomal aberrations.

Thus further particular embodiments relate to sets of marker genes which comprises marker genes indicative of each of the chromosomal aberrations. More particularly, such sets of marker genes comprise:
- at least 1, at least 2, at least 5, at least 10 or more marker genes selected from Table 2 (marker genes correlated with 8p loss) and
- at least 1, at least 2, at least 5, at least 10 or more marker genes selected from Table 3 (marker genes correlated with 8q gain) and
- at least 1, at least 2, at least 5, at least 10 or more marker genes selected from Table 4 (marker genes correlated with 13 q gain) and
- at least 1, at least 2, at least 5, at least 10 or more marker genes selected from Table 5 (marker genes correlated with 15q loss), and
- at least 1, at least 2, at least 5, 10 or more marker genes selected from Table 6 (marker genes correlate which correlate with 17p loss) and
- at least 1, at least 2, at least 5, at least 10 or more marker genes selected from Table 7 (marker genes correlated with 18q loss) and
- at least 1, at least 2, at least 5, at least 10 or more marker genes selected from Table 8 or 9 (marker genes correlates with 20q gain).

Additionally or alternatively, the marker gene(s) used in the methods of the present invention is/are selected from a subset of the sets of marker genes listed above corresponding to those marker genes for which the expression level is increased in colorectal adenocarcinoma cells compared to adenoma cells.

In other embodiments of the invention, the sets of marker genes indicative of each of the chromosomal aberrations used in the methods and tools of the present invention comprise genes which are located within the chromosomal region itself which is gained or lost. Accordingly, the sets of marker genes comprise:
- at least 1, at least 2, at least 5, at least 10 or all of the genes associated with chromosomal loss at 8p, listed in Table 10. More particularly, one or more marker genes are selected from the group consisting of NM_020749 and NM_004315 and/or selected from the group consisting of NM_003747, NM_016353, NM_152415, NM_006197, NM_000662, NM_000015 and D31887, and
- at least 1, at least 2, at least 5, at least 10 or all of the genes associated with chromosomal gain at 8q, listed in Table 11. More particular one or more genes are selected from the group consisting of NM_138455, NM_032611, NM_032862, and
- at least 1, at least 2, at least 5, at least 10 or all of the genes associated with chromosomal gain at 13q, listed in Table 12, particularly NM_145293 and or NM_005358. More particularly one or more genes selected from the group consisting of U50531, NM_012158, NM_017817, NM_003899, NM_003903, NM_018386, BC008975 and NM_023011, and
- at least 1, at least 2, at least 5, at least 10 or all of the genes associated with chromosomal loss at 15q listed in Table 13. More particularly NM030574 or one or more of the genes selected from NM_004255, NM_002573 and AB033025.
- at least 1, at least 2, at least 5, at least 10 or all of the genes associated with chromosomal loss at 17p listed in Table 14. More particularly one or more genes selected from the group consisting of NM_130766, NM_015721 and NM_031430, and
- at least 1, at least 2, at least 5, at least 10 or all of the genes associated with chromosomal loss at 18q and listed in Table 15. More particularly one or more marker genes selected from the group consisting of NM_004715, NM_006701 and NM_014913, and
- at least 1, at least 2, at least 5, at least 10, at least 25, at least 50, at least 100 or all of the genes associated with chromosomal gain at 20q and listed in Table 16. More particularly one or more marker genes selected from NM_016397 and NM_018270 and NM_006602. Other particular marker genes are one or both of NM_080476 and NM_017896. Other particular marker genes are one or more selected from the group consisting of NM_006097, NM_021809, NM_018840, NM_003600, NM_017495, NM_007002 and NM_016354. Other particular marker genes are one or more selected from the group consisting of NM_014071, NM_002212, NM_003185, NM_152255 and NM_022082. Yet other particular marker genes are one or more selected from the group consisting of NM_018244, NM_014902, NM_032013, NM_020182, NM_006886, NM_020673 and BC003122.

Additionally or alternatively, the marker gene(s) used in the methods of the present invention is/are selected from a subset of the genes listed above, corresponding to those marker genes for which the expression level is increased in colorectal adenocarcinoma cells compared to adenoma cells.

In general the marker genes described above are used in an *in vitro* method for detecting the presence of colorectal adenocarcinoma cells in a patient, the method comprising the steps of detecting in a test sample of that patient the expression level of one or more marker genes as defined in the above sets and subsets, comparing the expression level of the used marker gene(s) to that of a control sample (e.g. colorectal adenoma cells). An elevated or decreased expression level of the used marker gene(s) in the test sample, compared to the control sample, is indicative of the presence of colorectal adenocarcinoma cells in the patient.

The marker genes of the present invention are used in DNA, RNA or protein based expression analysis. A significant advantage of the marker genes of the present invention is that they are suitable for use in objective quantitative diagnostics. Methods based on markers identified in the prior art were limited because they relied on the examination of small numbers of histological samples comprising carcinoma cells.

In a particular embodiment, the marker genes are use in DNA or RNA-based expression analysis, which provides the advantage of increased sensitivity typical for DNA or RNA-based diagnostics. The methods and marker genes of the present invention allow to discriminate colorectal adenoma from carcinoma cells at a very early stage of the carcinogenic process.

The determination of the expression level of marker genes in a patient sample may be accomplished by any means known in the art. For example, expression levels of various marker genes may be assessed by separation of nucleic acid molecules (e.g. RNA or cDNA) obtained from the sample in agarose or polyacrylamide gels, followed by hybridisation with marker gene specific oligonucleotide probes. Alternatively, the difference in expression level may be determined by the labelling of nucleic acid obtained from the sample followed by separation on a sequencing gel. nucleic acid samples are placed on the gel such that patient and control or standard nucleic acid are in adjacent lanes. Comparison of expression levels is accomplished visually or by means of a densitometer.

In a particular embodiment, the expression of all marker genes used in the assay is assessed simultaneously by hybridisation to a DNA array (also called "microarray" or "DNA chip". Microarray based expression profiling can be carried out, for example, by the method as disclosed in "Microarray Biochip Technology" (Schena M., Eaton Publishing, 2000). A DNA array comprises immobilised high-density probes to detect a number of genes. The probes on the array are complementary to one or more parts of the sequence of a marker gene, or to the entire coding region of the marker gene. In the present invention, any type of polynucleotide can be used as probes for the DNA array. Typically, cDNAs, PCR products, and oligonucleotides are useful as probes. Thus, expression levels of a plurality of genes can be estimated at the same time by a single-round analysis.

A DNA array-based detection method generally comprises the following steps:
1) Isolating mRNA from a sample and optionally converting the mRNA to cDNA, and subsequently labelling this RNA or cDNA. Methods for isolating RNA, converting it into cDNA and for labelling nucleic acids are described in manuals for micro array technology.
2) Hybridising the nucleic acids from step 1 with probes for the marker genes. The nucleic acids from a sample can be labelled with a dye, such as the fluorescent dyes Cy3(red) or Cy5 (blue). Generally a control sample is labelled with a different dye.
3) Detecting the hybridisation of the nucleic acids from the sample with the probes and determining at least qualitatively, and more particularly quantitatively, the amounts of mRNA in the sample for the different marker genes investigated. The difference in the expression level between sample and control can be estimated based on a difference in the signal intensity. These can be measured and analysed by appropriate software such as, but not limited to the software provided for example by Affymetrix.

In a particular embodiment, the probes on an array can be arranged, for example according to marker genes correlated with a particular type of chromosomal aberration. Alternatively different arrays can be developed, each carrying probes for the detection of a particular type of chromosomal aberration.

There is no limitation on the number of probes corresponding to marker genes which are spotted on a DNA array. For example, one may select 1% or more, 5% or more, 20% or more, 50% or more, 70 % or more of any of the sets of the marker genes of the present invention. Also a marker gene can be represented by two or more probes hybridising to different parts of a gene. Probes are designed for each selected marker gene. Such a probe is typically an oligonucleotide comprising 5-50 nucleotide residues. Longer DNAs can be synthesised by PCR or chemically. Methods for synthesising such oligonucleotides and applying them on a substrate are well known in the field of micro-arrays.

Genes other than the marker genes may be also spotted on the DNA array. For example, a probe for a gene whose expression level is not significantly altered may be spotted on the DNA array to normalise assay results or to compare assay results of multiple arrays or different assays.

In particular embodiments of the methods of the present invention, the expression level of particular marker genes is assessed by determining the amount of protein expressed by the respective marker genes. For the analysis at the protein level, every marker gene described in the present invention can in principle be used, although some proteins may be less suitable, because of factors such as limited solubility, very high or small molecular weight or extreme isoelectric point.

Determination of expression level of a marker gene at the protein level can be accomplished, for example, by the separation of proteins from a sample on a polyacrylamide gel, followed by identification of a specific marker gene-derived protein using antibodies in a Western blot. Alternatively, proteins can be separated by two-dimensional gel electrophoresis systems. Two-dimensional gel electrophoresis is well-known in the art and typically involves isoelectric focusing along a first dimension followed by SDS-PAGE electrophoresis along a second dimension. The analysis of 2D SDS-PAGE gels can be performed by determining the intensity of protein spots on the gel, or can be performed using immune detection. In other embodiments, protein samples are analysed by mass spectroscopy.

The samples used *in vitro* assays generally will be colorectal biopsies or resections, more particularly adenomatous polyp biopsies or resections. For *in vitro* protein expression analysis, cells or cell lysates of biopsies or resections can be used. Accordingly, the localisation of the protein in the cell or the function of the protein to be assayed is of no importance for the analysis. The presence of adenocarcinoma cells in a patient is expected to be reflected by the presence of elevated or decreased levels of certain proteins secreted by adenocarcinoma cells. Such proteins can be present in blood, urine, sweat and other parts of the body. Equally, adenocarcinoma cells will release proteins to the colon lumen. In addition, intact adenocarcinoma cells or their lysed content may be released to the intestinal tract, and will be present in the faeces which can be used as a source for *in vitro* protein analysis. However, contrary to nucleic acids, proteins can not be amplified. Accordingly it is envisaged that, in particular embodiments the methods of the invention comprise an enrichment step, more particularly an enrichment of adenocarcinoma material. For instance a sample can be contacted with ligands specific for the cell membrane or organelles of adenoma and adenocarcinoma cells, functionalised for example with magnetic particles. The material concentrated by the magnetic particles can then be analysed for the detection of marker proteins.

In another aspect of the invention, the marker genes of the present invention are used for the differential detection of colorectal adenomas and adenocarcinomas in an *in vivo* analysis. The invention accordingly provides *in vivo* methods for detecting adenocarcinoma cells in a colorectal adenoma tissue said method comprising the steps of:
- contacting a labelled agent capable of specifically binding or interacting with a protein expressed by a marker gene with a colorectal lesion, and
- detecting the binding or interaction of the labelled agent with cells in the lesion;
whereby the detection of a locus in the lesion characterized by a difference in the binding or interaction of the labelled agent is indicative for the presence of adenocarcinoma cells in the colorectal adenoma.

The present invention provides a set of markers which have not been previously identified as markers for colorectal carcinomas and which can be used in the *in vivo* methods of the present invention (shown in Table 17). Any one of these markers is suitable for use in *the in vivo* diagnostic methods of the present invention. A particular embodiment of the invention provides a set of marker gene(s) which is/are selected from a subset of Table 17; corresponding to those marker genes for which the expression level is increased in colorectal adenocarcinoma cells compared to adenoma cells.

In a particular embodiment of the methods of the invention, a set of marker genes is analysed, so as to increase the reliability of detection. More particularly, according to a particular embodiment, a set of agents is used whereby each agent detects a marker associated with a different type of chromosomal aberration linked to colorectal adenocarcinoma.

Thus, according to one embodiment, a set of marker genes is used for the *in vivo* identification of the presence of carcinoma cells, which set of marker genes comprises marker genes indicative of each of the chromosomal aberrations. More particularly, such a set of marker genes comprises:
- one or more genes selected from Table 2 (marker genes correlated with 8p loss) and
- one or more genes selected from Table 3 (marker genes correlated with 8q loss) and
- one or more genes selected from Table 4 (marker genes correlated with 13 q gain) and
- one or more genes selected from Table 5 (marker genes correlated with 15q loss), and
- one or more genes selected from Table 6 (marker genes correlate which correlate with 17p loss) and
- one or more genes selected from Table 7 (marker genes correlated with 18q loss) and
- one or more genes selected from Table 8 or 9 (marker genes correlates with 20q gain).

Additionally or alternatively, sets of marker genes are provided for use in the *in vivo* methods of the invention which is/are selected from the sets of marker genes described above, corresponding to those marker genes for which the expression level is increased in colorectal adenocarcinoma cells compared to adenoma cells.

In other embodiments of *the in vivo* diagnostic methods invention, the sets of marker genes indicative of each of the chromosomal aberrations comprise genes which are located within the chromosomal regions itself which are gained or lost. Accordingly, the set of marker genes comprises:
- one or more genes selected from the marker genes listed in Table 10. More particularly, one or more marker genes are selected from the group consisting of NM_020749 and NM_004315 and/or selected from the group consisting of NM_003747, NM_016353, NM_152415, NM_006197, NM_000662, NM_000015 and D31887, and
- one or more genes selected from the marker genes listed in Table 11. More particular one or more genes are selected from the group consisting of NM_138455, NM_032611, NM_032862, and
- one or more genes selected from the marker genes listed in Table 12, particularly NM_145293 and or NM_005358. More particularly one or more genes selected from the group consisting of U50531, NM_012158, NM_017817, NM_003899, NM_003903, NM_018386, BC008975 and NM_023011, and
- one or more genes selected from the marker genes listed in Table 13. More particularly NM030574 or one or more of the genes selected from NM_004255, NM_002573 and AB033025.
- one or more genes selected from the marker genes listed in Table 14. More particularly one or more genes selected from the group consisting of NM_130766, NM_015721 and NM_031430, and
- one or more genes selected from the marker genes listed in Table 15. More particularly one or more marker genes selected from the group consisting of NM_004715, NM_006701 andNM_014913, and
- one or more genes selected from the marker genes listed in Table 16. More particularly one or more marker genes selected from NM_016397 and NM_018270 and NM_006602. Other particular marker genes are one or both of NM_080476 and NM_017896. Other particular marker genes are one or more selected from the group consisting of NM_006097, NM_021809, NM_018840, NM_003600, NM_017495, NM_007002 and NM_016354. Other particular marker genes are one or more selected from the group consisting of NM_014071, NM_002212, NM_003185, NM_152255 and NM_022082. Yet other particular marker genes are one or more selected from the group consisting of NM_018244, NM_014902, NM_032013, NM_020182, NM_006886, NM_020673 and BC003122.

Additionally or alternatively, sets of marker genes are provided for use in the *in vivo* methods of the invention which is/are selected from the sets of marker genes described above, corresponding to those marker genes for which the expression level is increased in colorectal adenocarcinoma cells compared to adenoma cells.

Analysis of the data of the present invention shows that the altered expression of one marker gene can be caused by different chromosomal aberrations. Table 18 shows marker genes which are altered by three, four, or even five different aberrations. According to a particular embodiment, *the in vivo* methods of the present invention are carried out using one or more marker genes selected from the marker genes listed in Table 18. Additionally or alternatively, *the in vivo* methods of the present invention are carried out using one or more marker genes selected from the marker genes listed in Table 18, corresponding to those marker genes for which the expression level is increased in colorectal adenocarcinoma cells compared to adenoma cells.

The digestive tract is well accessible for the administration of diagnostic compounds in a patient. In addition, colonoscopy can be used for the detection of labelled compounds. *In vivo* diagnostic techniques further allow the analysis of different polyps without collection of individual biopsies.

Marker genes which are particularly suitable for *in vivo* imaging are marker genes encoding proteins which are located on the cell surface or which are secreted. Alternatively, the detection of proteins encoded by marker genes is envisaged for proteins which remain in the cytoplasm or nucleus. Proteins can be detected by antibodies, binding peptides/proteins (e.g. receptor ligands, or parts thereof), and if appropriate by metabolites, enzyme substrates, substrate analogues and enzyme inhibitors. A number of proteins which are located inside a cell can be indirectly detected using compound which are internalised by a cell e.g. enzyme substrates or analogues, enzyme inhibitors or certain metabolites which are incorporated into a cell. Accordingly, adenocarcinoma cells can be identified *in vivo* by detecting a decreased or increased expression or activity of a protein encoded by a marker gene when compared to adenoma cells.

Any of the above compounds described above to be suitable for *the in vivo* detection of proteins can be further functionalised with chromophoric agents, MRI labels, labels for ultrasound detection, radioactive compounds or other tools for facilitating *in vivo* imaging.

Yet another aspect of the present invention relates to kits for performing the in vivo and/or in vitro detection methods of the present invention. Typically, the kits of the present invention contain one or more agents allowing the specific detection of one or more marker genes disclosed herein. In particular embodiments the kits comprise a set of agents which allow detection of a set of marker genes described herein. The nature of the agents is determined by the method of detection for which the kit is intended. Where detection at the DNA/RNA method is intended, the agents are typically marker-specific primers or probes, which are optionally labelled. Where detection is at the protein level, agents are typically antibodies or compounds containing an antigen-binding fragment of an antibody. However, as described above, protein expression can also be detected using other compounds which specifically interact with the marker of interest, such as specific substrates (in case of enzymes) or ligands (for receptors).

In particular embodiments the kits of the invention also comprise a control sample, in accordance with the methods of the present invention.

Particular and preferred aspects of the invention are set out in the accompanying independent and dependent claims. Features from the dependent claims may be combined with features of the independent claims and with features of other dependent claims as appropriate and not merely as explicitly set out in the claims.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### Examples

### Example 1: Determining differentially expressed genes in a microarray

### Selection of tumour samples

73 snapped frozen colorectal tumours (37 non-progressed adenomas and 36 carcinomas) were collected prospectively at the VU - University Medical Center (VUmc), Amsterdam, The Netherlands. All samples were used in compliance with the institution's ethical regulations.

The 73 frozen specimens corresponded to 65 patients (31 females and 34 males). From these, 6 patients had multiple tumours: 4 patients, multiple adenomas and 2 patients, 1 or more adenomas next to a carcinoma. The mean age of the patients was 69 (range 47-89).

Array-CGH and expression microarrays were done on the frozen set. RNA isolation

RNA from snap-frozen tissues was isolated with TRIzol reagent (Invitrogen, Breda, NL) following the supplier's instructions. Both RNA and DNA concentration and purity were measured in a Nanodrop ND-1000 spectrophotometer (Isogen, IJsselstein, NL) and integrity was evaluated in a 1% agarose gel, stained with ethidium bromide. Expression microarrays

### a) Array platform

The Human Release 2.0 oligonucleotide library, containing 60-mer oligonucleotides representing 28830 unique genes, designed by Compugen (San Jose, CA, USA) was obtained from Sigma-Genosys (Zwijndrecht, The Netherlands). The oligonucleotides were dissolved at 10 mM concentration in 50 mM sodium phosphate buffer pH 8.5 and single spotted onto Codelink^{™} slides (Amersham Biosciences, Roosendaal, NL), using the OmniGrid® 100 microarrayer (Genomic Solutions, Ann Arbor, MI, USA) equipped with SMP3 pins (TeleChem International, Sunnyvale, CA, USA). After printing slides were processed according to the manufacturers protocol (Codelink^{™} slides; Amersham BioSciences, Roosendaal, NL).

### b) labelling and hybridisation

First, 30µg messenger RNA was reverse-transcribed to cDNA using SuperScript^{™} II Reverse transcriptase (Invitrogen, Breda, NL) with oligo-dT priming (Isogen, IJsselstein, NL). In samples with limiting amount of RNA, less was used as starting material, but at least 15µg). cDNA was coupled to Fluorolink Cy3 and Cy5 Monofunctional Dye 5-pack (Amersham BioSciences, Rosendaal, NL). Cy3 labelled tumour cDNA and Cy5 labelled reference cDNA were combined and co- precipitated with 12µg pd(A)₄₀₋₆₀ (Amersham BioSciences, Rosendaal, NL), 60µg of tRNA (Sigma-Aldrich, Zwijndrecht, NL) and 24µg of human Cot-1 DNA (Invitrogen, Breda, NL) by adding 0.1 volume of 3 M sodium acetate (pH 5.2) and 2.5 volumes of ice-cold 100% ethanol. The precipitate was collected by centrifugation at 14,000 rpm for 10 minutes at 4 °C. After air-drying the pellet was dissolved in 126.7µl hybridisation mixture with a final concentration of 50% formamide, 2 x SCC, 10% dextran sulfate and 4% SDS.The cDNA samples were denatured for 10 minutes at 73 °C followed by a 60 minutes incubation at 37 °C to allow the Cot-1 DNA to block repetitive sequences. The arrays were incubated for 14h at 37°C with the denatured and blocked hybridisation mixture in a hybridisation station (HybArray12^{™} - Perkin Elmer Life Sciences, Zaventem, BE). After hybridisation, slides were washed in a solution containing 50% formamide, 2x SCC, pH 7 for 3 minutes at 45 °C, followed by 1 minute wash steps at room temperature with PN buffer (PN: 0.1 M sodium phosphate, 0.1% nonidet P40, pH 8), 0.2 x SSC, 0.1 x SCC and 0.01 x SCC. Slides were dried by centrifugation at 1000 rpm for 3 min at room temperature.

### c) image acquisition, feature extraction and normalization

Images of the arrays were acquired by scanning (Agilent DNA Microarray scanner- Agilent technologies, Palo Alto, USA) and Imagene 5.6 software (Biodiscovery Ltd, Marina del Rey, California) was used for automatic feature extraction (segmentation of the spots and quantification of the signal and background intensities for each spot for the two channels Cy3 and Cy5). Default settings for the flagging of poor quality spots was used. A microsoft Excel sheet was used to subtract local background from the signal median intensities of both test and reference cDNA. MA-plots of intensities of raw data were done to judge overall quality and flag out poor quality experiments. Normalization was done either with TIGR Midas, using "loess" correction or with "Median" normalization and implemented in the maNorm function (marray R bioconductor package), with identical results. Inter-array normalization was also performed for clustering purposes. Low intensity values were replaced by the intensity value of 50. Genes with more than 20% missing values in all tumours were excluded from further analysis.

### Data analysis

Unsupervised cluster analysis was done using the software TIGR multi experiment viewer (TMev). Complete linkage and Euclidian distances were applied.

As all hybridisations were performed against a common reference, all comparisons were relative between different groups of colorectal neoplasias, no differences compared to normal colorectal mucosa were considered. Supervised analysis for comparison of expression between carcinomas and adenomas was done using the Wilcoxon ranking test and a new method which takes into account subpopulations.

### Results

The genes which were identified by microarray to be differentially expressed between adenomas and carcinomas are provided in Table 1.

### Example 2: Integration of expression data and CGH analysis

To investigate the effects of chromosomal instability on gene expression in colorectal adenoma to carcinoma progression, whole-genome copy number changes were analysed, by array-CGH, on a series of 114 colorectal tumours (37 non-progressed adenomas, 41 progressed adenomas (malignant polyps) and 36 carcinomas) .

The determination of the SROs as disclosed in the present invention is illustrated in detail herein for the region of chromosomal gain at 20q. For the 41 progressed adenomas, the adenoma and the carcinoma components were analysed for DNA copy number alterations. Losses of 1p, 4, 8p, 14q, 15q, 17p and 18 and gains of 1q, 6p, 7, 8q, 13q, 17q, 19p, 20q and 22q were observed in >20% of cases, of which 8p and 18 loss and 13q and 20q gains were the most frequent, occurring in more than 35% of the cases. Gain of chromosome 20 alone occurred in more than 60% of the cases. Genome wide, the pattern of copy number changes did not differ between adenoma and carcinoma components in progressed adenomas, i.e. the aberrations found in the carcinoma component were already present on the adenoma component, although with lower frequencies or amplitudes.

Next, the copy number changes of the 37 non-progressed adenomas and 36 carcinomas were analysed. From the 73 tumours, 67 (34 adenomas and 33 carcinomas) showed high quality genomic profiles (corresponding to a 8% drop-out). In adenomas the frequency of aberrations obviously was very low. In contrast, carcinomas showed frequent (>20% of cases) 1p, 4, 8p, 14q, 15q, 17p and 18 losses and 1q, 6p, 7, 8q, 13q, 17q, 19p, 20q and 22q gains, with 8p and 18 deletions and 13q and 20q gains present in more than 35% of the cases (like in the progressed adenomas). Chromosome 20 gains occurred in less than 15% of the adenomas but in more than 60% of the carcinomas, mostly affecting either the whole chromosome or the long arm, like in the progressed adenomas.

Hierarchical clustering of these 67 tumours (non-progressed adenomas and carcinomas) on DNA copy number profiles showed a clear separation of carcinomas and adenomas into two different clusters, cluster 1 and 2, respectively, with χ² test p < 0.001. In search for those DNA copy number changes that were significantly different (p<0.05) between non-progressed adenomas and carcinomas, we observed that 4q, 8p, 8q, 13q, 15q, 18 and 20 were the relevant regions, of which loci on 20q differed most significantly (p <0.00001).

In order to determine the most relevant regions harbouring putative oncogenes with a role in colorectal cancer progression, STAC was applied to the combined set of paraffin-embedded malignant polyps (n=41) and frozen carcinomas (n=33). For 20q, analysis of these samples revealed 3 relevant regions of aberrant copy gains, one spanning 4 Mb (32-36Mb), one spanning 3 Mb (56-59Mb) and the third one spanning 2Mb (61-64Mb). These three regions (smallest regions of overlap - SROs) still contained 80, 35 and 94 genes, respectively. Similar analyses were performed for the other regions of chromosomal aberration linked with adenocarcinomas and the SROs identified for each of these regions.

Microarray expression analysis was performed in the 37 non-progressed adenomas and 36 carcinomas of which snap-frozen material was available. High quality expression data were obtained in 68 cases (37 adenomas and 31 carcinomas, 7% drop-out).

The array-CGH data were related to the microarray expression data genome-widely, independently of adenoma or carcinoma status. To compare expression between tumours with a certain chromosomal aberration and tumours without such aberration, in order to disclose genes expression of which is influenced by the chromosomal aberration, a statistical algorithm (R environment) was developed (de Wiel, unpublished data).

For each region of chromosomal aberration, a list of genes for which gene dosage affected expression levels was obtained (Tables 2-9). Genes were identified which mapped within the regions of chromosomal abberation and showing differential expression between colorectal adenoma and adenocarcinoma cells with the relevant chromosomal aberration (Tables 10-16).

This process is described more in detail for the region of chormosomal gain at 20q hereafter. Supervised analysis of expression data, with the aim of identifying putative oncogenes on 20q, was done in two different ways. First, differential expression of genes was investigated between carcinomas and adenomas (Table 1), and secondly the expression genes in tumours with 20q gain was compared with tumours without 20q gain (Table 8), to determine in which genes the expression level was influenced by the occurrence of 20q gain. The first approach revealed genome wide 122 up-regulated genes and 219 down-regulated genes, in carcinomas when compared to adenomas (Wilcoxon test p-value <1e-5 or Thas score >10.47). Of the 122 up-regulated genes, 14 map at chromosome 20q. For the second approach, only tumours (adenomas and carcinomas) were used of which both array-CGH data and expression data were available (n=64). As a pre selection, genes were used of which expression values were differentially expressed (both up- and down) between carcinomas and adenomas (cut-off p-value<0.05), obtained in the first approach, to focus on genes on 20q which are involved in the progression from adenoma to carcinoma. With this analysis 127 genes were identified, throughout the genome, whose expression levels are due to the occurrence of 20q gain. When we compared the genes mapping on 20q which are common between the two approaches, that is, which are up-regulated in carcinomas as a result of the gain of this chromosome arm, 9 genes were found, namely *TPX2, c20orf24, AURKA(STK6), RNPC1, TH1L, ADRM1, C20orf20, TCFL5* and *C20orf11.*

A similar analysis was performed for the other regions of chromosomal loss or gain linked to colorectal adenocarcinoma.

**Table 1: Human genes differentially expressed between colorectal adenoma and adenocarcinoma cells. (date ranked on p value)**

| Gene ID | Description (Table 1) | Corrrected p-value |
|---|---|---|
| NM_000963 | prostaglandin-endoperoxide synthase 2 (prostaglandin G/H synthase and cyclooxygenase) (PTGS2), mRNA. | 0 |
| BC017906 | clone IMAGE:4273917, mRNA. | 0 |
| NM_031285 | hypothetical protein PP1057 (PP1057), mRNA. | 0 |
| AF086401 | full length insert cDNA clone ZD75H06. | 0 |
| AI546979 | PN2.1_12_B02.r mynorm cDNA 5', mRNA sequence. | 0 |
| NM_000439 | proprotein convertase subtilisin/kexin type 1 (PCSK1), mRNA. | 0 |
| NM_032827 | hypothetical protein FLJ14708 (FLJ14708), mRNA. | 0 |
| AK024180 | cDNA FLJ14118 fis, clone MAMMA1001848. | 0 |
| AK001058 | cDNA FLJ10196 fis, clone HEMBA1004776. | 0 |
| NM_145341 | programmed cell death 4 (neoplastic transformation inhibitor) (PDCD4), transcript variant 2, mRNA. | 0 |
| NM_016651 | heptacellular carcinoma novel gene 3 (DAPPER1), mRNA. | 0 |
| NM_013943 | chloride intracellular channel 4 (CLIC4), mRNA. | 0 |
| NM_006922 | sodium channel, voltage-gated, type III, alpha polypeptide (SCN3A), mRNA. | 0 |
| NM_005424 | tyrosine kinase with immunoglobulin and epidermal growth factor homology domains (TIE), mRNA. | 0 |
| U80770 | EST clone 251800 mariner transposon Hsmarl sequence. | 0 |
| BC039491 | clone IMAGE:5547271, mRNA. | 0 |
| NM_145274 | LOC147184 (LOC147184), mRNA. | 0 |
| AF056437 | clone HEA2 Cri-du-chat critical region mRNA. | 0 |
| AB033100 | mRNA for KIAA1274 protein, partial cds. | 0 |
| NM_002731 | protein kinase, cAMP-dependent, catalytic, beta (PRKACB), mRNA. | 0 |
| NM_152621 | hypothetical protein MGC26963 (MGC26963), mRNA. | 0 |
| NM_006396 | Sjogren's syndrome/scleroderma autoantigen 1 (SSSCA1), mRNA. | 0 |
| BC009558 | clone IMAGE:3899550, mRNA, partial cds. | 0 |
| NM_005101 | interferon, alpha-inducible protein (clone IFI-15K) (G1P2), | 0 |
| | mRNA. | |
| NM_018484 | solute carrier family 22 (organic anion/cation transporter), member 11 (SLC22A11), mRNA. | 0 |
| NM_006944 | secreted phosphoprotein 2, 24kDa (SPP2), mRNA. | 0 |
| BF683837 | 602140129F1 NIH_MGC_46 cDNA clone IMAGE:4301287 5', mRNA sequence. | 0 |
| NM_017896 | chromosome 20 open reading frame 11 (C20orf11), mRNA. | 0 |
| NM_020169 | latexin protein (LXN), mRNA. | 0 |
| NM_013372 | cysteine knot superfamily 1, BMP antagonist 1 (CKTSF1B1), mRNA. | 0 |
| AK094784 | cDNA FLJ37465 fis, clone BRAWH2011823, highly similar to BONE MORPHOGENETIC PROTEIN 7 PRECURSOR. | 0 |
| BC022571 | clone MGC:27130 IMAGE:4794763, mRNA, complete cds. | 0 |
| NM_019079 | hypothetical protein FLJ10884 (FLJ10884), mRNA. | 0 |
| NM_003279 | troponin C2, fast (TNNC2), mRNA. | 0 |
| NM_012445 | spondin 2, extracellular matrix protein (SPON2), mRNA. | 0 |
| AF304443 | B lymphocyte activation-related protein BC-2048 mRNA, complete cds. | 0 |
| NM_002989 | chemokine (C-C motif) ligand 21 (CCL21), mRNA. | 0 |
| NM_021945 | hypothetical protein FLJ22174 (FLJ22174), mRNA. | 0 |
| NM_139058 | aristaless related homeobox (ARX), mRNA. | 0 |
| BC036390 | UDP-N-acetyl-alpha-D-galactosamine:polypeptide N-acetylgalactosaminyltransferase 4 (GalNAc-T4), clone MGC:41784 IMAGE:5261236, mRNA, complete cds. | 0 |
| AK025346 | cDNA: FLJ21693 fis, clone COL09609. | 0 |
| BC041467 | clone IMAGE:5215233, mRNA. | 0 |
| NM_017459 | microfibrillar-associated protein 2 (MFAP2), transcript variant 1, mRNA. | 0 |
| NM_138780 | synaptotagmin-like 5 (SYTL5), mRNA. | 0 |
| NM_002609 | platelet-derived growth factor receptor, beta polypeptide (PDGFRB), mRNA. | 0 |
| NM_003600 | serine/threonine kinase 6 (STK6), mRNA. | 0 |
| NM_138409 | hypothetical protein BC010003 (LOC112609), mRNA. | 0 |
| NM_021911 | gamma-aminobutyric acid (GABA) A receptor, beta 2 (GABRB2), transcript variant 1, mRNA. | 0 |
| NM_004669 | chloride intracellular channel 3 (CLIC3), mRNA. | 0 |
| NM_003014 | secreted frizzled-related protein 4 (SFRP4), mRNA. | 0 |
| NM_014312 | cortical thymocyte receptor (X. laevis CTX) like (CTXL), mRNA. | 0 |
| NM_024934 | hypothetical protein FLJ22659 (FLJ22659), mRNA. | 0 |
| NM_000088 | collagen, type I, alpha 1 (COL1A1), mRNA. | 0 |
| NM_001641 | APEX nuclease (multifunctional DNA repair enzyme) 1 (APEX1), transcript variant 1, mRNA. | 0 |
| AK022116 | cDNA FLJ12054 fis, clone HEMBB1002045. | 0 |
| NM_000958 | prostaglandin E receptor 4 (subtype EP4) (PTGER4), mRNA. | 0 |
| AK074150 | mRNA for FLJ00223 protein. | 0 |
| AL833463 | mRNA, cDNA DKFZp686P07116 (from clone DKFZp686P07116). | 0 |
| NM_024505 | NADPH oxidase, EF hand calcium-binding domain 5 (NOX5), mRNA. | 0 |
| BC033540 | RA-regulated nuclear matrix-associated protein, clone MGC:42657 IMAGE:4826434, mRNA, complete cds. | 0 |
| AF339788 | clone IMAGE:208561, mRNA sequence. | 0 |
| BG186690 | RST5665 Athersys RAGE Library cDNA, mRNA sequence. | 0 |
| NM_018662 | disrupted in schizophrenia 1 (DISC1), mRNA. | 0 |
| NM_001546 | inhibitor of DNA binding 4, dominant negative helix-loop-helix protein (ID4), mRNA. | 0 |
| AK091130 | cDNA FLJ33811 fis, clone CTONG2002095. | 0 |
| NM_006030 | calcium channel, voltage-dependent, alpha 2/delta subunit 2 (CACNA2D2), mRNA. | 0 |
| NM_016569 | T-box 3 (ulnar mammary syndrome) (TBX3), transcript variant 2, mRNA. | 0 |
| NM_152315 | hypothetical protein MGC34290 (MGC34290), mRNA. | 0 |
| NM_006602 | transcription factor-like 5 (basic helix-loop-helix) (TCFL5), mRNA. | 0 |
| AK095147 | cDNA FLJ37828 fis, clone BRSSN2006575. | 0 |
| NM_019000 | hypothetical protein FLJ20152 (FLJ20152), mRNA. | 0 |
| NM_024533 | hypothetical protein FLJ22167 (FLJ22167), mRNA. | 0 |
| NM_080661 | similar to RIKEN cDNA 0610008P16 gene (MGC15937), mRNA. | 0 |
| NM_014729 | thymus high mobility group box protein TOX (TOX), mRNA. | 0 |
| NM_032579 | colon and small intestine-specific cysteine-rich protein precursor (HXCP2), mRNA. | 0 |
| NM_002928 | regulator of G-protein signalling 16 (RGS 16), mRNA. | 0 |
| NM_014310 | RASD family, member 2 (RASD2), mRNA. | 0 |
| NM_001249 | ectonucleoside triphosphate diphosphohydrolase 5 (ENTPD5), mRNA. | 0 |
| BC015962 | clone IMAGE:4081125, mRNA, partial cds. | 0 |
| NM_016938 | EGF-containing fibulin-like extracellular matrix protein 2 (EFEMP2), mRNA. | 0 |
| AF085835 | full length insert cDNA clone YI41B09. | 0 |
| AK096002 | cDNA FLJ38683 fis, clone KIDNE2000777. | 0 |
| BM545871 | AGENCOURT_6500358 NIH_MGC_125 cDNA clone IMAGE:5588228 5', mRNA sequence. | 0 |
| AW975332 | EST387440 MAGE resequences, MAGN cDNA, mRNA sequence. | 0 |
| NM_138455 | collagen triple helix repeat containing 1(CTHRC1), mRNA. | 0 |
| NM_014176 | HSPC150 protein similar to ubiquitin-conjugating enzyme (HSPC150), mRNA. | 0 |
| NM_019012 | phosphoinositol 3-phosphate-binding protein-2 (PEPP2), mRNA. | 0 |
| NM_006809 | translocase of outer mitochondrial membrane 34 (TOMM34), mRNA. | 0 |
| BC035027 | clone IMAGE:4828469, mRNA. | 0 |
| NM_001072 | UDP glycosyltransferase 1 family, polypeptide A6 (UGT1A6), mRNA. | 0 |
| NM_007283 | monoglyceride lipase (MGLL), mRNA. | 0 |
| NM_003655 | chromobox homolog 4 (Pc class homolog, Drosophila) (CBX4), mRNA. | 0 |
| AK000245 | cDNA FLJ20238 fis, clone COLF5890. | 0 |
| BC029775 | hypothetical gene LOC127421, clone MGC:35394 IMAGE:5186268, mRNA, complete cds. | 0 |
| NM_001888 | crystallin, mu (CRYM), mRNA. | 0 |
| NM_018414 | GalNAc alpha-2, 6-sialyltransferase I, long form (ST6GalNAcI), mRNA. | 0 |
| AL355711 | EST from clone 208499, full insert. | 0 |
| NM_018270 | chromosome 20 open reading frame 20 (C20orf20), mRNA. | 0 |
| NM_033225 | CUB and Sushi multiple domains 1 (CSMD1), mRNA. | 0 |
| AK025017 | cDNA: FLJ21364 fis, clone COL02989. | 0 |
| AF147324 | full length insert cDNA clone YB22D01. | 0 |
| AK025522 | cDNA: FLJ21869 fis, clone HEP02442. | 0 |
| NM_033262 | solute carrier family 8 (sodium-calcium exchanger), member 3 (SLC8A3), transcript variant a, mRNA. | 0 |
| NM_005518 | 3-hydroxy-3-methylglutaryl-Coenzyme A synthase 2 (mitochondrial) (HMGCS2), mRNA. | 0 |
| BC008502 | clone MGC:14841 IMAGE:4295121, mRNA, complete cds. | 0 |
| NM_014326 | death-associated protein kinase 2 (DAPK2), mRNA. | 0 |
| AF086094 | full length insert cDNA clone YZ87H06. | 0 |
| AB037842 | mRNA for KIAA1421 protein, partial cds. | 0 |
| NM_025087 | hypothetical protein FLJ21511 (FLJ21511), mRNA. | 0 |
| NM_003682 | MAP-kinase activating death domain (MADD), transcript variant 4, mRNA. | 0 |
| NM_003657 | breast carcinoma amplified sequence 1 (BCAS1), mRNA. | 0 |
| NM_005904 | MAD, mothers against decapentaplegic homolog 7 (Drosophila) (MADH7), mRNA. | 0 |
| NM_004417 | dual specificity phosphatase 1 (DUSP1), mRNA. | 0 |
| NM_014766 | KIAA0193 gene product (KIAA0193), mRNA. | 0 |
| AK026378 | cDNA: FLJ22725 fis, clone HSI14917. | 0 |
| BC034757 | Indian hedgehog homolog (Drosophila), clone MGC:34815 IMAGE:5182642, mRNA, complete cds. | 0 |
| NM_005172 | atonal homolog 1 (Drosophila) (ATOH1), mRNA. | 0 |
| NM_012254 | solute carrier family 27 (fatty acid transporter), member 5 (SLC27A5), mRNA. | 0 |
| NM_133367 | chromosome 6 open reading frame 33 (C6orf33), mRNA. | 0 |
| NM_012413 | glutaminyl-peptide cyclotransferase (glutaminyl cyclase) (QPCT), mRNA. | 0 |
| AL548449 | AL548449 LTI_NFL006_PL2 cDNA clone CSODI014YH21 3 prime, mRNA sequence. | 0 |
| NM 003289 | tropomyosin 2 (beta) (TPM2), mRNA. | 0 |
| NM_153751 | chromosome 21 open reading frame 82 (C21ort82), mRNA. | 0 |
| NM_016308 | UMP-CMP kinase (UMP-CMPK), mRNA. | 0 |
| NM_005375 | v-myb myeloblastosis viral oncogene homolog (avian) (MYB), mRNA. | 0 |
| U01038 | pLK mRNA, complete cds. | 0 |
| NM_032951 | Williams Beuren syndrome chromosome region 14 (WBSCR14), transcript variant 1, mRNA. | 0 |
| NM_003882 | WNT1 inducible signaling pathway protein 1 (WISP1), transcript variant 1, mRNA. | 0 |
| NM_033126 | serine/threonine kinase PSKH2 (PSKH2), mRNA. | 0 |
| BC033971 | clone IMAGE:5288527, mRNA. | 0 |
| NM_004221 | natural killer cell transcript 4 (NK4), mRNA. | 0 |
| NM_002346 | lymphocyte antigen 6 complex, locus E (LY6E), mRNA. | 0 |
| NM_020360 | phospholipid scramblase 3 (PLSCR3), mRNA. | 0 |
| NM_002167 | inhibitor of DNA binding 3, dominant negative helix-loop-helix protein (ID3), mRNA. | 0 |
| NM_003839 | tumor necrosis factor receptor superfamily, member 11a, activator of NFKB (TNFRSF11A), mRNA. | 0 |
| NM_012326 | microtubule-associated protein, RP/EB family, member 3 (MAPRE3), mRNA. | 0 |
| NM_001761 | cyclin F (CCNF), mRNA. | 0 |
| NM_002345 | lumican (LUM), mRNA. | 0 |
| NM_009590 | amine oxidase, copper containing 2 (retina-specific) (AOC2), transcript variant 2, mRNA. | 0 |
| NM_005480 | trophinin associated protein (tastin) (TROAP), mRNA. | 0 |
| NM_014465 | sulfotransferase family, cytosolic, 1B, member 1 | 0 |
| | (suLT1B1), mRNA. | |
| BC031648 , | KIAA1324 protein, clone MGC:35166 IMAGE:5169952, mRNA, complete cds. | 0 |
| NM_020980 | aquaporin 9 (AQP9), mRNA. | 0 |
| NM_003890 | Fc fragment of IgG binding protein (FCGBP), mRNA. | 0 |
| NM_014631 | likely ortholog of mouse five SH3 domains (FISH), mRNA. | 0 |
| NM_022909 | centromere protein H (CENPH), mRNA. | 0 |
| NM_001715 | B lymphoid tyrosine kinase (BLK), mRNA. | 0 |
| NM_145235 | similar to RIKEN cDNA 1700007B22 (LOC92565), mRNA. | 0 |
| NM_006259 | protein kinase, cGMP-dependent, type II (PRKG2), mRNA. | 0 |
| NM_032229 | hypothetical protein FLJ22774 (FLJ22774), mRNA. | 0 |
| NM_003118 | secreted protein, acidic, cysteine-rich (osteonectin) (SPARC), mRNA. | 0 |
| NM_006615 | calpain 9 (nCL-4) (CAPN9), mRNA. | 0 |
| NM_023028 | fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) (FGFR2), transcript variant 10, mRNA. | 0 |
| NM_138706 | beta-1,3-N-acetylglucosaminyltransferase protein (IMAGE:4907098), mRNA. | 0,0012662 |
| NM_001343 | disabled homolog 2, mitogen-responsive phosphoprotein (Drosophila) (DAB2), mRNA. | 0,00127427 |
| NM_014276 | recombining binding protein suppressor of hairless (Drosophila)-like (RBPSUHL), mRNA. | 0,00128244 |
| NM_005476 | UDP-N-acetylglucosamine-2-epimerase/N-acetylmannosamine kinase (GNE), mRNA. | 0,00129071 |
| AL117475 | mRNA, cDNA DKFZp727C211 (from clone DKFZp727C211). | 0,00181782 |
| BC040548 | Similar to hypothetical protein MGC38960, clone IMAGE:5288206, mRNA. | 0,0018289 |
| NM_133489 | solute carrier family 26, member 10 (SLC26A10), mRNA. | 0,00184012 |
| NM_009587 | lectin, galactoside-binding, soluble, 9 (galectin 9) | 0,00185148 |
| | (LGALS9), transcript variant long, mRNA. | |
| AK055091 | cDNA FLJ30529 fis, clone BRAWH2001052. | 0,00186298 |
| AK001669 | cDNA FLJ10807 fis, clone NT2RP4000865, moderately similar to ZINC FINGER PROTEIN ZFP-36. | 0,00187463 |
| BC036237 | clone IMAGE:5297125, mRNA. | 0,00188642 |
| NM_006306 | SMC1 structural maintenance of chromosomes 1-like 1 (yeast) (SMC1L1), mRNA. | 0,00238874 |
| NM_016397 | TH1-like (Drosophila) (TH1L), mRNA. | 0,00240313 |
| NM_000442 | platelet/endothelial cell adhesion molecule (CD31 antigen) (PECAM1), mRNA. | 0,00298155 |
| NM_017898 | hypothetical protein FLJ20605 (FLJ20605), mRNA. | 0,00344759 |
| NM_153025 | hypothetical protein FLJ31606 (FLJ31606), mRNA. | 0,00346751 |
| NM_018840 | putative Rab5-interacting protein (RIP5), mRNA. | 0,00348767 |
| NM_004315 | N-acylsphingosine amidohydrolase (acid ceramidase) 1 (ASAH1), mRNA. | 0,00350807 |
| NM_014353 | RAB26, member RAS oncogene family (RAB26), mRNA. | 0,00352871 |
| BQ425855 | AGENCOURT_7894797 NIH_MGC_72 cDNA clone IMAGE:6158390 5', mRNA sequence. | 0,00354959 |
| NM_014052 | GW128 protein (GW128), mRNA. | 0,00367821 |
| NM_003186 | transgelin (TAGLN), mRNA. | 0,00369767 |
| AK092226 | cDNA FLJ34907 fis, clone NT2RI2003392. | 0,00371734 |
| NM_007069 | HRAS-like suppressor 3 (HRASLS3), mRNA. | 0,00373722 |
| NM_144646 | immunoglobulin J polypeptide, linker protein for immunoglobulin alpha and mu polypeptides (IGJ), mRNA. | 0,00375731 |
| NM_003977 | aryl hydrocarbon receptor interacting protein (AIP), mRNA. | 0,00377762 |
| NM_002216 | inter-alpha (globulin) inhibitor, H2 polypeptide (ITIH2), mRNA. | 0,00379815 |
| AK075365 | cDNA PSEC0052 fis, clone NT2RP2000279, weakly similar to AQUALYSIN I PRECURSOR (EC 3.4.21.-). | 0,00381891 |
| NM_002915 | replication factor C (activator 1) 3, 38kDa (RFC3), mRNA. | 0,00383989 |
| NM_006946 | spectrin, beta, non-erythrocytic 2 (SPTBN2), mRNA. | 0,00386111 |
| NM_003966 | sema domain, seven thrombospondin repeats (type 1 and type 1-like), transmembrane domain (TM) and short cytoplasmic domain, (semaphorin) 5A (SEMA5A), mRNA. | 0,00388256 |
| NM_005398 | protein phosphatase 1, regulatory (inhibitor) subunit 3C (PPP1R3C), mRNA. | 0,00390425 |
| BC040302 | clone IMAGE:4837012, mRNA. | 0,00392618 |
| NM_004915 | ATP-binding cassette, sub-family G (WHITE), member 1 (ABCG1), transcript variant 1, mRNA. | 0,00394836 |
| AF086547 | full length insert cDNA clone ZE12B03. | 0,0039708 |
| AK095226 | cDNA FLJ37907 fis, clone COLON2009337. | 0,00399349 |
| AL832060 | mRNA, cDNA DKFZp313L1514 (from clone DKFZp313L1514). | 0,00414943 |
| AF075039 | full length insert cDNA YN61C04. | 0,00417104 |
| NM_032711 | hypothetical protein MGC13090 (MGC13090), mRNA. | 0,00419288 |
| NM_016014 | CGI-67 protein (CGI-67), mRNA. | 0,00463825 |
| AK023616 | cDNA FLJ13554 fis, clone PLACE1007478. | 0,00509592 |
| U32597 | pre-B cell Ig heavy chain mRNA, third complementarity-determining region, clone PBUF-DE14, partial cds. | 0,00512205 |
| NM_002899 | retinol binding protein 1, cellular (RBP1), mRNA. | 0,00591015 |
| NM_001432 | epiregulin (EREG), mRNA. | 0,00593941 |
| NM_024933 | hypothetical protein FLJ12056 (FLJ12056), mRNA. | 0,00596051 |
| AW842526 | MR2-CN0035-170300-201-a06 CN0035 cDNA, mRNA sequence. | 0,00596896 |
| BC002877 | Similar to hypothetical protein FLJ11585, clone MGC:11258 IMAGE:3942160, mRNA, complete cds. | 0,00598598 |
| NM_022061 | mitochondrial ribosomal protein L17 (MRPL17), nuclear gene encoding mitochondrial protein, mRNA. | 0,0059988 |
| NM_002102 | glycophorin E (GYPE), mRNA. | 0,00601167 |
| NM_003173 | suppressor of variegation 3-9 homolog 1 (Drosophila) (SUV39H1), mRNA. | 0,00602895 |
| NM_032130 | hypothetical protein DKFZp434J0113 (DKFZP434J0113), mRNA. | 0,00603759 |
| BC037944 | clone IMAGE:5285703, mRNA. | 0,00605939 |
| NM_004385 | chondroitin sulfate proteoglycan 2 (versican) (CSPG2), mRNA. | 0,00606372 |
| NM_024513 | FYVE and coiled-coil domain containing 1 (FYCO1), mRNA. | 0,00609009 |
| AL834283 | mRNA, cDNA DKFZp547N0315 (from clone DKFZp547N0315). | 0,00609015 |
| NM_005144 | hairless (HR), transcript variant 1, mRNA. | 0,00611668 |
| NM_001920 | decorin (DCN), transcript variant A1, mRNA. | 0,00614351 |
| NM_018690 | apolipoprotein B48 receptor (APOB48R), mRNA. | 0,00617057 |
| AK000932 | cDNA FLJ10070 fis, clone HEMBA1001581. | 0,00619788 |
| NM_003645 | solute carrier family 27 (fatty acid transporter), member 2 (SLC27A2), mRNA. | 0,00622542 |
| BC000712 | Similar to kinesin family member C1, clone MGC:1202 IMAGE:3506669, mRNA, complete cds. | 0,00625321 |
| AF085969 | full length insert cDNA clone YT85B06. | 0,00628126 |
| NM_133646 | sterile alpha motif and leucine zipper containing kinase AZK (ZAK), mRNA. | 0,00630955 |
| NM_002135 | nuclear receptor subfamily 4, group A, member 1 (NR4A1), mRNA. | 0,0063381 |
| NM_021969 | nuclear receptor subfamily 0, group B, member 2 (NR0B2), mRNA. | 0,00636637 |
| BC033139 | clone IMAGE:3844353, mRNA. | 0,00636691 |
| NM_006874 | E74-like factor 2 (ets domain transcription factor) (ELF2), mRNA. | 0,00639598 |
| NM_001822 | chimerin (chimaerin) 1 (CHN1), mRNA. | 0,00642532 |
| NM_006097 | myosin regulatory light chain 2, smooth muscle isoform (MYRL2), mRNA. | 0,00645493 |
| AK098571 | cDNA FLJ25705 fis, clone TST04816. | 0,00648481 |
| AL832272 | mRNA, cDNA DKFZp667P1917 (from clone DKFZp667P1917). | 0,00651498 |
| NM_032746 | hypothetical protein MGC12538 (MGC12538), mRNA. | 0,00654542 |
| NM_138639 | BCL2-like 12 (proline rich) (BCL2L12), transcript variant 1, mRNA. | 0,00657615 |
| NM_006361 | homeo box B13 (HOXB13), mRNA. | 0,00660717 |
| BQ230145 | AGENCOURT_7592688 NIH_MGC_72 cDNA clone IMAGE:6050363 5', mRNA sequence. | 0,00663848 |
| NM_006150 | LIM domain only 6 (LMO6), mRNA. | 0,00666117 |
| AK023718 | cDNA FLJ13656 fis, clone PLACE1011520. | 0,00667009 |
| BC025345 | Similar to LOC149651, clone MGC:39393 IMAGE:4862156, mRNA, complete cds. | 0,00668904 |
| NM_012162 | F-box and leucine-rich repeat protein 6 (FBXL6), transcript variant 1, mRNA. | 0,00670201 |
| NM_001711 | biglycan (BGN), mRNA. | 0,00671714 |
| BC041362 | clone IMAGE:5273088, mRNA. | 0,00673423 |
| NM_001155 | annexin A6 (ANXA6), transcript variant 1, mRNA. | 0,00674549 |
| NM_001809 | centromere protein A, 17kDa (CENPA), mRNA. | 0,00676676 |
| NM_016341 | phospholipase C, epsilon 1 (PLCE1), mRNA. | 0,00677407 |
| NM_017678 | hypothetical protein FLJ20127 (FLJ20127), mRNA. | 0,00679961 |
| BC022357 | clone MGC:23866 IMAGE:4297017, mRNA, complete cds. | 0,00683278 |
| AK058066 | cDNA FLJ25337 fis, clone TST00714. | 0,00694147 |
| NM_003128 | spectrin, beta, non-erythrocytic 1 (SPTBN1), mRNA. | 0,00696992 |
| AB019210 | BM32A mRNA for PMMLP, complete cds. | 0,0069986 |
| AL359603 | mRNA, cDNA DKFZp547E096 (from clone DKFZp547E096). | 0,00702752 |
| NM_172127 | calcium/calmodulin-dependent protein kinase (CaM kinase) II delta (CAMK2D), transcript variant 1, mRNA. | 0,00747488 |
| AK022350 | cDNA FLJ12288 fis, clone MAMMA1001783. | 0,00750443 |
| AK001903 | cDNA FLJ11041 fis, clone PLACE1004405. | 0,00753421 |
| NM_138764 | BCL2-associated X protein (BAX), transcript variant epsilon, mRNA. | 0,00756422 |
| AK075287 | cDNA FLJ90806 fis, clone Y79AA1000750. | 0,00759448 |
| NM_032242 | hypothetical protein DKFZp564A176 (DKFZp564A176), mRNA. | 0,00762498 |
| BC015413 | clone MGC:21964 IMAGE:4394326, mRNA, complete cds. | 0,00765573 |
| NM_002318 | lysyl oxidase-like 2 (LOXL2), mRNA. | 0,00768672 |
| NM_017843 | breast carcinoma amplified sequence 4 (BCAS4), mRNA. | 0,00771797 |
| NM_000196 | hydroxysteroid (11-beta) dehydrogenase 2 (HSD11B2), mRNA. | 0,00774753 |
| NM_018685 | anillin, actin binding protein (scraps homolog, Drosophila) (ANLN), mRNA. | 0,00777622 |
| NM_001218 | carbonic anhydrase XII (CA12), mRNA. | 0,00780513 |
| NM_017495 | RNA-binding region (RNP1, RRM) containing 1 (RNPC1), | 0,00783425 |
| | mRNA. | |
| AL833091 | mRNA, cDNA DKFZp451M0319 (from clone DKFZp451M0319). | 0,0078636 |
| NM_033339 | caspase 7, apoptosis-related cysteine protease (CASP7), transcript variant gamma, mRNA. | 0,00789316 |
| NM_004202 | thymosin, beta 4, Y chromosome (TMSB4Y), mRNA. | 0,00792294 |
| NM 000835 | glutamate receptor, ionotropic, N-methyl D-aspartate 2C (GRIN2C), mRNA. | 0,00795296 |
| NM_004453 | electron-transferring-flavoprotein dehydrogenase (ETFDH), nuclear gene encoding mitochondrial protein, mRNA. | 0,00798319 |
| NM_000633 | B-cell CLL/lymphoma 2 (BCL2), nuclear gene encoding mitochondrial protein, transcript variant alpha, mRNA. | 0,00801366 |
| AL832848 | mRNA, cDNA DKFZp667H0525 (from clone DKFZp667H0525). | 0,00804437 |
| NM_138478 | hypothetical protein BC010734 (LOC147632), mRNA. | 0,00807531 |
| NM_017902 | hypoxia-inducible factor 1, alpha subunit inhibitor (HIF1AN), mRNA. | 0,00808294 |
| NM_006699 | mannosidase, alpha, class 1A, member 2 (MAN1A2), mRNA. | 0,00810649 |
| NM_025067 | hypothetical protein FLJ14106 (FLJ14106), mRNA. | 0,00813791 |
| NM_139248 | lipase, member H (LIPH), mRNA. | 0,00816957 |
| NM_018259 | hypothetical protein FLJ10890 (FLJ10890), mRNA. | 0,00820148 |
| NM_000629 | interferon (alpha, beta and omega) receptor 1 (IFNAR1), mRNA. | 0,00823365 |
| NM_018354 | chromosome 20 open reading frame 46 (C20orf46), mRNA. | 0,00842689 |
| NM_007002 | adhesion regulating molecule 1 (ADRM1), mRNA. | 0,00872553 |
| AY006312 | clone 09npa41 T cell receptor beta chain mRNA, partial cds. | 0,00875737 |
| NM_016353 | zinc finger, DHHC domain containing 2 (ZDHHC2), mRNA. | 0,0089552 |
| NM_012112 | chromosome 20 open reading frame 1 (C20orf1), mRNA. | 0,00898741 |
| AF353674 | BTB domain protein (BDPL) mRNA, partial cds. | 0,00901986 |
| NM_002659 | plasminogen activator, urokinase receptor (PLAUR), mRNA. | 0,00905254 |
| NM_020859 | Shroom-related protein (ShrmL), mRNA. | 0,00928743 |
| AL833307 | mRNA, cDNA DKFZp451O249 (from clone DKFZp4510249). | 0,00950514 |
| NM_052889 | CARD only protein (COP), mRNA. | 0,00953873 |
| AK095139 | cDNA FLJ37820 fis, clone BRSSN2004142. | 0,00957255 |
| NM_014444 | gamma tubulin ring complex protein (76p gene) (76P), mRNA. | 0,00960662 |
| AY101175 | extracellular sulfatase SULF-1 mRNA, complete cds. | 0,00971681 |
| NM_003987 | paired box gene 2 (PAX2), transcript variant a, mRNA. | 0,00975066 |
| BC018995 | clone MGC:20579 IMAGE:4300679, mRNA, complete cds. | 0,00978476 |
| BC036258 | clone IMAGE:5270538, mRNA. | 0,00981909 |
| NM_001855 | collagen, type XV, alpha 1 (COL15A1), mRNA. | 0,01000145 |
| NM_001405 | ephrin-A2 (EFNA2), mRNA. | 0,01003606 |
| NM_032385 | chromosome 5 open reading frame 4 (C5orf4), mRNA. | 0,01020204 |
| BC034571 | similar to phospholipase A2, group IVB (cytosolic), clone MGC:35120 IMAGE:5167964, mRNA, complete cds. | 0,01023686 |
| NM_152777 | chromosome 14 open reading frame 48 (C14orf48), mRNA. | 0,01027192 |
| NM_025244 | testis specific, 10 (TSGA10), mRNA. | 0,01030722 |
| NM_015973 | galanin-related peptide (LOC51083), mRNA. | 0,01036579 |
| BI791911 | ie10c04.x1 HR85 islet cDNA clone IMAGE:5086471 3', mRNA sequence. | 0,01040057 |
| NM_000435 | Notch homolog 3 (Drosophila) (NOTCH3), mRNA. | 0,01043559 |
| NM_024817 | hypothetical protein FLJ13710 (FLJ13710), mRNA. | 0,01047085 |
| AK022362 | cDNA FLJ12300 fis, clone MAMMA1001854. | 0,01050634 |
| AK026367 | cDNA: FLJ22714 fis, clone HSI13646. | 0,01086042 |
| AK095808 | cDNA FLJ38489 fis, clone FEBRA2023550, weakly similar to P3 PROTEIN. | 0,01089522 |
| AK093557 | cDNA FLJ36238 fis, clone THYMU2001422. | 0,01093026 |
| NM_006416 | solute carrier family 35 (CMP-sialic acid transporter), member 1 (SLC35A1), mRNA. | 0,01096126 |
| AB002339 | mRNA for KIAA0341 gene, partial cds. | 0,01096552 |
| NM_017590 | ubiquitous tetratricopeptide containing protein RoXaN (RoXaN), mRNA. | 0,0109978 |
| NM_018590 | hypothetical protein MGC40204 (MGC40204), mRNA. | 0,011001 |
| AK055120 | cDNA FLJ30558 fis, clone BRAWH2004044. | 0,01103672 |
| NM_006410 | HIV-1 Tat interactive protein 2, 30kDa (HTATIP2), mRNA. | 0,01107267 |
| NM_001752 | catalase (CAT), mRNA. | 0,01110886 |
| NM_000027 | aspartylglucosaminidase (AGA), mRNA. | 0,01114232 |
| AK023072 | cDNA FLJ13010 fis, clone NT2RP3000542. | 0,01114528 |
| NM_018182 | hypothetical protein FLJ10700 (FLJ10700), mRNA. | 0,01117598 |
| NM_016062 | CGI-128 protein (CGI-128), mRNA. | 0,01118194 |
| NM_021738 | supervillin (SVIL), transcript variant 2, mRNA. | 0,01120985 |
| AK023713 | cDNA FLJ13651 fis, clone PLACE1011452. | 0,01121885 |
| BG109597 | 602280715F1 NIH_MGC_86 cDNA clone IMAGE:4368554 5', mRNA sequence. | 0,01124392 |
| NM_002691 | polymerase (DNA directed), delta 1, catalytic subunit 125kDa (POLD1), mRNA. | 0,01125599 |
| NM_005618 | delta-like 1 (Drosophila) (DLL1), mRNA. | 0,0112782 |
| NM_018453 | chromosome 14 open reading frame 11 (C14orf11), mRNA. | 0,01128301 |
| NM_005538 | inhibin, beta C (INHBC), mRNA. | 0,01130729 |
| NM_018244 | chromosome 20 open reading frame 44 (C20orf44), mRNA. | 0,01131269 |
| Z83936 | mRNA, clone CD 36. | 0,01131849 |
| NM_014302 | Sec61 gamma (SEC61G), mRNA. | 0,01134105 |
| NM_000396 | cathepsin K (pycnodysostosis) (CTSK), mRNA. | 0,01134739 |
| BC006270 | clone MGC:11291 IMAGE:3946683, mRNA, complete cds. | 0,0113542 |
| NM_000698 | arachidonate 5-lipoxygenase (ALOX5), mRNA. | 0,011375 |
| BC040672 | clone IMAGE:4816952, mRNA. | 0,01138231 |
| NM_014142 | nudix (nucleoside diphosphate linked moiety X)-type motif 5 (NUDT5), mRNA. | 0,01139013 |
| AK056937 | cDNA FLJ32375 fis, clone SALGL1000065, weakly similar to POLYHOMEOTIC-PROXIMAL CHROMATIN PROTEIN. | 0,01140916 |
| BC040599 | clone IMAGE:5270494, mRNA. | 0,01141744 |
| NM_018180 | DEAD/H (Asp-Glu-Ala-Asp/His) box polypeptide 32 (DDX32), mRNA. | 0,01142629 |
| NM_003155 | stanniocalcin 1 (STC1), mRNA. | 0,01143466 |
| AK094846 | cDNA FLJ37527 fis, clone BRCAN2011946. | 0,01145279 |
| NM_003359 | UDP-glucose dehydrogenase (UGDH), mRNA. | 0,01146268 |
| NM_002840 | protein tyrosine phosphatase, receptor type, F (PTPRF), transcript variant 1, mRNA. | 0,01146829 |
| NM_020299 | aldo-keto reductase family 1, member B10 (aldose reductase) (AKR1B10), mRNA. | 0,01148835 |
| NM_000107 | damage-specific DNA binding protein 2, 48kDa (DDB2), mRNA. | 0,01150212 |
| NM_002250 | potassium intermediate/small conductance calcium-activated channel, subfamily N, member 4 (KCNN4), mRNA. | 0,01152414 |
| AK090685 | cDNA FLJ33366 fis, clone BRACE2005556. | 0,01153615 |
| NM_030574 | START domain containing 5 (STARD5), mRNA. | 0,01156016 |
| NM_138784 | hypothetical protein BC014341 (LOC116123), mRNA. | 0,01157039 |
| NM_005164 | ATP-binding cassette, sub-family D (ALD), member 2 (ABCD2), mRNA. | 0,01195099 |
| AK000899 | cDNA FLJ10037 fis, clone HEMBA1000968. | 0,01198594 |
| NM_024068 | hypothetical protein MGC2731 (MGC2731), mRNA. | 0,01220686 |
| AK093573 | cDNA FLJ36254 fis, clone THYMU2002157. | 0,01246125 |
| NM_017682 | vitelliform macular dystrophy 2-like protein 1 (VMD2L1), mRNA. | 0,01249753 |
| NM_005585 | MAD, mothers against decapentaplegic homolog 6 (Drosophila) (MADH6), mRNA. | 0,01253313 |
| NM_007055 | polymerase (RNA) III (DNA directed) (155kD) (RPC155), mRNA. | 0,01256894 |
| NM_024312 | MGC4170 protein (MGC4170), mRNA. | 0,01260496 |
| NM_016305 | synovial sarcoma translocation gene on chromosome 18-like 2 (SS18L2), mRNA. | 0,01264118 |
| BC030750 | clone IMAGE:4795773, mRNA. | 0,01267761 |
| NM_006067 | neighbor of COX4 (NOC4), mRNA. | 0,01270932 |
| AF506819 | URB mRNA, complete cds. | 0,01271425 |
| NM_152299 | hypothetical protein 384D8_6 (384D8-2), mRNA. | 0,01274533 |
| NM_005570 | lectin, mannose-binding, 1 (LMAN1), mRNA. | 0,01288255 |
| AB032983 | mRNA for KIAA1157 protein, partial cds. | 0,01291874 |
| NM_016614 | TRAF and TNF receptor-associated protein (TTRAP), | 0,01295513 |
| | mRNA. | |
| AK097377 | cDNA FLJ40058 fis, clone TCOLN1000180. | 0,01298086 |
| AL122071 | mRNA, cDNA DKFZp434H1235 (from clone DKFZp434H1235) | 0,01301681 |
| AK095100 | cDNA FLJ37781 fis, clone BRHIP2027054. | 0,01305297 |
| NM_002225 | isovaleryl Coenzyme A dehydrogenase (IVD), nuclear gene encoding mitochondrial protein, mRNA. | 0,01308933 |
| NM_030790 | T-cell immunomodulatory protein (CDA08), mRNA. | 0,01312589 |
| NM_001145 | angiogenin, ribonuclease, RNase A family, 5 (ANG), mRNA. | 0,0134382 |
| NM_014548 | tropomodulin 2 (neuronal) (TMOD2), mRNA. | 0,01345882 |
| NM_014747 | KIAA0237 gene product (KIAA0237), mRNA. | 0,01347442 |
| NM_012477 | WW domain binding protein 1 (WBP1), mRNA. | 0,0134959 |
| U05597 | anion exchanger 3 cardiac isoform (cAE3) mRNA, partial cds. | 0,01351084 |
| NM_016400 | Huntingtin interacting protein K (HYPK), mRNA. | 0,01354745 |
| NM_024855 | hypothetical protein FLJ12785 (FLJ12785), mRNA. | 0,01358427 |
| AL833742 | mRNA, cDNA DKFZp666E186 (from clone DKFZp666E186). | 0,01362128 |
| NM_033118 | myosin light chain kinase 2, skeletal muscle (MYLK2), mRNA. | 0,01363364 |
| NM_003283 | troponin T1, skeletal, slow (TNNT1), mRNA. | 0,0136585 |
| AL832954 | mRNA, cDNA DKFZp666M1310 (from clone DKFZp666M1310). | 0,01367019 |
| NM_018643 | triggering receptor expressed on myeloid cells 1 (TREM1), mRNA. | 0,01369592 |
| NM_016073 | likely ortholog of mouse hepatoma-derived growth factor, related protein 3 (HDGFRP3), mRNA. | 0,01386387 |
| AF130090 | clone FLB9530 PR02574 mRNA, complete cds. | 0,01405557 |
| AK000777 | cDNA FLJ20770 fis, clone COL06509. | 0,01409295 |
| NM_004407 | dentin matrix acidic phosphoprotein (DMP1), mRNA. | 0,0140964 |
| BC039263 | Similar to glutamate receptor, ionotropic, delta 1clone MGC:33869 IMAGE:5273869, mRNA, complete cds. | 0,0141333 |
| CA314483 | UI-CF-FN0-afh-g-23-0-UI.sl UI-CF-FN0 cDNA clone UI- CF-FN0-afh-g-23-0-UI 3', mRNA sequence. | 0,01417039 |
| NM_053286 | aquaporin 6, kidney specific (AQP6), transcript variant 2, mRNA. | 0,01420768 |
| AK055915 | cDNA FLJ31353 fis, clone MESAN2000264. | 0,01424517 |
| AK022426 | cDNA FLJ12364 fis, clone MAMMA1002384. | 0,01428286 |
| BQ646410 | AGENCOURT_8511770 NIH_MGC_100 cDNA clone IMAGE:6296949 5', mRNA sequence. | 0,01432003 |
| NM_030789 | histocompatibility (minor) 13 (HM13), mRNA. | 0,01446411 |
| NM_016357 | epithelial protein lost in neoplasm beta (EPLIN), mRNA. | 0,01450092 |
| AK096555 | cDNA FLJ39236 fis, clone OCBBF2007892, highly similar to zinc-finger helicase (hZFH) mRNA. | 0,0145049 |
| NM_018147 | Fas apoptotic inhibitory molecule (FAIM), mRNA. | 0,01453791 |
| NM_030878 | cytochrome P450, subfamily IIC (mephenytoin 4-hydroxylase), polypeptide 8 (CYP2C8), transcript variant Hp1-2, mRNA. | 0,01454257 |
| NM_001168 | baculoviral IAP repeat-containing 5 (survivin) (BIRC5), mRNA. | 0,01457509 |
| M96843 | striated muscle contraction regulatory protein (Id2B) mRNA, complete cds. | 0,01461246 |
| NM_002305 | lectin, galactoside-binding, soluble, 1 (galectin 1) (LGALS1), mRNA. | 0,01464351 |
| NM_004748 | cell cycle progression 8 protein (CPR8), mRNA. | 0,01465003 |
| BM456096 | AGENCOURT_6410321 NIH_MGC_85 cDNA clone IMAGE:5499473 5', mRNA sequence. | 0,01468058 |
| AK095403 | cDNA FLJ38084 fis, clone CTONG2016499. | 0,01468778 |
| NM_153225 | hypothetical protein FLJ40021 (FLJ40021), mRNA. | 0,01472574 |
| NM_015873 | villin-like (VILL), mRNA. | 0,01474708 |
| NM_006511 | regulatory solute carrier protein, family 1, member 1 (RSC1A1), mRNA. | 0,01478404 |
| NM_002971 | special AT-rich sequence binding protein 1 (binds to nuclear matrix/scaffold-associating DNA's) (SATB1), mRNA. | 0,01481185 |
| NM_006141 | dynein, cytoplasmic, light intermediate polypeptide 2 (DNCLI2), mRNA. | 0,01482118 |
| AL833743 | mRNA, cDNA DKFZp666E036 (from clone DKFZp666E036). | 0,01484852 |
| NM_003121 | Spi-B transcription factor (Spi-1/PU.1 related) (SPIB), mRNA. | 0,01485851 |
| NM_020403 | protocadherin 9 (PCDH9), mRNA. | 0,01488536 |
| NM_001807 | carboxyl ester lipase (bile salt-stimulated lipase) (CEL), mRNA. | 0,01492239 |
| NM_017959 | hypothetical protein FLJ20802 (FLJ20802), mRNA. | 0,0149596 |
| NM_000900 | matrix Gla protein (MGP), mRNA. | 0,01506876 |
| NM_080672 | Q9H4T4 like (H17739), mRNA. | 0,01510489 |
| NM_014622 | loss of heterozygosity, 11, chromosomal region 2, gene A (LOH11CR2A), mRNA. | 0,0151412 |
| NM_018085 | importin 9 (FLJ10402), mRNA. | 0,01517769 |
| NM_015666 | GTP binding protein 5 (putative) (GTPBP5), mRNA. | 0,01519306 |
| AW382764 | PMO-HT0339-081199-001-a01 HT0339 cDNA, mRNA sequence. | 0,01521435 |
| NM_014899 | Rho-related BTB domain containing 3 (RHOBTB3), mRNA. | 0,01523039 |
| NM_021830 | chromosome 10 open reading frame 2 (C10orf2), mRNA. | 0,01525119 |
| NM_012101 | tripartite motif-containing 29 (TRIM29), transcript variant 1, mRNA. | 0,01526791 |
| NM_000311 | prion protein (p27-30) (Creutzfeld-Jakob disease, Gerstmann-Strausler-Scheinker syndrome, fatal familial insomnia) (PRNP), mRNA. | 0,01527138 |
| NM_002644 | polymeric immunoglobulin receptor (PIGR), mRNA. | 0,0152882 |
| NM_013361 | zinc finger protein 223 (ZNF223), mRNA. | 0,0153254 |
| BC002782 | clone IMAGE:3621749, mRNA, partial cds. | 0,01532715 |
| NM_001285 | chloride channel, calcium activated, family member 1 (CLCA1), mRNA. | 0,01536278 |
| AW386993 | MR0-ST0019-290999-002-f03 ST0019 cDNA, mRNA sequence. | 0,01536338 |
| AK023519 | cDNA FLJ13457 fis, clone PLACE1003343. | 0,01539979 |
| NM_017699 | hypothetical protein FLJ20174 (FLJ20174), mRNA. | 0,01540034 |
| NM_015102 | nephronophthisis 4 (NPHP4), mRNA. | 0,01543637 |
| NM_147175 | heparan sulfate 6-O-sulfotransferase 2 (HS6ST2), transcript variant S, mRNA. | 0,01545358 |
| AW970134 | EST382215 MAGE resequences, MAGK cDNA, mRNA sequence. | 0,01547312 |
| NM_002452 | nudix (nucleoside diphosphate linked moiety X)-type motif 1 (NUDT1), mRNA. | 0,01548986 |
| NM_000288 | peroxisomal biogenesis factor 7 (PEX7), mRNA. | 0,01552631 |
| NM_016354 | solute carrier family 21 (organic anion transporter), member 12 (SLC21A12), mRNA. | 0,01611827 |
| NM_145293 | similar to hypothetical protein FLJ20897 (LOC196549), mRNA. | 0,01620044 |
| BC030701 | KIAA1764 protein, clone MGC:26917 IMAGE:4837403, mRNA, complete cds. | 0,01623803 |
| BC039414 | clone IMAGE:5302158, mRNA. | 0,01627579 |
| NM_017521 | FEV protein (HSRNAFEV), mRNA. | 0,01628117 |
| NM_017680 | asporin (LRR class 1) (ASPN), mRNA. | 0,01631373 |
| AA195192 | zr34g08.r1 Soares_NhHMPu_S1 cDNA clone IMAGE:665342 5', mRNA sequence. | 0,0163186 |
| AK098553 | cDNA FLJ25687 fis, clone TST04292. | 0,01632327 |
| NM_000773 | cytochrome P450, subfamily IIE (ethanol-inducible), polypeptide 1 (CYP2E1), mRNA. | 0,0163562 |
| NM_139033 | mitogen-activated protein kinase 7 (MAPK7), transcript variant 1, mRNA. | 0,01636036 |
| NM_031472 | hypothetical protein MGC11134 (MGC11134), mRNA. | 0,01639397 |
| AK025758 | cDNA: FLJ22105 fis, clone HEP17660. | 0,01639763 |
| AF131834 | clone 24841 mRNA sequence. | 0,01643507 |
| NM_025194 | inositol 1,4,5-trisphosphate 3-kinase C (ITPKC), mRNA. | 0,01647268 |
| NM_144971 | hypothetical protein MGC26641 (MGC26641), mRNA. | 0,01711367 |
| BQ921112 | AGENCOURT_8926187 NIH_MGC_101 cDNA clone IMAGE:6463013 5', mRNA sequence. | 0,0171523 |
| AK024356 | cDNA FLJ14294 fis, clone PLACE1008181. | 0,01719111 |
| NM_152340 | hypothetical protein FLJ39075 (FLJ39075), mRNA. | 0,01726114 |
| BC036419 | clone IMAGE:4797520, mRNA. | 0,01729993 |
| AF257167 | intestinal mucin 2.1 (MUC2) mRNA, partial cds. | 0,01736846 |
| NM_004172 | solute carrier family 1 (glial high affinity glutamate transporter), member 3 (SLC1A3), nuclear gene encoding mitochondrial protein, mRNA. | 0,01740723 |
| AL833444 | mRNA, cDNA DKFZp313M0542 (from clone DKFZp313M0542). | 0,01744617 |
| NM_152760 | hypothetical protein FLJ30934 (FLJ30934), mRNA. | 0,01755202 |
| NM_138573 | neuregulin 4 (LOC145957), mRNA. | 0,01768205 |
| BG107100 | 602291004F1 NIH_MGC_85 cDNA clone IMAGE:4385712 5', mRNA sequence. | 0,01772074 |
| NM_032833 | protein phosphatase 1, regulatory (inhibitor) subunit 15B (PPP1R15B), mRNA. | 0,01775961 |
| NM_002497 | NIMA (never in mitosis gene a)-related kinase 2 (NEK2), mRNA. | 0,01779864 |
| NM_005651 | tryptophan 2,3-dioxygenase (TDO2), mRNA. | 0,0178225 |
| NM_000669 | alcohol dehydrogenase 1C (class I), gamma polypeptide (ADH1C), mRNA. | 0,01783784 |
| NM_025198 | transcription termination factor-like protein (LOC80298), mRNA. | 0,01786133 |
| BC039527 | clone IMAGE:5742065, mRNA. | 0,01787722 |
| BC022423 | clone IMAGE:4247831, mRNA. | 0,01791677 |
| NM_013443 | CMP-NeuAC:(beta)-N-acetylgalactosaminide (alpha)2,6-sialyltransferase member VI (ST6GALNAC6), mRNA. | 0,01794513 |
| AK091117 | cDNA FLJ33798 fis, clone CTONG2000063. | 0,0179565 |
| NM_144676 | hypothetical protein MGC23911 (MGC23911), mRNA. | 0,01798356 |
| NM_022914 | hypothetical protein 24432 (24432), mRNA. | 0,01800076 |
| NM_003847 | peroxisomal biogenesis factor 11A (PEX11A), mRNA. | 0,01802215 |
| AK025703 | cDNA: FLJ22050 fis, clone HEP09454. | 0,01804308 |
| BG822407 | 602725454F1 NIH_MGC_15 cDNA clone IMAGE:4865053 5', mRNA sequence. | 0,0180609 |
| NM_145284 | similar to hypothetical protein MGC17347 (LOC159090), mRNA. | 0,01808147 |
| U44954 | NMDA receptor glutamate-binding chain (hnrgw) mRNA, partial cds. | 0,01809983 |
| AL832540 | mRNA, cDNA DKFZp547A0117 (from clone DKFZp547A0117). | 0,01812002 |
| BC040627 | clone IMAGE:5745252, mRNA. | 0,01813892 |
| AK094649 | cDNA FLJ37330 fis, clone BRAMY2019509. | 0,01817818 |
| AB053318 | ALS2CR16 mRNA, complete cds. | 0,01821672 |
| NM_000310 | palmitoyl-protein thioesterase 1 (ceroid-lipofuscinosis, neuronal 1, infantile) (PPT1), mRNA. | 0,01827366 |
| AF327347 | DnaJ protein SB73 mRNA, complete cds. | 0,01831213 |
| NM_019006 | protein associated with PRK1(AWP1), mRNA. | 0,01835076 |
| NM_138340 | abhydrolase domain containing 3 (ABHD3), mRNA. | 0,01838956 |
| NM_025098 | hypothetical protein FLJ22644 (FLJ22644), mRNA. | 0,01874625 |
| NM_152583 | hypothetical protein MGC40053 (MGC40053), mRNA. | 0,01878539 |
| AK075564 | cDNA PSEC0264 fis, clone NT2RP3002337. | 0,01882469 |
| NM_016013 | CGI-65 protein (CIA30), mRNA. | 0,01886415 |
| BC040901 | clone IMAGE:5743779, mRNA. | 0,01941758 |
| NM_032744 | hypothetical protein MGC12335 (MGC12335), mRNA. | 0,01945779 |
| NM_015446 | ELYS transcription factor-like protein TMBS62 (elys), mRNA. | 0,01949815 |
| NM_006850 | interleukin 24 (IL24), mRNA. | 0,01953869 |
| NM_152460 | hypothetical protein FLJ31882 (FLJ31882), mRNA. | 0,01958373 |
| NM_004657 | serum deprivation response (phosphatidylserine binding protein) (SDPR), mRNA. | 0,01974914 |
| NM_032848 | hypothetical protein FLJ14827 (FLJ14827), mRNA. | 0,01991386 |
| NM_002759 | protein kinase, interferon-inducible double stranded RNA dependent (PRKR), mRNA. | 0,01995569 |
| NM_000322 | retinal degeneration, slow (retinitis pigmentosa 7) (RDS), mRNA. | 0,019996 |
| NM_033280 | similar to signal peptidase complex (18kD) (LOC90701), mRNA. | 0,02003648 |
| NM_030912 | tripartite motif-containing 8 (TRIM8), mRNA. | 0,02003691 |
| NM_002896 | RNA binding motif protein 4 (RBM4), mRNA. | 0,02007712 |
| BI005815 | MR4-HN0058-250401-002-c08 HN0058 cDNA, mRNA sequence. | 0,02007797 |
| AF055007 | clone 24707 mRNA sequence. | 0,02011793 |
| AK022434 | cDNA FLJ12372 fis, clone MAMMA1002446. | 0,0201589 |
| NM_012332 | likely ortholog of mouse acyl-Coenzyme A thioesterase 2, mitochondrial (ACATE2), mRNA. | 0,02020004 |
| NM_020404 | tumor endothelial marker 1 precursor (TEM1), mRNA. | 0,02023169 |
| AL080129 | mRNA, cDNA DKFZp434D193 (from clone DKFZp434D193) | 0,02023645 |
| NM_014950 | KIAA0997 protein (KIAA0997), mRNA. | 0,02027152 |
| NM_014420 | dickkopf homolog 4 (Xenopus laevis) (DKK4), mRNA. | 0,02027709 |
| NM_000901 | nuclear receptor subfamily 3, group C, member 2 (NR3C2), mRNA. | 0,0203115 |
| AK024101 | cDNA FLJ14039 fis, clone HEMBA1005472. | 0,02031789 |
| BC041405 | clone IMAGE:5284125, mRNA. | 0,02035164 |
| NM_013368 | RPA-binding trans-activator (RBT1), mRNA. | 0,02039194 |
| NM_004112 | fibroblast growth factor 11 (FGF11), mRNA. | 0,02039592 |
| NM_002839 | protein tyrosine phosphatase, receptor type, D (PTPRD), transcript variant 1, mRNA. | 0,0204324 |
| AW178737 | IL5-HT0118-070999-001-H09 HT0118 cDNA, mRNA sequence. | 0,02047302 |
| AB067497 | mRNA for KIAA1910 protein, partial cds. | 0,02051381 |
| NM_153498 | CamKI-like protein kinase (CKLiK), transcript variant 2, mRNA. | 0,02055475 |
| NM_024522 | hypothetical protein FLJ12650 (FLJ12650), mRNA. | 0,02095733 |
| NM_014376 | cytoplasmic FMRl interacting protein 2 (CYFIP2), mRNA. | 0,02099771 |
| NM_052904 | KIAA1900 protein (KIAA1900), mRNA. | 0,02103824 |
| AB033050 | mRNA for KIAA1224 protein, partial cds. | 0,02107894 |
| NM_000425 | L1 cell adhesion molecule (hydrocephalus, stenosis of aqueduct of Sylvius 1, MASA (mental retardation, aphasia, shuffling gait and adducted thumbs) syndrome, spastic paraplegia 1) (L1CAM), transcript variant 1, mRNA. | 0,02111979 |
| NM_152613 | hypothetical protein MGC26816 (MGC26816), mRNA. | 0,0211608 |
| NM_016552 | testis specific ankyrin-like protein 1 (LOC51281), mRNA. | 0,02117224 |
| NM_004368 | calponin 2 (CNN2), mRNA. | 0,02120197 |
| NM_014047 | HSPC023 protein (HSPC023), mRNA. | 0,02124329 |
| AK027183 | cDNA: FLJ23530 fis, clone LNG06055. | 0,02128479 |
| NM_003594 | transcription termination factor, RNA polymerase II (TTF2), mRNA. | 0,02130092 |
| NM_003061 | slit homolog 1 (Drosophila) (SLIT1), mRNA. | 0,02132644 |
| NM_002267 | karyopherin alpha 3 (importin alpha 4) (KPNA3), mRNA. | 0,0216432 |
| NM_005637 | synovial sarcoma translocation, chromosome 18 (SS18), mRNA. | 0,02194216 |
| NM_024050 | hypothetical protein MGC2594 (MGC2594), mRNA. | 0,02198411 |
| NM_138933 | apobec-1 complementation factor (ACF), transcript variant 3, mRNA. | 0,02204884 |
| NM_002435 | mannose phosphate isomerase (MPI), mRNA. | 0,02209084 |
| BQ014489 | UI-H-ED1-axt-o-07-0-ULsl NCI_CGAP_ED1 cDNA clone IMAGE:5833566 3', mRNA sequence. | 0,02215466 |
| NM_005161 | angiotensin II receptor-like 1 (AGTRL1), mRNA. | 0,0221967 |
| NM_000983 | ribosomal protein L22 (RPL22), mRNA. | 0,02225968 |
| NM_021910 | FXYD domain containing ion transport regulator 3 (FXYD3), transcript variant 2, mRNA. | 0,02230176 |
| AK022111 | cDNA FLJ12049 fis, clone HEMBB1001996. | 0,02240608 |
| NM_004219 | pituitary tumor-transforming 1 (PTTG1), mRNA. | 0,0233409 |
| NM_006868 | RAB31, member RAS oncogene family (RAB31), mRNA. | 0,02335983 |
| BC033667 | clone IMAGE:5110701, mRNA. | 0,02337195 |
| NM_015198 | KIAA0633 protein (COBL), mRNA. | 0,02338389 |
| AK096389 | cDNA FLJ39070 fis, clone NT2RP7015676. | 0,02340358 |
| AK075059 | cDNA FLJ90578 fis, clone OVARC1002178. | 0,02341539 |
| NM_000135 | Fanconi anemia, complementation group A (FANCA), mRNA. | 0,02342703 |
| NM_000221 | ketohexokinase (fructokinase) (KHK), transcript variant a, mRNA. | 0,02344749 |
| BC037864 | clone IMAGE:5272804, mRNA. | 0,02345899 |
| BQ900993 | AGENCOURT_8585098 Lupski_sympathetic_trunk cDNA clone IMAGE:6192409 5', mRNA sequence. | 0,02347033 |
| AK000930 | cDNA FLJ10068 fis, clone HEMBA1001533. | 0,02348156 |
| NM_015512 | DKFZP434A236 protein (DKFZP434A236), mRNA. | 0,02349156 |
| NM_153338 | hypothetical protein FLJ90165 (FLJ90165), mRNA. | 0,02350276 |
| BC012852 | Similar to RIKEN cDNA 4930433D19 gene, clone MGC:9811 IMAGE:3860705, mRNA, complete cds. | 0,0235138 |
| NM_006229 | pancreatic lipase-related protein 1 (PNLIPRP1), mRNA. | 0,02362165 |
| BF332972 | MRI-BT0796-160600-001-c02 BT0796 cDNA, mRNA sequence. | 0,02430956 |
| NM_013438 | ubiquilin 1 (UBQLN1), transcript variant 1, mRNA. | 0,02444768 |
| CA426336 | UI-H-DF0-bek-n-06-0-UI.s1 NCI_CGAP_DFO cDNA clone UI-H-DFO-bek-n-06-0-UI 3', mRNA sequence. | 0,02454055 |
| NM_021127 | phorbol-12-myristate-13-acetate-induced protein 1 (PMAIP1), mRNA. | 0,02454381 |
| AK023734 | cDNA FLJ13672 fis, clone PLACE1011749. | 0,02458525 |
| AK056150 | cDNA FLJ31588 fis, clone NT2RI2002252. | 0,02458819 |
| Z34278 | (JER58) MUC5AC mRNA for mucin (partial). | 0,02463274 |
| NM_021101 | claudin 1 (CLDN1), mRNA. | 0,02467744 |
| NM_052965 | chromosome 1 open reading frame 19 (C1orf19), mRNA. | 0,02467977 |
| BC028630 | similar to putative, clone MGC:33835 IMAGE:5262164, mRNA, complete cds. | 0,02499498 |
| AF147791 | mucin 11 (MUC11) mRNA, partial cds. | 0,02508461 |
| AK001130 | cDNA FLJ10268 fis, clone HEMBB1001058, weakly similar to neuronal thread protein AD7c-NTP mRNA. | 0,02512964 |
| AK093095 | cDNA FLJ35776 fis, clone TESTI2005326. | 0,02521857 |
| NM_015438 | intermediate filament-like MGC:2625 (DKFZP586I2223), transcript variant 1, mRNA. | 0,02548512 |
| BC039385 | clone IMAGE:5298460, mRNA. | 0,02553063 |
| NM_004156 | protein phosphatase 2 (formerly 2A), catalytic subunit, beta isoform (PPP2CB), mRNA. | 0,02561765 |
| NM_003731 | Sjogren's syndrome nuclear autoantigen 1 (SSNA1), mRNA. | 0,02574443 |
| AL122070 | mRNA, cDNA DKFZp434E0535 (from clone DKFZp434E0535) | 0,02578991 |
| NM_024611 | similar to NMDA receptor-regulated gene 2 (mouse) (FLJ11896), mRNA. | 0,02582963 |
| NM_022754 | sideroflexin 1 (SFXN1), mRNA. | 0,02583556 |
| NM_014902 | KIAA0964 protein (KIAA0964), mRNA. | 0,02587511 |
| BQ312908 | PM4-BN0143-010600-003-h03 BN0143 cDNA, mRNA sequence. | 0,02588137 |
| NM_015469 | DKFZp564D177 protein (DKFZp564D177), mRNA. | 0,02592734 |
| NM_005415 | solute carrier family 20 (phosphate transporter), member 1 (SLC20A1), mRNA. | 0,0259597 |
| NM_006271 | S100 calcium binding protein A1 (S100A1), mRNA. | 0,02604376 |
| BC041626 | clone MGC:52394 IMAGE:4554923, mRNA, complete cds. | 0,02608937 |
| NM_016646 | mesenchymal stem cell protein DSCD28 (LOC51336), mRNA. | 0,02617278 |
| J05581 | polymorphic epithelial mucin (PEM) mRNA, complete cds. | 0,02629234 |
| AB033070 | mRNA for KIAA1244 protein, partial cds. | 0,02630134 |
| BC015977 | clone IMAGE:4042121, mRNA, partial cds. | 0,0263379 |
| NM_004869 | vacuolar protein sorting 4B (yeast) (VPS4B), mRNA. | 0,02638363 |
| NM_004496 | forkhead box A1 (FOXA1), mRNA. | 0,02641959 |
| NM_025042 | Williams-Beuren syndrome chromosome region 23 (WBSCR23), mRNA. | 0,02642951 |
| NM_145691 | ATP synthase mitochondrial F1 complex assembly factor 2 (ATPAF2), nuclear gene encoding mitochondrial protein, mRNA. | 0,02650478 |
| NM_005451 | enigma (LIM domain protein) (ENIGMA), mRNA. | 0,02652531 |
| NM_030763 | nucleosomal binding protein 1 (NSBP1), mRNA. | 0,0265464 |
| AJ292079 | partial mRNA for MUC5AC protein (mucin gene, MUC5AC). | 0,02654933 |
| NM_152369 | hypothetical protein MGC45474 (MGC45474), mRNA. | 0,02657049 |
| BC039098 | Similar to desmoglein 3 (pemphigus vulgaris antigen), clone IMAGE:4822945, mRNA, partial cds. | 0,02658132 |
| NM_139279 | neural stem cell derived neuronal survival protein (SDNSF), mRNA. | 0,02658331 |
| NM_005154 | ubiquitin specific protease 8 (USP8), mRNA. | 0,02659402 |
| NM_080476 | CDC91 cell division cycle 91-like 1 (S. cerevisiae) (CDC91L1), mRNA. | 0,02661584 |
| NM_006812 | amplified in osteosarcoma (OS-9), mRNA. | 0,02662699 |
| AL833081 | mRNA, cDNA DKFZp451J1719 (from clone DKFZp451J1719). | 0,02662784 |
| NM_012280 | FtsJ homolog 1 (E. coli) (FTSJ1), mRNA. | 0,02663887 |
| NM_025137 | hypothetical protein FLJ21439 (FLJ21439), mRNA. | 0,02666133 |
| NM_004929 | calbindin 1, 28kDa (CALB1), mRNA. | 0,02667282 |
| NM_000177 | gelsolin (amyloidosis, Finnish type) (GSN), mRNA. | 0,02668387 |
| AF074988 | full length insert cDNA YH78H07. | 0,02670699 |
| BC036321 | clone IMAGE:4837485, mRNA. | 0,02672902 |
| NM_005362 | melanoma antigen, family A, 3 (MAGEA3), mRNA. | 0,02677432 |
| NM_004056 | carbonic anhydrase VIII (CA8), mRNA. | 0,02681978 |
| NM_003091 | small nuclear ribonucleoprotein polypeptides B and B1 (SNRPB), mRNA. | 0,02687277 |
| BC039324 | clone IMAGE:5267076, mRNA. | 0,02698134 |
| NM_004252 | solute carrier family 9 (sodium/hydrogen exchanger), isoform 3 regulatory factor 1 (SLC9A3R1), mRNA. | 0,0269946 |
| NM_016391 | hypothetical protein HSPC111 (HSPC111), mRNA. | 0,02702609 |
| NM_017903 | hypothetical protein FLJ20618 (FLJ20618), mRNA. | 0,02707098 |
| NM_152324 | hypothetical protein MGC35169 (MGC35169), mRNA. | 0,02711602 |
| NM_003821 | receptor-interacting serine-threonine kinase 2 (RIPK2), mRNA. | 0,02767157 |
| NM_000572 | interleukin 10 (IL10), mRNA. | 0,02771723 |
| NM_006317 | brain abundant, membrane attached signal protein 1 (BASP1), mRNA. | 0,02776304 |
| NM_017874 | chromosome 20 open reading frame 27 (C20orf27), mRNA. | 0,02777224 |
| NM_013390 | transmembrane protein 2 (TMEM2), mRNA. | 0,02781769 |
| NM 019853 | protein phosphatase 4, regulatory subunit 2 (PPP4R2), mRNA. | 0,0278633 |
| NM_016331 | zinc finger protein ANC_2H01 (LOC51193), mRNA. | 0,02789002 |
| NM_031304 | hypothetical protein MGC4293 (MGC4293), mRNA. | 0,02790905 |
| NM_017431 | protein kinase, AMP-activated, gamma 3 non-catalytic subunit (PRKAG3), mRNA. | 0,02795495 |
| NM_080564 | SAC2 suppressor of actin mutations 2-like (yeast) (SACM2L), transcript variant 1, mRNA. | 0,02800741 |
| AK026659 | cDNA: FLJ23006 fis, clone LNG00414. | 0,02812447 |
| NM_153713 | hypothetical protein MGC46719 (MGC46719), mRNA. | 0,0282411 |
| NM_080678 | NEDDB-conjugating enzyme (NCE2), mRNA. | 0,02826574 |
| BC040879 | clone MGC:50479 IMAGE:5725643, mRNA, complete cds. | 0,02831147 |
| BC033760 | clone IMAGE:3864726, mRNA. | 0,02833573 |
| AK025416 | cDNA: FLJ21763 fis, clone COLF6967. | 0,02835736 |
| NM_024115 | hypothetical protein MGC4309 (MGC4309), mRNA. | 0,02838144 |
| NM_004036 | adenylate cyclase 3 (ADCY3), mRNA. | 0,0284288 |
| NM_005901 | MAD, mothers against decapentaplegic homolog 2 (Drosophila) (MADH2), mRNA. | 0,0284509 |
| NM_001548 | interferon-induced protein with tetratricopeptide repeats 1 (IFIT1), mRNA. | 0,02847429 |
| NM_003318 | TTK protein kinase (TTK), mRNA. | 0,02851992 |
| NM_022486 | hypothetical protein DKFZp761E1824 (DKFZP761E1824), mRNA. | 0,02854295 |
| NM_013261 | peroxisome proliferative activated receptor, gamma, coactivator 1 (PPARGC1), mRNA. | 0,0285657 |
| NM_058222 | tectorin beta (TECTB), mRNA. | 0,02865669 |
| NM_001829 | chloride channel 3 (CLCN3), mRNA. | 0,02877006 |
| AK055631 | cDNA FLJ31069 fis, clone HSYRA2001255. | 0,02947113 |
| NM_020532 | reticulon 4 (RTN4), mRNA. | 0,02951791 |
| NM_012483 | granulysin (GNLY), transcript variant 519, mRNA. | 0,0297409 |
| BF998244 | MR2-GN0158-101100-004-c01 GN0158 cDNA, mRNA sequence. | 0,02977459 |
| NM_000662 | N-acetyltransferase 1 (arylamine N-acetyltransferase) (NAT1), mRNA. | 0,02982133 |
| NM_022444 | solute carrier family 13 (sodium/sulfate symporters), member 1 (SLC13A1), mRNA. | 0,0298378 |
| NM_031284 | hypothetical protein DKFZp434B195 (DKFZP434B195), mRNA. | 0,0298402 |
| AK055590 | cDNA FLJ31028 fis, clone HLUNG2000570, weakly similar to 40S RIBOSOMAL PROTEIN S10. | 0,02985185 |
| NM_007243 | nurim (nuclear envelope membrane protein) (NRM), mRNA. | 0,02985406 |
| NM_032828 | ubiquitin UBF-fl (UBF-fl), mRNA. | 0,02986822 |
| BC004287 | clone IMAGE:3618365, mRNA. | 0,02988449 |
| NM_001062 | transcobalamin I (vitamin B12 binding protein, R binder family) (TCN1), mRNA. | 0,02988618 |
| NM_001609 | acyl-Coenzyme A dehydrogenase, short/branched chain (ACADSB), nuclear gene encoding mitochondrial protein, mRNA. | 0,02990056 |
| NM_152313 | hypothetical protein FLJ38932 (FLJ38932), mRNA. | 0,02991526 |
| AB051475 | mRNA for KIAA1688 protein, partial cds. | 0,02991648 |
| NM_018473 | uncharacterized hypothalamus protein HT012 (HT012), mRNA. | 0,0299323 |
| NM_021158 | chromosome 20 open reading frame 97 (C20orf97), mRNA. | 0,02994721 |
| NM_015541 | leucine-rich repeats and immunoglobulin-like domains 1 (LRIG1), mRNA. | 0,02996245 |
| AF289596 | clone pp7882 unknown mRNA. | 0,02996294 |
| BC041376 | Similar to hypothetical protein 5830442F04, clone MGC:43895 IMAGE:5274634, mRNA, complete cds. | 0,02997856 |
| NM_000295 | serine (or cysteine) proteinase inhibitor, clade A (alpha-1 antiproteinase, antitrypsin), member 1 (SERPINA1), mRNA. | 0,03002497 |
| NM_007019 | ubiquitin-conjugating enzyme E2C (UBE2C), mRNA. | 0,03007034 |
| NM_015141 | KIAA0089 protein (KIAA0089), mRNA. | 0,03010149 |
| AK025765 | cDNA: FLJ22112 fis, clone HEP18319. | 0,03011632 |
| NM_153246 | hypothetical protein MGC45491 (MGC45491), mRNA. | 0,03016244 |
| NM_004369 | collagen, type VI, alpha 3 (COL6A3), transcript variant 1, mRNA. | 0,03020545 |
| NM_002257 | kallikrein 1, renal/pancreas/salivary (KLK1), mRNA. | 0,0302087 |
| AK025311 | cDNA: FLJ21658 fis, clone COL08688. | 0,03025115 |
| NM_020139 | oxidoreductase UCPA (LOC56898), mRNA. | 0,03028312 |
| NM_138818 | hypothetical protein BC019095 (LOC158471), mRNA. | 0,03029699 |
| NM_024725 | hypothetical protein FLJ23518 (FLJ23518), mRNA. | 0,03031068 |
| Z34281 | (MAR10) MUC5AC mRNA for mucin (partial). | 0,03032928 |
| NM_016641 | membrane interacting protein of RGS16 (MIR16), mRNA. | 0,03034296 |
| NM_016479 | scotin (SCOTIN), mRNA. | 0,03038907 |
| NM_005011 | nuclear respiratory factor 1 (NRF1), mRNA. | 0,03092476 |
| NM_000410 | hemochromatosis (HFE), transcript variant 1, mRNA. | 0,03097126 |
| NM_002097 | general transcription factor IIIA (GTF3A), mRNA. | 0,03101791 |
| NM_006930 | S-phase kinase-associated protein 1A (p19A) (SKP1A), transcript variant 1, mRNA. | 0,03102828 |
| NM_021095 | solute carrier family 5 (sodium-dependent vitamin transporter), member 6 (SLC5A6), mRNA. | 0,03113148 |
| BC008115 , | clone MGC:16981 IMAGE:2900378, mRNA, complete cds. | 0,03138386 |
| BC017428 , | clone IMAGE:4703783, mRNA. | 0,03169009 |
| AK056929 | cDNA FLJ32367 fis, clone PUAEN1000239. | 0,03173732 |
| NM_152535 | hypothetical protein FLJ31131 (FLJ31131), mRNA. | 0,03178469 |
| NM_032316 | nicolin 1 (NICN1), mRNA. | 0,03194013 |
| NM_032287 | hypothetical protein DKFZp761O17121 (DKFZp761O17121), mRNA. | 0,03243796 |
| NM_004760 | serine/threonine kinase 17a (apoptosis-inducing) (STK17A), mRNA. | 0,03283367 |
| AF339814 | clone IMAGE:32106, mRNA sequence. | 0,0331792 |
| N23022 | yx65e12.s1 Soares melanocyte 2NbHM cDNA clone IMAGE:266638 3', mRNA sequence. | 0,03322821 |
| BC029776 | similar to CG12393 gene product, clone IMAGE:5188623, mRNA, partial cds. | 0,03327757 |
| BC032716 | clone MGC:45425 IMAGE:5518697, mRNA, complete cds. | 0,03327802 |
| NM_001453 | forkhead box C1 (FOXC1), mRNA. | 0,03332667 |
| NM_005060 | RAR-related orphan receptor C (RORC), mRNA. | 0,03337546 |
| AW510657 | hc89b09.x1 Soares_NFL_T_GBC_S1 cDNA clone IMAGE:2907161 3', mRNA sequence. | 0,03337569 |
| NM_006733 | FSH primary response (LRPR1, rat) homolog 1 (FSHPRH1), mRNA. | 0,0334244 |
| NM_004037 | adenosine monophosphate deaminase 2 (isoform L) (AMPD2), mRNA. | 0,03347092 |
| NM_152705 | hypothetical protein MGC9850 (MGC9850), mRNA. | 0,03347348 |
| NM_152320 | hypothetical protein FLJ31295 (FLJ31295), mRNA. | 0,03351929 |
| AK001604 | cDNA FLJ10742 fis, clone NT2RP3001629. | 0,0335678 |
| U55105 | isolate HR035 T cell receptor V-beta complementarity determining region 3 mRNA, partial cds. | 0,03361397 |
| AL833538 | mRNA, cDNA DKFZp686D123 (from clone DKFZp686D123). | 0,03361645 |
| NM_022790 | matrix metalloproteinase 19 (MMP19), transcript variant rasi-3, mRNA. | 0,03361773 |
| NM_000433 | neutrophil cytosolic factor 2 (65kDa, chronic granulomatous disease, autosomal 2) (NCF2), mRNA. | 0,03366233 |
| NM_018211 | hypothetical protein FLJ10770 (KIAA1579), mRNA. | 0,03366524 |
| NM_003940 | ubiquitin specific protease 13 (isopeptidase T-3) (USP13), mRNA. | 0,03366674 |
| AK094894 | cDNA FLJ37575 fis, clone BRCOC2003125, moderately similar to TRIOSEPHOSPHATE ISOMERASE (EC 5.3.1.1). | 0,03370917 |
| NM_033542 | chromosome 20 open reading frame 169 (C20orf169), mRNA. | 0,03371084 |
| NM_002165 | inhibitor of DNA binding 1, dominant negative helix-loop-helix protein (ID1), mRNA. | 0,03371417 |
| BG192666 | RST11783 Athersys RAGE Library cDNA, mRNA sequence. | 0,03380414 |
| NM_012421 | rearranged L-myc fusion sequence (RLF), mRNA. | 0,0343279 |
| BF242982 | 601877758FI NIH-MGC-55 cDNA clone IMAGE:4106055 5', mRNA sequence. | 0,03446119 |
| NM_000183 | hydroxyacyl-Coenzyme A dehydrogenase/3-ketoacyl-Coenzyme A thiolase/enoyl-Coenzyme A hydratase (trifunctional protein), beta subunit (HADHB), mRNA. | 0,03446605 |
| NM_000682 | adrenergic, alpha-2B-, receptor (ADRA2B), mRNA. | 0,03450511 |
| NM_003022 | SH3 domain binding glutamic acid-rich protein like (SH3BGRL), mRNA. | 0,03451014 |
| AK056047 | cDNA FLJ31485 fis, clone NT2NE2001698. | 0,03451522 |
| NM_020188 | DC13 protein (DC13), mRNA. | 0,03454365 |
| NM_052947 | heart alpha-kinase (HAK), mRNA. | 0,03455398 |
| NM_144704 | hypothetical protein FLJ30473 (FLJ30473), mRNA. | 0,03455923 |
| AK024933 | cDNA: FLJ21280 fis, clone COL01884. | 0,03456453 |
| AK027107 | cDNA: FLJ23454 fis, clone HSI06959. | 0,03459224 |
| BF056245 | 7k03b03.xl NCI_CGAP_GC6 cDNA clone IMAGE:3443260 3', mRNA sequence. | 0,03459761 |
| NM_014241 | protein tyrosine phosphatase-like (proline instead of catalytic arginine), member a (PTPLA), mRNA. | 0,03463541 |
| NM_000577 | interleukin 1 receptor antagonist (IL1RN), mRNA. | 0,03464096 |
| AB006909 | mRNA for A-type microphthalmia associated transcription factor, complete cds. | 0,03468982 |
| NM_005622 | SA hypertension-associated homolog (rat) (SAH), mRNA. | 0,03500699 |
| BC033549 | clone IMAGE:4826963, mRNA. | 0,03545883 |
| AK094613 | cDNA FLJ37294 fis, clone BRAMY2015211. | 0,03549913 |
| BC030557 | clone MGC:40430 IMAGE:5218944, mRNA, complete cds. | 0,03550822 |
| BG460041 | RST42470 Athersys RAGE Library cDNA, mRNA sequence. | 0,03551739 |
| NM_005981 | sarcoma amplified sequence (SAS), mRNA. | 0,03554843 |
| NM_004819 | symplekin, Huntingtin interacting protein I (SPK), mRNA. | 0,03555774 |
| AK074657 | cDNA FLJ90176 fis, clone MAMMA1000528. | 0,0356074 |
| NM 017423 | UDP-N-acetyl-alpha-D-galactosamine:polypeptide N-acetylgalactosaminyltransferase 7 (GalNAc-T7) (GALNT7), mRNA. | 0,03613069 |
| NM_153446 | beta 1,4 N-acetylgalactosaminyltransferase (B4GALT), mRNA. | 0,03618059 |
| NM_018043 | hypothetical protein FLJ10261 (FLJ10261), mRNA. | 0,03621866 |
| NM_145059 | L-fucose kinase (FUK), mRNA. | 0,03628082 |
| BC030133 | similar to syndecan 2 (heparan sulfate proteoglycan 1, cell surface-associated, fibroglycan), clone IMAGE:4138627, mRNA. | 0,0367413 |
| NM_004237 | thyroid hormone receptor interactor 13 (TRIP13), mRNA. | 0,03675532 |
| NM_016063 | CGI-130 protein (CGI-130), mRNA. | 0,03679156 |
| BC039474 | clone IMAGE:5528009, mRNA. | 0,03684196 |
| AK056395 | cDNA FLJ31833 fis, clone NT2RP6000130. | 0,03685644 |
| NM_080658 | aspartoacylase-3 (ACY-3), mRNA. | 0,03696394 |
| NM_031413 | cat eye syndrome chromosome region, candidate 2 (CECR2), mRNA. | 0,03703465 |
| NM_004613 | transglutaminase 2 (C polypeptide, protein-glutamine-gamma-glutamyltransferase) (TGM2), mRNA. | 0,03708497 |
| BC039181 | , Similar to diacylglycerol O-acyltransferase 2, clone IMAGE:4746146, mRNA. | 0,03711993 |
| NM_006732 | FBJ murine osteosarcoma viral oncogene homolog B (FOSB), mRNA. | 0,03713543 |
| NM_013248 | NTF2-like export factor 1 (NXT1), mRNA. | 0,03718602 |
| NM_033664 | cadherin 11, type 2, OB-cadherin (osteoblast) (CDH11), transcript variant 2, mRNA. | 0,0373403 |
| AK026195 | cDNA: FLJ22542 fis, clone HSI00196. | 0,0374925 |
| AK094771 | cDNA FLJ37452 fis, clone BRAWH2010618. | 0,03750938 |
| NM_015367 | BCL2-like 13 (apoptosis facilitator) (BCL2L13), nuclear gene encoding mitochondrial protein, mRNA. | 0,03752564 |
| BC011002 | clone IMAGE:3946502, mRNA, partial cds. | 0,03754242 |
| NM_000676 | adenosine A2b receptor (ADORA2B), mRNA. | 0,03756007 |
| B1052992 | RCO-GN0275-230101-031-f06 GN0275 cDNA, mRNA sequence. | 0,03757547 |
| BC034434 | clone IMAGE:4824111, mRNA. | 0,03759248 |
| NM_013230 | CD24 antigen (small cell lung carcinoma cluster 4 antigen) (CD24), mRNA. | 0,03760838 |
| U17623 | chromosome 21, Down syndrome critical region transcript, T7 end of clone a-2-d9. | 0,03764267 |
| AK091173 | cDNA FLJ33854 fis, clone CTONG2005890. | 0,03769087 |
| AF090925 | clone HQ0452 PRO0452 mRNA, partial cds. | 0,037693 |
| NM_152487 | hypothetical protein FLJ31842 (FLJ31842), mRNA. | 0,03772829 |
| NM_001992 | coagulation factor II (thrombin) receptor (F2R), mRNA. | 0,03774345 |
| BC035372 | clone MGC:35266 IMAGE:5174235, mRNA, complete cds. | 0,03779405 |
| NM_002619 | platelet factor 4 (chemokine (C-X-C motif) ligand 4) (PF4), mRNA. | 0,0378121 |
| NM_005340 | histidine triad nucleotide binding protein 1 (HINT1), mRNA. | 0,03784478 |
| NM_005855 | receptor (calcitonin) activity modifying protein 1 (RAMP1), mRNA. | 0,03789565 |
| AV648632 | AV648632 GLC cDNA clone GLCBLE06 3', mRNA sequence. | 0,03798712 |
| BU855835 | AGENCOURT_10453986 NIH_MGC_109 cDNA clone IMAGE:6645406 5', mRNA sequence. | 0,03803731 |
| AF258564 | PP3781 mRNA, complete cds. | 0,03806879 |
| NM_005263 | growth factor independent 1 (GFI1), mRNA. | 0,03815028 |
| NM_017855 | hypothetical protein FLJ20513 (FLJ20513), mRNA. | 0,03836288 |
| NM_024599 | hypothetical protein FLJ22341 (FLJ22341), mRNA. | 0,03839338 |
| NM_014682 | suppression of tumorigenicity 18 (breast carcinoma) (zinc finger protein) (ST18), mRNA. | 0,03844377 |
| AF054994 | clone 23832 mRNA sequence. | 0,03855437 |
| NM_003053 | solute carrier family 18 (vesicular monoamine), member 1 (SLC18A1), mRNA. | 0,03860483 |
| NM_004165 | Ras-related associated with diabetes (RRAD), mRNA. | 0,03863458 |
| AK093993 | cDNA FLJ36674 fis, clone UTERU2004197. | 0,03864336 |
| NM_152259 | hypothetical protein MGC45866 (MGC45866), mRNA. | 0,03866414 |
| NM_022006 | FXYD domain containing ion transport regulator 7 (FXYD7), mRNA. | 0,03869355 |
| NM_001177 | ADP-ribosylation factor-like 1 (ARL1), mRNA. | 0,03871455 |
| NM_004831 | cofactor required for Sp1 transcriptional activation, subunit 7, 70kDa (CRSP7), mRNA. | 0,03874386 |
| AK054814 | cDNA FLJ30252 fis, clone BRACE2002409, highly similar to HOMEOBOX PROTEIN MEIS3. | 0,03880207 |
| NM_021809 | TGFB-induced factor 2 (TALE family homeobox) (TGIF2), mRNA. | 0,03883093 |
| NM_022082 | chromosome 20 open reading frame 59 (C20orf59), mRNA. | 0,03885233 |
| NM_001979 | epoxide hydrolase 2, cytoplasmic (EPHX2), mRNA. | 0,0388811 |
| NM_024081 | transmembrane gamma-carboxyglutamic acid protein 4 (TMG4), mRNA. | 0,03903858 |
| AF147359 | full length insert cDNA clone YB62B07. | 0,0390447 |
| AK093330 | cDNA FLJ36011 fis, clone TESTI2015947. | 0,03906672 |
| NM_130847 | angiomotin like 1 (AMOTL1), mRNA. | 0,03909476 |
| NM_144641 | hypothetical protein FLJ32332 (FLJ32332), mRNA. | 0,039117 |
| NM_015079 | KIAA1055 protein (KIAA1055), mRNA. | 0,03914494 |
| NM_016291 | mammalian inositol hexakisphosphate kinase 2 (IP6K2), mRNA. | 0,03950616 |
| NM_004872 | chromosome 1 open reading frame 8 (Clorf8), mRNA. | 0,03960992 |
| NM_001647 | apolipoprotein D (APOD), mRNA. | 0,03966045 |
| NM_006630 | zinc finger protein 234 (ZNF234), mRNA. | 0,0397111 |
| NM_013330 | NME7 (NME7), mRNA. | 0,03983976 |
| NM_005940 | matrix metalloproteinase 11 (stromelysin 3) (MMP11), mRNA. | 0,03986544 |
| NM_025190 | KIAA1641 protein (KIAA1641), mRNA. | 0,03989038 |
| BM927308 | AGENCOURT_6688596 NIH_MGC_121 cDNA clone IMAGE:5768300 5', mRNA sequence. | 0,03991597 |
| NM_033405 | peroxisomal proliferator-activated receptor A interacting complex 285 (PRIC285), mRNA. | 0,03994113 |
| Y14488 | mRNA for putative 14kD protein containing SHMT homology, clone pUS1215. | 0,04006785 |
| NM_020240 | non-kinase Cdc42 effector protein SPEC2 (SPEC2), mRNA. | 0,04018803 |
| NM_032709 | hypothetical protein MGC13047 (MGC13047), mRNA. | 0,04023734 |
| NM_002296 | lamin B receptor (LBR), mRNA. | 0,04028677 |
| Y16713 | mRNA from HIV associated non-Hodgkin's lymphoma (clone hll-5). | 0,04033632 |
| NM_014684 | KIAA0373 gene product (KIAA0373), mRNA. | 0,040386 |
| BE501771 | hw35a01.x1 NCI_CGAP_Kid11 cDNA clone IMAGE:3184872 3', mRNA sequence. | 0,04039597 |
| NM_006833 | COP9 subunit 6 (MOV34 homolog, 34 kD) (COPS6), mRNA. | 0,0404358 |
| NM_016042 | exosome component Rrp40 (RRP40), mRNA. | 0,04048572 |
| AI652393 | wb20f02.x1 NCI_CGAP_GC6 cDNA clone IMAGE:2306235 3', mRNA sequence. | 0,04053576 |
| NM_017702 | hypothetical protein FLJ20186 (FLJ20186), mRNA. | 0,04058593 |
| BM918075 | AGENCOURT_6611357 NIH_MGC_106 cDNA clone IMAGE:5485442 5', mRNA sequence. | 0,04059716 |
| BC037917 , | clone IMAGE:5277954, mRNA. | 0,04062836 |
| BU957874 | AGENCOURT_10625720 NIH_MGC_141 cDNA clone IMAGE:6731986 5', mRNA sequence. | 0,04063622 |
| NM_144660 | hypothetical protein FLJ25082 (FLJ25082), mRNA. | 0,04068664 |
| NM_004403 | deafness, autosomal dominant 5 (DFNA5), mRNA. | 0,04073718 |
| NM_003859 | dolichyl-phosphate mannosyltransferase polypeptide 1, catalytic subunit (DPM1), mRNA. | 0,04074186 |
| NM_031966 | cyclin B1 (CCNB1), mRNA. | 0,04078785 |
| AF210651 | NAG18 (NAG18) mRNA, complete cds. | 0,04079285 |
| AL832390 | mRNA, cDNA DKFZp667H2012 (from clone DKFZp667H2012). | 0,04083864 |
| AK096306 | cDNA FLJ38987 fis, clone NT2RI2005818. | 0,04084397 |
| BU509077 | AGENCOURT_10100056 NIH_MGC_71 cDNA clone IMAGE:6503621 5', mRNA sequence. | 0,04088956 |
| NM_032387 | protein kinase, lysine deficient 4 (PRKWNK4), mRNA. | 0,04089522 |
| AB046786 | mRNA for KIAA1566 protein, partial cds. | 0,04092379 |
| AK091592 | cDNA FLJ34273 fis, clone FEBRA2003181. | 0,04094061 |
| NM_018593 | solute carrier family 16 (monocarboxylic acid transporters), member 10 (SLC16A10), mRNA. | 0,0409466 |
| NM_006392 | nucleolar protein 5A (56kDa with KKE/D repeat) (NOL5A), mRNA. | 0,0409981 |
| AF161414 | HSPC296 mRNA, partial cds. | 0,04106758 |
| AJ489592 | mRNA for glycosylation-dependent cell adhesion molecule 1 (GLYCAM1 gene), isolate KH c15. | 0,04211612 |
| NM_018293 | hypothetical protein FLJ10997 (FLJ10997), mRNA. | 0,04220568 |
| NM_005877 | splicing factor 3a, subunit 1, 120kDa (SF3A1), mRNA. | 0,04225709 |
| NM_014172 | phosphohistidine phosphatase (PHP14), mRNA. | 0,04230862 |
| NM_031299 | likely ortholog of mouse gene rich cluster, C8 gene (GRCC8), mRNA. | 0,04255496 |
| BU737015 | UI-E-DWO-agh-g-01-0-UI.s2 UI-E-DWO cDNA clone UI-E-DWO-agh-g-01-0-UI 3', mRNA sequence. | 0,04260648 |
| NM_002158 | T-cell leukemia virus enhancer factor (HTLF), mRNA. | 0,04264032 |
| NM_002192 | inhibin, beta A (activin A, activin AB alpha polypeptide) (INHBA), mRNA. | 0,04265812 |
| NM_025263 | CAT56 protein (CAT56), mRNA. | 0,04270989 |
| NM_152429 | hypothetical protein MGC39320 (MGC39320), mRNA. | 0,04310732 |
| NM_006293 | TYRO3 protein tyrosine kinase (TYRO3), mRNA. | 0,04312389 |
| BC033243 | clone IMAGE:5433475, mRNA. | 0,04314042 |
| NM_006283 | transforming, acidic coiled-coil containing protein 1 (TACC1), mRNA. | 0,04317591 |
| AK021616 | cDNA FLJ11554 fis, clone HEMBA1003037. | 0,04319234 |
| AK094421 | cDNA FLJ37102 fis, clone BRACE2019258. | 0,04320864 |
| AF191020 | E2IG5 (E2IG5) mRNA, complete cds. | 0,0432767 |
| NM_004853 | syntaxin 8 (STX8), mRNA. | 0,04335626 |
| NM_001461 | flavin containing monooxygenase 5 (FMO5), mRNA. | 0,0433605 |
| NM_025212 | Dvl-binding protein IDAX (inhibition of the Dvl and Axin complex) (IDAX), mRNA. | 0,04339205 |
| Z34280 | (JUL32) MUC5AC mRNA for mucin (partial). | 0,04340769 |
| BQ310594 | MR0-BT3002-230701-205-d09 BT3002 cDNA, mRNA sequence. | 0,04341237 |
| NM_018710 | hypothetical protein DKFZp762O076 (DKFZp762O076), mRNA. | 0,0434437 |
| NM_014262 | likely ortholog of mouse gene rich cluster, B gene (GRCB), mRNA. | 0,04345924 |
| NM_020831 | megakaryoblastic leukemia (translocation) 1 (MKL1), mRNA. | 0,04346436 |
| NM_016429 | coatomer protein complex, subunit zeta 2 (COPZ2), mRNA. | 0,04349548 |
| BC000885 | clone MGC:5619 IMAGE:3462332, mRNA, complete cds. | 0,04354739 |
| AF183570 | unknown mRNA. | 0,04365991 |
| NM_144589 | hypothetical protein FLJ23841 (FLJ23841), mRNA. | 0,04372647 |
| NM_001100 | actin, alpha 1, skeletal muscle (ACTA1), mRNA. | 0,04391093 |
| NM_004486 | golgi autoantigen, golgin subfamily a, 2 (GOLGA2), mRNA. | 0,04439631 |
| NM_138802 | hypothetical protein BC018415 (LOC130617), mRNA. | 0,04444867 |
| NM_012469 | chromosome 20 open reading frame 14 (C20orf14), mRNA. | 0,04481457 |
| NM_001957 | endothelin receptor type A (EDNRA), mRNA. | 0,04499689 |
| NM_020233 | x 006 protein (MDS006), mRNA. | 0,04508445 |
| NM_152332 | chromosome 14 open reading frame 47 (C14orf47), mRNA. | 0,04513718 |
| BF919368 | CMO-NT0131-311000-644-g09 NT0131 cDNA, mRNA sequence. | 0,04519004 |
| NM_033332 | CDC14 cell division cycle 14 homolog B (S. cerevisiae) (CDC14B), transcript variant 3, mRNA. | 0,04523724 |
| NM_013327 | parvin, beta (PARVB), mRNA. | 0,04524301 |
| BC033186 | clone IMAGE:4589300, mRNA, partial cds. | 0,04528711 |
| BC019669 | eukaryotic translation elongation factor 1 alpha 1, clone MGC:25051 IMAGE:4478650, mRNA, complete cds. | 0,04528912 |
| NM_007030 | brain-specific protein p25 alpha (p25), mRNA. | 0,04529612 |
| NM_006830 | ubiquinol-cytochrome c reductase (6.4kD) subunit (UQCR), mRNA. | 0,04531965 |
| AF441770 | Tho2 mRNA, complete cds. | 0,04533039 |
| NM_005080 | X-box binding protein 1 (XBP1), mRNA. | 0,04533977 |
| NM_014890 | downregulated in ovarian cancer 1 (DOC1), mRNA. | 0,04534111 |
| BM669556 | UI-E-DX1-agw-c-13-0-UI.s1 UI-E-DX1 cDNA clone UI-E-DX1-agw-c-13-0-UI 3', mRNA sequence. | 0,04536131 |
| AL832858 | mRNA, cDNA DKFZp667A182 (from clone DKFZp667A182). | 0,04537199 |
| NM_001185 | alpha-2-glycoprotein 1, zinc (AZGP1), mRNA. | 0,04538183 |
| NM_007187 | WW domain binding protein 4 (formin binding protein 21) (WBP4), mRNA. | 0,04538262 |
| NM_014244 | a disintegrin-like and metalloprotease (reprolysin type) with thrombospondin type 1 motif, 2 (ADAMTS2), transcript variant 1, mRNA. | 0,04539255 |
| AL831866 | mRNA, cDNA DKFZp761O0117 (from clone DKFZp76100117). | 0,04539323 |
| AF350451 | C6orf37 mRNA, complete cds. | 0,04541387 |
| NM_004873 | BCL2-associated athanogene 5 (BAG5), mRNA. | 0,04542444 |
| NM_004731 | solute carrier family 16 (monocarboxylic acid transporters), member 7 (SLC16A7), mRNA. | 0,04544546 |
| NM_001379 | DNA (cytosine-5-)-methyltransferase 1 (DNMT1), mRNA. | 0,04546656 |
| NM_005631 | smoothened homolog (Drosophila) (SMOH), mRNA. | 0,04559088 |
| AK096208 | cDNA FLJ38889 fis, clone NB9N42000314. | 0,04564304 |
| NM_004633 | interleukin 1 receptor, type II (IL1R2), mRNA. | 0,04572464 |
| AK091332 | cDNA FLJ34013 fis, clone FCBBF2002111. | 0,04577672 |
| NM_004616 | transmembrane 4 superfamily member 3 (TM4SF3), mRNA. | 0,04578628 |
| NM_015442 | hypothetical protein FLJ12890 (FLJ12890), mRNA. | 0,04582892 |
| NM_004742 | BAIl-associated protein 1 (BAIAP1), mRNA. | 0,04588123 |
| NM_002662 | phospholipase D1, phophatidylcholine-specific (PLD1), mRNA. | 0,04596129 |
| NM_005376 | v-myc myelocytomatosis viral oncogene homolog 1, lung carcinoma derived (avian) (MYCL1), mRNA. | 0,04597041 |
| NM_012190 | formyltetrahydrofolate dehydrogenase (FTHFD), transcript variant 1, mRNA. | 0,04599754 |
| AL833544 | mRNA, cDNA DKFZp686J113 (from clone DKFZp686J113). | 0,04602253 |
| NM_152400 | hypothetical protein FLJ39370 (FLJ39370), mRNA. | 0,04604934 |
| AK057953 | cDNA FLJ25224 fis, clone STM00905. | 0,04605821 |
| AF086087 | full length insert cDNA clone YZ84G08. | 0,04610125 |
| NM_152312 | hypothetical protein FLJ35207 (FLJ35207), mRNA. | 0,04615328 |
| NM_003078 | SWI/SNF related, matrix associated, actin dependent regulator of chromatin, subfamily d, member 3 (SMARCD3), mRNA. | 0,04620544 |
| NM_015969 | mitochondrial ribosomal protein S17 (MRPS17), nuclear gene encoding mitochondrial protein, mRNA. | 0,0462577 |
| BC035561 | clone MGC:40554 IMAGE:5211583, mRNA, complete cds. | 0,0462912 |
| NM_145160 | mitogen-activated protein kinase kinase 5 (MAP2K5), transcript variant A, mRNA. | 0,04634315 |
| NM_005837 | POP7 (processing of precursor, S. cerevisiae) homolog (RPP20), mRNA. | 0,04691112 |
| NM_032644 | hypothetical protein MGC2452 (MGC2452), mRNA. | 0,04708229 |
| D86519 | mRNA for neuropeptide y/peptide YY Y6 receptor, complete cds. | 0,04725312 |
| NM_006418 | differentially expressed in hematopoietic lineages (GW112), mRNA. | 0,04731195 |
| NM_021258 | interleukin 22 receptor (IL22R), mRNA. | 0,04742927 |
| AK054951 | cDNA FLJ30389 fis, clone BRACE2008276. | 0,04748214 |
| NM_004699 | DNA segment on chromosome X (unique) 9928 expressed sequence (DXS9928E), mRNA. | 0,04749329 |
| NM_003504 | CDC45 cell division cycle 45-like (S. cerevisiae) (CDC45L), mRNA. | 0,04754606 |
| NM_019038 | hypothetical protein FLJ11045 (FLJ11045), mRNA. | 0,04755146 |
| BC013389 | clone IMAGE:3932143, mRNA. | 0,04759894 |
| AB058758 | mRNA for KIAA1855 protein, partial cds. | 0,04760951 |
| NM_004313 | arrestin, beta 2 (ARRB2), mRNA. | 0,04772526 |
| NM_130777 | G antigen, family D, 3 (GAGED3), mRNA. | 0,04777805 |
| AI222167 | qg96g02.x1 Soares_NFL_T_GBC_S1 cDNA clone IMAGE:1843058 3', mRNA sequence. | 0,04784522 |
| NM_005630 | solute carrier family 21 (prostaglandin transporter), member 2 (SLC21A2), mRNA. | 0,04789791 |
| NM_152629 | hypothetical protein MGC33662 (MGC33662), mRNA. | 0,0479025 |
| AK021691 | cDNA FLJ11629 fis, clone HEMBA1004241. | 0,04795072 |
| NM_145654 | RAD52B (RAD52B), mRNA. | 0,04800364 |
| AK000839 | cDNA FLJ20832 fis, clone ADKA03033. | 0,04806942 |
| AK093454 | cDNA FLJ36135 fis, clone TESTI2024718. | 0,04823598 |
| AF088032 | full length insert cDNA clone ZC24D06. | 0,04846192 |
| NM_003266 | toll-like receptor 4 (TLR4), transcript variant 3, mRNA. | 0,04851167 |
| NM_032251 | hypothetical protein DKFZp434G0920 (DKFZp434G0920), mRNA. | 0,04851489 |
| NM_014035 | SBBI31 protein (SBBI31), mRNA. | 0,04856797 |
| AF086365 | full length insert cDNA clone ZD66D02. | 0,04862714 |
| AK098031 | cDNA FLJ40712 fis, clone THYMU2027249. | 0,04868307 |
| NM_003134 | signal recognition particle 14kDa (homologous Alu RNA binding protein) (SRP14), mRNA. | 0,04879975 |
| NM_152904 | sperm antigen HCMOGT-1 (HCMOGT-1), mRNA. | 0,04884769 |
| NM_138414 | hypothetical protein BC011981 (LOC112869), mRNA. | 0,04885262 |
| NM_152374 | hypothetical protein FLJ38984 (FLJ38984), mRNA. | 0,04890326 |
| AK024457 | mRNA for FLJ00049 protein, partial cds. | 0,04890561 |
| BC012476 | clone IMAGE:4537124, mRNA, partial cds. | 0,04895871 |
| AK090949 | cDNA FLJ33630 fis, clone BRAMY2022525. | 0,04939551 |
| AK055934 | cDNA FLJ31372 fis, clone NB9N42000281. | 0,04966748 |
| NM_005358 | LIM domain only 7 (LMO7), transcript variant 1, mRNA. | 0,04966817 |
| AK024118 | cDNA FLJ14056 fis, clone HEMBB1000335. | 0,04966888 |
| B1256572 | 602976939F1 NIH_MGC_12 cDNA clone IMAGE:5116233 5', mRNA sequence. | 0,04972095 |
| AK091169 | cDNA FLJ33850 fis, clone CTONG2005615. | 0,04972152 |
| NM_005416 | small proline-rich protein 3 (SPRR3), mRNA. | 0,04972211 |
| M57423 | phosphoribosylpyrophosphate synthetase subunit III mRNA, 3' end. | 0,04977453 |
| AK022101 | cDNA FLJ12039 fis, clone HEMBB1001930. | 0,04982822 |
| AF297014 | RGS8 mRNA, partial sequence. | 0,04988203 |
| NM_005173 | ATPase, Ca++ transporting, ubiquitous (ATP2A3), mRNA. | 0,04993655 |
| NM_014141 | contactin associated protein-like 2 (CNTNAP2), mRNA. | 0,04999 |

**Table 2: Human genes differentially expressed between colorectal adenoma and adenocarcinoma cells with chromosomal loss at 8p. (univariant analysis). Data are ranked on FDR value**

| Gene ID | Description (8 p loss, Table 2) | FDR | Effect |
|---|---|---|---|
| NM_018388 | CHCR (CHCR), mRNA. | 0,005045 | -0,54868 |
| AK054799 | cDNA FLJ30237 fis, clone BRACE2002034. | 0,005971 | -0,39081 |
| NM_016397 | TH1-like (Drosophila) (TH1L), mRNA. | 0,006725 | 0,298588 |
| NM_000637 | glutathione reductase (GSR), mRNA. | 0,006725 | -0,33537 |
| D31887 | mRNA for KIAA0062 gene, partial cds. | 0,006725 | -0,49409 |
| NM_006602 | transcription factor-like 5 (basic helix-loop-helix) (TCFL5), mRNA. | 0,006725 | 0,638295 |
| AK026883 | cDNA: FLJ23230 fis, clone CAE07143. | 0,006725 | 0,426682 |
| NM_018270 | chromosome 20 open reading frame 20 (C20orf20), mRNA. | 0,006725 | 0,23481 |
| NM_004901 | lysosomal apyrase-like 1 (LYSAL1), mRNA. | 0,006725 | -0,23079 |
| NM_021928 | hypothetical protein FLJ22649 similar to signal peptidase SPC22/23 (FLJ22649), mRNA. | 0,007549 | -0,22392 |
| NM_003747 | tankyrase, TRF1-interacting ankyrin-related ADP-ribose polymerase (TNKS), mRNA. | 0,011251 | -0,39827 |
| NM_030579 | cytochrome b5 outer mitochondrial membrane precursor (CYB5-M), mRNA. | 0,012808 | 0,247417 |
| NM_002609 | platelet-derived growth factor receptor, beta polypeptide (PDGFRB), mRNA. | 0,020483 | 0,51344 |
| NM_033126 | serine/threonine kinase PSKH2 (PSKH2), mRNA. | 0,020483 | 0,472905 |
| NM_003014 | secreted frizzled-related protein 4 (SFRP4), mRNA. | 0,021177 | 0,842529 |
| NM_020749 | AT2 receptor-interacting protein 1 (ATIP1), mRNA. | 0,021177 | -0,36043 |
| NM_007002 | adhesion regulating molecule 1 (ADRM1), mRNA. | 0,021177 | 0,285899 |
| NM_057093 | crystallin, beta A2 (CRYBA2), transcript variant 2, mRNA. | 0,023543 | -0,41107 |
| NM_172250 | methylmalonic aciduria type A (MMAA), mRNA. | 0,023543 | -0,34183 |
| BC015412 | clone IMAGE:4393471, mRNA. | 0,023543 | 0,397184 |
| NM_002640 | serine (or cysteine) proteinase inhibitor, clade B (ovalbumin), member 8 (SERPINB8), mRNA. | 0,023543 | -0,40671 |
| NM_031285 | hypothetical protein PP1057 (PP1057), mRNA. | 0,024446 | 0,44688 |
| NM_001696 | ATPase, H+ transporting, lysosomal 31kDa, V1 subunit E isoform 1 (ATP6V1E1), mRNA. | 0,024446 | 0,186487 |
| AK094784 | cDNA FLJ37465 fis, clone BRAWH2011823, highly similar to BONE MORPHOGENETIC PROTEIN 7 PRECURSOR. | 0,024935 | 0,506101 |
| NM_018710 | hypothetical protein DKFZp762O076 (DKFZp762O076), mRNA. | 0,027424 | 0,387812 |
| NM_006809 | translocase of outer mitochondrial membrane 34 (TOMM34), mRNA. | 0,029001 | 0,404823 |
| BM476468 | AGENCOURT_6476297 NIH_MGC_85 cDNA clone IMAGE:5553395 5', mRNA sequence. | 0,030444 | 0,429324 |
| NM_152332 | chromosome 14 open reading frame 47 (C14orf47), mRNA. | 0,030466 | -0,3124 |
| AW977106 | EST389215 MAGE resequences, MAGO cDNA, mRNA sequence. | 0,031602 | 0,317498 |
| NM_000238 | potassium voltage-gated channel, subfamily H (eag-related), member 2 (KCNH2), transcript variant 1, mRNA. | 0,031646 | 0,440776 |
| NM_012155 | echinoderm microtubule associated protein like 2 (EML2), mRNA. | 0,033412 | -0,45142 |
| AK097647 | cDNA FLJ40328 fis, clone TESTI2031356. | 0,033412 | 0,296624 |
| BC004890 | clone IMAGE:3937917, mRNA. | 0,033412 | -0,44593 |
| NM_006493 | ceroid-lipofuscinosis, neuronal 5 (CLN5), mRNA. | 0,033965 | -0,31853 |
| BC032316 | clone IMAGE:5219499, mRNA. | 0,035205 | -0,38849 |
| NM_003118 | secreted protein, acidic, cysteine-rich (osteonectin) (SPARC), mRNA. | 0,035205 | 0,443985 |
| AK090998 | cDNA FLJ33679 fis, clone BRAWH2002352. | 0,036982 | -0,53683 |
| NM_022062 | PBX/knotted 1 homeobox 2 (PKNOX2), mRNA. | 0,03775 | 0,266646 |
| NM_017872 | hypothetical protein FLJ20546 (FLJ20546), mRNA. | 0,038899 | -0,24664 |
| NM_003186 | transgelin (TAGLN), mRNA. | 0,038899 | 0,363435 |
| BC025370 | clone IMAGE:3945331, mRNA. | 0,039111 | 0,486875 |
| BC013389 | clone IMAGE:3932143, mRNA. | 0,039111 | 0,437633 |
| NM_006251 | protein kinase, AMP-activated, alpha 1 catalytic subunit (PRKAA1), mRNA. | 0,039111 | -0,32277 |
| BQ921112 | AGENCOURT_8926187 NIH_MGC_101 cDNA clone IMAGE:6463013 5', mRNA sequence. | 0,039111 | 0,492923 |
| AK024156 | cDNA FLJ14094 fis, clone MAMMA1000372. | 0,039111 | -0,42942 |
| NM_021095 | solute carrier family 5 (sodium-dependent vitamin transporter), member 6 (SLC5A6), mRNA. | 0,039111 | 0,378796 |
| NM_004704 | U3 snoRNP-associated 55-kDa protein (U3-55K), mRNA. | 0,039111 | 0,27622 |
| NM_001933 | dihydrolipoamide S-succinyltransferase (E2 component of 2-oxo-glutarate complex) (DLST), mRNA. | 0,039111 | -0,25677 |
| NM_020169 | latexin protein (LXN), mRNA. | 0,040379 | -0,22413 |
| AK023018 | cDNA FLJ12956 fis, clone NT2RP2005501. | 0,040379 | 0,306279 |
| NM_000285 | peptidase D (PEPD), mRNA. | 0,040711 | 0,263666 |
| BC011877 | Similar to dedicator of cyto-kinesis 1, clone IMAGE:3347029, mRNA. | 0,041668 | -0,33248 |
| AK026367 | cDNA: FLJ22714 fis, clone HSI13646. | 0,041668 | 0,388352 |
| NM_025138 | hypothetical protein FLJ12661 (FLJ12661), transcript variant 1, mRNA. | 0,042841 | 0,266503 |
| NM_020650 | hypothetical protein LOC57333 (LOC57333), mRNA. | 0,043572 | 0,474606 |
| NM_006197 | pericentriolar material 1 (PCM1), mRNA. | 0,045178 | -0,27764 |
| NM_000698 | arachidonate 5-lipoxygenase (ALOX5), mRNA. | 0,045178 | -0,44187 |
| AK021812 | cDNA FLJ11750 fis, clone HEMBA1005568. | 0,045178 | -0,35545 |
| AW296028 | UI-H-BWO-aiu-d-04-0-UI.s1 NCI-CGAP-Sub6 cDNA clone IMAGE:2730654 3', mRNA sequence. | 0,045452 | 0,340944 |
| NM_017933 | hypothetical protein FLJ20701 (FLJ20701), mRNA. | 0,048261 | -0,35263 |
| NM_138784 | hypothetical protein BC014341 (LOC116123), mRNA. | 0,048697 | -0,36672 |
| NM_080476 | CDC91 cell division cycle 91-like 1 (S. cerevisiae) (CDC91L1), mRNA. | 0,048697 | 0,276457 |
| NM_032242 | hypothetical protein DKFZp564A176 (DKFZp564A176), mRNA. | 0,052079 | 0,249169 |
| NM_003276 | thymopoietin (TMPO), mRNA. | 0,053471 | -0,27181 |
| NM_002562 | purinergic receptor P2X, ligand-gated ion channel, 7 (P2RX7), mRNA. | 0,056426 | 0,295663 |
| NM_007236 | calcium binding protein P22 (CHP), mRNA. | 0,057301 | -0,20633 |
| NM_152745 | neurexophilin 1 (NXPH1), mRNA. | 0,058683 | -0,30813 |
| NM_003804 | receptor (TNFRSF)-interacting serine-threonine kinase 1 (RIPK1), mRNA. | 0,059712 | -0,30649 |
| AL137279 | mRNA, cDNA DKFZp434O1214 (from clone DKFZp434O1214). | 0,061202 | 0,363935 |
| BM561501 1 | AGENCOURT_6567312 NIH_MGC_88 cDNA clone IMAGE:5739715 5', mRNA sequence. | 0,061202 | 0,411812 |
| NM_018203 | hypothetical protein FLJ10748 (FLJ10748), mRNA. | 0,061202 | -0,38393 |
| NM_021724 | nuclear receptor subfamily 1, group D, member 1 (NR1D1), mRNA. | 0,061202 | 0,392836 |
| NM_016127 | hypothetical protein MGC8721 (MGC8721), mRNA. | 0,061202 | -0,20445 |
| NM_015705 | hypothetical protein DJ1042K10.2 (DJ1042K10.2), mRNA. | 0,061202 | -0,39612 |
| NM_007019 | ubiquitin-conjugating enzyme E2C (UBE2C), mRNA. | 0,061202 | 0,292396 |
| NM_000177 | gelsolin (amyloidosis, Finnish type) (GSN), mRNA. | 0,061202 | -0,16679 |
| NM_012461 | TERF1 (TRF1)-interacting nuclear factor 2 (TINF2), mRNA. | 0,061202 | -0,19784 |
| AK022220 | cDNA FLJ12158 fis, clone MAMMA1000522. | 0,061202 | -0,32507 |
| NM_022157 | Rag C protein (GTR2), mRNA. | 0,061202 | 0,180955 |
| NM_018488 | T-box 4 (TBX4), mRNA. | 0,06159 | 0,311215 |
| NM_004993 | Machado-Joseph disease (spinocerebellar ataxia 3, olivopontocerebellar ataxia 3, autosomal dominant, ataxin 3) (MJD), transcript variant 1, mRNA. | 0,06159 | -0,3148 |
| AL831872 | mRNA, cDNA DKFZp547A1310 (from clone DKFZp547A1310). | 0,061791 1 | -0,46465 |
| BM921036 | AGENCOURT_6633196 NIH_MGC_115 cDNA clone IMAGE:5752355 5', mRNA sequence. | 0,061791 | 0,313878 |
| NM_014282 | hyaluronan binding protein 4 (HABP4), mRNA. | 0,062383 | 0,27001 |
| AB033100 | mRNA for KIAA1274 protein, partial cds. | 0,062383 | 0,495481 |
| NM_007253 | cytochrome P450, subfamily IVF, polypeptide 8 (CYP4F8), mRNA. | 0,062383 | -0,40672 |
| NM_014636 | Ral guanine nucleotide exchange factor RaIGPS1A (RALGPS1A), mRNA. | 0,062383 | -0,19961 |
| AY102069 | surfactant associated protein F mRNA, partial sequence. | 0,062383 | -0,40876 |
| NM_006097 | myosin regulatory light chain 2, smooth muscle isoform (MYRL2), mRNA. | 0,062383 | 0,338708 |
| NM_016938 | EGF-containing fibulin-like extracellular matrix protein 2 (EFEMP2), mRNA. | 0,062383 | 0,32609 |
| NM_018414 | GalNAc alpha-2, 6-sialyltransferase I, long form (ST6GalNAcI), mRNA. | 0,062383 | -0,60664 |
| NM_003617 | regulator of G-protein signalling 5 (RGS5), mRNA. | 0,062383 | 0,377022 |
| NM_015540 | DKFZP727M111 protein (DKFZP727M111), mRNA. | 0,062383 | 0,167647 |
| NM_033120 | naked cuticle H.log 2 (Drosophila) (NKD2), mRNA. | 0,062383 | 0,675064 |
| NM_015512 | DKFZP434A236 protein (DKFZP434A236), mRNA. | 0,063004 | 0,254703 |
| AF085900 | full length insert cDNA clone YQ28C05. | 0,065715 | -0,27278 |
| NM_001008 | ribosomal protein S4, Y-linked (RPS4Y), mRNA. | 0,065715 | -0,85366 |
| NM_017896 | chromosome 20 open reading frame 11 (C20orf11), mRNA. | 0,067243 | 0,227182 |
| NM_000367 | thiopurine S-methyltransferase (TPMT), mRNA. | 0,067243 | -0,32273 |
| NM_007011 | abhydrolase domain containing 2 (ABHD2), transcript variant 1, mRNA. | 0,067243 | -0,33685 |
| NM_002305 | lectin, galactoside-binding, soluble, 1 (galectin 1) (LGALS1), mRNA. | 0,069165 | 0,454091 |
| AF001892 | MEN1 region clone epsilon/beta mRNA, 5' fragment. | 0,069489 | -0,58133 |
| NM_005877 | splicing factor 3a, subunit 1, 120kDa (SF3A1), mRNA. | 0,06978 | -0,23195 |
| AB033070 | mRNA for KIAA1244 protein, partial cds. | 0,06978 | -0,35261 |
| BC034374 | similar to FKSG76, clone MGC:35390 IMAGE:5185621, mRNA, complete cds. | 0,069786 | 0,23969 |
| NM_032125 | hypothetical protein DKFZp564D0478 (DKFZP564D0478), mRNA. | 0,069786 | 0,212384 |
| NM_004403 | deafness, autosomal dominant 5 (DFNA5), mRNA. | 0,069786 | 0,283821 |
| NM_020806 | gephyrin (GPHN), mRNA. | 0,069786 | -0,19149 |
| NM_005959 | melatonin receptor 1B (MTNR1B), mRNA. | 0,069786 | -0,31553 |
| NM_005469 | peroxisomal acyl-CoA thioesterase (PTE1), mRNA. | 0,069786 | 0,293167 |
| AL713790 | mRNA, cDNA DKFZp564F1062 (from clone DKFZp564F1062). | 0,069786 | -0,25592 |
| B1520375 | 603071853F1 NIH_MGC_119 cDNA clone IMAGE:5163756 5', mRNA sequence. | 0,069786 | -0,2411 |
| NM_000119 | erythrocyte membrane protein band 4.2 (EPB42), mRNA. | 0,069786 | -0,24152 |
| AB002339 | mRNA for KIAA0341 gene, partial cds. | 0,069786 | -0,26811 |
| NM_004139 | lipopolysaccharide binding protein (LBP), mRNA. | 0,069786 | 0,18535 |
| NM_012331 | methionine sulfoxide reductase A (MSRA), mRNA. | 0,069786 | -0,22314 |
| NM_018422 | hypothetical protein DKFZp761K1423 (DKFZp761K1423), mRNA. | 0,070503 | -0,44371 |
| NM_001951 | E2F transcription factor 5, p130-binding (E2F5), mRNA. | 0,070596 | 0,274336 |
| NM_018043 | hypothetical protein FLJ10261 (FLJ10261), mRNA. | 0,070596 | -0,50301 |
| BC002877 | Similar to hypothetical protein FLJ11585, clone MGC:11258 IMAGE:3942160, mRNA, complete cds. | 0,070596 | -0,20351 |
| NM_015721 | gem (nuclear organelle) associated protein 4 (GEMIN4), mRNA. | 0,070946 | 0,228449 |
| BQ230145 | AGENCOURT_7592688 NIH_MGC_72 cDNA clone IMAGE:6050363 5', mRNA sequence. | 0,072458 | 0,310216 |
| AK057962 | cDNA FLJ25233 fis, clone STM01789. | 0,072458 | -0,32341 |
| AK021887 | cDNA FLJ11825 fis, clone HEMBA1006494. | 0,072998 | -0,51913 |
| AK022479 | cDNA FLJ12417 fis, clone MAMMA1003039. | 0,074457 | 0,407503 |
| AK057664 | cDNA FLJ33102 fis, clone TRACH2000898. | 0,074457 | -0,32304 |
| NM_138706 | beta-1,3-N-acetylglucosaminyltransferase protein (IMAGE:4907098), mRNA. | 0,074457 | -0,59194 |
| NM_032803 | solute carrier family 7 (cationic amino acid transporter, y+ system), member 3 (SLC7A3), mRNA. | 0,074519 | 0,707353 |
| U17623 | chromosome 21, Down syndrome critical region transcript, T7 end of clone a-2-d9. | 0,07505 | 0,255058 |
| NM_017762 | hypothetical protein FLJ20313 (FLJ20313), mRNA. | 0,075595 | -0,32046 |
| NM_007125 | ubiquitously transcribed tetratricopeptide repeat gene, Y chromosome (UTY), mRNA. | 0,077966 | -0,61812 |
| NM_144498 | oxysterol binding protein-like 2 (OSBPL2), transcript variant 2, mRNA. | 0,079602 | 0,344545 |
| NM_018569 | hypothetical protein PR00971 (PR00971), mRNA. | 0,079602 | 0,253986 |
| NM_024075 | LENG5 protein (LENG5), mRNA. | 0,079602 | 0,149324 |
| NM_002525 | nardilysin (N-arginine dibasic convertase) (NRD1), mRNA. | 0,079602 | -0,32216 |
| NM_015962 | CGI-35 protein (CGI-35), mRNA. | 0,079602 | -0,30982 |
| NM_017709 | hypothetical protein FLJ20202 (FLJ20202), mRNA. | 0,079602 | -0,5167 |
| M27316 | transfer RNA-Ser. | 0,079602 | 0,78553 |
| NM_002691 | polymerase (DNA directed), delta 1, catalytic subunit 125kDa (POLD1), mRNA. | 0,080006 | 0,221819 |
| NM_004603 | syntaxin 1A (brain) (STX1A), mRNA. | 0,080006 | 0,402681 |
| NM_032878 | hypothetical protein MGC15677 (MGC15677), mRNA. | 0,080006 | 0,284438 |
| AK022101 | cDNA FLJ12039 fis, clone HEMBB1001930. | 0,080006 | -0,23547 |
| BC038767 | clone IMAGE:5270478, mRNA. | 0,080006 | -0,51549 |
| AK027819 | cDNA FLJ14913 fis, clone PLACE1006782. | 0,080006 | -0,38327 |
| NM_138963 | ribosomal protein S4, Y-linked 2 (RPS4Y2), mRNA. | 0,080006 | -0,53287 |
| NM_024490 | ATPase, Class V, type 10A (ATP10A), mRNA. | 0,080006 | 0,351773 |
| BQ072652 | AGENCOURT_6763016 NIH_MGC_118 cDNA clone IMAGE:5756116 5', mRNA sequence. | 0,080006 | 0,302702 |
| NM_019016 | hypothetical protein FLJ20261 (FLJ20261), mRNA. | 0,081109 | -0,26741 |
| BE410139 | 60130245IF1 NIH_MGC_21 cDNA clone IMAGE:3636877 5', mRNA sequence. | 0,081109 | 0,4566 |
| NM_021258 | interleukin 22 receptor (IL22R), mRNA. | 0,081132 | 0,515985 |
| NM_014786 | Rho-specific guanine-nucleotide exchange factor 164 kDa (P164RHOGEF), mRNA. | 0,081408 | 0,18099 |
| NM_001997 | Finkel-Biskis-Reilly murine sarcoma virus (FBR-MuSV) ubiquitously expressed (fox derived) ribosomal protein S30 (FAU), mRNA. | 0,081888 | 0,25712 |
| NM_032611 | protein tyrosine phosphatase type IVA, member 3 (PTP4A3), transcript variant 1, mRNA. | 0,081888 | 0,362214 |
| AB011131 | mRNA for KIAA0559 protein, partial cds. | 0,081888 | -0,2282 |
| NM_018142 | hypothetical protein FLJ10569 (FLJ10569), mRNA. | 0,081888 | -0,30228 |
| AK001903 | cDNA FLJ11041 fis, clone PLACE1004405. | 0,081888 | 0,583886 |
| AL122070 | mRNA, cDNA DKFZp434E0535 (from clone DKFZp434E0535) | 0,081888 | -0,3407 |
| NM_004669 | chloride intracellular channel 3 (CLIC3), mRNA. | 0,082476 | -0,69518 |
| NM_016305 | synovial sarcoma translocation gene on chromosome 18-like 2 (SS18L2), mRNA. | 0,082476 | 0,194934 |
| NM_144664 | hypothetical protein MGC33371 (MGC33371), mRNA. | 0,082476 | -0,30557 |
| NM_002573 | platelet-activating factor acetylhydrolase, isoform Ib, gamma subunit 29kDa (PAFAH1B3), mRNA. | 0,082476 | 0,189294 |
| AW084755 | xc57d02.x1 NCI_CGAP _Eso2 cDNA clone IMAGE:2588355 3' similar to SW:CHH2_BOMMO P05687 CHORION CLASS HIGH-CYSTEINE HCA PROTEIN 12 PRECURSOR contains element MSR1 repetitive element | 0,082476 | 0,443077 |
| NM_014071 | nuclear receptor coactivator 6 (NCOA6), mRNA. | 0,082476 | 0,270178 |
| AF085837 | full length insert cDNA clone YI41H11. | 0,082476 | 0,268544 |
| NM_012392 | PEF protein with a long N-terminal hydrophobic domain (peflin) (PEF), mRNA. | 0,082476 | 0,169837 |
| AK094413 | cDNA FLJ37094 fis, clone BRACE2018337. | 0,082586 | -0,32562 |
| NM_000435 | Notch H.log 3 (Drosophila) (NOTCH3), mRNA. | 0,082586 | 0,17514 |
| NM_152312 | hypothetical protein FLJ35207 (FLJ35207), mRNA. | 0,082776 | 0,498205 |
| NM_172171 | calcium/calmodulin-dependent protein kinase (CaM kinase) II gamma (CAMK2G), transcript variant 1, mRNA. | 0,082776 | 0,205556 |
| AK001130 | cDNA FLJ10268 fis, clone HEMBB1001058, weakly similar to neuronal thread protein AD7c-NTP mRNA. | 0,08327 | 0,318442 |
| BC014441 | Similar to RIKEN cDNA 2810405F18 gene, clone MGC:22960 IMAGE:4865283, mRNA, complete cds. | 0,083328 | -0,23912 |
| BC038852 | clone IMAGE:5168364, mRNA. | 0,083328 | -0,57493 |
| NM_019846 | chemokine (C-C motif) ligand 28 (CCL28), transcript variant 1, mRNA. | 0,083402 | -0,40981 |
| NM_003298 | nuclear receptor subfamily 2, group C, member 2 (NR2C2), mRNA. | 0,085307 | -0,20389 |
| AK022976 | cDNA FLJ12914 fis, clone NT2RP2004523. | 0,085349 | 0,348863 |
| AL831875 | mRNA, cDNA DKFZp547E1510 (from clone DKFZp547E1510). | 0,085549 | 0,206302 |
| AK056573 | cDNA FLJ32011 fis, clone NT2RP7009507. | 0,085549 | -0,25456 |
| AK022947 | cDNA FLJ12885 fis, clone NT2RP2003988. | 0,085549 | 0,273694 |
| NM_024513 | FYVE and coiled-coil domain containing 1 (FYCO1), mRNA. | 0,085549 | -0,21209 |
| AB033107 | mRNA for KIAA1281 protein, partial cds. | 0,086768 | 0,231675 |
| AF087970 | full length insert cDNA clone YU75B05. | 0,086768 | -0,26453 |
| NM_005276 | glycerol-3-phosphate dehydrogenase 1 (soluble) (GPD1), mRNA. | 0,08722 | -0,23882 |
| NM_004315 | N-acylsphingosine amidohydrolase (acid ceramidase) 1 (ASAH1), mRNA. | 0,08747 | -0,26787 |
| NM_006752 | surfeit 5 (SURF5), transcript variant a, mRNA. | 0,08747 | 0,233567 |
| NM_003185 | TAF4 RNA polymerase II, TATA box binding protein (TBP)-associated factor, 135kDa (TAF4), mRNA. | 0,08747 | 0,255894 |
| AB002314 | mRNA for KIAA0316 protein, partial cds. | 0,08747 | -0,322 |
| AB037842 | mRNA for KIAA1421 protein, partial cds. | 0,08747 | -0,32792 |
| NM_002979 | sterol carrier protein 2 (SCP2), mRNA. | 0,08865 | -0,19443 |
| BC021575 | clone IMAGE:3161966, mRNA. | 0,08865 | 0,136964 |
| AK022745 | cDNA FLJ12683 fis, clone NT2RM4002457. | 0,08865 | 0,379521 |
| NM_025209 | enhancer of polycomb H.log 1, (Drosophila) (EPC1), mRNA. | 0,08865 | 0,132535 |
| CA314430 | UI-CF-FNO-afm-n-08-0-ULsl UI-CF-FN0 cDNA clone UI-CF-FNO-afm-n-08-0-UI 3', mRNA sequence. | 0,08865 | -0,23737 |
| NM_020673 | RAB22A, member RAS oncogene family (RAB22A), mRNA. | 0,08865 | 0,229513 |
| BQ220757 | AGENCOURT_7589763 NIH_MGC_92 cDNA | 0,08865 | 0,288766 |
| | clone IMAGE:6067696 5', mRNA sequence. | | |
| NM_025047 | hypothetical protein FLJ22595 (FLJ22595), mRNA. | 0,08865 | -0,24253 |
| NM_152415 | hypothetical protein FLJ32642 (FLJ32642), mRNA. | 0,08865 | -0,17664 |
| NM_016569 | T-box 3 (ulnar mammary syndrome) (TBX3), transcript variant 2, mRNA. | 0,08865 | -0,41763 |
| AK075564 | cDNA PSEC0264 fis, clone NT2RP3002337. | 0,08865 | -0,60426 |
| NM_002586 | pre-B-cell leukemia transcription factor 2 (PBX2), mRNA. | 0,08865 | 0,299901 |
| AF070620 | clone 24694 mRNA sequence. | 0,08865 | 0,190677 |
| BM674956 | UI-E-EJ0-ahn-c-06-0-UI.s1 UI-E-EJ0 cDNA clone UI-E-EJ0-ahn-c-06-0-UI 3', mRNA sequence. | 0,08865 | -0,26423 |
| NM_005104 | bromodomain containing 2 (BRD2), mRNA. | 0,08865 | -0,16248 |
| AK092106 | cDNA FLJ34787 fis, clone NT2NE2004740. | 0,08969 | -0,3318 |
| NM_153246 | hypothetical protein MGC45491 (MGC45491), mRNA. | 0,08969 | 0,520345 |
| NM_013974 | dimethylarginine dimethylaminohydrolase 2 (DDAH2), mRNA. | 0,08969 | 0,211115 |
| NM_005415 | solute carrier family 20 (phosphate transporter), member 1 (SLC20A1), mRNA. | 0,08969 | 0,258846 |
| NM_031299 | likely ortholog of mouse gene rich cluster, C8 gene (GRCC8), mRNA. | 0,08969 | 0,337448 |
| BC002782 | clone IMAGE:3621749, mRNA, partial cds. | 0,091053 | 0,314213 |
| NM_138284 | interleukin 17D (ILI7D), mRNA. | 0,091053 | 0,509744 |
| NM_004776 | UDP-Gal:betaGlcNAc beta 1,4- galactosyltransferase, polypeptide 5 (B4GALT5), mRNA. | 0,091532 | 0,160436 |
| NM_020750 | exportin 5 (XPO5), mRNA. | 0,091934 | 0,216215 |
| AK096002 | cDNA FLJ38683 fis, clone KIDNE2000777. | 0,093478 | -0,89719 |
| NM_005313 | glucose regulated protein, 58kDa (GRP58), mRNA. | 0,093989 | -0,29814 |
| NM_030763 | nucleosomal binding protein 1 (NSBP1), mRNA. | 0,094327 | -0,31628 |
| NM_006644 | heat shock 105kD (HSP105B), mRNA. | 0,094327 | 0,321667 |
| AF304443 | B lymphocyte activation-related protein BC-2048 mRNA, complete cds. | 0,094327 | -0,7255 |
| NM_005015 | oxidase (cytochrome c) assembly 1-like (OXA1L), mRNA. | 0,094327 | -0,15643 |
| NM_005339 | huntingtin interacting protein 2 (HIP2), mRNA. | 0,094327 | -0,31967 |
| AL833655 | mRNA, cDNA DKFZp667O0320 (from clone DKFZp667O0320). | 0,094327 | -0,34752 |
| AK094651 | cDNA FLJ37332 fis, clone BRAMY2019710. | 0,094327 | -0,20019 |
| NM_000088 | collagen, type I, alpha 1 (COL1A1), mRNA. | 0,094327 | 0,510085 |
| NM_000135 | Fanconi anemia, complementation group A (FANCA), mRNA. | 0,094327 | 0,192872 |
| BC016795 | clone IMAGE:4067166, mRNA. | 0,094327 | -0,46023 |
| AB006909 | mRNA for A-type microphthalmia associated transcription factor, complete cds. | 0,094327 | 0,353937 |
| NM_000073 | CD3G antigen, gamma polypeptide (TiT3 complex) (CD3G), mRNA. | 0,094327 | 0,232673 |
| NM_020182 | transmembrane, prostate androgen induced RNA (TMEPAI), mRNA. | 0,094327 | 0,390529 |
| BI769135 | 603053727FI NIH_MGC_122 cDNA clone IMAGE:5203207 5', mRNA sequence. | 0,094327 | 0,284732 |
| NM_005080 | X-box binding protein 1 (XBP1), mRNA. | 0,094327 | -0,24926 |
| AB007875 | KIAA0415 mRNA, complete cds. | 0,094327 | 0,299914 |
| AK024940 | cDNA: FLJ21287 fis, clone COL01918. | 0,094327 | -0,45615 |
| AK056609 | cDNA FLJ32047 fis, clone NTONG2001137. | 0,094327 | -0,26822 |
| NM_018246 | hypothetical protein FLJ10853 (FLJ10853), mRNA. | 0,094327 | -0,19685 |
| NM_001829 | chloride channel 3 (CLCN3), mRNA. | 0,094327 | -0,28227 |
| NM_015666 | GTP binding protein 5 (putative) (GTPBP5), mRNA. | 0,094327 | 0,261554 |
| NM_009590 | amine oxidase, copper containing 2 (retina-specific) (AOC2), transcript variant 2, mRNA. | 0,094327 | 0,458948 |
| NM_014052 | GW128 protein (GW128), mRNA. | 0,094327 | 0,254987 |
| NM_144641 | hypothetical protein FLJ32332 (FLJ32332), mRNA. | 0,095434 | 0,252008 |
| NM_024035 | hypothetical protein MGC3113 (MGC3113), mRNA. | 0,095434 | 0,287896 |
| NM_002398 | Meisl, myeloid ecotropic viral integration site 1 H.log (mouse) (MEIS1), mRNA. | 0,095862 | 0,5196 |
| NM_017417 | UDP-N-acetyl-alpha-D-galactosamine:polypeptide N-acetylgalactosaminyltransferase 8 (GalNAc-T8) (GALNT8), mRNA. | 0,095888 | -0,40833 |
| AL137326 | mRNA, cDNA DKFZp434B0650 (from clone | 0,096591 | 0,416007 |
| | DKFZp434B0650). | | |
| NM_002894 | retinoblastoma binding protein 8 (RBBP8), mRNA. | 0,097349 | -0,15438 |
| NM_003359 | UDP-glucose dehydrogenase (UGDH), mRNA. | 0,09769 | -0,32815 |
| NM_022461 | hypothetical protein FLJ21939 similar to 5-azacytidine induced gene 2 (FLJ21939), mRNA. | 0,09769 | 0,167686 |
| NM_152437 | hypothetical protein DKFZp761B128 (DKFZp761B128), mRNA. | 0,09769 | 0,400912 |
| AK056178 | cDNA FLJ31616 fis, clone NT2RI2003019. | 0,098067 | 0,313366 |
| BC020895 | clone IMAGE:4704474, mRNA. | 0,099308 | -0,31829 |
| NM_000862 | hydroxy-delta-5-steroid dehydrogenase, 3 beta- and steroid delta-isomerase 1 (HSD3B1), mRNA. | 0,099331 | 0,199437 |
| NM_004657 | serum deprivation response (phosphatidylserine binding protein) (SDPR), mRNA. | 0,099331 | 0,247845 |
| NM_020962 | likely ortholog of mouse neighbor of Punc E11 (NOPE), mRNA. | 0,099737 | 0,320891 |
| NM_000574 | decay accelerating factor for complement (CD55, Cromer blood group system) (DAF), mRNA. | 0,099737 | -0,39095 |

**Table 3: Human genes differentially expressed between colorectal adenoma and adenocarcinoma cells with chromosomal gain at 8q (Univariante analysis). Data are ranked on FDR value.**

| Gene id | Description (8 q gain Table 3) | FDR | Effect |
|---|---|---|---|
| NM_001951 | E2F transcription factor 5, p130-binding (E2F5), mRNA. | 0,000321 | 0,37466 |
| BM921036 | AGENCOURT_6633196 NIH_MGC_115 cDNA clone IMAGE:5752355 5', mRNA sequence. | 0,002075 | 0,387272 |
| NM_016308 | UMP-CMP kinase (UMP-CMPK), mRNA. | 0,002075 | -0,25909 |
| NM_000285 | peptidase D (PEPD), mRNA. | 0,006291 | 0,293134 |
| NM_145293 | similar to hypothetical protein FLJ20897 (LOC196549), mRNA. | 0,023161 | 0,187785 |
| NM_004232 | suppressor of cytokine signaling 4 (SOCS4), mRNA. | 0,041674 | -0,30051 |
| NM_022130 | golgi phosphoprotein 3 (coat-protein) (GOLPH3), mRNA. | 0,041674 | -0,30666 |
| AB033100 | mRNA for KIAA1274 protein, partial cds. | 0,041674 | 0,523579 |
| NM_003014 | secreted frizzled-related protein 4 (SFRP4), mRNA. | 0,041674 | 0,764632 |
| NM_004891 | mitochondrial ribosomal protein L33 (MRPL33), nuclear gene encoding mitochondrial protein, transcript variant 1, mRNA. | 0,041674 | 0,175935 |
| NM_032862 | tigger transposable element derived 5 (TIGD5), mRNA. | 0,041674 | 0,319249 |
| NM_033126 | serine/threonine kinase PSKH2 (PSKH2), mRNA. | 0,041674 | 0,432724 |
| NM_144660 | hypothetical protein FLJ25082 (FLJ25082), mRNA. | 0,050505 | 0,302561 |
| NM_015127 | Mid-1-related chloride channel 1 (MCLC), mRNA. | 0,050505 | -0,25616 |
| NM_031920 | ARG99 protein (ARG99), mRNA. | 0,050505 | 0,208365 |
| NM_007055 | polymerase (RNA) III (DNA directed) (155kD) (RPC155), mRNA. | 0,050505 | 0,14563 |
| NM_024491 | p10-binding protein (BITE), mRNA. | 0,050505 | -0,26034 |
| NM_006117 | peroxisomal D3,D2-enoyl-CoA isomerase (PECI), mRNA. | 0,050505 | -0,36647 |
| NM_033120 | naked cuticle H.log 2 (Drosophila) (NKD2), mRNA. | 0,050505 | 0,693186 |
| AF161353 | HSPC090 mRNA, partial cds. | 0,053587 | 0,234453 |
| NM_019016 | hypothetical protein FLJ20261 (FLJ20261), mRNA. | 0,054369 | -0,28294 |
| NM_024522 | hypothetical protein FLJ12650 (FLJ12650), mRNA. | 0,054369 | 0,220378 |
| NM_013354 | CCR4-NOT transcription complex, subunit 7 (CNOT7), transcript variant 1, mRNA. | 0,054537 | -0,19074 |
| NM_031472 | hypothetical protein MGC11134 (MGC11134), mRNA. | 0,060975 | -0,26471 |
| NM_138425 | hypothetical protein BC009925 (LOC113246), mRNA. | 0,060975 | -0,27155 |
| NM_003186 | transgelin (TAGLN), mRNA. | 0,078491 | 0,330037 |
| NM_016397 | TH1-like (Drosophila) (TH1L), mRNA. | 0,088863 | 0,233221 |
| NM_003839 | tumor necrosis factor receptor superfamily, member 11a, activator ofNFKB (TNFRSF11A), mRNA. | 0,099913 | -0,41856 |

**Table 4: Human genes differentially expressed between colorectal adenoma and adenocarcinoma cells with chromosomal gain at 13 q. Data are ranked on FDR value.**

| Gene ID | Description (13 q gain, Table 4) | FDR | Effect |
|---|---|---|---|
| NM_003850 | succinate-CoA ligase, ADP-forming, beta subunit (SUCLA2), mRNA. | 5,99E-07 | 0,268062 |
| NM_025138 | hypothetical protein FLJ12661 (FLJ12661), transcript variant 1, mRNA. | 0,002424 | 0,273094 |
| NM_003839 | tumor necrosis factor receptor superfamily, member 11a, activator ofNFKB (TNFRSF11A), mRNA. | 0,002424 | -0,46738 |
| NM_003576 | serine/threonine kinase 24 (STE20 H.log, yeast) (STK24), mRNA. | 0,002424 | 0,304764 |
| NM_032813 | hypothetical protein FLJ14624 (FLJ14624), mRNA. | 0,003078 | 0,266088 |
| NM_138425 | hypothetical protein BC009925 (LOC113246), mRNA. | 0,005352 | -0,28087 |
| NM_003899 | Rho guanine nucleotide exchange factor (GEF) 7 (ARHGEF7), transcript variant 1, mRNA. | 0,005352 | 0,20227 |
| BC006008 | clone IMAGE:4285740, mRNA. | 0,008614 | -0,24076 |
| NM_004001 | Fc fragment of IgG, low affinity IIb, receptor for (CD32) (FCGR2B), mRNA. | 0,008614 | 0,295299 |
| NM_006117 | peroxisomal D3,D2-enoyl-CoA isomerase (PECI), mRNA. | 0,0105 | -0,35631 |
| NM_021033 | RAP2A, member of RAS oncogene family (RAP2A), mRNA. | 0,010657 | 0,314425 |
| NM_002225 | isovaleryl Coenzyme A dehydrogenase (IVD), nuclear gene encoding mitochondrial protein, mRNA. | 0,012567 | -0,22827 |
| NM_007055 | polymerase (RNA) III (DNA directed) (155kD) (RPC155), mRNA. | 0,013994 | 0,136614 |
| NM_057158 | dual specificity phosphatase 4 (DUSP4), transcript variant 2, mRNA. | 0,015656 | -0,31358 |
| NM_032432 | actin binding LIM protein 2 (ABLIM2), mRNA. | 0,015656 | 0,232291 |
| NM_018206 | vacuolar protein sorting 35 (yeast) (VPS35), mRNA. | 0,015656 | 0,147472 |
| NM_014353 | RAB26, member RAS oncogene family (RAB26), mRNA. | 0,016631 | -0,43657 |
| NM_024090 | long-chain fatty-acyl elongase (LCE), mRNA. | 0,016631 | 0,199665 |
| NM_016646 | mesenchymal stem cell protein DSCD28 (LOC51336), mRNA. | 0,016631 | 0,215541 |
| NM_024110 | caspase recruitment domain family, member 14 (CARD 14), transcript variant 1, mRNA. | 0,016631 | 0,235476 |
| NM_002267 | karyopherin alpha 3 (importin alpha 4) (KPNA3), mRNA. | 0,016631 | 0,160517 |
| NM_032229 | hypothetical protein FLJ22774 (FLJ22774), mRNA. | 0,016631 | -0,76877 |
| NM_004445 | EphB6 (EPHB6), mRNA. | 0,017186 | 0,337104 |
| NM_002296 | lamin B receptor (LBR), mRNA. | 0,018744 | 0,219703 |
| AW972072 | EST384056 MAGE resequences, MAGL cDNA, mRNA sequence. | 0,020341 | 0,31753 |
| NM_012449 | six transmembrane epithelial antigen of the prostate (STEAP), mRNA. | 0,020473 | -0,32771 |
| NM_005870 | sin3-associated polypeptide, 18kDa (SAP18), mRNA. | 0,020691 | 0,219704 |
| NM_018386 | hypothetical protein FLJ11305 (FLJ11305), mRNA. | 0,022216 | 0,248688 |
| AF075082 | full length insert cDNA YQ80H06. | 0,022216 | -0,29092 |
| AL109727 | mRNA full length insert cDNA clone EUROIMAGE 200247. | 0,022702 | -0,18078 |
| AK055903 | cDNA FLJ31341 fis, clone MESAN1000050. | 0,023674 | -0,44115 |
| NM_014177 | HSPC154 protein (HSPC154), mRNA. | 0,02626 | -0,1951 |
| NM_006825 | cytoskeleton-associated protein 4 (CKAP4), mRNA. | 0,02765 | -0,1768 |
| L38282 | 30a mRNA fragment. | 0,028321 | 0,468719 |
| NM_025187 | hypothetical protein FLJ12076 (FLJ12076), mRNA. | 0,028321 | 0,19899 |
| NM_021945 | hypothetical protein FLJ22174 (FLJ22174), mRNA. | 0,039902 | -0,41007 |
| NM_139167 | sarcoglycan zeta (SGCZ), mRNA. | 0,039902 | 0,228483 |
| NM_006022 | transforming growth factor beta-stimulated protein TSC-22 (TSC22), mRNA. | 0,04116 | 0,203489 |
| NM_006215 | serine (or cysteine) proteinase inhibitor, clade A (alpha-1 antiproteinase, antitrypsin), member 4 (SERPINA4), mRNA. | 0,042174 | 0,444696 |
| NM_018559 | lipopolysaccharide specific response-7 protein (LSR7), mRNA. | 0,042174 | 0,270419 |
| AK024254 | cDNA FLJ14192 fis, clone NT2RP3000584. | 0,042174 | 0,224429 |
| NM_005358 | LIM domain only 7 (LMO7), transcript variant 1, | 0,042174 | 0,251855 |
| | mRNA. | | |
| NM_032827 | hypothetical protein FLJ14708 (FLJ14708), mRNA. | 0,047016 | -0,48154 |
| BC018758 | Similar to RIKEN cDNA 1110032022 gene, clone MGC:31967 IMAGE:4907976, mRNA, complete cds. | 0,051706 | -0,23603 |
| NM_030579 | cytochrome b5 outer mitochondrial membrane precursor (CYB5-M), mRNA. | 0,051706 | 0,176881 |
| AF085893 | full length insert cDNA clone YP95A10. | 0,051706 | 0,217617 |
| NM_152322 | hypothetical protein FLJ33957 (FLJ33957), mRNA. | 0,051731 | 0,373213 |
| NM_152707 | hypothetical protein MGC39851 (MGC39851), mRNA. | 0,053698 | 0,195109 |
| BC001092 | Similar to hypothetical protein FLJ20378, clone IMAGE:3506144, mRNA. | 0,056537 | -0,24494 |
| NM_002389 | membrane cofactor protein (CD46, trophoblast-lymphocyte cross-reactive antigen) (MCP), transcript variant a, mRNA. | 0,056648 | 0,241236 |
| AF086417 | full length insert cDNA clone ZD78A02. | 0,056648 | 0,538334 |
| NM_153751 | chromosome 21 open reading frame 82 (C21orf82), mRNA. | 0,057336 | -0,30873 |
| NM_003731 | Sjogren's syndrome nuclear autoantigen 1 (SSNA1), mRNA. | 0,058099 | 0,218273 |
| AL832390 | mRNA, cDNA DKFZp667H2012 (from clone DKFZp667H2012). | 0,063252 | -0,30417 |
| NM_012158 | F-box and leucine-rich repeat protein 3A (FBXL3A), mRNA. | 0,063252 | 0,264664 |
| NM_005374 | membrane protein, palmitoylated 2 (MAGUK p55 subfamily member 2) (MPP2), mRNA. | 0,063252 | -0,174 |
| NM_002097 | general transcription factor IIIA (GTF3A), mRNA. | 0,063252 | 0,268556 |
| W02242 | za57b04.r1 Soares fetal liver spleen 1NFLS cDNA clone IMAGE:296623 5', mRNA sequence. | 0,063252 | 0,244961 |
| NM_004255 | cytochrome c oxidase subunit Va (COX5A), nuclear gene encoding mitochondrial protein, mRNA. | 0,063252 | -0,1947 |
| AF350451 | C6orf37 mRNA, complete cds. | 0,064674 | -0,32683 |
| BC028119 | similar to putative, clone MGC:40181 | 0,065315 | -0,31424 |
| | IMAGE:5172473, mRNA, complete cds. | | |
| NM_016594 | FK506 binding protein 11, 19 kDa (FKBP11), mRNA. | 0,066266 | -0,23327 |
| NM_014849 | synaptic vesicle glycoprotein 2 (SV2), mRNA. | 0,066266 | 0,151549 |
| NM_032352 | hypothetical protein MGC11296 (MGC11296), mRNA. | 0,066266 | 0,132448 |
| NM_078467 | cyclin-dependentkinase inhibitor 1A (p21, Cip1) (CDKN1A), transcript variant 2, mRNA. | 0,066266 | 0,196894 |
| NM_007342 | nucleoporin-like protein 1 (NLP_1), mRNA. | 0,066671 | 0,137201 |
| AK057085 | cDNA FLJ32523 fis, clone SMINT2000032. | 0,06872 | 0,393846 |
| AF085955 | full length insert cDNA clone YR86G04. | 0,06872 | 0,220771 |
| NM_144736 | hypothetical protein PRO1853 (PRO1853), transcript variant 1, mRNA. | 0,06872 | 0,165416 |
| NM_007035 | keratocan (KERA), mRNA. | 0,06872 | 0,345311 |
| NM_152255 | proteasome (prosome, macropain) subunit, alpha type, 7 (PSMA7), transcript variant 2, mRNA. | 0,06872 | 0,141922 |
| U50531 | BRCA2 region, mRNA sequence CG030. | 0,06872 | 0,305838 |
| NM_004128 | general transcription factor IIF, polypeptide 2 (30kD subunit) (GTF2F2), mRNA. | 0,06872 | 0,200892 |
| BC010857 | clone MGC:9168 IMAGE:3876839, mRNA, complete cds. | 0,06872 | 0,156838 |
| BC011242 | clone IMAGE:4153763, mRNA. | 0,06872 | 0,385364 |
| BC035600 | Similar to RIKEN cDNA 2810405J04 gene, clone IMAGE:4430622, mRNA. | 0,06872 | -0,21368 |
| NM_080429 | aquaporin 10 (AQP10), mRNA. | 0,069317 | 0,175585 |
| NM_003903 | CDC16 cell division cycle 16 H.log (S. cerevisiae) (CDC16), mRNA. | 0,069317 | 0,138743 |
| NM_002435 | mannose phosphate isomerase (MPI), mRNA. | 0,069317 | -0,23954 |
| AB023233 | mRNA for KIAA1016 protein, partial cds. | 0,069317 | 0,146398 |
| NM_018270 | chromosome 20 open reading frame 20 (C20orf20), mRNA. | 0,069317 | 0,151877 |
| NM_014786 | Rho-specific guanine-nucleotide exchange factor 164 kDa (P164RHOGEF), mRNA. | 0,069317 | 0,154829 |
| NM_030789 | histocompatibility (minor) 13 (HM13), mRNA. | 0,069317 | 0,163094 |
| AK023616 | cDNA FLJ13554 fis, clone PLACE1007478. | 0,069317 | 0,315352 |
| NM_006602 | transcription factor-like 5 (basic helix-loop-helix) (TCFL5), mRNA. | 0,071441 | 0,406339 |
| AF191020 | E2IG5 (E2IG5) mRNA, complete cds. | 0,071441 | -0,25437 |
| NM_032846 | RAB2B (RAB2B), mRNA. | 0,071755 | -0,24439 |
| NM_144660 | hypothetical protein FLJ25082 (FLJ25082), mRNA. | 0,071755 | 0,236038 |
| NM_012180 | F-box only protein 8 (FBXO8), mRNA. | 0,071755 | 0,252412 |
| NM_002657 | pleiomorphic adenoma gene-like 2 (PLAGL2), mRNA. | 0,071755 | 0,283922 |
| NM_153336 | hypothetical protein MGC35392 (MGC35392), mRNA. | 0,071755 | -0,26883 |
| BC033204 | similar to excision repair protein (clone pcDE-72), clone MGC:45855 IMAGE:4876082, mRNA, complete cds. | 0,07337 | -0,21593 |
| AJ489980 | mRNA for solute carrier family 13 (sodium-dependent dicarboxylate transporter), member 2 (SLC13A2 gene). | 0,076544 | -0,45616 |
| AK056680 | cDNA FLJ32118 fis, clone PEBLM1000029. | 0,076544 | 0,189024 |
| U58663 | mRNA upregulated during camptothecin-induced apoptosis of U937 cells. | 0,076544 | 0,204923 |
| NM_005955 | metal-regulatory transcription factor 1 (MTF1), mRNA. | 0,076544 | 0,192013 |
| AK024423 | mRNA for FLJ00012 protein, partial cds. | 0,076544 | -0,22477 |
| NM_144726 | hypothetical protein FLJ31951 (FLJ31951), mRNA. | 0,078188 | -0,18645 |
| BC033940 | clone IMAGE:5272723, mRNA, partial cds. | 0,078188 | 0,242817 |
| NM_000107 | damage-specific DNA binding protein 2, 48kDa (DDB2), mRNA. | 0,078188 | -0,22344 |
| NM_144778 | muscleblind-like protein MBLL39 (MBLL39), transcript variant 1, mRNA. | 0,078188 | 0,218234 |
| NM_015070 | KIAA0853 protein (KIAA0853), mRNA. | 0,078188 | 0,196796 |
| NM_002212 | integrin beta 4 binding protein (ITGB4BP), mRNA. | 0,078188 | 0,185317 |
| NM_021258 | interleukin 22 receptor (IL22R), mRNA. | 0,079085 | 0,431196 |
| NM_014887 | hypothetical protein from BCRA2 region (CG005), mRNA. | 0,080338 | 0,233766 |
| NM_004755 | ribosomal protein S6 kinase, 90kDa, polypeptide 5 (RPS6KA5), mRNA. | 0,085964 | -0,18116 |
| NM_031266 | heterogeneous nuclear ribonucleoprotein A/B (HNRPAB), transcript variant 1, mRNA. | 0,085964 | -0,1542 |
| NM_018147 | Fas apoptotic inhibitory molecule (FAIM), mRNA. | 0,085964 | -0,34657 |
| NM_002431 | menage a trois 1 (CAK assembly factor) (MNAT1), mRNA. | 0,087716 | -0,1658 |
| NM_031472 | hypothetical protein MGC11134 (MGC11134), mRNA. | 0,087716 | -0,20932 |
| BC009210 | Similar to mesoderm development candiate 2, clone MGC:16185 IMAGE:3637449, mRNA, complete cds. | 0,087717 | -0,15961 |
| NM_002915 | replication factor C (activator 1) 3, 38kDa (RFC3), mRNA. | 0,089927 | 0,196477 |
| AF514992 | nuclear protein p30 mRNA, partial cds. | 0,090807 | -0,16537 |
| NM_013974 | dimethylarginine dimethylaminohydrolase 2 (DDAH2), mRNA. | 0,090807 | 0,17908 |
| BC008502 | clone MGC:14841 IMAGE:4295121, mRNA, complete cds. | 0,090807 | -0,28609 |
| BF967668 | 602287374T1 NIH_MGC_96 cDNA clone IMAGE:4374499 3', mRNA sequence. | 0,092551 | 0,241666 |
| AK092490 | cDNA FLJ35171 fis, clone PLACE6013220, weakly similar to TRICHOHYALIN. | 0,092847 | -0,20427 |
| AL390178 | mRNA, cDNA DKFZp547N064 (from clone DKFZp547N064). | 0,092928 | 0,259092 |
| NM_024618 | hypothetical protein FLJ21478 (FLJ21478), transcript variant 1, mRNA. | 0,093324 | -0,23144 |
| AK097101 | cDNA FLJ39782 fis, clone SPLEN2002175. | 0,093866 | 0,323348 |
| AK024933 | cDNA: FLJ21280 fis, clone COL01884. | 0,093866 | -0,36332 |
| NM_004545 | NADH dehydrogenase (ubiquinone) 1 beta subcomplex, 1, 7kDa (NDUFB1), mRNA. | 0,093866 | -0,17512 |
| NM_006811 | tumor differentially expressed 1(TDE1), mRNA. | 0,093866 | 0,185639 |
| NM_017817 | RAB20, member RAS oncogene family (RAB20), mRNA. | 0,093866 | 0,157212 |
| AK022295 | cDNA FLJ12233 fis, clone MAMMA1001215. | 0,093866 | 0,230759 |
| NM_001613 | actin, alpha 2, smooth muscle, aorta (ACTA2), mRNA. | 0,093866 | 0,296416 |
| NM_138792 | hypothetical protein BC018147 (LOC123169), mRNA. | 0,093866 | -0,17206 |
| BC014259 | clone MGC:20791 IMAGE:4763971, mRNA, complete cds. | 0,093866 | -0,23018 |
| NM_006769 | LIM domain only 4 (LMO4), mRNA. | 0,093866 | -0,26606 |
| AL137645 | mRNA, cDNA DKFZp586D0924 (from clone DKFZp586D0924). | 0,09471 | 0,282151 |
| BC007089 | Similar to hypothetical protein FLJ20489, clone IMAGE:4248707, mRNA. | 0,09471 | -0,27466 |
| NM_001500 | GDP-mannose 4,6-dehydratase (GMDS), mRNA. | 0,095468 | -0,23619 |
| NM_024537 | hypothetical protein FLJ12118 (FLJ12118), mRNA. | 0,095468 | 0,173686 |
| NM_006492 | aristaless-like homeobox 3 (ALX3), mRNA. | 0,097652 | 0,242826 |
| NM_032013 | NDRG family member 3 (NDRG3), mRNA. | 0,097652 | 0,159868 |
| NM_172366 | F-box protein 16 (FBX16), mRNA. | 0,09855 | -0,31506 |
| BC031266 | clone IMAGE:5277162, mRNA. | 0,09855 | -0,14471 |
| NM_024868 | hypothetical protein FLJ14124 (FLJ14124), mRNA. | 0,099014 | -0,31056 |
| AK098024 | cDNA FLJ40705 fis, clone THYMU2026530. | 0,099014 | -0,20783 |
| AK096958 | cDNA FLJ39639 fis, clone SMINT2003340. | 0,099014 | 0,226423 |
| NM_023011 | similar to yeast Upf3, variant A (UPF3A), transcript variant 1, mRNA. | 0,099014 | 0,151456 |
| BC040662 | clone IMAGE:4799788, mRNA. | 0,099014 | 0,217656 |
| NM_144618 | hypothetical protein MGC29891 (MGC29891), mRNA. | 0,099417 | 0,189802 |
| BQ719012 | AGENCOURT_8100927 Lupski_sympathetic_trunk cDNA clone IMAGE:6189773 5', mRNA sequence. | 0,099417 | 0,307114 |
| AK022424 | cDNA FLJ12362 fis, clone MAMMA1002360. | 0,099417 | -0,36672 |
| BF683837 | 602140129F1 NIH_MGC_46 cDNA clone IMAGE:4301287 5', mRNA sequence. | 0,099954 | -0,26208 |

**Table 5: Human genes differentially expressed between colorectal adenoma and adenocarcinoma cells with chromosomal loss at 15q (Univariant analysis). Data are ranked on FDR value.**

| Gene ID | Description (15 q loss , Table 5) | FDR | Effect |
|---|---|---|---|
| AK057085 | cDNA FLJ32523 fis, clone SMINT2000032. | 0,023084 | 0,663367 |
| BC010701 | clone MGC:16795 IMAGE:3855397, mRNA, complete cds. | 0,028543 | -0,40007 |
| NM_016397 | TH1-like (Drosophila) (TH1L), mRNA. | 0,03823 | 0,312299 |
| NM_006809 | translocase of outer mitochondrial membrane 34 (TOMM34), mRNA. | 0,03823 | 0,466704 |
| AF156166 | putative tumor suppressor mRNA. | 0,04221 | -0,34763 |
| NM_004653 | Smcy H.log, Y chromosome (mouse) (SMCY), mRNA. | 0,042857 | -0,44454 |
| NM_003880 | WNT1 inducible signaling pathway protein 3 (WISP3), transcript variant 1, mRNA. | 0,042857 | -0,31789 |
| NM_002755 | mitogen-activated protein kinase kinase 1 (MAP2K1), mRNA. | 0,042857 | -0,29003 |
| NM_002267 | karyopherin alpha 3 (importin alpha 4) (KPNA3), mRNA. | 0,042857 | 0,213699 |
| NM_006726 | LPS-responsive vesicle trafficking, beach and anchor containing (LRBA), mRNA. | 0,04717 | -0,17132 |
| NM_004323 | BCL2-associated athanogene (BAG1), mRNA. | 0,048517 | -0,3164 |
| NM_003411 | zinc finger protein, Y-linked (ZFY), mRNA. | 0,050424 | -0,82401 |
| BC035268 | clone IMAGE:4779288, mRNA. | 0,050424 | -0,25257 |
| AK075564 | cDNA PSEC0264 fis, clone NT2RP3002337. | 0,057755 | -0,77583 |
| AF085861 | full length insert cDNA clone YN67C05. | 0,057755 | -0,37833 |
| NM_144726 | hypothetical protein FLJ31951 (FLJ31951), mRNA. | 0,057755 | -0,27891 |
| NM_014786 | Rho-specific guanine-nucleotide exchange factor 164 kDa (P164RHOGEF), mRNA. | 0,066275 | 0,223783 |
| AL713719 | mRNA, cDNA DKFZp667K1916 (from clone DKFZp667K1916). | 0,066275 | 0,488787 |
| BC038852 | clone IMAGE:5168364, mRNA. | 0,066417 | -0,70334 |
| NM_003121 | Spi-B transcription factor (Spi-1/PU.1 related) (SPIB), mRNA. | 0,066417 | -0,33326 |
| Z24725 | mitogen inducible gene mig-2, complete CDS. | 0,066417 | -0,22419 |
| NM_014885 | anaphase-promoting complex 10 (APC10), mRNA. | 0,066417 | 0,240729 |
| NM_016646 | mesenchymal stem cell protein DSCD28 (LOC51336),mRNA. | 0,066417 | 0,267465 |
| NM_002296 | lamin B receptor (LBR), mRNA. | 0,066417 | 0,270704 |
| NM_017895 | DEAD/H (Asp-Glu-Ala-Asp/His) box polypeptide 27 (DDX27), mRNA. | 0,066417 | 0,283457 |
| NM_001997 | Finkel-Biskis-Reilly murine sarcoma virus (FBR-MuSV) ubiquitously expressed (fox derived) ribosomal protein S30 (FAU), mRNA. | 0,066417 | 0,314198 |
| BC008975 | clone MGC:16774 IMAGE:4215274, mRNA, complete cds. | 0,066417 | 0,37827 |
| NM_006644 | heat shock 105kD (HSP105B), mRNA. | 0,066417 | 0,411448 |
| NM_133170 | protein tyrosine phosphatase, receptor type, T (PTPRT), transcript variant 1, mRNA. | 0,067154 | -0,48547 |
| NM_001324 | cleavage stimulation factor, 3' pre-RNA, subunit 1, 50kDa (CSTF1), mRNA. | 0,067154 | 0,271924 |
| NM_018439 | hypothetical protein IMPACT (IMPACT), mRNA. | 0,075897 | -0,35011 |
| NM_030579 | cytochrome b5 outer mitochondrial membrane precursor (CYB5-M), mRNA. | 0,080353 | 0,23272 |
| NM_005469 | peroxisomal acyl-CoA thioesterase (PTE1), mRNA. | 0,080556 | 0,336772 |
| H43963 | yo70a10.s1 Soares breast 3NbHBst cDNA clone IMAGE:183258 3', mRNA sequence. | 0,081086 | -0,30907 |
| NM_000374 | uroporphyrinogen decarboxylase (UROD), mRNA. | 0,081086 | 0,244062 |
| NM_057158 | dual specificity phosphatase 4 (DUSP4), transcript variant 2, mRNA. | 0,089012 | -0,3644 |
| NM_032576 | chromosome Y open reading frame 15B (CYorf15B),mRNA. | 0,092312 | -0,51647 |
| NM_005531 | interferon, gamma-inducible protein 16 (IFI16), mRNA. | 0,092312 | -0,36367 |
| N23022 | yx65e12.s1 Soares melanocyte 2NbHM cDNA clone IMAGE:266638 3', mRNA sequence. | 0,092312 | -0,29388 |
| NM_018360 | chromosome X open reading frame 15 (CXorf15), mRNA. | 0,092312 | 0,341923 |
| BC011242 | clone IMAGE:4153763, mRNA. | 0,092312 | 0,5178 |
| AF086417 | full length insert cDNA clone ZD78A02. | 0,093351 | 0,698622 |
| NM_021945 | hypothetical protein FLJ22174 (FLJ22174), mRNA. | 0,096636 | -0,51134 |
| U33787 | immature B cell Ig heavy chain mRNA, third complementarity-determining region, clone MBT-20, partial cds. | 0,097621 | -1,07139 |
| NM_024522 | hypothetical protein FLJ12650 (FLJ12650), mRNA. | 0,097621 | 0,239056 |
| NM_012142 | cyclin D-type binding-protein 1 (CCNDBP1), transcript variant 1, mRNA. | 0,098621 | -0,30022 |

**Table 6: Human genes differentially expressed between colorectal adenoma and adenocarcinoma cells with chromosomal loss at 17p (Univariante analysis). Data are ranked on FDR value**

| Gene ID | description (17p loss, table 6) | FDR | Effect |
|---|---|---|---|
| NM_024096 | hypothetical protein MGC5627 (MGC5627), mRNA. | 0,056597 | 0,218944 |
| AK023018 | cDNA FLJ12956 fis, clone NT2RP2005501. | 0,056597 | 0,323956 |
| AF086011 | full length insert cDNA clone YW18A11. | 0,056597 | 0,413724 |
| NM_003072 | SWI/SNF related, matrix associated, actin dependent regulator of chromatin, subfamily a, member 4 (SMARCA4), mRNA. | 0,056597 | -0,20215 |
| BC009198 | Similar to RNA polymerase I transcription factor RRN3, clone MGC:15321 IMAGE:3678732, mRNA, complete cds. | 0,056597 | 0,278908 |
| AF070620 | clone 24694 mRNA sequence. | 0,056597 | 0,230604 |
| NM_013362 | zinc finger protein 225 (ZNF225), mRNA. | 0,056597 | -0,48787 |
| NM_080752 | chromosome 20 open reading frame 164 (C20orf164), mRNA. | 0,081745 | 0,329131 |

**Table 7: Human genes differentially expressed between colorectal adenoma and adenocarcinoma cells with chromosomal loss at 18q (Univariante analysis). Data are ranked on FDR value**

| Gene ID | Description (18q loss, Table 7) | FDR | Effect |
|---|---|---|---|
| NM_016397 | TH1-like (Drosophila) (TH1L), mRNA. | 0,000824 | 0,282908 |
| NM_004715 | CTD (carboxy-terminal domain, RNA polymerase II, polypeptide A) phosphatase, subunit 1 (CTDP1), transcript variant FCP1a, mRNA. | 0,000824 | -0,19524 |
| NM_004539 | asparaginyl-tRNA synthetase (NARS), mRNA. | 0,000944 | -0,24154 |
| NM_002894 | retinoblastoma binding protein 8 (RBBP8), mRNA. | 0,001559 | -0,19184 |
| NM_021809 | TGFB-induced factor 2 (TALE family homeobox) (TGIF2), mRNA. | 0,001559 | 0,364009 |
| NM_002915 | replication factor C (activator 1) 3, 38kDa (RFC3), mRNA. | 0,001792 | 0,277825 |
| NM_016646 | mesenchymal stem cell protein DSCD28 (LOC51336), mRNA. | 0,003526 | 0,246432 |
| NM_013451 | fer-1-like 3, myoferlin (C. elegans) (FER1L3), transcript variant 1, mRNA. | 0,003816 | -0,26834 |
| NM_005469 | peroxisomal acyl-CoA thioesterase (PTE1), mRNA. | 0,003816 | 0,317782 |
| NM_024522 | hypothetical protein FLJ12650 (FLJ12650), mRNA. | 0,003816 | 0,234068 |
| NM_002212 | integrin beta 4 binding protein (ITGB4BP), mRNA. | 0,003816 | 0,24754 |
| BC033940 | clone IMAGE:5272723, mRNA, partial cds. | 0,004542 | 0,320051 |
| NM_014177 | HSPC154 protein (HSPC154), mRNA. | 0,004542 | -0,22578 |
| BC038852 | clone IMAGE:5168364, mRNA. | 0,006043 | -0,63205 |
| BQ189477 | UI-E-EJ1-aka-c-13-0-UI.r1 UI-E-EJ1 cDNA clone UI-E-EJ1-aka-c-13-0-UI 5', mRNA sequence. | 0,006686 | -0,57215 |
| NM_018439 | hypothetical protein IMPACT (IMPACT), mRNA. | 0,007012 | -0,31578 |
| NM_018270 | chromosome 20 open reading frame 20 (C20orf20), mRNA. | 0,007749 | 0,189734 |
| NM_138425 | hypothetical protein BC009925 (LOC113246), mRNA. | 0,009635 | -0,27373 |
| NM_002296 | lamin B receptor (LBR), mRNA. | 0,009635 | 0,236768 |
| NM_006602 | transcription factor-like 5 (basic helix-loop-helix) (TCFL5), mRNA. | 0,009886 | 0,501186 |
| NM_152777 | chromosome 14 open reading frame 48 (C14orf48), mRNA. | 0,010708 | 0,318223 |
| Z34281 | (MAR10) MUC5AC mRNA for mucin (partial). | 0,011328 | -1,33357 |
| BQ921112 | AGENCOURT_8926187 NIH_MGC_101 cDNA clone IMAGE:6463013 5', mRNA sequence. | 0,012033 | 0,451489 |
| AK024563 | cDNA: FLJ20910 fis, clone ADSE00492. | 0,012033 | 0,349917 |
| NM_032611 | protein tyrosine phosphatase type IVA, member 3 (PTP4A3), transcript variant 1, mRNA. | 0,01539 | 0,370319 |
| AK022220 | cDNA FLJ12158 fis, clone MAMMA1000522. | 0,01539 | -0,31078 |
| NM_017750 | hypothetical protein FLJ20296 (FLJ20296), mRNA. | 0,01679 | -0,24488 |
| NM_002655 | pleiomorphic adenoma gene 1 (PLAG1), mRNA. | 0,01679 | 0,575441 |
| BC002877 | Similar to hypothetical protein FLJ11585, clone MGC:11258 IMAGE:3942160, mRNA, complete cds. | 0,01679 | -0,20124 |
| NM_004107 | Fc fragment of IgG, receptor, transporter, alpha (FCGRT), mRNA. | 0,01799 | 0,326969 |
| AB033070 | mRNA for KIAA1244 protein, partial cds. | 0,020624 | -0,33961 |
| NM_018710 | hypothetical protein DKFZp762O076 (DKFZp762O076), mRNA. | 0,020624 | 0,327157 |
| BC008502 | clone MGC:14841 IMAGE:4295121, mRNA, complete cds. | 0,021539 | -0,34512 |
| NM_002640 | serine (or cysteine) proteinase inhibitor, clade B (ovalbumin), member 8 (SERPINB8), mRNA. | 0,022755 | -0,33415 |
| NM_152255 | proteasome (prosome, macropain) subunit, alpha type, 7 (PSMA7), transcript variant 2, mRNA. | 0,023501 | 0,161889 |
| NM_003839 | tumor necrosis factor receptor superfamily, member 11a, activator ofNFKB (TNFRSF11A), mRNA. | 0,023501 | -0,4074 |
| NM_019079 | hypothetical protein FLJ10884 (FLJ10884), mRNA. | 0,025102 | -0,63052 |
| NM_018840 | putative Rab5-interacting protein (RIP5), mRNA. | 0,025102 | 0,248901 |
| NM_020650 | hypothetical protein LOC57333 (LOC57333), mRNA. | 0,025102 | 0,418072 |
| AK055091 | cDNA FLJ30529 fis, clone BRAWH2001052. | 0,025102 | -0,32068 |
| NM_001546 | inhibitor of DNA binding 4, dominant negative helix-loop-helix protein (ID4), mRNA. | 0,025102 | -0,70017 |
| NM_006117 | peroxisomal D3,D2-enoyl-CoA isomerase (PECI), mRNA. | 0,025102 | -0,33373 |
| NM_001324 | cleavage stimulation factor, 3' pre-RNA, subunit 1, 50kDa (CSTF1), mRNA. | 0,025438 | 0,219884 |
| NM_001432 | epiregulin (EREG), mRNA. | 0,026555 | 0,711058 |
| AW815041 | CMO-ST0209-271099-082-c12 ST0209 cDNA, mRNA sequence. | 0,026555 | -0,29885 |
| NM_018043 | hypothetical protein FLJ10261 (FLJ10261), mRNA. | 0,027258 | -0,47338 |
| NM_018569 | hypothetical protein PRO0971 (PRO0971), mRNA. | 0,027258 | 0,24305 |
| NM_020232 | hepatocellular carcinoma susceptibility protein (HCCA3), mRNA. | 0,027924 | -0,1674 |
| NM_006097 | myosin regulatory light chain 2, smooth muscle isoform (MYRL2), mRNA. | 0,027924 | 0,308449 |
| NM_003118 | secreted protein, acidic, cysteine-rich (osteonectin) (SPARC), mRNA. | 0,027924 | 0,371898 |
| NM_138340 | abhydrolase domain containing 3 (ABHD3), mRNA. | 0,028702 | -0,34834 |
| NM_173168 | SPCX (SPCX), mRNA. | 0,028702 | 0,290214 |
| NM_000238 | potassium voltage-gated channel, subfamily H (eag-related), member 2 (KCNH2), transcript variant 1, mRNA. | 0,028702 | 0,362318 |
| NM_016569 | T-box 3 (ulnar mammary syndrome) (TBX3), transcript variant 2, mRNA. | 0,028702 | -0,40908 |
| NM_006809 | translocase of outer mitochondrial membrane 34 (TOMM34), mRNA. | 0,028702 | 0,327919 |
| NM_138573 | neuregulin 4 (LOC145957), mRNA. | 0,028702 | -0,33061 |
| BC016864 | clone IMAGE:3847536, mRNA. | 0,028702 | 0,335912 |
| K00629 | kpni repeat mma (cdna clone pcd-kpni-4), 3' end. | 0,028949 | 0,200056 |
| NM_005877 | splicing factor 3a, subunit 1, 120kDa (SF3A1), mRNA. | 0,028949 | -0,21253 |
| NM_006615 | calpain 9 (nCL-4) (CAPN9), mRNA. | 0,028949 | -0,56754 |
| NM_024426 | Wilms tumor 1 (WT1), transcript variant D, mRNA. | 0,02963 | 0,688737 |
| NM_025138 | hypothetical protein FLJ12661 (FLJ12661), transcript variant 1, mRNA. | 0,02963 | 0,226221 |
| AK074657 | cDNA FLJ90176 fis, clone MAMMA1000528. | 0,02963 | -0,3027 |
| BM921036 | AGENCOURT_6633196 NIH_MGC_115 cDNA clone IMAGE:5752355 5', mRNA sequence. | 0,02963 | 0,279055 |
| NM_003121 | Spi-B transcription factor (Spi-1/PU.1 related) (SPIB), mRNA. | 0,02963 | -0,25696 |
| BC031244 | dual-specificity tyrosine-(Y)-phosphorylation regulated kinase 4, clone MGC:39640 IMAGE:5266287, mRNA, complete cds. | 0,02963 | -0,31347 |
| AF088051 | full length insert cDNA clone ZD63G05. | 0,02963 | -0,22944 |
| NM_030815 | chromosome 20 open reading frame 126 (C20orf126), mRNA. | 0,02963 | 0,252626 |
| NM_014729 | thymus high mobility group box protein TOX (TOX), mRNA. | 0,029713 | -0,38597 |
| NM_021945 | hypothetical protein FLJ22174 (FLJ22174), mRNA. | 0,031107 | -0,41574 |
| NM_000439 | proprotein convertase subtilisin/kexin type 1 (PCSK1), mRNA. | 0,033688 | -1,05921 |
| NM_006644 | heat shock 105kD (HSP105B), mRNA. | 0,033688 | 0,312619 |
| BC034812 | Similar to HSPC182 protein, clone IMAGE:4825606, mRNA. | 0,033688 | 0,229524 |
| U44954 | NMDA receptor glutamate-binding chain (hnrgw) mRNA, partial cds. | 0,033688 | 0,235122 |
| NM_002885 | RAP1, GTPase activating protein 1 (RAP1GA1), mRNA. | 0,033688 | -0,17827 |
| NM_002573 | platelet-activating factor acetylhydrolase, isoform Ib, gamma subunit 29kDa (PAFAH1B3), mRNA. | 0,033688 | 0,176978 |
| NM_002097 | general transcription factor IIIA(GTF3A), mRNA. | 0,033688 | 0,283916 |
| NM_025067 | hypothetical protein FLJ14106 (FLJ14106), mRNA. | 0,034774 | 0,136761 |
| NM_130785 | TPTE and PTEN H.logous inositol lipid phosphatase (TPIP), mRNA. | 0,034774 | -0,20841 |
| NM_012190 | formyltetrahydrofolate dehydrogenase (FTHFD), transcript variant 1, mRNA. | 0,034774 | -0,4416 |
| NM_080476 | CDC91 cell division cycle 91-like 1 (S. cerevisiae) (CDC91L1), mRNA. | 0,036126 | 0,23289 |
| NM_016626 | hypothetical protein LOC51320 (LOC51320), mRNA. | 0,036126 | -0,22033 |
| AK096306 | cDNA FLJ38987 fis, clone NT2RI2005818. | 0,036126 | 0,295329 |
| AF339772 | clone IMAGE:128781, mRNA sequence. | 0,036825 | 0,386798 |
| NM_025047 | hypothetical protein FLJ22595 (FLJ22595), mRNA. | 0,036825 | -0,22813 |
| NM_021246 | lymphocyte antigen 6 complex, locus G6D (LY6G6D), mRNA. | 0,036825 | 0,781552 |
| NM_006839 | inner membrane protein, mitochondrial (mitofilin) (IMMT), mRNA. | 0,036825 | -0,32053 |
| NM_004786 | thioredoxin-like, 32kDa (TXNL), mRNA. | 0,036825 | -0,19712 |
| NM_030789 | histocompatibility (minor) 13 (HM13), mRNA. | 0,037928 | 0,173847 |
| AF304443 | B lymphocyte activation-related protein BC-2048 mRNA, complete cds. | 0,038975 | -0,69041 |
| NM_016400 | Huntingtin interacting protein K (HYPK), mRNA. | 0,038975 | -0,23071 |
| NM_153751 | chromosome 21 open reading frame 82 (C21orf82), mRNA. | 0,038975 | -0,31597 |
| AF086021 | full length insert cDNA clone YW21D11. | 0,038975 | -0,40572 |
| NM_002156 | heat shock 60kDa protein 1 (chaperonin) (HSPD1), mRNA. | 0,03977 | 0,174064 |
| AJ292079 | partial mRNA for MUC5AC protein (mucin gene, MUC5AC). | 0,043301 | -1,2742 |
| NM_053055 | C-terminal modulator protein (CTMP), mRNA. | 0,043301 | -0,18836 |
| NM_004669 | chloride intracellular channel 3 (CLIC3), mRNA. | 0,043458 | -0,63156 |
| NM_014066 | HT002 protein, hypertension-related calcium-regulated gene (HT002), mRNA. | 0,043458 | 0,183686 |
| NM_002267 | karyopherin alpha 3 (importin alpha 4) (KPNA3), mRNA. | 0,043458 | 0,14631 |
| NM_024928 | hypothetical protein FLJ22559 (FLJ22559), mRNA. | 0,044215 | -0,17755 |
| NM_000107 | damage-specific DNA binding protein 2, 48kDa (DDB2), mRNA. | 0,044215 | -0,23951 |
| NM_005359 | MAD, mothers against decapentaplegic H.log 4 (Drosophila) (MADH4), mRNA. | 0,044664 | -0,33258 |
| NM_005054 | RAN binding protein 2-like 1 (RANBP2L1), transcript variant 1, mRNA. | 0,04739 | -0,283 |
| AK057624 | cDNA FLJ33062 fis, clone TRACH2000032. | 0,047422 | 0,250799 |
| NM_144726 | hypothetical protein FLJ31951 (FLJ31951), mRNA. | 0,047422 | -0,19842 |
| NM_024898 | hypothetical protein FLJ22757 (FLJ22757), mRNA. | 0,047422 | -0,17724 |
| NM_018962 | Down syndrome critical region gene 6 (DSCR6), mRNA. | 0,047422 | 0,320234 |
| NM_018662 | disrupted in schizophrenia 1 (DISC1), mRNA. | 0,047422 | -0,44603 |
| NM_006886 | ATP synthase, H+ transporting, mitochondrial F1 complex, epsilon subunit (ATP5E), nuclear gene encoding mitochondrial protein, mRNA. | 0,047422 | 0,207215 |
| NM_001072 | UDP glycosyltransferase 1 family, polypeptide A6 (UGT1A6), mRNA. | 0,047422 | -0,37685 |
| NM_000285 | peptidase D (PEPD), mRNA. | 0,047422 | 0,210238 |
| NM_018154 | hypothetical protein FLJ10604 (FLJ10604), mRNA. | 0,047422 | 0,229376 |
| NM_138706 | beta-1,3-N-acetylglucosaminyltransferase protein (IMAGE:4907098), mRNA. | 0,047422 | -0,51921 |
| NM_000015 | N-acetyltransferase 2 (arylamine N-acetyltransferase) (NAT2), mRNA. | 0,047971 | -0,23243 |
| NM_020673 | RAB22A, member RAS oncogene family (RAB22A), mRNA. | 0,047971 | 0,208594 |
| NM_033342 | tripartite motif-containing 7 (TRIM7), mRNA. | 0,047971 | -0,35293 |
| NM_002899 | retinol binding protein 1, cellular (RBP1), mRNA. | 0,047971 | 0,417924 |
| NM_002755 | mitogen-activated protein kinase kinase 1 (MAP2K1), mRNA. | 0,049509 | -0,19374 |
| NM_003617 | regulator of G-protein signalling 5 (RGS5), mRNA. | 0,049509 | 0,316416 |
| AY069977 | anterior gradient protein 3 mRNA, complete cds. | 0,049992 | -0,60091 |
| NM_002905 | retinol dehydrogenase 5 (11-cisand 9-cis) (RDH5), mRNA. | 0,050001 | -0,20145 |
| AK057085 | cDNA FLJ32523 fis, clone SMINT2000032. | 0,055786 | 0,399947 |
| Y11166 | U71b small nucleolar RNA gene. | 0,055786 | 0,484431 |
| NM_004588 | sodium channel, voltage-gated, type II, beta polypeptide (SCN2B), mRNA. | 0,057233 | -0,19156 |
| NM_020749 | AT2 receptor-interacting protein 1 (ATIP1), mRNA. | 0,057233 | -0,2583 |
| AK095147 | cDNA FLJ37828 fis, clone BRSSN2006575. | 0,057233 | -0,79158 |
| NM_004925 | aquaporin 3 (AQP3), mRNA. | 0,057233 | -0,42326 |
| BQ055725 | AGENCOURT_6796360 NIH_MGC_99 cDNA clone IMAGE:5807946 5', mRNA sequence. | 0,057233 | -0,32696 |
| NM_002345 | lumican (LUM), mRNA. | 0,057233 | 0,385281 |
| NM_002102 | glycophorin E (GYPE), mRNA. | 0,057233 | -0,22666 |
| NM_133170 | protein tyrosine phosphatase, receptor type, T (PTPRT), transcript variant 1, mRNA. | 0,057233 | -0,35159 |
| BM669556 | UI-E-DX1-agw-c-13-0-UI.s1 UL-E-DX1 cDNA clone UI-E-DX1-agw-c-13-0-UI 3', mRNA sequence. | 0,057894 | 0,211554 |
| NM_002225 | isovaleryl Coenzyme A dehydrogenase (IVD), nuclear gene encoding mitochondrial protein, mRNA. | 0,058226 | -0,1931 |
| NM_152400 | hypothetical protein FLJ39370 (FLJ39370), mRNA. | 0,058226 | -0,22856 |
| NM_006796 | AFG3 ATPase family gene 3-like 2 (yeast) (AFG3L2), nuclear gene encoding mitochondrial protein, mRNA. | 0,058226 | -0,20738 |
| NM_033111 | CG016 (LOC88523), mRNA. | 0,058945 | 0,425126 |
| NM_004599 | sterol regulatory element binding transcription factor 2 (SREBF2), mRNA. | 0,059015 | -0,24902 |
| NM_020169 | latexin protein (LXN), mRNA. | 0,059328 | -0,1734 |
| NM_018414 | GalNAc alpha-2, 6-sialyltransferase I, long form (ST6GalNAcI), mRNA. | 0,059328 | -0,49984 |
| AB033100 | mRNA for KIAA1274 protein, partial cds. | 0,059616 | 0,406927 |
| AK056609 | cDNA FLJ32047 fis, clone NTONG2001137. | 0,060288 | -0,24176 |
| NM_014052 | GW128 protein (GW128), mRNA. | 0,060898 | 0,229394 |
| AK057045 | cDNA FLJ32483 fis, clone SKNMC2001503, weakly similar to P-SELECTIN GLYCOPROTEIN LIGAND 1 PRECURSOR. | 0,061898 | -0,38993 |
| NM_017895 | DEAD/H (Asp-Glu-Ala-Asp/His) box polypeptide 27 (DDX27), mRNA. | 0,061986 | 0,201229 |
| NM_030579 | cytochrome b5 outer mitochondrial membrane precursor (CYB5-M), mRNA. | 0,061986 | 0,168765 |
| NM_007019 | ubiquitin-conjugating enzyme E2C (UBE2C), mRNA. | 0,061998 | 0,236312 |
| NM_007039 | protein tyrosine phosphatase, non-receptor type 21 (PTPN21), mRNA. | 0,061998 | -0,24194 |
| NM_018360 | chromosome X open reading frame 15 (CXorf15), mRNA. | 0,061998 | 0,25321 |
| NM_003185 | TAF4 RNA polymerase II, TATA box binding protein (TBP)-associated factor, 135kDa (TAF4), mRNA. | 0,062237 | 0,224102 |
| AK075564 | cDNA PSEC0264 fis, clone NT2RP3002337. | 0,062237 | -0,53126 |
| NM_000177 | gelsolin (amyloidosis, Finnish type) (GSN), mRNA. | 0,062237 | -0,13412 |
| NM_016299 | likely ortholog of mouse heat shock protein, 70 kDa 4 (HSP70-4), mRNA. | 0,062237 | 0,168397 |
| BC014362 | clone IMAGE:3932221, mRNA. | 0,063385 | 0,253343 |
| BC009447 | clone MGC:15887 IMAGE:3530481, mRNA, complete cds. | 0,06351 | 0,288058 |
| BC041626 | clone MGC:52394 IMAGE:4554923, mRNA, complete cds. | 0,064346 | -0,33008 |
| NM_032709 | hypothetical protein MGC13047 (MGC13047), mRNA. | 0,065196 | -0,35449 |
| AL713719 | mRNA, cDNA DKFZp667K1916 (from clone DKFZp667K1916). | 0,0653 | 0,33683 |
| NM_030877 | catenin, beta like 1 (CTNNBL1), mRNA. | 0,066566 | 0,248269 |
| NM_024725 | hypothetical protein FLJ23518 (FLJ23518), mRNA. | 0,066566 | -0,22828 |
| NM_014657 | KIAA0406 gene product (KIAA0406), mRNA. | 0,067336 | 0,273531 |
| NM_032827 | hypothetical protein FLJ14708 (FLJ14708), mRNA. | 0,067336 | -0,44802 |
| NM_145284 | similar to hypothetical protein MGC17347 (LOC159090), mRNA. | 0,067336 | 0,250469 |
| NM_015832 | methyl-CpG binding domain protein 2 (MBD2), transcript variant testis-specific, mRNA. | 0,067336 | -0,17895 |
| NM_000901 | nuclear receptor subfamily 3, group C, member 2 (NR3C2), mRNA. | 0,067336 | -0,56338 |
| NM_032633 | hypothetical protein MGC5457 (MGC5457), mRNA. | 0,067336 | 0,171888 |
| NM_032527 | hypothetical protein FLJ14972 (KIAA1847), mRNA. | 0,067336 | 0,153376 |
| NM_001316 | CSE1 chromosome segregation 1-like (yeast) (CSE1L), mRNA. | 0,067336 | 0,212579 |
| AK095808 | cDNA FLJ38489 fis, clone FEBRA2023550, weakly similar to P3 PROTEIN. | 0,067336 | -0,25346 |
| NM_001933 | dihydrolipoamide S-succinyltransferase (E2 component of 2-oxo-glutarate complex) (DLST), | 0,067336 | -0,19324 |
| | mRNA. | | |
| BC030750 | clone IMAGE:4795773, mRNA. | 0,067391 | -0,31297 |
| NM_004453 | electron-transferring-flavoprotein dehydrogenase (ETFDH), nuclear gene encoding mitochondrial protein, mRNA. | 0,069033 | -0,20405 |
| NM_018326 | hypothetical protein FLJ11110 (FLJ11110), mRNA. | 0,069195 | 0,141442 |
| NM_005273 | guanine nucleotide binding protein (G protein), beta polypeptide 2 (GNB2), mRNA. | 0,070066 | 0,334504 |
| NM_003031 | seven in absentia H.log 1 (Drosophila) (SIAH1), mRNA. | 0,070066 | 0,182138 |
| AW975332 | EST387440 MAGE resequences, MAGN cDNA, mRNA sequence. | 0,070066 | -0,45859 |
| NM_002870 | RAB13, member RAS oncogene family (RAB13), mRNA. | 0,070066 | -0,16753 |
| NM_021637 | hypothetical protein FLJ14084 (FLJ14084), mRNA. | 0,071297 | -0,36458 |
| NM_017896 | chromosome 20 open reading frame 11 (C20orf11), mRNA. | 0,072695 | 0,182801 |
| NM_003265 | toll-like receptor 3 (TLR3), mRNA. | 0,072695 | -0,34201 |
| NM_007342 | nucleoporin-like protein 1 (NLP_1), mRNA. | 0,072792 | 0,132142 |
| NM_001800 | cyclin-dependent kinase inhibitor 2D (p19, inhibits CDK4) (CDKN2D), transcript variant 1, mRNA. | 0,072792 | 0,209575 |
| NM_024075 | LENG5 protein (LENG5), mRNA. | 0,075868 | 0,123473 |
| AI652393 | wb20f02.x1 NCI_CGAP_GC6 cDNA clone IMAGE:2306235 3', mRNA sequence. | 0,075868 | 0,223284 |
| AK056292 | cDNA FLJ31730 fis, clone NT2RI2006838. | 0,075868 | 0,40984 |
| NM_003731 | Sjogren's syndrome nuclear autoantigen 1 (SSNA1), mRNA. | 0,075868 | 0,204909 |
| NM_016305 | synovial sarcoma translocation gene on chromosome 18-like 2 (SS18L2), mRNA. | 0,076482 | 0,16357 |
| NM_024761 | hypothetical protein FLJ13204 (FLJ13204), mRNA. | 0,077076 | -0,18086 |
| NM_001951 | E2F transcription factor 5, p130-binding (E2F5), mRNA. | 0,077076 | 0,22251 |
| AB002339 | mRNA for KIAA0341 gene, partial cds. | 0,077076 | -0,21649 |
| NM_005637 | synovial sarcoma translocation, chromosome 18 | 0,077078 | -0,13342 |
| | (SS18),mRNA. | | |
| AK074192 | cDNA FLJ23612 fis, clone ADKA02523. | 0,077111 | 0,255663 |
| NM_000442 | platelet/endothelial cell adhesion molecule (CD31 antigen) (PECAM1), mRNA. | 0,077637 | -0,42079 |
| AK000165 | cDNA FLJ20158 fis, clone COL08935. | 0,077657 | -0,21885 |
| BC007399 | clone MGC:16308 IMAGE:3836116, mRNA, complete cds. | 0,078925 | 0,583373 |
| NM_001280 | cold inducible RNA binding protein (CIRBP), mRNA. | 0,079348 | -0,17706 |
| NM_016938 | EGF-containing fibulin-like extracellular matrix protein 2 (EFEMP2), mRNA. | 0,079348 | 0,256868 |
| AF086130 | full length insert cDNA clone ZA84A12. | 0,079582 | 0,117902 |
| BQ646410 | AGENCOURT_8511770 NIH_MGC_100 cDNA clone IMAGE:6296949 5', mRNA sequence. | 0,080045 | 0,706676 |
| NM_003359 | UDP-glucose dehydrogenase (UGDH), mRNA. | 0,080045 | -0,28442 |
| BC018995 | clone MGC:20579 IMAGE:4300679, mRNA, complete cds. | 0,080045 | 0,489487 |
| BC033250 | clone IMAGE:5441027, mRNA, partial cds. | 0,080045 | 0,215003 |
| NM_001830 | chloride channel 4 (CLCN4), mRNA. | 0,080045 | 0,159316 |
| NM_003014 | secreted frizzled-related protein 4 (SFRP4), mRNA. | 0,080045 | 0,569245 |
| AL833742 | mRNA, cDNA DKFZp666E186 (from clone DKFZp666E186). | 0,080045 | -0,39501 |
| NM_015512 | DKFZP434A236 protein (DKFZP434A236), mRNA. | 0,080045 | 0,200372 |
| AL834121 | mRNA, cDNA DKFZp761D2121 (from clone DKFZp761D2121). | 0,080045 | -0,30573 |
| NM_006833 | COP9 subunit 6 (MOV34 H.log, 34 kD) (COPS6), mRNA. | 0,080045 | 0,151558 |
| NM_013390 | transmembrane protein 2 (TMEM2), mRNA. | 0,080045 | -0,24358 |
| AK093557 | cDNA FLJ36238 fis, clone THYMU2001422. | 0,080045 | 0,236266 |
| NM_032044 | regenerating gene type IV (REG-IV), mRNA. | 0,080045 | -0,79212 |
| NM_017495 | RNA-binding region (RNP1, RRM) containing 1 (RNPC1), mRNA. | 0,080045 | 0,265594 |
| NM_152621 | hypothetical protein MGC26963 (MGC26963), mRNA. | 0,080739 | -0,39756 |
| AK092074 | cDNA FLJ34755 fis, clone NHNPC1000034. | 0,080739 | -0,31291 |
| NM_003615 | solute carrier family 4, sodium bicarbonate cotransporter, member 7 (SLC4A7), mRNA. | 0,080739 | 0,18244 |
| NM_152586 | hypothetical protein FLJ37318 (FLJ37318), mRNA. | 0,080739 | -0,22746 |
| NM_002466 | v-myb myeloblastosis viral oncogene H.log (avian)-like 2 (MYBL2), mRNA. | 0,080739 | 0,425478 |
| BC028376 | clone IMAGE:4838541, mRNA. | 0,080739 | -0,22249 |
| BC011242 | clone IMAGE:4153763, mRNA. | 0,080739 | 0,366334 |
| AL831862 | mRNA, cDNA DKFZp761I2317 (from clone DKFZp761I2317). | 0,081827 | -0,16968 |
| BC036876 | clone IMAGE:5245135, mRNA. | 0,082197 | 0,275752 |
| NM_033339 | caspase 7, apoptosis-related cysteine protease (CASP7), transcript variant gamma, mRNA. | 0,08233 | -0,22092 |
| NM_000373 | uridine monophosphate synthetase (orotate phosphoribosyl transferase and orotidine-5'-decarboxylase) (UMPS), mRNA. | 0,082581 | 0,129645 |
| NM_006922 | sodium channel, voltage-gated, type III, alpha polypeptide (SCN3A), mRNA. | 0,082581 | -0,207 |
| NM_014065 | HT001 protein (HT001), mRNA. | 0,082581 | 0,175696 |
| AB058758 | mRNA for KIAA1855 protein, partial cds. | 0,082581 | 0,275326 |
| NM_152429 | hypothetical protein MGC39320 (MGC39320), mRNA. | 0,082581 | -0,25357 |
| BC030520 | similar to hypothetical protein F33H2.2 - Caenorhabditis elegans, clone MGC:40403 IMAGE:5180533, mRNA, complete cds. | 0,082581 | 0,189826 |
| NM_024115 | hypothetical protein MGC4309 (MGC4309), mRNA. | 0,082581 | 0,160535 |
| NM_031920 | ARG99 protein (ARG99), mRNA. | 0,082581 | 0,158741 |
| AK021812 | cDNA FLJ11750 fis, clone HEMBA1005568. | 0,082581 | -0,26281 |
| BC036004 | clone IMAGE:4730399, mRNA. | 0,082581 | -0,28908 |
| NM_024631 | hypothetical protein FLJ23342 (FLJ23342), mRNA. | 0,082581 | 0,153323 |
| NM_020299 | aldo-keto reductase family 1, member B10 (aldose reductase) (AKR1B10), mRNA. | 0,083612 | -0,36368 |
| AL832390 | mRNA, cDNA DKFZp667H2012 (from clone DKFZp667H2012). | 0,083612 | -0,28133 |
| NM_002609 | platelet-derived growth factor receptor, beta polypeptide (PDGFRB), mRNA. | 0,084113 | 0,338394 |
| AK022263 | cDNA FLJ12201 fis, clone MAMMA1000906. | 0,084113 | -0,27479 |
| NM_032579 | colon and small intestine-specific cysteine-rich protein precursor (HXCP2), mRNA. | 0,084321 | -0,81763 |
| AK092490 | cDNA FLJ35171 fis, clone PLACE6013220, weakly similar to TRICHOHYALIN. | 0,085165 | -0,202 |
| NM_001807 | carboxyl ester lipase (bile salt-stimulated lipase) (CEL), mRNA. | 0,085355 | 0,327859 |
| NM_006701 | similar to S. pombe diml+ (DIM1), mRNA. | 0,085377 | -0,14439 |
| NM_014471 | serine protease inhibitor, Kazal type 4 (SPINK4), mRNA. | 0,086303 | -0,41374 |
| NM_002278 | keratin, hair, acidic, 2 (KRTHA2), mRNA. | 0,08655 | 0,15975 |
| BQ072652 | AGENCOURT_6763016 NIH_MGC_118 cDNA clone IMAGE:5756116 5', mRNA sequence. | 0,086909 | 0,24376 |
| NM_024556 | hypothetical protein FLJ21103 (FLJ21103), mRNA. | 0,088833 | -0,18109 |
| NM_139016 | hypothetical gene LOC128439 (LOC128439), mRNA. | 0,089422 | 0,233019 |
| BC039555 | clone IMAGE:4184613, mRNA. | 0,089567 | -0,20617 |
| NM_014593 | CpG binding protein (CGBP), mRNA. | 0,089567 | -0,16544 |
| NM_001920 | decorin (DCN), transcript variant A1, mRNA. | 0,089651 | 0,342227 |
| NM_015338 | KIAA0978 protein (KIAA0978), mRNA. | 0,089651 | 0,250409 |
| AL080280 | mRNA full length insert cDNA clone EUROIMAGE 85905. | 0,089798 | -0,40611 |
| NM_001249 | ectonucleoside triphosphate diphosphohydrolase 5 (ENTPD5), mRNA. | 0,091582 | -0,30505 |
| NM_032857 | lactamase, beta (LACTB), transcript variant 1, nuclear gene encoding mitochondrial protein, mRNA. | 0,092221 | -0,22271 |
| Z36818 | (xs166) mRNA, 400bp. | 0,092641 | 0,202657 |
| BU594226 | AGENCOURT_8843927 NIH_MGC_142 cDNA clone IMAGE:6449644 5', mRNA sequence. | 0,092641 | -0,20224 |
| NM_031453 | hypothetical protein MGC11034 (MGC11034), mRNA. | 0,092641 | -0,30736 |
| NM_003859 | dolichyl-phosphate mannosyltransferase polypeptide | 0,092641 | 0,25132 |
| | 1, catalytic subunit (DPM1), mRNA. | | |
| NM_001500 | GDP-mannose 4,6-dehydratase (GMDS), mRNA. | 0,09289 | -0,233 |
| NM_016014 | CGI-67 protein (CGI-67), mRNA. | 0,09289 | -0,24249 |
| NM_016332 | selenoprotein X, 1 (SEPX1), mRNA. | 0,09289 | 0,201764 |
| U32248 | mRNA with TGG repeat, clone 83. | 0,093518 | -0,30275 |
| AK056401 | cDNA FLJ31839 fis, clone NT2RP7000086. | 0,093844 | 0,375509 |
| NM_033126 | serine/threonine kinase PSKH2 (PSKH2), mRNA. | 0,093844 | 0,30743 |
| NM_014071 | nuclear receptor coactivator 6 (NCOA6), mRNA. | 0,094529 | 0,216909 |
| NM_003526 | H2B histone family, member L (H2BFL), mRNA. | 0,094617 | 0,195215 |
| NM_030582 | collagen, type XVIII, alpha 1 (COL18A1), transcript variant 1, mRNA. | 0,094767 | 0,319311 |
| AK002162 | cDNA FLJ11300 fis, clone PLACE1009886. | 0,094934 | -0,269 |
| NM_005944 | antigen identified by monoclonal antibody MRC OX-2 (MOX2), mRNA. | 0,094934 | -0,20733 |
| NM_138432 | likely ortholog of mouse serine dehydratase related sequence 1 (SDS-RS1), mRNA. | 0,094934 | -0,17005 |
| BC003122 | clone IMAGE:3357127, mRNA, partial cds. | 0,094934 | 0,274503 |
| NM_032813 | hypothetical protein FLJ14624 (FLJ14624), mRNA. | 0,094934 | 0,189731 |
| NM_007345 | zinc finger protein 236 (ZNF236), mRNA. | 0,094934 | -0,17085 |
| NM_004891 | mitochondrial ribosomal protein L33 (MRPL33), nuclear gene encoding mitochondrial protein, transcript variant 1, mRNA. | 0,094934 | 0,125686 |
| AY006312 | clone 09npa41 T cell receptor beta chain mRNA, partial cds. | 0,094934 | -0,36656 |
| AB033025 | mRNA for KIAA1199 protein, partial cds. | 0,094934 | -0,34034 |
| NM_003212 | teratocarcinoma-derived growth factor 1 (TDGF1), mRNA. | 0,094934 | 0,213788 |
| NM_017540 | UDP-N-acetyl-alpha-D-galactosamine:polypeptide N-acetylgalactosaminyltransferase 10 (GalNAc-T10) (GALNT10), mRNA. | 0,094934 | -0,1997 |
| BC034459 | clone IMAGE:5185460, mRNA. | 0,094934 | -0,2396 |
| BE672687 | 7b71e04.x1 NCI_CGAP _Lu24 cDNA clone IMAGE:3233694 3', mRNA sequence. | 0,094934 | -0,53881 |
| NM_016004 | chromosome 20 open reading frame 9 (C20orf9), | 0,094934 | 0,21615 |
| | mRNA. | | |
| BQ932364 | AGENCOURT_8854114 NIH_MGC_71 cDNA clone IMAGE:6473695 5', mRNA sequence. | 0,095037 | -0,21294 |
| NM_000134 | fatty acid binding protein 2, intestinal (FABP2), mRNA. | 0,095037 | -0,49638 |
| NM_015949 | CGI-20 protein (CGI-20), mRNA. | 0,095037 | 0,227496 |
| NM_018235 | hypothetical protein FLJ10830 (FLJ10830), mRNA. | 0,095161 | -0,18303 |
| NM_017707 | up-regulated in liver cancer 1 (UPLC1), mRNA. | 0,096054 | -0,32197 |
| AK097488 | cDNA FLJ40169 fis, clone TESTI2016583. | 0,096059 | 0,208604 |
| NM_024533 | hypothetical protein FLJ22167 (FLJ22167), mRNA. | 0,096125 | -0,3448 |
| AL110141 | mRNA, cDNA DKFZp564D0164 (from clone DKFZp564D0164). | 0,096125 | -0,19982 |
| NM_024861 | hypothetical protein FLJ22671 (FLJ22671), mRNA. | 0,09616 | 0,405449 |
| NM_004242 | high mobility group nucleosomal binding domain 3 (HMGN3), transcript variant 1, mRNA. | 0,09616 | -0,1732 |
| U50524 | BRCA2 region, mRNA sequence CG017. | 0,096218 | 0,266439 |
| NM_007055 | polymerase (RNA) III (DNA directed) (155kD) (RPC155), mRNA. | 0,097724 | 0,105862 |
| NM_006746 | sex comb on midleg-like 1 (Drosophila) (SCML1), mRNA. | 0,09878 | 0,24864 |
| NM_024792 | membrane protein expressed in epithelial-like lung adenocarcinoma (CT120), mRNA. | 0,09878 | -0,17414 |
| NM_015476 | DKFZP586M1523 protein (DKFZP586M1523), mRNA. | 0,098987 | -0,1933 |
| NM_032862 | tigger transposable element derived 5 (TIGD5), mRNA. | 0,099429 | 0,220793 |
| NM_004407 | dentin matrix acidic phosphoprotein (DMP1), mRNA. | 0,099429 | 0,268602 |
| NM_003880 | WNT1 inducible signaling pathway protein 3 (WISP3), transcript variant 1, mRNA. | 0,099712 | -0,19109 |
| AK090401 | mRNA for FLJ00278 protein. | 0,099712 | 0,283364 |

**Table 8: Human genes differentially expressed between colorectal adenoma and adenocarcinoma cells with chromosomal gain at 20q (Univariante analysis). Data are ranked on FDR value**

| Gene ID | Description (20q gain, Table 8) | FDR | Effect |
|---|---|---|---|
| NM_003859 | dolichyl-phosphate mannosyltransferase polypeptide 1, catalytic subunit (DPM1), mRNA. | 9,74E-07 | 0,418628 |
| NM_021809 | TGFB-induced factor 2 (TALE family homeobox) (TGIF2), mRNA. | 7,16E-06 | 0,394353 |
| NM_024522 | hypothetical protein FLJ12650 (FLJ12650), mRNA. | 1,06E-05 | 0,261245 |
| NM_018840 | putative Rab5-interacting protein (RIP5), mRNA. | 1,15E-05 | 0,317908 |
| NM_030815 | chromosome 20 open reading frame 126 (C20orf126), mRNA. | 0,000079 | 0,315633 |
| NM_014052 | GW128 protein (GW128), mRNA. | 0,000079 | 0,31413 |
| AK024563 | cDNA: FLJ20910 fis, clone ADSE00492. | 0,000079 | 0,400716 |
| NM_017896 | chromosome 20 open reading frame 11 (C20orf11), mRNA. | 0,000111 | 0,253047 |
| NM_032527 | hypothetical protein FLJ14972 (KIAA1847), mRNA. | 0,000111 | 0,210606 |
| NM_033342 | tripartite motif-containing 7 (TRIM7), mRNA. | 0,000111 | -0,46251 |
| NM_152777 | chromosome 14 open reading frame 48 (C14orf48), mRNA. | 0,000148 | 0,349834 |
| BC033940 | clone IMAGE:5272723, mRNA, partial cds. | 0,000219 | 0,328968 |
| NM_016397 | TH1-like (Drosophila) (TH1L), mRNA. | 0,000277 | 0,256703 |
| NM_006602 | transcription factor-like 5 (basic helix-loop-helix) (TCFL5), mRNA. | 0,000277 | 0,535295 |
| NM_016646 | mesenchymal stem cell protein DSCD28 (LOC51336), mRNA. | 0,000283 | 0,243076 |
| NM_002436 | membrane protein, palmitoylated 1, 55kDa (MPP1), mRNA. | 0,000352 | 0,425174 |
| NM_024115 | hypothetical protein MGC4309 (MGC4309), mRNA. | 0,000369 | 0,217642 |
| NM_012105 | beta-site APP-cleaving enzyme 2 (BACE2), transcript variant a, mRNA. | 0,000392 | -0,24343 |
| AB033070 | mRNA for KIAA1244 protein, partial cds. | 0,000485 | -0,3724 |
| NM_012190 | formyltetrahydrofolate dehydrogenase (FTHFD), transcript variant 1, mRNA. | 0,000485 | -0,52114 |
| NM_002296 | lamin B receptor (LBR), mRNA. | 0,000632 | 0,242622 |
| AL832390 | mRNA, cDNA DKFZp667H2012 (from clone DKFZp667H2012). | 0,000691 | -0,37264 |
| NM_024898 | hypothetical protein FLJ22757 (FLJ22757), mRNA. | 0,000704 | -0,21365 |
| NM_024592 | hypothetical protein FLJ13352 (FLJ13352), mRNA. | 0,000704 | -0,28523 |
| Y11166 | U71b small nucleolar RNA gene. | 0,001202 | 0,582884 |
| NM_030789 | histocompatibility (minor) 13 (HM13), mRNA. | 0,001222 | 0,199872 |
| NM_018270 | chromosome 20 open reading frame 20 (C20orf20), mRNA. | 0,001223 | 0,186523 |
| NM_022105 | death associated transcription factor 1 (DATF1), transcript variant 1, mRNA. | 0,001431 | 0,272385 |
| NM_005077 | transducin-like enhancer of split 1(E(spl)H.1og, Drosophila) (TLE1), mRNA. | 0,001704 | -0,33036 |
| AY069977 | anterior gradient protein 3 mRNA, complete cds. | 0,001712 | -0,70293 |
| NM_018439 | hypothetical protein IMPACT (IMPACT), mRNA. | 0,001712 | -0,30337 |
| NM_018710 | hypothetical protein DKFZp762O076 (DKFZp762O076), mRNA. | 0,001712 | 0,338698 |
| NM_003839 | tumor necrosis factor receptor superfamily, member 11a, activator ofNFKB (TNFRSF11A), mRNA. | 0,001802 - | 0,4257757 |
| NM_005710 | polyglutamine binding protein 1 (PQBP1), mRNA. | 0,002247 | 0,146744 |
| NM_030877 | catenin, beta like 1 (CTNNBL1), mRNA. | 0,002247 | 0,296816 |
| NM_014593 | CpG binding protein (CGBP), mRNA. | 0,002247 | -0,20906 |
| NM_007039 | protein tyrosine phosphatase, non-receptor type 21 (PTPN21), mRNA. | 0,002254 | -0,28594 |
| BQ189477 | UI-E-EJ1-aka-c-13-0-UI.r1 UI-E-EJ1 cDNA clone UI-E-EJ1-aka-c-13-0-UI 5', mRNA sequence. | 0,00229 | -0,53703 |
| NM_002915 | replication factor C (activator 1) 3, 38kDa (RFC3), mRNA. | 0,00229 | 0,24125 |
| NM_018414 | GalNAc alpha-2, 6-sialyltransferase I, long form (ST6GalNAcI), mRNA. | 0,00229 | -0,5849 |
| NM_024792 | membrane protein expressed in epithelial-like lung adenocarcinoma (CT120), mRNA. | 0,002393 | -0,223 |
| NM_015338 | KIAA0978 protein (KIAA0978), mRNA. | 0,00309 | 0,310714 |
| NM_018569 | hypothetical protein PRO0971 (PRO0971), mRNA. | 0,003334 | 0,250694 |
| NM_014214 | inositol(myo)-1(or 4)-monophosphatase 2 (IMPA2), mRNA. | 0,00338 | -0,27004 |
| AF085835 | full length insert cDNA clone YI41B09. | 0,00338 | 0,554618 |
| NM_001432 | epiregulin (EREG), mRNA. | 0,00338 | 0,72949 |
| NM_017495 | RNA-binding region (RNP1, RRM) containing 1 (RNPC1), mRNA. | 0,00338 | 0,318526 |
| NM_002212 | integrin beta 4 binding protein (ITGB4BP), mRNA. | 0,00338 | 0,219412 |
| NM_033120 | naked cuticle H.log 2 (Drosophila) (NKD2), mRNA. | 0,00338 | 0,635327 |
| BC002877 | Similar to hypothetical protein FLJ11585, clone MGC:11258 IMAGE:3942160, mRNA, complete cds. | 0,003386 | -0,19597 |
| BC038852 | clone IMAGE:5168364, mRNA. | 0,003386 | -0,5747 |
| AK057624 | cDNA FLJ33062 fis, clone TRACH2000032. | 0,003485 | 0,276663 |
| NM_014657 | KIAA0406 gene product (KIAA0406), mRNA. | 0,003625 | 0,31674 |
| NM_020673 | RAB22A, member RAS oncogene family (RAB22A), mRNA. | 0,003832 | 0,230507 |
| NM_006886 | ATP synthase, H+ transporting, mitochondrial F1 complex, epsilon subunit (ATP5E), nuclear gene encoding mitochondrial protein, mRNA. | 0,003832 | 0,227079 |
| NM_032872 | NADPH oxidase-related, C2 domain-containing protein (JFC1), mRNA. | 0,003846 | -0,30115 |
| AK056292 | cDNA FLJ31730 fis, clone NT2RI2006838. | 0,003846 | 0,480505 |
| NM_006839 | inner membrane protein, mitochondrial (mitofilin) (IMMT), mRNA. | 0,003901 | -0,34137 |
| NM_006714 | acid sphingomyelinase-like phosphodiesterase (ASM3A), mRNA. | 0,004209 | -0,39427 |
| NM_002267 | karyopherin alpha 3 (importin alpha 4) (KPNA3), mRNA. | 0,004209 | 0,157742 |
| NM_006746 | sex comb on midleg-like 1 (Drosophila) (SCML1), mRNA. | 0,004499 | 0,305508 |
| NM_016839 | RNA binding motif, single stranded interacting protein 1 (RBMS1), transcript variant MSSP-2, mRNA. | 0,004629 | 0,264982 |
| AK094894 | cDNA FLJ37575 fis, clone BRCOC2003125, moderately similar to TRIOSEPHOSPHATE ISOMERASE (EC 5.3.1.1). | 0,005114 | 0,195457 |
| NM_032044 | regenerating gene type IV (REG-IV), mRNA. | 0,005114 | -0,9253 |
| NM_021945 | hypothetical protein FLJ22174 (FLJ22174), mRNA. | 0,005173 | -0,4256 |
| BC029775 | hypothetical gene LOC127421, clone MGC:35394 IMAGE:5186268, mRNA, complete cds. | 0,005173 | -0,48807 |
| BC014971 | Similar to tubulin, beta, 2, clone IMAGE:4873024, mRNA. | 0,005173 | 0,243047 |
| BC040672 | clone IMAGE:4816952, mRNA. | 0,005206 | -0,33858 |
| NM_003185 | TAF4 RNA polymerase II, TATA box binding protein (TBP)-associated factor, 135kDa (TAF4), mRNA. | 0,005309 | 0,249808 |
| NM_138425 | hypothetical protein BC009925 (LOC113246), mRNA. | 0,005406 | -0,24799 |
| NM_080476 | CDC91 cell division cycle 91-like 1 (S. cerevisiae) (CDC91L1), mRNA. | 0,005474 | 0,241093 |
| NM_053286 | aquaporin 6, kidney specific (AQP6), transcript variant 2, mRNA. | 0,005474 | -0,47621 |
| NM_000030 | alanine-glyoxylate aminotransferase (oxalosis I hyperoxaluria I | 0,006588 | 0,231166 |
| NM_024868 | hypothetical protein FLJ14124 (FLJ14124), mRNA. | 0,006588 | -0,36372 |
| AF441770 | Tho2 mRNA, complete cds. | 0,006588 | 0,210436 |
| NM_016004 | chromosome 20 open reading frame 9 (C20orf9), mRNA. | 0,006588 | 0,256153 |
| NM_000442 | platelet/endothelial cell adhesion molecule (CD31 antigen) (PECAM1), mRNA. | 0,007012 | -0,47652 |
| AK022277 | cDNA FLJ12215 fis, clone MAMMA1001021. | 0,007842 | 0,392955 |
| NM_016938 | EGF-containing fibulin-like extracellular matrix protein 2 (EFEMP2), mRNA. | 0,008237 | 0,289147 |
| NM_016407 | chromosome 20 open reading frame 43 (C20orf43), mRNA. | 0,008237 | 0,235892 |
| NM_016400 | Huntingtin interacting protein K (HYPK), mRNA. | 0,008254 | -0,23536 |
| NM_020532 | reticulon 4 (RTN4), mRNA. | 0,008407 | 0,239667 |
| NM_032342 | hypothetical protein MGC12992 (MGC12992), mRNA. | 0,008407 | -0,4736 |
| NM_014071 | nuclear receptor coactivator 6 (NCOA6), mRNA. | 0,008407 | 0,252263 |
| NM_138340 | abhydrolase domain containing 3 (ABHD3), mRNA. | 0,008449 | -0,33528 |
| NM_032827 | hypothetical protein FLJ14708 (FLJ14708), mRNA. | 0,008449 | -0,48847 |
| NM_021990 | gamma-aminobutyric acid (GABA) A receptor, epsilon (GABRE), transcript variant 4, mRNA. | 0,008449 | 0,330516 |
| NM_003600 | serine/threonine kinase 6 (STK6), mRNA. | 0,008449 | 0,319726 |
| NM_017699 | hypothetical protein FLJ20174 (FLJ20174), mRNA. | 0,008449 | -0,48325 |
| NM_003518 | H2B histone family, member A (H2BFA), mRNA. | 0,008449 | 0,190364 |
| AF353674 | BTB domain protein (BDPL) mRNA, partial cds. | 0,008449 | -0,25392 |
| NM_012112 | chromosome 20 open reading frame 1 (C20orf1), mRNA. | 0,008449 | 0,309192 |
| NM_021100 | NFS1 nitrogen fixation 1 (S. cerevisiae) (NFS1), mRNA. | 0,008449 | 0,251411 |
| NM_007019 | ubiquitin-conjugating enzyme E2C (UBE2C), mRNA. | 0,008449 | 0,254903 |
| BU682808 | UI-CF-EC1-aci-j-11-0-UI.sl UL-CF-EC1 cDNA clone UI-CF-EC1-aci-j-11-0-UI 3', mRNA sequence. | 0,008449 | 0,311729 |
| NM_017954 | hypothetical protein FLJ20761 (FLJ20761), mRNA. | 0,008449 | -0,28226 |
| BC008502 | clone MGC:14841 IMAGE:4295121, mRNA, complete cds. | 0,008449 | -0,31933 |
| AF350451 | C6orf37 mRNA, complete cds. | 0,008449 | -0,3448 |
| NM_015675 | growth arrest and DNA-damage-inducible, beta (GADD45B), mRNA. | 0,008449 | 0,116265 |
| NM_014766 | KIAA0193 gene product (KIAA0193), mRNA. | 0,008449 | 0,63088 |
| NM_002846 | protein tyrosine phosphatase, receptor type, N (PTPRN), mRNA. | 0,008449 | -0,22025 |
| NM_005159 | actin, alpha, cardiac muscle (ACTC), mRNA. | 0,008449 | 0,153351 |
| NM_024725 | hypothetical protein FLJ23518 (FLJ23518), mRNA. | 0,008449 | -0,24707 |
| NM_000273 | ocular albinism 1 (Nettleship-Falls) (OA1), mRNA. | 0,008562 | 0,388237 |
| AK093418 | cDNA FLJ36099 fis, clone TESTI2021057, weakly similar to RETINAL-BINDING PROTEIN. | 0,008852 | 0,400544 |
| NM_001062 | transcobalamin I (vitamin B12 binding protein, R binder family) (TCN1), mRNA. | 0,008928 | -0,58802 |
| BM669556 | UI-E-DX1-agw-c-13-0-UI.sl UI-E-DX1 cDNA clone UI-E-DXl-agw-c-13-0-UI 3', mRNA sequence. | 0,008928 | 0,222546 |
| NM_032633 | hypothetical protein MGC5457 (MGC5457), mRNA. | 0,009272 | 0,184803 |
| NM_012461 | TERF1 (TRF1)-interacting nuclear factor 2 (TINF2), mRNA. | 0,009331 | -0,16965 |
| NM_030672 | hypothetical protein FLJ10312 (FLJ10312), mRNA. | 0,009331 | 0,348971 |
| NM_014622 | loss of heterozygosity, 11, chromosomal region 2, gene A (LOH11CR2A), mRNA. | 0,00952 | -0,30272 |
| NM_021114 | serine protease inhibitor, Kazal type, 2 (acrosin-trypsin inhibitor) (SPINK2), mRNA. | 0,009691 | -0,30382 |
| NM_014177 | HSPC154 protein (HSPC154), mRNA. | 0,00984 | -0,18611 |
| NM_001316 | CSE1 chromosome segregation 1-like (yeast) (CSE1L), mRNA. | 0,010139 | 0,227071 |
| NM_020315 | hypothetical protein dJ37E16.5 (DJ37E16.5), mRNA. | 0,010203 | 0,194366 |
| NM_130785 | TPTE and PTEN H.logous inositol lipid phosphatase (TPIP), mRNA. | 0,010213 | -0,2038 |
| NM_014054 | chromosome 20 open reading frame 40 (C20orf40), mRNA. | 0,010213 | 0,208409 |
| AL833463 | mRNA, cDNA DKFZp686P07116 (from clone DKFZp686P07116). | 0,010237 | 0,458492 |
| NM_007002 | adhesion regulating molecule 1 (ADRM1), mRNA. | 0,01037 | 0,211848 |
| AK021916 | cDNA FLJ11854 fis, clone HEMBA1006767. | 0,010987 | 0,231398 |
| NM_004715 | CTD (carboxy-terminal domain, RNA polymerase II, polypeptide A) phosphatase, subunit 1 (CTDP1), transcript variant FCPla, mRNA. | 0,010995 | -0,14281 |
| NM_004902 | RNA-binding region (RNP1, RRM) containing 2 (RNPC2), mRNA. | 0,011173 | 0,208296 |
| NM_006615 | calpain 9 (nCL-4) (CAPN9), mRNA. | 0,011265 | -0,53614 |
| NM_006644 | heat shock 105kD (HSP105B), mRNA. | 0,011317 | 0,301377 |
| BC039527 | clone IMAGE:5742065, mRNA. | 0,011482 | 0,263524 |
| BC016864 | clone IMAGE:3847536, mRNA. | 0,011482 | 0,315896 |
| NM_003404 | tyrosine 3-monooxygenase/tryptophan 5-monooxygenase activation protein, beta polypeptide (YWHAB), transcript variant 1, mRNA. | 0,011625 | 0,23437 |
| NM_024609 | hypothetical protein FLJ21841 (FLJ21841), mRNA. | 0,011625 | 0,411411 |
| BC008975 | clone MGC:16774 IMAGE:4215274, mRNA, complete cds. | 0,011625 | 0,275597 |
| AB033045 | mRNA for KIAA1219 protein, partial cds. | 0,011763 | 0,196977 |
| AY057051 | putative integral membrane protein mRNA, complete cds. | 0,011763 | 0,384158 |
| NM_001151 | solute carrier family 25 (mitochondrial carrier adenine nucleotide translocator), member 4 (SLC25A4), nuclear gene encoding mitochondrial protein, mRNA. | 0,011763 | -0,18736 |
| AK093522 | cDNA FLJ36203 fis, clone TESTI2028426, weakly similar to HISTONE H2B F. | 0,011763 | -0,39299 |
| NM_024861 | hypothetical protein FLJ22671 (FLJ22671), mRNA. | 0,011763 | 0,457049 |
| NM_030579 | cytochrome b5 outer mitochondrial membrane precursor (CYB5-M), mRNA. | 0,012053 | 0,174642 |
| AJ227898 | partial mRNA ID EE2-8E. | 0,012185 | 0,280683 |
| NM_013390 | transmembrane protein 2 (TMEM2), mRNA. | 0,012188 | -0,263 |
| NM_014399 | transmembrane 4 superfamily member tetraspan NET-6 (NET-6), mRNA. | 0,012449 | -0,24464 |
| BC024318 | clone IMAGE:3915414, mRNA. | 0,012669 | 0,257688 |
| BC035561 | clone MGC:40554 IMAGE:5211583, mRNA, complete cds. | 0,012687 | -0,27115 |
| NM_032950 | matrix metalloproteinase 28 (MMP28), transcript variant 2, mRNA. | 0,012705 | -0,38736 |
| NM_002899 | retinol binding protein 1, cellular (RBP1), mRNA. | 0,013437 | 0,415611 |
| NM_031407 | upstream regulatory element binding protein 1 (UREB1), mRNA. | 0,013527 | 0,203237 |
| CA447552 | UI-H-EI0-aye-j-13-0-UI.s1 NCI_CGAP_EI0 cDNA clone UI-H-EI0-aye-j-13-0-UI 3', mRNA sequence. | 0,013527 | 0,242779 |
| AF086417 | full length insert cDNA clone ZD78A02. | 0,014121 | 0,530657 |
| NM_019610 | hypothetical protein 669 (LOC56267), mRNA. | 0,014244 | 0,20125 |
| NM_001363 | dyskeratosis congenita 1, dyskerin (DKC1), mRNA. | 0,014293 | 0,166155 |
| BC031648 | KIAA1324 protein, clone MGC:35166 IMAGE:5169952, mRNA, complete cds. | 0,014293 | -0,53032 |
| BC011242 | clone IMAGE:4153763, mRNA. | 0,014293 | 0,391582 |
| NM_024533 | hypothetical protein FLJ22167 (FLJ22167), mRNA. | 0,014448 | -0,38164 |
| NM_144676 | hypothetical protein MGC23911 (MGC23911), mRNA. | 0,014448 | -0,13111 |
| AL122070 | mRNA, cDNA DKFZp434E0535 (from clone DKFZp434E0535) | 0,014448 | -0,29939 |
| NM_003731 | Sjogren's syndrome nuclear autoantigen 1 (SSNA1), mRNA. | 0,014448 | 0,215103 |
| AK091840 | cDNA FLJ34521 fis, clone HLUNG2007041. | 0,014472 | -0,31834 |
| NM_000612 | insulin-like growth factor 2 (somatomedin A) (IGF2), mRNA. | 0,014848 | 0,990401 |
| NM_006892 | DNA (cytosine-5-)-methyltransferase 3 beta (DNMT3B), mRNA. | 0,015111 | 0,453912 |
| NM_017734 | palmdelphin (PALMD), mRNA. | 0,015111 | 0,256082 |
| NM_002398 | Meisl, myeloid ecotropic viral integration site 1 H.log (mouse) (MEIS1), mRNA. | 0,01539 | 0,474067 |
| NM_002894 | retinoblastoma binding protein 8 (RBBP8), mRNA. | 0,01539 | -0,14114 |
| NM_018066 | hypothetical protein FLJ10349 (FLJ10349), mRNA. | 0,01539 | -0,1972 |
| AW816421 | QV4-ST0234-241199-040-h07 ST0234 cDNA, mRNA sequence. | 0,015975 | -0,28802 |
| NM_152243 | CDC42 effector protein (Rho GTPase binding) 1 (CDC42EP1), transcript variant 1, mRNA. | 0,016006 | -0,34206 |
| NM_138446 | hypothetical protein BC012331 (LOCI 15416), mRNA. | 0,016006 | 0,14689 |
| NM_017895 | DEAD/H (Asp-Glu-Ala-Asp/His) box polypeptide 27 (DDX27), mRNA. | 0,016354 | 0,202609 |
| NM_016045 | chromosome 20 open reading frame 45 (C20orf45), mRNA. | 0,016429 | 0,275018 |
| AK055928 | cDNA FLJ31366 fis, clone NB9N41000142. | 0,016429 | 0,413379 |
| NM_033339 | caspase 7, apoptosis-related cysteine protease (CASP7), transcript variant gamma, mRNA. | 0,016429 | -0,23371 |
| BC033250 | clone IMAGE:5441027, mRNA, partial cds. | 0,016435 | 0,225752 |
| NM_152621 | hypothetical protein MGC26963 (MGC26963), mRNA. | 0,01681 | -0,41828 |
| NM_13 8432 | likely ortholog of mouse serine dehydratase related sequence 1 (SDS-RS1), mRNA. | 0,017141 | -0,18424 |
| AK074657 | cDNA FLJ90176 fis, clone MAMMA1000528. | 0,017147 | -0,27683 |
| NM_018235 | hypothetical protein FLJ10830 (FLJ10830), mRNA. | 0,017147 | -0,19875 |
| NM_006649 | serologically defined colon cancer antigen 16 (SDCCAG16),mRNA. | 0,017147 | 0,190769 |
| BC013148 | clone IMAGE:3925108, mRNA. | 0,017646 | 0,403509 |
| NM_005153 | ubiquitin specific protease 10 (USP10), mRNA. | 0,01772 | 0,118274 |
| NM_018530 | hypothetical protein PRO2521 (PRO2521), mRNA. | 0,018304 | -0,28343 |
| NM_007187 | WW domain binding protein 4 (formin binding protein 21) (WBP4), mRNA. | 0,018326 | 0,221965 |
| AB033037 | mRNA for KIAA1211 protein, partial cds. | 0,018326 | -0,20092 |
| NM_016315 | CED-6 protein (CED-6), mRNA. | 0,018326 | 0,161405 |
| NM_001145 | angiogenin, ribonuclease, RNase A family, 5 (ANG), mRNA. | 0,018326 | -0,26978 |
| NM_018690 | apolipoprotein B48 receptor (APOB48R), mRNA. | 0,018808 | -0,38986 |
| NM_016082 | CDK5 regulatory subunit associated protein 1 (CDKSRAP1), mRNA. | 0,018997 | 0,19906 |
| NM_006646 | WAS protein family, member 3 (WASF3), mRNA. | 0,019059 | 0,357576 |
| NM_021215 | chromosome 20 open reading frame 77 (C20orf77), mRNA. | 0,019187 | 0,292433 |
| NM_152255 | proteasome (prosome, macropain) subunit, alpha type, 7 (PSMA7), transcript variant 2, mRNA. | 0,019187 | 0,139936 |
| NM_003265 | toll-like receptor 3 (TLR3), mRNA. | 0,019498 | -0,34724 |
| NM_014786 | Rho-specific guanine-nucleotide exchange factor 164 kDa (P164RHOGEF), mRNA. | 0,019681 | 0,153977 |
| BG289994 | 602385142F1 NIH_MGC_93 cDNA clone IMAGE:4513882 5', mRNA sequence. | 0,019681 | -0,30448 |
| NM_031472 | hypothetical protein MGC11134 (MGC11134), mRNA. | 0,02001 | -0,21485 |
| AL713719 | mRNA, cDNA DKFZp667K1916 (from clone DKFZp667K1916). | 0,02001 | 0,335338 |
| NM_006704 | SGT1, suppressor of G2 allele of SKP1 (S. cerevisiae) (SUGT1), mRNA. | 0,02001 | 0,143388 |
| NM_138793 | Ca2+-dependent endoplasmic reticulum nucleoside diphosphatase (SHAPY), mRNA. | 0,02045 | -0,21529 |
| NM_006097 | myosin regulatory light chain 2, smooth muscle isoform (MYRL2), mRNA. | 0,020598 | 0,271796 |
| NM_014183 | dynein, cytoplasmic, light polypeptide 2A (DNCL2A), mRNA. | 0,020598 | 0,195279 |
| NM_001500 | GDP-mannose 4,6-dehydratase (GMDS), mRNA. | 0,020706 | -0,24621 |
| AK095896 | cDNA FLJ38577 fis, clone HCHON2007650. | 0,020928 | -0,23544 |
| NM_001155 | annexin A6 (ANXA6), transcript variant 1, mRNA. | 0,020928 | 0,252778 |
| AK095808 | cDNA FLJ38489 fis, clone FEBRA2023550, weakly similar to P3 PROTEIN. | 0,020928 | -0,25284 |
| NM_012339 | transmembrane 4 superfamily member tetraspan NET-7 (NET-7), mRNA. | 0,021375 | -0,249 |
| NM_003808 | tumor necrosis factor (ligand) superfamily, member 13 (TNFSF13), transcript variant alpha, mRNA. | 0,021611 | -0,33387 |
| NM_015932 | hypothetical protein HSPC014 (HSPC014), mRNA. | 0,02168 | 0,181839 |
| NM_021998 | zinc finger protein 6 (CMPX1) (ZNF6), mRNA. | 0,02168 | 0,229833 |
| AF085893 | full length insert cDNA clone YP95A10. | 0,02168 | 0,202777 |
| NM_003118 | secreted protein, acidic, cysteine-rich (osteonectin) (SPARC), mRNA. | 0,02168 | 0,325687 |
| NM_013451 | fer-1-like 3, myoferlin (C. elegans) (FER1L3), transcript variant 1, mRNA. | 0,022435 | -0,20185 |
| NM_006074 | tripartite motif-containing 22 (TRIM22), mRNA. | 0,022435 | -0,26807 |
| NM_001324 | cleavage stimulation factor, 3' pre-RNA, subunit 1, 50kDa (CSTF1), mRNA. | 0,022435 | 0,189653 |
| NM_015362 | HSPC002 protein (HSPC002), mRNA. | 0,022743 | -0,24258 |
| BC027604 | similar to CG14182 gene product, clone MGC:26782 IMAGE:4838212, mRNA, complete cds. | 0,022743 | 0,164779 |
| NM_015352 | protein O-fucosyltransferase 1 (POFUT1), transcript variant 1, mRNA. | 0,022743 | 0,305394 |
| NM_152429 | hypothetical protein MGC39320 (MGC39320), mRNA. | 0,022749 | -0,26023 |
| NM_018282 | paraspeckle protein 1 (PSP1), mRNA. | 0,022749 | 0,278469 |
| NM_007032 | Tara-like protein (HRIHFB2122), transcript variant 1, mRNA. | 0,022749 | -0,23412 |
| BC033196 | clone MGC:45840 IMAGE:4849453, mRNA, complete cds. | 0,022749 | -0,32489 |
| NM_032229 | hypothetical protein FLJ22774 (FLJ22774), mRNA. | 0,022749 | -0,64794 |
| AK090928 | cDNA FLJ33609 fis, clone BRAMY2015890. | 0,02286 | 0,219169 |
| NM_023011 | similar to yeast Upf3, variant A (UPF3A), transcript variant 1, mRNA. | 0,02286 | 0,156934 |
| AF339772 | clone IMAGE:128781, mRNA sequence. | 0,023342 | 0,350884 |
| NM_014353 | RAB26, member RAS oncogene family (RAB26), mRNA. | 0,023342 | -0,36521 |
| AL122043 | mRNA, cDNA DKFZp566G1424 (from clone DKFZp566G1424). | 0,023342 | 0,282538 |
| NM_021724 | nuclear receptor subfamily 1, group D, member 1 (NR1D1), mRNA. | 0,023342 | 0,306694 |
| NM_000532 | propionyl Coenzyme A carboxylase, beta polypeptide (PCCB), nuclear gene encoding mitochondrial protein, mRNA. | 0,023342 | -0,19244 |
| NM_013974 | dimethylarginine dimethylaminohydrolase 2 (DDAH2), mRNA. | 0,023342 | 0,18098 |
| AF161414 | HSPC296 mRNA, partial cds. | 0,023342 | -0,21477 |
| NM_002097 | general transcription factor IIIA (GTF3A), mRNA. | 0,023342 | 0,254443 |
| AK025683 | cDNA: FLJ22030 fis, clone HEP08669. | 0,023342 | 0,478539 |
| NM_024618 | hypothetical protein FLJ21478 (FLJ21478), transcript variant 1, mRNA. | 0,023342 | -0,23549 |
| BC031973 | similar to RIKEN cDNA 4921536K21, clone IMAGE:4827637, mRNA. | 0,023342 | 0,223172 |
| NM_003298 | nuclear receptor subfamily 2, group C, member 2 (NR2C2), mRNA. | 0,023342 | -0,17191 |
| NM_005877 | splicing factor 3a, subunit 1, 120kDa (SF3A1), mRNA. | 0,023522 | -0,1859 |
| AK024898 | cDNA: FLJ21245 fis, clone COL01184. | 0,023615 | -0,44329 |
| NM_000238 | potassium voltage-gated channel, subfamily H (eag-related), member 2 (KCNH2), transcript variant 1, mRNA. | 0,024092 | 0,314197 |
| BC015790 | clone IMAGE:4866059, mRNA. | 0,024564 | -0,46644 |
| NM_002216 | inter-alpha (globulin) inhibitor, H2 polypeptide (ITIH2), mRNA. | 0,024564 | 0,394375 |
| NM_002257 | kallikrein 1, renal/pancreas/salivary (KLK1), mRNA. | 0,024757 | -0,45029 |
| NM_025079 | hypothetical protein FLJ23231 (FLJ23231), mRNA. | 0,024757 | -0,27768 |
| NM_014484 | molybdenum cofactor synthesis 3 (MOCS3), mRNA. | 0,024912 | 0,368281 |
| NM_033126 | serine/threonine kinase PSKH2 (PSKH2), mRNA. | 0,025233 | 0,317575 |
| BC030520 | similar to hypothetical protein F33H2.2 - Caenorhabditis elegans, clone MGC:40403 IMAGE:5180533, mRNA, complete cds. | 0,025945 | 0,191189 |
| AK000852 | cDNA FLJ20845 fis, clone ADKA01901. | 0,025945 | 0,163988 |
| BG723246 | 602690864F1 NIH_MGC_97 cDNA clone IMAGE:4823295 5', mRNA sequence. | 0,025945 | -0,48691 |
| NM_032550 | hypothetical protein FLJ14564 (FLJ14564), mRNA. | 0,026121 | -0,39633 |
| NM_001788 | CDC10 cell division cycle 10 H.log (S. cerevisiae) (CDC10), mRNA. | 0,026931 | 0,179453 |
| NM 003908 | eukaryotic translation initiation factor 2, subunit 2 beta, 38kDa (EIF2S2), mRNA. | 0,026931 | 0,181861 |
| NM_005176 | ATP synthase, H+ transporting, mitochondrial F0 complex, subunit c (subunit 9), isoform 2 (ATP5G2), mRNA. | 0,026931 | 0,240383 |
| NM_030574 | START domain containing 5 (STARD5), mRNA. | 0,026933 | -0,19682 |
| NM_152366 | hypothetical protein MGC33338 (MGC33338), mRNA. | 0,027461 | -0,18439 |
| BC038512 | clone IMAGE:5262734, mRNA. | 0,027475 | 0,250234 |
| AK093993 | cDNA FLJ36674 fis, clone UTERU2004197. | 0,027475 | -0,28392 |
| NM_004738 | VAMP (vesicle-associated membrane protein)-associated protein B and C (VAPB), mRNA. | 0,027584 | 0,288854 |
| BC041376 | Similar to hypothetical protein 5830442F04, clone MGC:43895 IMAGE:5274634, mRNA, complete cds. | 0,027584 | -0,34671 |
| NM_012469 | chromosome 20 open reading frame 14 (C20orf14), mRNA. | 0,027584 | 0,228577 |
| NM_017423 | UDP-N-acetyl-alpha-D-galactosamine:polypeptide N-acetylgalactosaminyltransferase 7 (GalNAc-T7) (GALNT7), mRNA. | 0,02807 | -0,22549 |
| AK096918 | cDNA FLJ39599 fis, clone SKNSH2003174. | 0,028137 | 0,396244 |
| AB051495 | mRNA for KIAA1708 protein, partial cds. | 0,028349 | 0,118787 |
| NM_000456 | sulfite oxidase (SUOX), nuclear gene encoding mitochondrial protein, mRNA. | 0,028349 | -0,15531 |
| NM_002905 | retinol dehydrogenase 5 (11-cisand 9-cis) (RDH5), mRNA. | 0,02848 | -0,18593 |
| NM_003358 | UDP-glucose ceramide glucosyltransferase (UGCG), mRNA. | 0,028579 | 0,137583 |
| AL833275 | mRNA, cDNA DKFZp451C118 (from clone DKFZp451C118). | 0,028862 | 0,284558 |
| NM_015392 | neural proliferation, differentiation and control, 1 (NPDC1), mRNA. | 0,028862 | -0,29974 |
| NM_080672 | Q9H4T4 like (H17739), mRNA. | 0,028862 | -0,52739 |
| AF118079 | PRO1854 mRNA, complete cds. | 0,028878 | 0,231825 |
| NM_000835 | glutamate receptor, ionotropic, N-methyl D-aspartate 2C (GRIN2C), mRNA. | 0,028878 | 0,218899 |
| AB033100 | mRNA for KIAA1274 protein, partial cds. | 0,029154 | 0,382182 |
| AL831866 | mRNA, cDNA DKFZp761O0117 (from clone DKFZp76100117). | 0,029154 | -0,30015 |
| NM_001888 | crystallin, mu (CRYM), mRNA. | 0,029406 | -0,35197 |
| BC040628 | clone IMAGE:5745274, mRNA. | 0,029491 | -0,36081 |
| NM_018283 | hypothetical protein FLJ10956 (FLJ10956), mRNA. | 0,029774 | 0,120587 |
| NM_000537 | renin (REN), mRNA. | 0,029788 | -0,22482 |
| BM476468 | AGENCOURT_6476297 NIH_MGC_85 cDNA clone IMAGE:5553395 5', mRNA sequence. | 0,030174 | 0,296564 |
| AF084362 | lipoate-protein ligase B mRNA, partial cds. | 0,030307 | -0,20895 |
| AL834121 | mRNA, cDNA DKFZp761D2121 (from clone DKFZp761D2121). | 0,030486 | -0,29911 |
| NM_000921 | phosphodiesterase 3A, cGMP-inhibited (PDE3A), mRNA. | 0,030561 | 0,312171 |
| AK096267 | cDNA FLJ38948 fis, clone NT2NE2018165, weakly similar to Shb=Src protein. | 0,030561 | -0,26263 |
| NM_001933 | dihydrolipoamide S-succinyltransferase (E2 component of 2-oxo-glutarate complex) (DLST), mRNA. | 0,030561 | -0,18432 |
| BC040599 | clone IMAGE:5270494, mRNA. | 0,030594 | 0,516781 |
| NM_170695 | TGFB-induced factor (TALE family homeobox) (TGIF), mRNA. | 0,030594 | -0,17391 |
| NM_002814 | proteasome (prosome, macropain) 26S subunit, non-ATPase, 10 (PSMD10), mRNA. | 0,030594 | 0,141706 |
| NM_006416 | solute carrier family 35 (CMP-sialic acid transporter), member 1 (SLC35A1), mRNA. | 0,030594 | -0,28448 |
| NM_015328 | KIAA0828 protein (KIAA0828), mRNA. | 0,030594 | -0,29704 |
| NM_024636 | likely ortholog of mouse tumor necrosis-alpha-induced adipose-related protein (FLJ23153), mRNA. | 0,030794 | -0,19275 |
| NM_138706 | beta-1,3-N-acetylglucosaminyltransferase protein (IMAGE:4907098), mRNA. | 0,030794 | -0,4685 |
| NM_007238 | peroxisomal membrane protein 4, 24kDa (PXMP4), mRNA. | 0,030868 | 0,165557 |
| NM_000937 | polymerase (RNA) II (DNA directed) polypeptide A, 220kDa (POLR2A), mRNA. | 0,030998 | -0,19869 |
| NM_017798 | chromosome 20 open reading frame 21 (C20orf21), mRNA. | 0,031059 | 0,216003 |
| NM_015373 | chromosome 22 open reading frame 2 (C22orf2), mRNA. | 0,031238 | -0,19461 |
| AJ489592 | mRNA for glycosylation-dependent cell adhesion molecule 1 (GLYCAM1 gene), isolate KH c15. | 0,031344 | 0,275851 |
| BC037943 | clone IMAGE:5285657, mRNA. | 0,03141 | 0,303143 |
| NM_000295 | serine (or cysteine) proteinase inhibitor, clade A (alpha-1 antiproteinase, antitrypsin), member 1 (SERPINA1), mRNA. | 0,031556 | -0,41307 |
| NM_016232 | interleukin 1 receptor-like 1 (IL1RL1), mRNA. | 0,031556 | 0,15045 |
| NM_052854 | old astrocyte specifically induced substance (OASIS), mRNA. | 0,031556 | -0,28554 |
| AB028962 | mRNA for KIAA1039 protein, partial cds. | 0,031681 | -0,23145 |
| AK022220 | cDNA FLJ12158 fis, clone MAMMA1000522. | 0,031739 | -0,24435 |
| NM_012091 | adenosine deaminase, tRNA-specific 1 (ADATI), mRNA. | 0,032362 | 0,164394 |
| AK093281 | cDNA FLJ35962 fis, clone TESTI2012544. | 0,032759 | 0,207193 |
| NM_006667 | progesterone receptor membrane component 1 (PGRMC1), mRNA. | 0,033166 | 0,177531 |
| BG724120 | 602697424F1 NIH_MGC_97 cDNA clone IMAGE:4829788 5', mRNA sequence. | 0,033166 | 0,194064 |
| NM_002156 | heat shock 60kDa protein 1 (chaperonin) (HSPD1), mRNA. | 0,033166 | 0,152917 |
| NM_006809 | translocase of outer mitochondrial membrane 34 (TOMM34), mRNA. | 0,033311 | 0,274716 |
| NM_002303 | leptin receptor (LEPR), mRNA. | 0,033349 | -0,30981 |
| NM_002102 | glycophorin E (GYPE), mRNA. | 0,033956 | -0,20741 |
| AK094413 3 | cDNA FLJ37094 fis, clone BRACE2018337. | 0,034927 | -0,26101 |
| NM_013439 | paired immunoglobulin-like receptor alpha (PILR(ALPHA)), mRNA. | 0,035012 | -0,2427 |
| NM_018257 | chromosome 20 open reading frame 36 (C20orf36), mRNA. | 0,035012 | 0,189989 |
| NM_001887 | crystallin, beta B1 (CRYBB1), mRNA. | 0,035038 | 0,187768 |
| BU839667 | AGENCOURT_8494292 NIH_MGC_100 cDNA clone IMAGE:6285840 5', mRNA sequence. | 0,035078 | 0,330608 |
| NM_013248 | NTF2-like export factor 1 (NXT1), mRNA. | 0,035078 | 0,132344 |
| NM_000134 | fatty acid binding protein 2, intestinal (FABP2), mRNA. | 0,03518 | -0,49623 |
| NM_003683 | DNA segment on chromosome 21 (unique) 2056 expressed sequence (D2152056E), mRNA. | 0,035416 | 0,163274 |
| AF257167 | intestinal mucin 2.1 (MUC2) mRNA, partial cds. | 0,035646 | -0,73443 |
| NM_144726 | hypothetical protein FLJ31951 (FLJ31951), mRNA. | 0,035703 | -0,17616 |
| NM_003582 | dual-specificity tyrosine-(Y)-phosphorylation regulated kinase 3 (DYRK3), mRNA. | 0,035703 | 0,256838 |
| NM_000901 | nuclear receptor subfamily 3, group C, member 2 (NR3C2), mRNA. | 0,035703 | -0,52683 |
| NM_000965 | retinoic acid receptor, beta (RARB), transcript variant 1, mRNA. | 0,035703 | 0,34048 |
| NM_004385 | chondroitin sulfate proteoglycan 2 (versican) (CSPG2), mRNA. | 0,035703 | 0,286553 |
| BU739124 | UI-E-EJO-ahm-e-19-0-UI.sl UI-E-EJ0 cDNA clone UI-E-EJO-ahm-e-19-0-UI 3', mRNA sequence. | 0,035896 | -0,61874 |
| AK022464 | cDNA FLJ12402 fis, clone MAMMA1002807. | 0,036074 | 0,239522 |
| NM_170751 | chromodomain protein, Y chromosome-like (CDYL), transcript variant 2, mRNA. | 0,036074 | 0,155739 |
| NM_004700 | potassium voltage-gated channel, KQT-like subfamily, member 4 (KCNQ4), transcript variant 1, mRNA. | 0,036074 | -0,30596 |
| NM_002677 | peripheral myelin protein 2 (PMP2), mRNA. | 0,036074 | -0,37358 |
| NM_017781 | hypothetical protein FLJ20359 (FLJ20359), mRNA. | 0,036074 | 0,216438 |
| AJ417554 | partial mRNA for killer cell immunoglobulin receptor (KIR2DS4 gene), strain 3321. | 0,036074 | 0,236225 |
| NM_002619 | platelet factor 4 (chemokine (C-X-C motif) ligand 4) (PF4), mRNA. | 0,036467 | -0,36009 |
| AK021691 | cDNA FLJ11629 fis, clone HEMBA1004241. | 0,036467 | 0,272374 |
| NM_004798 | kinesin family member 3B (KIF3B), mRNA. | 0,036745 | 0,188924 |
| AK094156 | cDNA FLJ36837 fis, clone ASTR02011422. | 0,037271 | 0,379706 |
| NM_012391 | prostate epithelium-specific Ets transcription factor (PDEF), mRNA. | 0,037271 | -0,39702 |
| NM_012244 | solute carrier family 7 (cationic amino acid transporter, y+ system), member 8 (SLC7A8), mRNA. | 0,037271 | -0,21374 |
| AK055371 | cDNA FLJ30809 fis, clone FEBRA2001438. | 0,037271 | 0,362607 |
| NM_024761 | hypothetical protein FLJ13204 (FLJ13204), mRNA. | 0,03776 | -0,17087 |
| NM_004876 | zinc finger protein 254 (ZNF254), mRNA. | 0,03776 | 0,242645 |
| NM_005717 | actin related protein 2/3 complex, subunit 5, 16kDa (ARPC5), mRNA. | 0,038236 | 0,136888 |
| NM_052935 | hypothetical protein MGC20781 (MGC20781), mRNA. | 0,038574 | 0,222224 |
| BC035036 | clone IMAGE:5262128, mRNA, partial cds. | 0,0388 | 0,288315 |
| NM_138573 | neuregulin 4 (LOC145957), mRNA. | 0,039144 | -0,2714 |
| Z34281 | (MAR10) MUC5AC mRNA for mucin (partial). | 0,03927 | -1,00219 |
| NM_080663 | similar to RIKEN cDNA 4933424N09 gene (MGC16943), mRNA. | 0,03927 | -0,1697 |
| BF035279 | 601457165F1 NIH_MGC_66 cDNA clone IMAGE:3860633 5', mRNA sequence. | 0,039713 | 0,295041 |
| NM_004672 | mitogen-activated protein kinase kinase kinase 6 (MAP3K6), transcript variant 1, mRNA. | 0,039713 | -0,19613 |
| NM_013443 | CMP-NeuAC:(beta)-N-acetylgalactosaminide (alpha)2,6-sialyltransferase member VI (ST6GALNAC6), mRNA. | 0,039713 | -0,36877 |
| AJ001402 | mRNA for MUC5AC protein (tracheal), partial. | 0,039713 | -0,42807 |
| NM_009590 | amine oxidase, copper containing 2 (retina-specific) (AOC2), transcript variant 2, mRNA. | 0,039713 | 0,372385 |
| NM_002225 | isovaleryl Coenzyme A dehydrogenase (IVD), nuclear gene encoding mitochondrial protein, mRNA. | 0,039807 | -0,17344 |
| AK094441 | cDNA FLJ37122 fis, clone BRACE2022448. | 0,039807 | 0,309278 |
| NM_002827 | protein tyrosine phosphatase, non-receptor type 1 (PTPN1), mRNA. | 0,039807 | 0,167187 |
| NM_007035 | keratocan (KERA), mRNA. | 0,039807 | 0,313022 |
| NM_004508 | isopentenyl-diphosphate delta isomerase (IDI1), mRNA. | 0,039807 | 0,185147 |
| NM_145160 | mitogen-activated protein kinase kinase 5 (MAP2K5), transcript variant A, mRNA. | 0,039807 | -0,21378 |
| NM_000311 | prion protein (p27-30) (Creutzfeld-Jakob disease, Gerstmann-Strausler-Scheinker syndrome, fatal familial insomnia) (PRNP), mRNA. | 0,039807 | 0,204793 |
| NM_172002 | J-type co-chaperone HSC20 (HSC20), mRNA. | 0,039849 | -0,14898 |
| NM_017767 | solute carrier family 39 (zinc transporter), member 4 (SLC39A4), mRNA. | 0,039933 | 0,33716 |
| NM_018386 | hypothetical protein FLJ11305 (FLJ11305), mRNA. | 0,039941 | 0,20096 |
| AK001394 | cDNA FLJ10532 fis, clone NT2RP2001044. | 0,040018 | 0,207178 |
| BQ932364 | AGENCOURT_8854114 NIH_MGC_71 cDNA clone IMAGE:6473695 5', mRNA sequence. | 0,040054 | -0,20882 |
| NM_016948 | par-6 partitioning defective 6 H.log alpha (C.elegans) (PARD6A), mRNA. | 0,040054 | -0,17715 |
| NM_016569 | T-box 3 (ulnar mammary syndrome) (TBX3), transcript variant 2, mRNA. | 0,040054 | -0,33332 |
| NM_032579 | colon and small intestine-specific cysteine-rich protein precursor (HXCP2), mRNA. | 0,040147 | -0,78322 |
| NM_002657 | pleiomorphic adenoma gene-like 2 (PLAGL2), mRNA. | 0,040166 | 0,259643 |
| BE622355 | 601441142F1 NIH-MGC-72 cDNA clone IMAGE:3915971 5', mRNA sequence. | 0,040166 | -0,39107 |
| NM_145235 | similar to RIKEN cDNA 1700007B22 (LOC92565), mRNA. | 0,040937 | -0,3001 |
| NM_015099 | calmodulin binding transcription activator 2 (CAMTA2), mRNA. | 0,040937 | -0,35056 |
| NM₋172250 | methylmalonic aciduria type A (MMAA), mRNA. | 0,041121 | -0,22102 |
| AB029001 | mRNA for KIAA1078 protein, partial cds. | 0,041121 | 0,215228 |
| NM_000107 | damage-specific DNA binding protein 2, 48kDa (DDB2), mRNA. | 0,042056 | -0,20596 |
| AK026883 | cDNA: FLJ23230 fis, clone CAE07143. | 0,042416 | 0,243452 |
| BC038569 | clone IMAGE:4341708, mRNA. | 0,042416 | -0,48565 |
| NM_002570 | paired basic amino acid cleaving system 4 (PACE4), transcript variant 1, mRNA. | 0,042475 | -0,21394 |
| AK097981 | cDNA FLJ40662 fis, clone THYMU2020198, highly similar to Mus musculus faciogenital dysplasia protein 2 (Fgd2) mRNA. | 0,042475 | -0,19761 |
| NM_014905 | glutaminase (GLS), mRNA. | 0,042475 | 0,172472 |
| AK098482 | cDNA FLJ25616 fis, clone STM01779. | 0,042499 | 0,36903 |
| NM_002476 | myosin, light polypeptide 4, alkali atrial, embryonic (MYL4), mRNA. | 0,042499 | 0,205055 |
| NM_022088 | zinc finger protein 64 H.log (mouse) (ZFP64), mRNA. | 0,042618 | 0,211673 |
| BC035130 | clone IMAGE:5263749, mRNA. | 0,042635 | 0,268553 |
| NM_016354 | solute carrier family 21 (organic anion transporter), member 12 (SLC21A12), mRNA. | 0,042956 | 0,225776 |
| NM_005589 | aldehyde dehydrogenase 6 family, member A1 (ALDH6A1), nuclear gene encoding mitochondrial protein, mRNA. | 0,042956 | -0,17344 |
| NM_133367 | chromosome 6 open reading frame 33 (C6orf33), mRNA. | 0,042956 | -0,27696 |
| NM_032013 | NDRG family member 3 (NDRG3), mRNA. | 0,042973 | 0,152285 |
| NM_003291 | tripeptidyl peptidase II (TPP2), mRNA. | 0,042973 | 0,141957 |
| NM_000958 | prostaglandin E receptor 4 (subtype EP4) (PTGER4), mRNA. | 0,043212 | -0,22516 |
| AK021963 | cDNA FLJ11901 fis, clone HEMBA1007347. | 0,043212 | 0,249382 |
| NM_025247 | hypothetical protein MGC5601 (MGC5601), mRNA. | 0,043708 | 0,131305 |
| AK055652 | cDNA FLJ31090 fis, clone IMR321000102. | 0,043708 | 0,272011 |
| NM_016598 | zinc finger, DHHC domain containing 3 (ZDHHC3), mRNA. | 0,043708 | -0,13051 |
| BC010544 | clone IMAGE:3462401, mRNA, partial cds. | 0,043708 | 0,378366 |
| NM_001218 | carbonic anhydrase XII (CA12), mRNA. | 0,043708 | -0,23047 |
| NM_020372 | organic cation transporter (LOC57100), mRNA. | 0,043856 | 0,188354 |
| BF683837 | 602140129F1 NIH_MGC_46 cDNA clone IMAGE:4301287 5', mRNA sequence. | 0,043921 | -0,2505 |
| NM_022127 | solute carrier family 28 (sodium-coupled nucleoside transporter), member 3 (SLC28A3), mRNA. | 0,043921 | -0,37039 |
| AF111804 | MSTP023 (MST023) mRNA, complete cds. | 0,043921 | -0,25497 |
| NM_032709 | hypothetical protein MGC13047 (MGC13047), mRNA. | 0,043921 | -0,31906 |
| NM_130766 | skeletal muscle and kidney enriched inositol phosphatase (SKIP), transcript variant 2, mRNA. | 0,044459 | -0,20572 |
| NM_012325 | microtubule-associated protein, RP/EB family, member 1 (MAPRE1), mRNA. | 0,04498 | 0,223772 |
| BC036876 | clone IMAGE:5245135, mRNA. | 0,045702 | 0,257759 |
| BC032582 | similar to data source:SPTR, source key:Q18357, evidence:ISS~putative~related to C33A12.3 PROTEIN, clone MGC:45381 IMAGE:5494700, mRNA, complete cds. | 0,045702 | -0,21731 |
| AW968387 | EST380463 MAGE resequences, MAGJ cDNA, mRNA sequence. | 0,045702 | 0,259431 |
| AL831837 | mRNA, cDNA DKFZp547L2215 (from clone DKFZp547L2215). | 0,045745 | 0,264002 |
| NM_007342 | nucleoporin-like protein 1 (NLP_1), mRNA. | 0,045745 | 0,12047 |
| NM_145728 | desmuslin (DMN), transcript variant A, mRNA. | 0,045745 | 0,422901 |
| AK022426 | cDNA FLJ12364 fis, clone MAMMA1002384. | 0,045745 | -0,20569 |
| NM_052889 | CARD only protein (COP), mRNA. | 0,045745 | -0,18935 |
| NM_004001 | Fc fragment of IgG, low affinity IIb, receptor for (CD32) (FCGR2B), mRNA. | 0,045745 | 0,214078 |
| NM_133170 | protein tyrosine phosphatase, receptor type, T (PTPRT), transcript variant 1, mRNA. | 0,045745 | -0,30842 |
| AI291524 | qm84f08.x1 NCI_CGAP_Lu5 cDNA clone IMAGE: 1895463 3', mRNA sequence. | 0,045936 | -0,36473 |
| NM_004606 | TAF1 RNA polymerase II, TATA box binding protein (TBP)-associated factor, 250kDa (TAF1), transcript variant 1, mRNA. | 0,046757 | 0,181068 |
| NM_016029 | retinal short-chain dehydrogenase/reductase 4 (retSDR4), mRNA. | 0,046757 | -0,21314 |
| NM_003977 | aryl hydrocarbon receptor interacting protein (AIP), mRNA. | 0,046757 | 0,169957 |
| NM_080658 | aspartoacylase-3 (ACY-3), mRNA. | 0,046757 | -0,26083 |
| NM_000149 | fucosyltransferase 3 (galactoside 3(4)-L-fucosyltransferase, Lewis blood group included) (FUT3), mRNA. | 0,0469 | -0,43568 |
| NM_006377 | unc-13-like (C. elegans) (UNC13), mRNA. | 0,047389 | -0,25996 |
| NM_000900 | matrix Gla protein (MGP), mRNA. | 0,047679 | 0,265979 |
| NM_003998 | nuclear factor of kappa light polypeptide gene enhancer in B-cells 1 (p105) (NFKB1), mRNA. | 0,048139 | -0,17353 |
| BC002696 | Similar to heterogeneous nuclear ribonucleoprotein C (C1/C2), clone IMAGE:3608642, mRNA. | 0,048198 | 0,221145 |
| NM_000767 | cytochrome P450, subfamily IIB (phenobarbital-inducible), polypeptide 6 (CYP2B6), mRNA. | 0,048579 | 0,31483 |
| NM_018245 | hypothetical protein FLJ10851 (FLJ10851), mRNA. | 0,04867 | 0,193797 |
| BC037887 | Similar to hypothetical protein MGC36672, clone MGC:43900 IMAGE:5274706, mRNA, complete cds. | 0,048767 | 0,115209 |
| BC014362 | clone IMAGE:3932221, mRNA. | 0,049588 | 0,224049 |
| NM_014384 | acyl-Coenzyme A dehydrogenase family, member 8 (ACAD8), mRNA. | 0,049588 | -0,19906 |
| NM_003732 | eukaryotic translation initiation factor 4E binding protein 3 (EIF4EBP3), mRNA. | 0,049823 | -0,12559 |
| NM_002979 | sterol carrier protein 2 (SCP2), mRNA. | 0,050282 | -0,15034 |
| NM_019079 | hypothetical protein FLJ10884 (FLJ10884), mRNA. | 0,050282 | -0,48899 |
| AI432199 | tg77g09.x1 Soares_NhHMPu_S1 cDNA clone IMAGE:2114848 3', mRNA sequence. | 0,050282 | -0,18395 |
| NM_002345 | lumican (LUM), mRNA. | 0,050767 | 0,333592 |
| NM_001450 | four and a half LIM domains 2 (FHL2), mRNA. | 0,050816 | -0,24746 |
| AK000850 | cDNA FLJ20843 fis, clone ADKA01954. | 0,05177 | -0,39317 |
| NM_016484 | hypothetical protein LOC51248 (LOC51248), mRNA. | 0,05177 | 0,10943 |
| BC013942 | clone IMAGE:4044872, mRNA. | 0,05177 | -0,29374 |
| NM_020232 | hepatocellular carcinoma susceptibility protein (HCCA3), mRNA. | 0,052438 | -0,13085 |
| NM_003500 | acyl-Coenzyme A oxidase 2, branched chain (ACOX2), mRNA. | 0,052438 | -0,15457 |
| NM_001999 | fibrillin 2 (congenital contractural arachnodactyly) (FBN2), mRNA. | 0,052438 | 0,242282 |
| AK056609 | cDNA FLJ32047 fis, clone NTONG2001137. | 0,052553 | -0,21041 |
| NM_006811 | tumor differentially expressed 1(TDE1), mRNA. | 0,052698 | 0,170883 |
| NM_000720 | calcium channel, voltage-dependent, L type, alpha 1D subunit (CACNA1D), mRNA. | 0,052698 | 0,264575 |
| NM_024595 | hypothetical protein FLJ12666 (FLJ12666), mRNA. | 0,052698 | 0,100821 |
| NM_031420 | mitochondrial ribosomal protein L9 (MRPL9), nuclear gene encoding mitochondrial protein, mRNA. | 0,05272 | 0,122943 |
| AL137279 | mRNA, cDNA DKFZp434O1214 (from clone DKFZp434O1214). | 0,052915 | 0,254814 |
| NM_024101 | melanophilin (MLPH), mRNA. | 0,052945 | -0,3441 |
| NM_012384 | glucocorticoid modulatory element binding protein 2 (GMEB2), mRNA. | 0,052945 | 0,268879 |
| NM_153336 | hypothetical protein MGC35392 (MGC35392), mRNA. | 0,052945 | -0,23733 |
| NM_004816 | Friedreich ataxia region gene X123 (X123), mRNA. | 0,052945 | -0,36986 |
| NM_000698 | arachidonate 5-lipoxygenase (ALOX5), mRNA. | 0,05332 | -0,29991 |
| NM_021067 | KIAA0186 gene product (KIAA0186), mRNA. | 0,05332 | 0,29867 |
| NM_005415 | solute carrier family 20 (phosphate transporter), member 1 (SLC20A1), mRNA. | 0,05332 | 0,199915 |
| NM_032442 | G protein pathway suppressor 2 (GPS2), transcript variant 1, mRNA. | 0,053509 | -0,18127 |
| AK022745 | cDNA FLJ12683 fis, clone NT2RM4002457. | 0,05362 | 0,291644 |
| BM551183 | AGENCOURT_6545271 NIH_MGC_88 cDNA clone IMAGE:5738611 5', mRNA sequence. | 0,05362 | -0,54343 |
| NM_024593 | hypothetical protein FLJ11767 (FLJ11767), mRNA. | 0,05368 | -0,17954 |
| NM_018227 | hypothetical protein FLJ10808 (FLJ10808), mRNA. | 0,05368 | 0,180226 |
| Y16713 | mRNA from HIV associated non-Hodgkin's lymphoma (clone hl1-5). | 0,053915 | 0,263963 |
| AY040873 | C21orf57 isoform A protein (C21orf57) mRNA, partial cds, alternatively spliced. | 0,054371 | -0,22453 |
| AY006312 | clone 09npa41 T cell receptor beta chain mRNA, partial cds. | 0,054722 | -0,3437 |
| NM_000126 | electron-transfer-flavoprotein, alpha polypeptide (glutaric aciduria II) (ETFA), nuclear gene encoding mitochondrial protein, mRNA. | 0,054899 | -0,16374 |
| NM_014321 | origin recognition complex, subunit 6 H.log-like (yeast) (ORC6L), mRNA. | 0,054899 | 0,157622 |
| NM_003129 | squalene epoxidase (SQLE), mRNA. | 0,054899 | 0,220846 |
| NM_003655 | chromobox H.log 4 (Pc class H.log, Drosophila) (CBX4), mRNA. | 0,055056 | 0,169295 |
| NM_006282 | serine/threonine kinase 4 (STK4), mRNA. | 0,055229 | 0,326113 |
| BC034812 | Similar to HSPC182 protein, clone IMAGE:4825606, mRNA. | 0,05549 | 0,183791 |
| NM_025047 | hypothetical protein FLJ22595 (FLJ22595), mRNA. | 0,05549 | -0,18492 |
| NM_000161 | GTP cyclohydrolase 1 (dopa-responsive dystonia) (GCH1), mRNA. | 0,05549 | -0,15084 |
| BC020862 | clone IMAGE:4556692, mRNA. | 0,055696 | 0,553282 |
| NM_017542 | pogo transposable element with KRAB domain (POGK), mRNA. | 0,055696 | 0,148446 |
| NM_015369 | TP53TG3 protein (TP53TG3), mRNA. | 0,055867 | 0,143507 |
| NM_145284 | similar to hypothetical protein MGC17347 (LOC159090), mRNA. | 0,055867 | 0,219551 |
| NM_032181 | hypothetical protein FLJ13391 (FLJ13391), mRNA. | 0,055867 | 0,205933 |
| NM_152332 | chromosome 14 open reading frame 47 (C14orf47), mRNA. | 0,055867 | -0,19863 |
| AK022343 | cDNA FLJ12281 fis, clone MAMMA1001745. | 0,055867 | -0,1953 |
| NM_016299 | likely ortholog of mouse heat shock protein, 70 kDa 4 (HSP70-4), mRNA. | 0,055867 | 0,14589 |
| NM_033198 | phosphatidylinositol glycan, class S (PIGS), mRNA. | 0,055867 | -0,18236 |
| NM_005479 | frequently rearranged in advanced T-cell lymphomas (FRAT1), mRNA. | 0,056284 | -0,13499 |
| U58662 | mRNA upregulated during camptothecin-induced apoptosis of U937 cells. | 0,056284 | 0,44522 |
| NM_032591 | solute carrier family 9 (sodium/hydrogen exchanger), isoform 7 (SLC9A7), mRNA. | 0,056284 | -0,15822 |
| NM_013314 | B-cell linker (BLNK), mRNA. | 0,056318 | -0,17279 |
| NM_005224 | dead ringer-like 1 (Drosophila) (DRIL1), mRNA. | 0,056318 | 0,33645 |
| Z36818 | (xs166) mRNA, 400bp. | 0,056357 | 0,187585 |
| NM_018298 | mucolipin-3 (MCOLN3), mRNA. | 0,056774 | 0,208523 |
| NM_004496 | forkhead box A1 (FOXA1), mRNA. | 0,05704 | -0,22788 |
| AK093069 | cDNA FLJ35750 fis, clone TESTI2004539, weakly similar to adlican mRNA. | 0,057517 | 0,236889 |
| NM_004493 | hydroxyacyl-Coenzyme A dehydrogenase, type II (HADH2), mRNA. | 0,057641 | 0,125987 |
| BC040924 | clone MGC:50642 IMAGE:5767986, mRNA, complete cds. | 0,057799 | -0,35669 |
| BC039320 | clone IMAGE:5266755, mRNA. | 0,057824 | -0,30209 |
| NM_014735 | KIAA0215 gene product (KIAA0215), mRNA. | 0,058144 | 0,223283 |
| NM_002895 | retinoblastoma-like 1 (p107) (RBL1), mRNA. | 0,058144 | 0,18465 |
| NM_003478 | cullin 5 (CUL5), mRNA. | 0,058144 | -0,18019 |
| NM_025082 | hypothetical protein FLJ13111 (FLJ13111), mRNA. | 0,058144 | 0,14792 |
| NM_139312 | YME1-like 1 (S. cerevisiae) (YME1L1), nuclear gene encoding mitochondrial protein, transcript variant 1, mRNA. | 0,059117 | 0,13519 |
| NM_004164 | retinol binding protein 2, cellular (RBP2), mRNA. | 0,059148 | 0,251131 |
| BC039363 | clone IMAGE:5270672, mRNA. | 0,05921 | -0,29807 |
| NM_013402 | fatty acid desaturase 1 (FADS1), mRNA. | 0,059688 | 0,215917 |
| BQ055725 | AGENCOURT_6796360 NIH_MGC_99 cDNA clone IMAGE:5807946 5', mRNA sequence. | 0,060886 | -0,27551 |
| NM_016357 | epithelial protein lost in neoplasm beta (EPLIN), mRNA. | 0,061361 | -0,22316 |
| NM_023018 | NAD kinase (FLJ13052), mRNA. | 0,062491 | -0,14889 |
| NM_000880 | interleukin 7 (IL7), mRNA. | 0,062764 | -0,23231 |
| NM_138969 | retinal short chain dehydrogenase reductase (RDH-E2), mRNA. | 0,062843 | -0,39108 |
| NM_022154 | BCG-induced gene in monocytes, clone 103 (BIGM103), mRNA. | 0,062843 | -0,21219 |
| BC003122 | clone IMAGE:3357127, mRNA, partial cds. | 0,063027 | 0,25271 |
| NM_022082 | chromosome 20 open reading frame 59 (C20orf59), mRNA. | 0,063027 | 0,31222 |
| NM_021738 | supervillin (SVIL), transcript variant 2, mRNA. | 0,063027 | -0,11199 |
| NM_014055 | carnitine deficiency-associated gene expressed in ventricle 1 (CDV-1), mRNA. | 0,063291 | 0,197908 |
| NM_153751 | chromosome 21 open reading frame 82 (C21ort82), mRNA. | 0,063623 | -0,25359 |
| NM_004107 | Fc fragment of IgG, receptor, transporter, alpha (FCGRT), mRNA. | 0,06368 | 0,237504 |
| NM_018246 | hypothetical protein FLJ10853 (FLJ10853), mRNA. | 0,06368 | -0,14981 |
| U91153 | T-cell receptor delta chain (TCRDV1J2) mRNA, partial cds. | 0,063693 | -0,2968 |
| NM_002623 | prefoldin 4 (PFDN4), mRNA. | 0,063697 | 0,195181 |
| NM_139016 | hypothetical gene LOC128439 (LOC128439), mRNA. | 0,063697 | 0,211135 |
| BC016787 | clone IMAGE:4064953, mRNA. | 0,063697 | -0,24544 |
| AK096002 | cDNA FLJ38683 fis, clone KIDNE2000777. | 0,063697 | -0,6784 |
| NM_032487 | actin related protein M1 (ARPM1), mRNA. | 0,063697 | 0,130603 |
| AK097488 | cDNA FLJ40169 fis, clone TESTI2016583. | 0,063697 | 0,192104 |
| NM_003143 | single-stranded DNA binding protein (SSBP1), mRNA. | 0,063697 | 0,097306 |
| AK093065 | cDNA FLJ35746 fis, clone TESTI2004399, moderately similar to APOMUCIN. | 0,063697 | -0,21135 |
| NM_024561 | hypothetical protein FLJ22054 (FLJ22054), mRNA. | 0,063697 | 0,202144 |
| AB037809 | mRNA for KIAA1388 protein, partial cds. | 0,063697 | -0,17468 |
| NM_001455 | forkhead box 03A(FOX03A), mRNA. | 0,063697 | 0,140705 |
| NM_001807 | carboxyl ester lipase (bile salt-stimulated lipase) (CEL), mRNA. | 0,063922 | 0,294631 |
| NM_017898 | hypothetical protein FLJ20605 (FLJ20605), mRNA. | 0,063922 | 0,249514 |
| AK055694 | cDNA FLJ31132 fis, clone IMR322000953. | 0,063922 | 0,316206 |
| AB023156 | mRNA for KIAA0939 protein, partial cds. | 0,063922 | 0,127129 |
| NM_020422 | hypothetical protein from clone 24796 (LOC57146), mRNA. | 0,063922 | -0,20346 |
| NM_014471 | serine protease inhibitor, Kazal type 4 (SPINK4), mRNA. | 0,06447 | -0,37202 |
| BC031244 | dual-specificity tyrosine-(Y)-phosphorylation regulated kinase 4, clone MGC:39640 IMAGE:5266287, mRNA, complete cds. | 0,06447 | -0,24158 |
| NM_002435 | mannose phosphate isomerase (MPI), mRNA. | 0,06447 | -0,20337 |
| AW975332 | EST387440 MAGE resequences, MAGN cDNA, mRNA sequence. | 0,06447 | -0,39613 |
| NM_024041 | hypothetical protein MGC3180 (MGC3180), mRNA. | 0,06447 | 0,13974 |
| NM_024010 | 5-methyltetrahydrofolate-H.cysteine methyltransferase reductase (MTRR), transcript variant 2, mRNA. | 0,064622 | 0,163187 |
| NM_002655 | pleiomorphic adenoma gene 1 (PLAG1), mRNA. | 0,064794 | 0,413313 |
| AB011250 | mRNA for T cell receptor V beta14-D-J, partial cds. | 0,064824 | -0,25105 |
| AF288405 | G protein interaction factor 1-like mRNA sequence. | 0,065091 | 0,315483 |
| AB033025 | mRNA for KIAA1199 protein, partial cds. | 0,065091 | -0,3105 |
| BC030091 | clone IMAGE:4793171, mRNA. | 0,065428 | -0,22288 |
| NM_002079 | glutamic-oxaloacetic transaminase 1, soluble (aspartate aminotransferase 1) (GOT1), mRNA. | 0,065727 | -0,13124 |
| NM_003742 | ATP-binding cassette, sub-family B (MDR/TAP), member 11 (ABCB11), mRNA. | 0,065727 | -0,26287 |
| BC027919 | clone IMAGE:5226180, mRNA. | 0,065926 | 0,380672 |
| AB002339 | mRNA for KIAA0341 gene, partial cds. | 0,065926 | -0,18872 |
| NM_153367 | hypothetical protein FLJ90798 (FLJ90798), mRNA. | 0,066058 | 0,328357 |
| BC011002 | clone IMAGE:3946502, mRNA, partial cds. | 0,066272 | -0,27545 |
| NM_017787 | hypothetical protein FLJ20154 (FLJ20154), mRNA. | 0,066703 | -0,14052 |
| AK021881 | cDNA FLJ11819 fis, clone HEMBA1006426. | 0,066703 | 0,355501 |
| NM_018302 | hypothetical protein FLJ11017 (FLJ11017), mRNA. | 0,066703 | -0,36098 |
| NM_022766 | ceramide kinase (CERK), mRNA. | 0,067252 | 0,211298 |
| NM_000383 | autoimmune regulator (automimmune polyendocrinopathy candidiasis ectodermal dystrophy) (AIRE), transcript variant AIRE-1, mRNA. | 0,067441 | 0,282071 |
| NM_021727 | fatty acid desaturase 3 (FADS3), mRNA. | 0,067441 | 0,193475 |
| NM_014934 | zinc-finger protein DZIP1 (DZIP1), mRNA. | 0,067441 | 0,113776 |
| AF229803 | endozepine-like protein type 1 mutant mRNA, complete cds. | 0,067749 | -0,22423 |
| BC018312 | clone IMAGE:4153775, mRNA. | 0,068313 | -0,20871 |
| NM_005721 | ARP3 actin-related protein 3 H.log (yeast) (ACTR3), mRNA. | 0,0689 | 0,107506 |
| AK093028 | cDNA FLJ35709 fis, clone SPLEN2020512. | 0,068911 | 0,275535 |
| NM_004255 | cytochrome c oxidase subunit Va (COX5A), nuclear gene encoding mitochondrial protein, mRNA. | 0,068911 | -0,15969 |
| NM_002148 | homeo box D10 (HOXD10), mRNA. | 0,06928 | 0,414467 |
| NM_004202 | thymosin, beta 4, Y chromosome (TMSB4Y), mRNA. | 0,069653 | -0,30904 |
| BQ646410 | AGENCOURT_8511770 NIH_MGC_100 cDNA clone IMAGE:6296949 5', mRNA sequence. | 0,069733 | 0,61722 |
| AK025511 | cDNA: FLJ21858 fis, clone HEP02301. | 0,070013 | 0,158336 |
| NM_019888 | melanocortin 3 receptor (MC3R), mRNA. | 0,070013 | -0,22795 |
| AF147414 | full length insert cDNA clone YP60H04. | 0,070013 | 0,222292 |
| NM_006454 | MAX dimerization protein 4 (MXD4), mRNA. | 0,070013 | -0,155 |
| NM_002466 | v-myb myeloblastosis viral oncogene H.log (avian)-like 2 (MYBL2), mRNA. | 0,070124 | 0,372111 |
| AK074150 | mRNA for FLJ00223 protein. | 0,070325 | -0,16585 |
| AK097563 | cDNA FLJ40244 fis, clone TESTI2023951, moderately similar to Ubiquitin specific protease 6. | 0,070692 | -0,30429 |
| BC000885 | clone MGC:5619 IMAGE:3462332, mRNA, complete cds. | 0,0708 | -0,20226 |
| AV726231 | AV726231 HTC cDNA clone HTCAOH09 5', mRNA sequence. | 0,0708 | -0,33475 |
| AK091220 | cDNA FLJ33901 fis, clone CTONG2008321, highly similar to HISTONE H2B F. | 0,070837 | 0,199733 |
| NM_016472 | hypothetical protein HSPC210 (HSPC210), mRNA. | 0,070837 | -0,20875 |
| NM_024692 | hypothetical protein FLJ21069 (FLJ21069), mRNA. | 0,070837 | 0,214351 |
| BE502147 | hy13c02.x1 NCI_CGAP_GC6 cDNA clone IMAGE:3197186 3', mRNA sequence. | 0,070837 | -0,38178 |
| AK026378 | cDNA: FLJ22725 fis, clone HSI14917. | 0,070837 | -0,58821 |
| BC025999 | clone IMAGE:4291396, mRNA. | 0,07091 | 0,205068 |
| NM_005135 | solute carrier family 12 (potassium/chloride transporters), member 6 (SLC12A6), mRNA. | 0,071586 | -0,12351 |
| BQ921112 | AGENCOURT_8926187 NIH_MGC_101 cDNA clone IMAGE:6463013 5', mRNA sequence. | 0,071763 | 0,312261 |
| BC029776 | similar to CG12393 gene product, clone IMAGE:5188623, mRNA, partial cds. | 0,07184 | -0,2622 |
| AB037842 | mRNA for KIAA1421 protein, partial cds. | 0,072155 | -0,23954 |
| NM_014587 | SRY (sex determining region Y)-box 8 (SOX8), mRNA. | 0,0726 | 0,345598 |
| BC034459 | clone IMAGE:5185460, mRNA. | 0,072826 | -0,21496 |
| NM_006721 | adenosine kinase (ADK), transcript variant ADK-long, mRNA. | 0,072919 | 0,106291 |
| NM_016463 | hypothetical protein HSPC195 (HSPC195), mRNA. | 0,073054 | 0,175255 |
| AK096306 | cDNA FLJ38987 fis, clone NT2RI2005818. | 0,073091 | 0,228787 |
| NM_145293 | similar to hypothetical protein FLJ20897 (LOC196549), mRNA. | 0,073114 | 0,114053 |
| NM_018360 | chromosome X open reading frame 15 (CXorfl5), mRNA. | 0,073275 | 0,210295 |
| NM_025206 | fer-1-like 4 (C. elegans) (FER1L4), mRNA. | 0,07339 | 0,195812 |
| NM_013279 | chromosome 11 open reading frame 9 (C11orf9), mRNA. | 0,073526 | -0,25629 |
| AK021733 | cDNA FLJ11671 fis, clone HEMBA1004730. | 0,073745 | 0,233873 |
| BU567822 | AGENCOURT_10398839 NIH_MGC_82 cDNA clone IMAGE:6614524 5', mRNA sequence. | 0,074029 | -0,25351 |
| NM_006668 | cytochrome P450, subfamily 46 (cholesterol 24-hydroxylase) (CYP46), mRNA. | 0,074029 | -0,23363 |
| NM_025051 | hypothetical protein FLJ23022 (FLJ23022), mRNA. | 0,074384 | -0,26598 |
| NM_014714 | KIAA0590 gene product (KIAA0590), mRNA. | 0,07479 | -0,17485 |
| NM_018206 | vacuolar protein sorting 35 (yeast) (VPS35), mRNA. | 0,07479 | 0,104322 |
| NM_022101 | hypothetical protein FLJ22965 (FLJ22965), mRNA. | 0,075006 | 0,130346 |
| BC035372 | clone MGC:35266 IMAGE:5174235, mRNA, complete cds. | 0,075061 | -0,2973 |
| NM_001910 | cathepsin E (CTSE), transcript variant 1, mRNA. | 0,075061 | -0,45924 |
| BC038767 | clone IMAGE:5270478, mRNA. | 0,075213 | -0,36496 |
| NM_057158 | dual specificity phosphatase 4 (DUSP4), transcript variant 2, mRNA. | 0,075244 | -0,22098 |
| BC020844 | clone MGC:23971 IMAGE:4714217, mRNA, complete cds. | 0,075708 | -0,17112 |
| NM_133373 | similar to phospholipase C, delta (LOC113026), mRNA. | 0,076165 | -0,21763 |
| NM_005128 | chromosome 21 open reading frame 5 (C21orf5), mRNA. | 0,076313 | -0,22382 |
| NM_004394 | death-associated protein (DAP), mRNA. | 0,076313 | -0,15565 |
| NM_080752 | chromosome 20 open reading frame 164 (C20orf164), mRNA. | 0,076388 | 0,203084 |
| AF088051 | full length insert cDNA clone ZD63G05. | 0,0767 | -0,17243 |
| AK026418 | cDNA: FLJ22765 fis, clone KAIA1180. | 0,076954 | 0,259588 |
| NM_014489 | FGF receptor activating protein 1 (FRAG1), mRNA. | 0,077447 | -0,14138 |
| BC020896 | clone IMAGE:4710650, mRNA. | 0,077606 | -0,55274 |
| NM_016183 | chromosome 1 open reading frame 33 (C1orf33), mRNA. | 0,077697 | 0,104503 |
| NM_005944 | antigen identified by monoclonal antibody MRC OX-2 (MOX2), mRNA. | 0,078387 | -0,18431 |
| NM_004403 | deafness, autosomal dominant 5 (DFNA5), mRNA. | 0,078387 | 0,193986 |
| NM_033292 | caspase 1, apoptosis-related cysteine protease (interleukin 1, beta, convertase) (CASP1), transcript variant alpha, mRNA. | 0,078387 | -0,34641 |
| AF147436 | full length insert cDNA clone YP87B10. | 0,078387 | -0,22072 |
| NM_012401 | plexin B2 (PLXNB2), mRNA. | 0,078387 | -0,13448 |
| NM_130810 | EKN1 (EKN1), mRNA. | 0,078387 | -0,31601 |
| NM_144658 | hypothetical protein FLJ32122 (FLJ32122), mRNA. | 0,078859 | 0,153119 |
| NM_001085 | serine (or cysteine) proteinase inhibitor, clade A (alpha-1 antiproteinase, antitrypsin), member 3 (SERPINA3), mRNA. | 0,078863 | -0,39441 |
| NM_018043 | hypothetical protein FLJ10261 (FLJ10261), mRNA. | 0,078863 | -0,34572 |
| AK058068 | cDNA FLJ25339 fis, clone TST00959. | 0,078863 | 0,215292 |
| NM_022045 | Mdm2, transformed 3T3 cell double minute 2, p53 binding protein (mouse) binding protein, 104kDa (MTBP), mRNA. | 0,079107 | 0,251406 |
| NM_030925 | hypothetical protein FLJ12577 (FLJ12577), mRNA. | 0,079318 | 0,347034 |
| NM_024556 | hypothetical protein FLJ21103 (FLJ21103), mRNA. | 0,080434 | -0,1579 |
| AK025793 | cDNA: FLJ22140 fis, clone HEP20977. | 0,080434 | -0,28128 |
| NM_024123 | lymphocyte antigen 6 complex, locus G6E (LY6G6E), mRNA. | 0,080698 | 0,21583 |
| NM_024426 | Wilms tumor 1 (WT1), transcript variant D, mRNA. | 0,08101 | 0,511174 |
| NM_001313 | collapsin response mediator protein 1 (CRMP1), mRNA. | 0,081105 | 0,152517 |
| NM_005900 | MAD, mothers against decapentaplegic H.log 1 (Drosophila) (MADH1), mRNA. | 0,081105 | -0,17975 |
| NM_022445 | thiamin pyrophosphokinase 1 (TPK1), mRNA. | 0,081334 | -0,2798 |
| AB013384 | mRNA for HIP1R, complete cds. | 0,081334 | -0,17225 |
| BC040982 | clone IMAGE:4798675, mRNA. | 0,08137 | -0,1738 |
| BC035660 | clone IMAGE:5721900, mRNA. | 0,081442 | 0,420216 |
| NM_014080 | dual oxidase 2 (DUOX2), mRNA. | 0,081442 | -0,49211 |
| NM_004092 | enoyl Coenzyme A hydratase, short chain, 1, mitochondrial (ECHS1), nuclear gene encoding mitochondrial protein, mRNA. | 0,081442 | -0,13245 |
| NM_002874 | RAD23 H.log B (S. cerevisiae) (RAD23B), mRNA. | 0,081442 | 0,115098 |
| NM_001800 | cyclin-dependent kinase inhibitor 2D (p19, inhibits CDK4) (CDKN2D), transcript variant 1, mRNA. | 0,081442 | 0,175601 |
| BI789172 | ie52h09.xl Melton Normalized Human Islet 4 N4-HIS 1 cDNA clone IMAGE:5670761 3', mRNA sequence. | 0,081442 | -0,25886 |
| AF264629 | uncharacterized gastric protein ZA43P mRNA, partial cds. | 0,081442 | 0,222508 |
| NM_024908 | hypothetical protein FLJ12973 (FLJ12973), mRNA. | 0,081442 | -0,24063 |
| NM_016084 | RAS, dexamethasone-induced 1 (RASD1), mRNA. | 0,081443 | -0,30879 |
| NM_005263 | growth factor independent 1 (GFI1), mRNA. | 0,08166 | -0,21716 |
| AK024346 | cDNA FLJ14284 fis, clone PLACE1005898. | 0,08192 | -0,27611 |
| NM_000274 | ornithine aminotransferase (gyrate atrophy) (OAT), nuclear gene encoding mitochondrial protein, mRNA. | 0,082401 | 0,275897 |
| M64247 | cardiac troponin I mRNA, complete cds. | 0,082401 | 0,314683 |
| NM_004925 | aquaporin 3 (AQP3), mRNA. | 0,082401 | -0,33953 |
| NM_015926 | putative secreted protein ZSIG11 (ZSIG11), mRNA. | 0,082401 | -0,16468 |
| AL137442 | mRNA, cDNA DKFZp566C114 (from clone DKFZp566C114) partial cds. | 0,082604 | 0,274811 |
| AF116695 | PRO2221 mRNA, complete cds. | 0,082735 | 0,302746 |
| AK096129 | cDNA FLJ38810 fis, clone LIVER2006251. | 0,082877 | 0,208516 |
| AL834283 | mRNA, cDNA DKFZp547N0315 (from clone DKFZp547N0315). | 0,083016 | 0,181979 |
| NM_006825 | cytoskeleton-associated protein 4 (CKAP4), mRNA. | 0,08318 | -0,1303 |
| BC038356 | clone IMAGE:4825207, mRNA. | 0,08334 | -0,21392 |
| NM_003850 | succinate-CoA ligase, ADP-forming, beta subunit (SUCLA2), mRNA. | 0,08334 | 0,134511 |
| NM_032661 | hypothetical protein MGC5139 (MGC5139), mRNA. | 0,08334 | -0,15739 |
| BC014162 | clone IMAGE:4547814, mRNA. | 0,08334 | 0,318786 |
| BG107100 | 602291004F1 NIH_MGC_85 cDNA clone IMAGE:4385712 5', mRNA sequence. | 0,08334 | 0,224361 |
| AK093715 | cDNA FLJ36396 fis, clone THYMU2009526. | 0,08334 | -0,1926 |
| NM_006259 | protein kinase, cGMP-dependent, type II (PRKG2), mRNA. | 0,08334 | -0,22058 |
| AK021913 | cDNA FLJ11851 fis, clone HEMBA1006744. | 0,08334 | -0,1134 |
| NM_153425 | TNFRSF1A-associated via death domain (TRADD), transcript variant 2, mRNA. | 0,083859 | -0,10067 |
| BC015142 | clone IMAGE:4043205, mRNA. | 0,083859 | 0,268352 |
| AL833020 | mRNA, cDNA DKFZp666C238 (from clone DKFZp666C238). | 0,084035 | 0,276271 |
| AF258564 | PP3781 mRNA, complete cds. | 0,084193 | 0,497796 |
| AF339822 | clone IMAGE:451939, mRNA sequence. | 0,084273 | 0,20404 |
| NM_003336 | ubiquitin-conjugating enzyme E2A (RAD6 H.log) (UBE2A), mRNA. | 0,084273 | 0,121124 |
| NM_032045 | kringle containing transmembrane protein 1 (KREMEN1), transcript variant 2, mRNA. | 0,084273 | -0,25929 |
| NM_030674 | solute carrier family 38, member 1 (SLC38A1), mRNA. | 0,084992 | 0,171705 |
| NM_005768 | putative protein similar to nessy (Drosophila) (C3F), mRNA. | 0,084992 | -0,17362 |
| NM_018379 | hypothetical protein FLJ11280 (FLJ11280), mRNA. | 0,084992 | -0,13727 |
| AF070612 | clone 24771 mRNA sequence. | 0,084992 | -0,21798 |
| NM_004808 | N-myristoyltransferase 2 (NMT2), mRNA. | 0,084992 | 0,157143 |
| BC037919 | clone IMAGE:5278089, mRNA. | 0,085036 | -0,36831 |
| BC033546 | clone IMAGE:5163906, mRNA. | 0,085134 | -0,43665 |
| NM_004221 | natural killer cell transcript 4 (NK4), mRNA. | 0,085745 | 0,201959 |
| NM_015057 | KIAA0916 protein (KIAA0916), mRNA. | 0,085993 | 0,157345 |
| AK000144 | cDNA FLJ20137 fis, clone COL07137. | 0,085993 | -0,29644 |
| NM_006150 | LIM domain only 6 (LMO6), mRNA. | 0,086383 | 0,157022 |
| NM_139033 | mitogen-activated protein kinase 7 (MAPK7), transcript variant 1, mRNA. | 0,086383 | -0,13906 |
| NM_138455 | collagen triple helix repeat containing 1 (CTHRC1), mRNA. | 0,086452 | 0,44781 |
| NM_024552 | hypothetical protein FLJ12089 (FLJ12089), mRNA. | 0,086452 | 0,345846 |
| NM_000321 | retinoblastoma 1 (including osteosarcoma) (RB1), mRNA. | 0,086452 | 0,21893 |
| BC039513 | clone IMAGE:5580856, mRNA. | 0,086606 | 0,220768 |
| AB044555 | mRNA for PAR-6 beta, complete cds. | 0,086893 | 0,147086 |
| NM_002157 | heat shock 10kDa protein 1 (chaperonin 10) (HSPE1), mRNA. | 0,08732 | 0,133269 |
| NM_006175 | nebulin-related anchoring protein (NRAP), mRNA. | 0,08732 | -0,24442 |
| AK096053 | cDNA FLJ38734 fis, clone KIDNE2010863. | 0,087551 | 0,184234 |
| NM_014849 | synaptic vesicle glycoprotein 2 (SV2), mRNA. | 0,087756 | 0,120051 |
| NM_005618 | delta-like 1 (Drosophila) (DLL1), mRNA. | 0,087756 | -0,19063 |
| NM_020182 | transmembrane, prostate androgen induced RNA (TMEPAI), mRNA. | 0,087756 | 0,281365 |
| NM_152400 | hypothetical protein FLJ39370 (FLJ39370), mRNA. | 0,087756 | -0,18205 |
| AK055903 | cDNA FLJ31341 fis, clone MESAN1000050. | 0,087756 | -0,31828 |
| NM_032483 | HTPAP protein (HTPAP), mRNA. | 0,087963 | -0,15815 |
| AK090706 | cDNA FLJ33387 fis, clone BRACE2006636. | 0,087963 | -0,29997 |
| AF130090 | clone FLB9530 PR02574 mRNA, complete cds. | 0,087963 | 0,404278 |
| BC036799 | clone IMAGE:5590162, mRNA. | 0,087963 | -0,20596 |
| AK091562 | cDNA FLJ34243 fis, clone FCBBF3028593, highly similar to ZINC FINGER PROTEIN ZFP-1. | 0,087967 | 0,170231 |
| NM_007048 | butyrophilin, subfamily 3, member A1 (BTN3A1), mRNA. | 0,087967 | -0,19681 |
| NM_003496 | transformation/transcription domain-associated protein (TRRAP), mRNA. | 0,088048 | -0,17804 |
| NM_022658 | homeo box C8 (HOXCB), mRNA. | 0,088244 | 0,177866 |
| NM_002023 | fibromodulin (FMOD), mRNA. | 0,088474 | -0,27322 |
| NM_020865 | DEAD/H (Asp-Glu-Ala-Asp/His) box polypeptide 36 (DDX36), mRNA. | 0,088831 | 0,130167 |
| NM_014350 | TNF-induced protein (GG2-1), mRNA. | 0,089078 | -0,17898 |
| AF086476 | full length insert cDNA clone ZD88F12. | 0,089078 | 0,27836 |
| NM_003155 | stanniocalcin 1 (STC1), mRNA. | 0,089078 | 0,280653 |
| NM_002389 | membrane cofactor protein (CD46, trophoblast-lymphocyte cross-reactive antigen) (MCP), transcript variant a, mRNA. | 0,089248 | 0,18632 |
| NM_003635 | N-deacetylase/N-sulfotransferase (heparan glucosaminyl) 2 (NDST2), mRNA. | 0,089646 | 0,149154 |
| AF344194 | clone 423 active transcribed Alu sequence. | 0,089652 | -0,15984 |
| AJ292079 | partial mRNA for MUC5AC protein (mucin gene, MUC5AC). | 0,089795 | -0,97009 |
| NM_014747 | KIAA0237 gene product (KIAA0237), mRNA. | 0,089874 | -0,22134 |
| NM_002447 | macrophage stimulating 1 receptor (c-met-related tyrosine kinase) (MST1R), mRNA. | 0,089948 | -0,20784 |
| NM_013261 | peroxisome proliferative activated receptor, gamma, coactivator 1 (PPARGC1), mRNA. | 0,08999 | -0,18143 |
| AK098381 | cDNA FLJ25515 fis, clone CBR06479. | 0,090079 | -0,10508 |
| NM_016129 | COP9 constitutive photomorphogenic H.log subunit 4 (Arabidopsis) (COPS4), mRNA. | 0,090079 | -0,09671 |
| NM_139286 | CDC26 subunit of anaphase promoting complex (CDC26), mRNA. | 0,090095 | -0,15937 |
| AK093095 | cDNA FLJ35776 fis, clone TESTI2005326. | 0,090875 | -0,18112 |
| AK023472 | cDNA FLJ13410 fis, clone PLACE1001720. | 0,09119 | -0,26007 |
| AK094659 | cDNA FLJ37340 fis, clone BRAMY2020574. | 0,09119 | -0,23842 |
| NM_020357 | PEST-containing nuclear protein (PCNP), mRNA. | 0,09119 | 0,114111 |
| D86961 | mRNA for KIAA0206 gene, partial cds. | 0,09119 | -0,18452 |
| NM_022450 | likely ortholog of mouse epidermal growth factor receptor, related sequence (EGFR-RS), mRNA. | 0,091634 | 0,129786 |
| BG206080 | RST25515 Athersys RAGE Library cDNA, mRNA sequence. | 0,091867 | -0,32668 |
| NM_015420 | DKFZP56400463 protein (DKFZP564O0463), mRNA. | 0,092018 | 0,146258 |
| NM_014077 | DKFZP586O0120 protein (DKFZP586O0120), mRNA. | 0,092097 | 0,116794 |
| NM_004294 | mitochondrial translational release factor 1 (MTRF1), mRNA. | 0,092541 | 0,133047 |
| NM_000862 | hydroxy-delta-5-steroid dehydrogenase, 3 beta- and steroid delta-isomerase 1 (HSD3B1), mRNA. | 0,092742 | 0,143905 |
| NM_003289 | tropomyosin 2 (beta) (TPM2), mRNA. | 0,092916 | 0,256793 |
| BM908191 | AGENCOURT_6707396 NIH_MGC_119 cDNA clone IMAGE:5745241 5', mRNA sequence. | 0,093098 | -0,37837 |
| NM_173168 | SPCX (SPCX), mRNA. | 0,093205 | 0,207812 |
| NM_032139 | hypothetical protein DKFZp434L0718 (DKFZP434L0718), mRNA. | 0,093274 | 0,175473 |
| NM_014298 | quinolinate phosphoribosyltransferase (nicotinate-nucleotide pyrophosphorylase (carboxylating)) (QPRT), mRNA. | 0,093274 | 0,26915 |
| AB046863 | mRNA for KIAA1643 protein, partial cds. | 0,093274 | 0,174842 |
| NM_006579 | emopamil binding protein (sterol isomerase) (EBP), mRNA. | 0,09343 | 0,147465 |
| NM_005327 | L-3-hydroxyacyl-Coenzyme A dehydrogenase, short chain (HADHSC), mRNA. | 0,093457 | -0,19638 |
| NM_003266 | toll-like receptor 4 (TLR4), transcript variant 3, mRNA. | 0,093457 | -0,17937 |
| BC029797 | clone IMAGE:5196511, mRNA. | 0,093486 | -0,18475 |
| AF119900 | PRO2822 mRNA, complete cds. | 0,094031 | 0,16485 |
| NM_003522 | H2B histone family, member G (H2BFG), mRNA. | 0,094031 | 0,185873 |
| NM_007283 | monoglyceride lipase (MGLL), mRNA. | 0,094536 | -0,18844 |
| NM_001920 | decorin (DCN), transcript variant A1, mRNA. | 0,094536 | 0,289786 |
| BM804045 | AGENCOURT_6460684 NIH_MGC_88 cDNA clone IMAGE:5561240 5', mRNA sequence. | 0,094889 | 0,238679 |
| AL833743 | mRNA, cDNA DKFZp666E036 (from clone DKFZp666E036). | 0,095197 | 0,279238 |
| NM_000607 | orosomucoid 1 (ORM1), mRNA. | 0,095197 | 0,515915 |
| NM_032857 | lactamase, beta (LACTB), transcript variant 1, nuclear gene encoding mitochondrial protein, mRNA. | 0,095306 | -0,18893 |
| NM_005908 | mannosidase, beta A, lysosomal (MANBA), mRNA. | 0,095306 | -0,17218 |
| NM_152368 | hypothetical protein MGC9629 (MGC9629), mRNA. | 0,09556 | 0,158407 |
| AK097081 | cDNA FLJ39762 fis, clone SPLEN1000156. | 0,095575 | 0,229516 |
| AK000671 | cDNA FLJ20664 fis, clone KAIA795. | 0,095575 | -0,28289 |
| L10404 | surfactant protein B-binding protein mRNA, complete cds. | 0,095672 | -0,23267 |
| NM_015446 | ELYS transcription factor-like protein TMBS62 (elys), mRNA. | 0,095672 | 0,133758 |
| NM_018662 | disrupted in schizophrenia 1 (DISC1), mRNA. | 0,095672 | -0,34094 |
| BG774620 | 602662836F1 NIH_MGC_21 cDNA clone IMAGE:4811193 5', mRNA sequence. | 0,095739 | 0,104531 |
| NM_019067 | hypothetical protein FLJ10613 (FLJ10613), mRNA. | 0,096101 | 0,132872 |
| AF058804 | clone G4-10-3 mucin 4 (MUC4) mRNA, partial cds. | 0,09618 | -0,4132 |
| NM_021182 | minor histocompatibility antigen HB-1 (HB-1), mRNA. | 0,096794 | 0,224357 |
| NM_020233 | x 006 protein (MDS006), mRNA. | 0,097063 | -0,2374 |
| NM_016940 | chromosome 21 open reading frame 6 (C21orf6), mRNA. | 0,097245 | -0,15326 |
| AK092373 | cDNA FLJ35054 fis, clone OCBBF2018380. | 0,097265 | -0,16439 |
| NM_001550 | interferon-related developmental regulator 1 (IFRD1), mRNA. | 0,097485 | 0,12646 |
| NM_030792 | hypothetical protein PP1665 (PP1665), mRNA. | 0,09786 | 0,20522 |
| AK024511 | cDNA: FLJ20858 fis, clone ADKA01561. | 0,09786 | -0,23219 |
| NM_032313 | hypothetical protein MGC3232 (MGC3232), mRNA. | 0,09786 | -0,16 |
| NM_005141 | fibrinogen, B beta polypeptide (FGB), mRNA. | 0,097942 | -0,57243 |
| AB062438 | mRNA for OK/SW-CL.30, complete cds. | 0,097942 | 0,495277 |
| NM_005759 | abl-interactor 2 (ABI-2), mRNA. | 0,097942 | 0,14493 |
| NM_053012 | hypothetical protein LOC114137 (LOC114137), mRNA. | 0,097942 | -0,18464 |
| NM_002511 | neuromedin B receptor (NMBR), mRNA. | 0,097942 | -0,16282 |
| NM_004479 | fucosyltransferase 7 (alpha (1,3) fucosyltransferase) (FUT7), mRNA. | 0,097942 | -0,23954 |
| AW842526 | MR2-CN0035-170300-201-a06 CN0035 cDNA, mRNA sequence. | 0,098061 | -0,4273 |
| AK025522 | cDNA: FLJ21869 fis, clone HEP02442. | 0,098061 | 0,237754 |
| AK024962 | cDNA: FLJ21309 fis, clone COL02152. | 0,098075 | -0,24916 |
| NM_006030 | calcium channel, voltage-dependent, alpha 2/delta subunit 2 (CACNA2D2), mRNA. | 0,098075 | -0,44673 |
| BG208226 | RST27718 Athersys RAGE Library cDNA, mRNA sequence. | 0,098075 | 0,152109 |
| AL110290 | EST from clone 76558, 5' end. | 0,098075 | -0,20695 |
| AF086181 | full length insert cDNA clone ZB97E03. | 0,098075 | -0,25429 |
| NM_004453 | electron-transferring-flavoprotein dehydrogenase (ETFDH), nuclear gene encoding mitochondrial protein, mRNA. | 0,098368 | -0,16337 |
| NM_001692 | ATPase, H+ transporting, lysosomal 56/58kDa, VI subunit B, isoform 1 (Renal tubular acidosis with deafness) (ATP6V1B1), mRNA. | 0,098646 | -0,1475 |
| AK000778 | cDNA FLJ20771 fis, clone COL06394. | 0,098646 | -0,2914 |
| AF085925 | full length insert cDNA clone YR48C03. | 0,098646 | 0,249705 |
| BC017347 | clone MGC:29484 IMAGE:5014808, mRNA, complete cds. | 0,098646 | 0,234165 |
| NM_031216 | sec13-like protein (SEC13L), mRNA. | 0,098646 | -0,14614 |
| AF506819 | URB mRNA, complete cds. | 0,099548 | 0,32602 |
| NM_023036 | dynein, axonemal, intermediate polypeptide 2 (DNAI2), mRNA. | 0,099548 | 0,169981 |
| NM_031430 | rab interacting lysosomal protein (RILP), mRNA. | 0,099548 | -0,24121 |
| NM_018092 | neuropilin (NRP) and tolloid (TLL)-like 2 (NETO2), mRNA. | 0,099564 | -0,25884 |
| NM_018683 | zinc finger protein 313 (ZNF313), mRNA. | 0,099564 | 0,200528 |
| NM_002927 | regulator of G-protein signalling 13 (RGS13), transcript variant 1, mRNA. | 0,099749 | -0,2247 |
| NM_152333 | chromosome 14 open reading frame 69 (C14orf69), mRNA. | 0,099899 | -0,14178 |
| NM_007289 | membrane metallo-endopeptidase (neutral endopeptidase, enkephalinase, CALLA, CD10) (MME), transcript variant 2b, mRNA. | 0,099917 | 0,246843 |
| AK095491 | cDNA FLJ38172 fis, clone FCBBF1000038. | 0,099917 | 0,199477 |
| NM_014060 | MCT-1 protein (MCT-1), mRNA. | 0,099917 | 0,1335 |
| NM_152703 | hypothetical protein FLJ39885 (FLJ39885), mRNA. | 0,099917 | -0,24777 |
| NM_003049 | solute carrier family 10 (sodium/bile acid cotransporter family), member 1 (SLC10A1), mRNA. | 0,099917 | -0,31754 |

**Table 9: Human genes differentially expressed between colorectal adenoma and adenocarcinoma cells with chromosomal gain at 8q (multivariant analysis).**

| | Gene ID | Description (20q gain, Table 9) | FDR | Effect |
|---|---|---|---|---|
| 1 | NM_003859 | dolichyl-phosphate mannosyltransferase polypeptide 1, catalytic subunit (DPM1), mRNA. | 1,29E-06 | 3,32635 |
| 2 | NM_014052 | GW128 protein (GW128), mRNA. | 0,000219 | 2,75826 |
| 3 | NM_024115 | hypothetical protein MGC4309 (MGC4309), mRNA. | 0,000227 | 1,946961 |
| 4 | NM_018840 | putative Rab5-interacting protein (RIP5), mRNA. | 0,000614 | 2,36608 |
| 5 | NM_024522 | hypothetical protein FLJ12650 (FLJ12650), mRNA. | 0,000722 | 2,084454 |
| 6 | NM_017896 | chromosome 20 open reading frame 11 (C20orf11), mRNA. | 0,000925 | 1,93846 |
| 7 | AF085835 | full length insert cDNA clone YI41B09. | 0,000966 | 3,998038 |
| 8 | NM_021809 | TGFB-induced factor 2 (TALE family homeobox) (TGIF2), mRNA. | 0,002932 | 2,265688 |
| 9 | AK025758 | cDNA: FLJ22105 fis, clone HEP17660. | 0,003082 | 2,552564 |
| 10 | NM_152777 | chromosome 14 open reading frame 48 (C14orf48), mRNA. | 0,00421 | 2,082743 |
| 11 | BC039527 | clone IMAGE:5742065, mRNA. | 0,00421 | 1,937765 |
| 12 | AF441770 | Tho2 mRNA, complete cds. | 0,00421 | 1,754557 |
| 13 | NM_016397 | TH1-like (Drosophila) (TH1L), mRNA. | 0,006583 | 1,918519 |
| 14 | AK095226 | cDNA FLJ37907 fis, clone COLON2009337. | 0,006964 | -4,31359 |
| 15 | NM_022082 | chromosome 20 open reading frame 59 (C20orf59), mRNA. | 0,006964 | 3,252232 |
| 16 | Y16713 | mRNA from HIV associated non-Hodgkin's lymphoma (clone hl1-5). | 0,007383 | 2,258568 |
| 17 | NM_001432 | epiregulin (EREG), mRNA. | 0,009669 | 4,65224 |
| 18 | NM_007019 | ubiquitin-conjugating enzyme E2C (UBE2C), mRNA. | 0,009792 | 2,34477 |
| 19 | AK094894 | cDNA FLJ37575 fis, clone BRCOC2003125, moderately similar to TRIOSEPHOSPHATE ISOMERASE (EC 5.3.1.1). | 0,01059 | 1,456139 |
| 20 | NM_021158 | chromosome 20 open reading frame 97 (C20orf97), mRNA. | 0,01059 | 2,067057 |
| 21 | NM_020532 | reticulon 4 (RTN4), mRNA. | 0,010896 | 1,712038 |
| 22 | AL832390 | mRNA, cDNA DKFZp667H2012 (from clone DKFZp667H2012). | 0,01232 | -1,72106 |
| 23 | NM_014766 | KIAA0193 gene product (KIAA0193), mRNA. | 0,013744 | 4,253342 |
| 24 | AB033070 | mRNA for KIAA1244 protein, partial cds. | 0,014074 | -1,89188 |
| 25 | BC039098 | Similar to desmoglein 3 (pemphigus vulgaris antigen), clone IMAGE:4822945, mRNA, partial cds. | 0,014074 | -5,58975 |
| 26 | BC029775 | hypothetical gene LOC127421, clone MGC:35394 IMAGE:5186268, mRNA, complete cds. | 0,015926 | -2,59845 |
| 27 | NM_018690 | apolipoprotein B48 receptor (APOB48R), mRNA. | 0,016058 | -2,61738 |
| 28 | NM_021911 | gamma-aminobutyric acid (GABA) A receptor, beta 2 (GABRB2), transcript variant 1, mRNA. | 0,019169 | -6,04422 |
| 29 | NM_016646 | mesenchymal stem cell protein DSCD28 (LOC51336),mRNA. | 0,021802 | 1,439666 |
| 30 | BC040672 | clone IMAGE:4816952, mRNA. | 0,023629 | -1,58839 |
| 31 | NM_138409 | hypothetical protein BC010003 (LOC112609), mRNA. | 0,023629 | -1,73317 |
| 32 | NM_005877 | splicing factor 3a, subunit 1, 120kDa (SF3A1), mRNA. | 0,023629 | -1,49304 |
| 33 | NM_012190 | formyltetrahydrofolate dehydrogenase (FTHFD), transcript variant 1, mRNA. | 0,023629 | -2,42935 |
| 34 | NM_003600 | serine/threonine kinase 6 (STK6), mRNA. | 0,024589 | 2,388597 |
| 35 | NM_018414 | GalNAc alpha-2, 6-sialyltransferase I, long form (ST6GalNAcI), mRNA. | 0,024589 | -3,79484 |
| 36 | NM_002759 | protein kinase, interferon-inducible double stranded RNA dependent (PRKR), mRNA. | 0,024589 | 1,141759 |
| 37 | NM_080476 | CDC91 cell division cycle 91-like 1 (S. cerevisiae) (CDC91L1), mRNA. | 0,024686 | 1,600234 |
| 38 | NM_000311 | prion protein (p27-30) (Creutzfeld-Jakob disease, Gerstmann-Strausler-Scheinker syndrome, fatal familial insomnia) (PRNP), mRNA. | 0,024686 | 1,773454 |
| 39 | NM_002296 | lamin B receptor (LBR), mRNA. | 0,025015 | 1,545491 |
| 40 | NM_053286 | aquaporin 6, kidney specific (AQP6), transcript variant 2, mRNA. | 0,029067 | -2,35392 |
| 41 | NM_006602 | transcription factor-like 5 (basic helix-loop-helix) (TCFL5), mRNA. | 0,032959 | 2,252188 |
| 42 | BM669556 | UI-E-DX1-agw-c-13-0-UI.s1 UI-E-DX1 cDNA clone UI-E-DX1-agw-c-13-0-UI 3', mRNA sequence. | 0,032959 | 1,420023 |
| 43 | NM_030789 | histocompatibility (minor) 13 (HM13), mRNA. | 0,032959 | 1,338115 |
| 44 | NM_012112 | chromosome 20 open reading frame 1 (C20orf1), mRNA. | 0,033163 | 2,151017 |
| 45 | AF258564 | PP3781 mRNA, complete cds. | 0,033429 | 3,63228 |
| 46 | NM_000835 | glutamate receptor, ionotropic, N-methyl D-aspartate 2C (GRIN2C), mRNA. | 0,033429 | 1,500558 |
| 47 | NM_012469 | chromosome 20 open reading frame 14 (C20orf14), mRNA. | 0,033429 | 1,617033 |
| 48 | BC002877 | Similar to hypothetical protein FLJ11585, clone MGC:11258 IMAGE:3942160, mRNA, complete cds. | 0,033429 | -1,00202 |
| 49 | NM_021738 | supervillin (SVIL), transcript variant 2, mRNA. | 0,03532 | -1,04373 |
| 50 | NM_018710 | hypothetical protein DKFZp7620076 (DKFZp7620076), mRNA. | 0,039255 | 1,675976 |
| 51 | NM_017495 | RNA-binding region (RNP1, RRM) containing 1 (RNPC1), mRNA. | 0,039255 | 1,951461 |
| 52 | NM_017699 | hypothetical protein FLJ20174 (FLJ20174), mRNA. | 0,039648 | -2,23626 |
| 53 | NM_014622 | loss of heterozygosity, 11, chromosomal region 2, gene A (LOH11CR2A), mRNA. | 0,039648 | -1,81638 |
| 54 | BG107100 | 602291004F1 NIH_MGC_85 cDNA clone IMAGE:4385712 5', mRNA sequence. | 0,039648 | 2,110365 |
| 55 | NM_006615 | calpain 9 (nCL-4) (CAPN9), mRNA. | 0,039648 | -2,76165 |
| 56 | NM_002216 | inter-alpha (globulin) inhibitor, H2 polypeptide (ITIH2), mRNA. | 0,040599 | 2,63875 |
| 57 | AI546979 | PN2.1_12_B02.r mynorm cDNA 5', mRNA sequence. | 0,041285 | -3,01754 |
| 58 | NM_018270 | chromosome 20 open reading frame 20 (C20orf20), mRNA. | 0,042112 | 0,977697 |
| 59 | NM_002899 | retinol binding protein 1, cellular (RBP1), mRNA. | 0,044713 | 2,777886 |
| 60 | NM_031966 | cyclin B1 (CCNB1), mRNA. | 0,047922 | 1,637206 |
| 61 | NM_021945 | hypothetical protein FLJ22174 (FLJ22174), mRNA. | 0,047922 | -1,93072 |
| 62 | NM_007002 | adhesion regulating molecule 1 (ADRM1), mRNA. | 0,047922 | 1,658766 |
| 63 | NM_014902 | KIAA0964 protein (KIAA0964), mRNA. | 0,047922 | 1,345732 |
| 64 | NM_013390 | transmembrane protein 2 (TMEM2), mRNA. | 0,047922 | -1,58866 |
| 65 | AL833463 | mRNA, cDNA DKFZp686P07116 (from clone DKFZp686P07116). | 0,048294 | 2,360742 |
| 66 | NM_020188 | DC13 protein (DC13), mRNA. | 0,048384 | 2,401407 |
| 67 | AJ489592 | mRNA for glycosylation-dependent cell adhesion molecule 1 (GLYCAM1 gene), isolate KH c15. | 0,050642 | 1,402198 |
| 68 | NM_002915 | replication factor C (activator 1) 3, 38kDa (RFC3), mRNA. | 0,050642 | 1,281867 |
| 69 | NM_030574 | START domain containing 5 (STARD5), mRNA. | 0,051215 | -1,1568 |
| 70 | NM_000698 | arachidonate 5-lipoxygenase (ALOX5), mRNA. | 0,051215 | -2,14809 |
| 71 | NM_014747 | KIAA0237 gene product (KIAA0237), mRNA. | 0,051215 | -1,51108 |
| 72 | NM_145235 | similar to RIKEN cDNA 1700007B22 (LOC92565), mRNA. | 0,051215 | -2,31424 |
| 73 | NM_001062 | transcobalamin I (vitamin B12 binding protein, R binder family) (TCN1), mRNA. | 0,052267 | -3,27926 |
| 74 | BC041376 | Similar to hypothetical protein 5830442F04, clone MGC:43895 IMAGE:5274634, mRNA, complete cds. | 0,055936 | -1,86045 |
| 75 | AK021691 | cDNA FLJ11629 fis, clone HEMBA1004241. | 0,055936 | 1,502259 |
| 76 | AF353674 | BTB domain protein (BDPL) mRNA, partial cds. | 0,05615 | -1,35116 |
| 77 | AL834283 | mRNA, cDNA DKFZp547N0315 (from clone DKFZp547N0315). | 0,057809 | 1,423136 |
| 78 | NM_032744 | hypothetical protein MGC12335 (MGC12335), mRNA. | 0,057886 | -3,23807 |
| 79 | NM_000442 | platelet/endothelial cell adhesion molecule (CD31 | 0,057886 | -2,7086 |
| | | antigen) (PECAM1), mRNA. | | |
| 80 | NM_005538 | inhibin, beta C (INHBC), mRNA. | 0,057886 | 1,147071 |
| 81 | Z34278 | (JER58)MUC5AC mRNA for mucin (partial). | 0,058672 | -4,85491 |
| 82 | NM_033339 | caspase 7, apoptosis-related cysteine protease (CASP7), transcript variant gamma, mRNA. | 0,058672 | -1,40297 |
| 83 | NM_005618 | delta-like 1 (Drosophila) (DLL1), mRNA. | 0,058883 | -1,2942 |
| 84 | NM_016938 | EGF-containing fibulin-like extracellular matrix protein 2 (EFEMP2), mRNA. | 0,059763 | 1,522942 |
| 85 | NM_152369 | hypothetical protein MGC45474 (MGC45474), mRNA. | 0,064718 | -1,60257 |
| 86 | NM_018244 | chromosome 20 open reading frame 44 (C20orf44), mRNA. | 0,067511 | 1,488465 |
| 87 | NM_133367 | chromosome 6 open reading frame 33 (C6orf33), mRNA. | 0,070585 | -1,81794 |
| 88 | BC031648 | KIAA1324 protein, clone MGC:35166 IMAGE:5169952, mRNA, complete cds. | 0,070585 | -3,16718 |
| 89 | BC008502 | clone MGC:14841 IMAGE:4295121, mRNA, complete cds. | 0,071165 | -1,66179 |
| 90 | NM_138340 | abhydrolase domain containing 3 (ABHD3), mRNA. | 0,071967 | -1,61731 |
| 91 | NM_006259 | protein kinase, cGMP-dependent, type II (PRKG2), mRNA. | 0,071967 | -1,74885 |
| 92 | NM_024533 | hypothetical protein FLJ22167 (FLJ22167), mRNA. | 0,072191 | -1,97266 |
| 93 | NM_001155 | annexin A6 (ANXA6), transcript variant 1, mRNA. | 0,075565 | 1,760937 |
| 94 | NM_020831 | megakaryoblastic leukemia (translocation) 1 (MKL1), mRNA. | 0,075565 | -1,03031 |
| 95 | AK095808 | cDNA FLJ38489 fis, clone FEBRA2023550, weakly similar to P3 PROTEIN. | 0,075565 | -1,19307 |
| 96 | NM_138573 | neuregulin 4 (LOC145957), mRNA. | 0,075565 | -1,28984 |
| 97 | NM_003839 | tumor necrosis factor receptor superfamily, member 11a, activator ofNFKB (TNFRSF11A), mRNA. | 0,075565 | -1,63975 |
| 98 | NM_004237 | thyroid hormone receptor interactor 13 (TRIP13), mRNA. | 0,075565 | 2,118122 |
| 99 | BC000885 | clone MGC:5619 IMAGE:3462332, mRNA, complete cds. | 0,077064 | -1,22335 |
| 100 | NM_032579 | colon and small intestine-specific cysteine-rich protein precursor (HXCP2), mRNA. | 0,077064 | -4,92307 |
| 101 | NM_006416 | solute carrier family 35 (CMP-sialic acid transporter), member 1 (SLC35A1), mRNA. | 0,077064 | -1,64993 |
| 102 | NM_016354 | solute carrier family 21 (organic anion transporter), member 12 (SLC21A12), mRNA. | 0,080013 | 1,550246 |
| 103 | AF257167 | intestinal mucin 2.1 (MUC2) mRNA, partial cds. | 0,080013 | -4,56423 |
| 104 | NM_033126 | serine/threonine kinase PSKH2 (PSKH2), mRNA. | 0,080206 | 1,480433 |
| 105 | AB033100 | mRNA for KIAA1274 protein, partial cds. | 0,082429 | 1,828137 |
| 106 | AK022426 | cDNA FLJ12364 fis, clone MAMMA1002384. | 0,082429 | -0,99196 |
| 107 | NM_024725 | hypothetical protein FLJ23518 (FLJ23518), mRNA. | 0,084091 | -1,23218 |
| 108 | NM_025194 | inositol 1,4,5-trisphosphate 3-kinase C (ITPKC), mRNA. | 0,084748 | -1,99891 |
| 109 | AF161414 | HSPC296 mRNA, partial cds. | 0,084748 | -1,0946 |
| 110 | NM_004699 | DNA segment on chromosome X (unique) 9928 expressed sequence (DXS9928E), mRNA. | 0,084748 | 1,004674 |
| III | BC035372 | clone MGC:35266 IMAGE:5174235, mRNA, complete cds. | 0,085475 | -1,75397 |
| 112 | BC029776 | similar to CG12393 gene product, clone IMAGE:5188623, mRNA, partial cds. | 0,085475 | -1,31163 |
| 113 | NM_031413 | cat eye syndrome chromosome region, candidate 2 (CECR2), mRNA. | 0,085475 | -1,83919 |
| 114 | NM_000295 | serine (or cysteine) proteinase inhibitor, clade A (alpha-1 antiproteinase, antitrypsin), member 1 (SERPINA1), mRNA. | 0,087886 | -2,34172 |
| 115 | NM_007187 | WW domain binding protein 4 (formin binding protein 21) (WBP4), mRNA. | 0,088138 | 1,148173 |
| 116 | Z34281 | (MAR10) MUC5AC mRNA for mucin (partial). | 0,088138 | -5,17796 |
| 117 | NM_014444 | gamma tubulin ring complex protein (76p gene) (76P), mRNA. | 0,089116 | 0,843388 |
| 118 | BQ312908 | PM4-BN0143-010600-003-h03 BN0143 cDNA, | 0,089116 | 0,948883 |
| | | mRNA sequence. | | |
| 119 | NM_006150 | LIM domain only 6 (LM06), mRNA. | 0,089785 | 1,102005 |
| 120 | NM_019853 | protein phosphatase 4, regulatory subunit 2 (PPP4R2), mRNA. | 0,090725 | 0,668805 |
| 121 | NM_004403 | deafness, autosomal dominant 5 (DFNA5), mRNA. | 0,090912 | 1,171547 |
| 122 | NM_002619 | platelet factor 4 (chemokine (C-X-C motif) ligand 4) (PF4), mRNA. | 0,090912 | -1,93058 |
| 123 | NM_016400 | Huntingtin interacting protein K (HYPK), mRNA. | 0,090912 | -1,04747 |
| 124 | AL122070 | mRNA, cDNA DKFZp434E0535 (from clone DKFZp434E0535) | 0,090912 | -1,26611 |
| 125 | NM_020233 | x 006 protein (MDS006), mRNA. | 0,09234 | -1,24087 |
| 126 | AF130090 | clone FLB9530 PR02574 mRNA, complete cds. | 0,094572 | 1,989331 |
| 127 | AK074657 | cDNA FLJ90176 fis, clone MAMMA1000528. | 0,096835 | -1,13323 |
| 128 | NM_004385 | chondroitin sulfate proteoglycan 2 (versican) (CSPG2), mRNA. | 0,099564 | 1,774975 |

**Table 10: Human genes located within regions of chromosomal loss and showing differential expression between colorectal adenoma and adenocarcinoma cells with chromosomal loss at 8p.**

| 8p | Description (8p loss, Table 10) | Nr. hits^{##} |
|---|---|---|
| NM_020749 | AT2 receptor-interacting protein 1 (ATIP1), mRNA. | 3x |
| NM_004315 | N-acylsphingosine amidohydrolase (acid ceramidase) 1 (ASAH1), mRNA. | 3x |
| NM_003747 | tankyrase, TRF1-interacting ankyrin-related ADP-ribose polymerase (TNKS), mRNA. | 2x |
| NM_016353 | zinc finger, DHHC domain containing 2 (ZDHHC2), mRNA. | 2x |
| NM_152415 | hypothetical protein FLJ32642 (FLJ32642), mRNA. | 2x |
| NM_006197 | pericentriolar material 1(PCM1), mRNA. | 2x |
| NM_000662 | N-acetyltransferase 1 (arylamine N-acetyltransferase) (NAT1), mRNA. | 2x |
| NM_000015 | N-acetyltransferase 2 (arylamine N-acetyltransferase) (NAT2), mRNA. | 2x |
| D31887 | mRNA for KIAA0062 gene, partial cds. | 2x |
| NM_017884 | PIN2-interacting protein 1 (PINX1), mRNA. | 1x |
| NM_004462 | farnesyl-diphosphate farnesyltransferase 1 (FDFT1), mRNA. | 1x |
| NM_006765 | Putative prostate cancer tumor suppressor (N33), mRNA. | 1x |
| NM_001715 | B lymphoid tyrosine kinase (BLK), mRNA. | 1x |
| NM_012331 | methionine sulfoxide reductase A (MSRA), mRNA. | 1x |
| NM_139167 | sarcoglycan zeta (SGCZ), mRNA. | 1x |
| NM_013354 | CCR4-NOT transcription complex, subunit 7 (CNOT7), transcript variant 1, mRNA. | 1x |
| NM_005144 | hairless (HR), transcript variant 1, mRNA. | 1x |

| | | |
|---|---|---|
| ##: "Number of hits" in table 10 to 16, refers to the number of independent assays wherein a correlation between altered expression and the presence of colorectal carcinoma cells is observed for a given gene (altered expression regardless of the genomic background of the sample, altered expression in sample pools selected on the type of chromosomal aberration). | | |

**Table 11: Human genes located within regions of chromosomal loss and showing differential expression between colorectal adenoma and adenocarcinoma cells with chromosomal gain at 8q.**

| Gene ID | Description (8q gain, Table 11) | Nr. hits |
|---|---|---|
| NM_138455 | collagen triple helix repeat containing 1 (CTHRC1), mRNA. | 2x |
| NM_032611 | protein tyrosine phosphatase type IVA, member 3 (PTP4A3), transcript variant 1, mRNA. | 2x |
| NM_032862 | tigger transposable element derived 5 (TIGD5), mRNA. | 2x |
| AL713790 | mRNA, cDNA DKFZp564F1062 (from clone DKFZp564F1062). | 1x |
| BC030520 | similar to hypothetical protein F33H2.2 - Caenorhabditis elegans, clone MGC:40403 IMAGE:5180533, mRNA, complete cds. | 1x |
| NM_024035 | hypothetical protein MGC3113 (MGC3113), mRNA. | 1x |
| NM_017767 | solute carrier family 39 (zinc transporter), member 4 (SLC39A4), mRNA. | 1x |
| NM_002346 | lymphocyte antigen 6 complex, locus E (LY6E), mRNA. | 1x |
| AF289596 | clone pp7882 unknown mRNA. | 1x |
| NM_012162 | F-box and leucine-rich repeat protein 6 (FBXL6), transcript variant 1, mRNA. | 1x |
| AB051475 | mRNA for KIAA1688 protein, partial cds. | 1x |

**Table 12: Human genes located within regions of chromosomal loss and showing differential expression between colorectal adenoma and adenocarcinoma cells with chromosomal gain at 13q.**

| Gene ID | Description (13 q gain, table 12) | Nr. hits |
|---|---|---|
| NM_145293 | similar to hypothetical protein FLJ20897 (LOC196549), mRNA. | 3x |
| NM_005358 | LIM domain only 7 (LMO7), transcript variant 1, mRNA. | 3x |
| U50531 | BRCA2 region, mRNA sequence CG030. | 2x |
| NM_012158 | F-box and leucine-rich repeat protein 3A (FBXL3A), mRNA. | 2x |
| NM_017817 | RAB20, member RAS oncogene family (RAB20), mRNA. | 2x |
| NM_003899 | Rho guanine nucleotide exchange factor (GEF) 7 (ARHGEF7), transcript variant 1, mRNA. | 2x |
| NM_003903 | CDC16 cell division cycle 16 homolog (S. cerevisiae) (CDC16), mRNA. | 2x |
| NM_018386 | hypothetical protein FLJ11305 (FLJ11305), mRNA. | 2x |
| BC008975 | clone MGC:16774 IMAGE:4215274, mRNA, complete cds. | 2x |
| NM_023011 | similar to yeast Upf3, variant A (UPF3A), transcript variant 1, mRNA. | 2x |
| NM_001260 | cyclin-dependent kinase 8 (CDK8), mRNA. | 1x |
| NM_006646 | WAS protein family, member 3 (WASF3), mRNA. | 1x |
| U50524 | BRCA2 region, mRNA sequence CG017. | 1x |
| NM_033111 | CG016 (LOC88523), mRNA. | 1x |
| NM_024808 | hypothetical protein FLJ22624 (FLJ22624), mRNA. | 1x |
| NM_014832 | TBC1 domain family, member 4 (TBC1D4), mRNA. | 1x |
| NM_006002 | ubiquitin carboxyl-terminal esterase L3 (ubiquitin thiolesterase) (UCHL3), mRNA. | 1x |
| NM_015057 | KIAA0916 protein (KIAA0916), mRNA. | 1x |
| NM_024546 | hypothetical protein FLJ13449 (FLJ13449), mRNA. | 1x |
| BC026126 | hypothetical protein KIAA1165, clone MGC:3415 IMAGE:3027907, mRNA, complete cds. | 1x |
| NM_006493 | ceroid-lipofuscinosis, neuronal 5 (CLN5), mRNA. | 1x |
| NM_018210 | hypothetical protein FLJ10769 (FLJ10769), mRNA. | 1x |
| NM_017664 | hypothetical protein FLJ20093 (FLJ20093), mRNA. | 1x |

**Table 13: Human genes located within regions of chromosomal loss and showing differential expression between colorectal adenoma and adenocarcinoma cells with chromosomal loss at 15 q.**

| Gene ID | Description (15q loss table 13) | Nr. hits |
|---|---|---|
| NM_030574 | START domain containing 5 (STARD5), mRNA. | 3x |
| NM_004255 | cytochrome c oxidase subunit Va (COX5A), nuclear gene encoding mitochondrial protein, mRNA. | 2x |
| NM_002573 | platelet-activating factor acetylhydrolase, isoform Ib, gamma subunit 29kDa (PAFAH1B3), mRNA. | 2x |
| AB033025 | mRNA for KIAA1199 protein, partial cds. | 2x |
| NM_033240 | promyelocytic leukemia (PML), transcript variant 2, mRNA. | 1x |
| NM_000126 | electron-transfer-flavoprotein, alpha polypeptide (glutaric aciduria II) (ETFA), nuclear gene encoding mitochondrial protein, mRNA. | 1x |
| NM_015079 | KIAA1055 protein (KIAA1055), mRNA. | 1x |
| NM_015969 | mitochondrial ribosomal protein S17 (MRPS17), nuclear gene encoding mitochondrial protein, mRNA. | 1x |
| NM_016073 | likely ortholog of mouse hepatoma-derived growth factor, related protein 3 (HDGFRP3), mRNA. | 1x |

**Table 14: Human genes located within regions of chromosomal loss and showing differential expression between colorectal adenoma and adenocarcinoma cells with chromosomal loss at 17p.**

| Gene ID | Description (17 p loss, Table 14) | Nr. hits |
|---|---|---|
| NM_130766 | skeletal muscle and kidney enriched inositol phosphatase (SKIP), transcript variant 2, mRNA. | 2x |
| NM_015721 | gem (nuclear organelle) associated protein 4 (GEMIN4), mRNA. | 1x |
| NM_031430 | rab interacting lysosomal protein (RILP), mRNA. | 1x |

**Table 15: Human genes located within regions of chromosomal loss and showing differential expression between colorectal adenoma and adenocarcinoma cells with chromosomal loss at 18q.**

| Gene ID | Description (18q loss, Table 15) | Nr. hits |
|---|---|---|
| NM_004715 | CTD (carboxy-terminal domain, RNA polymerase II, polypeptide A) phosphatase, subunit 1 (CTDP1), transcript variant FCPla, mRNA. | 2x |
| NM_006701 | similar to S. pombe diml+ (DIM1), mRNA. | 2x |
| NM_014913 | KIAA0863 protein (KIAA0863), mRNA. | 1x |

**Table 16: Human genes located within regions of chromosomal loss and showing differential expression between colorectal adenoma and adenocarcinoma cells with chromosomal gain at 20q.**

| Gene ID | Description (20q gain, Table 16) | Nr. hits |
|---|---|---|
| NM_016397 | TH1-like (Drosophila) (TH1L), mRNA. | 8x |
| NM_018270 | chromosome 20 open reading frame 20 (C20orf20), mRNA. | 8x |
| NM_006602 | transcription factor-like 5 (basic helix-loop-helix) (TCFL5), mRNA. | 7x |
| NM_080476 | CDC91 cell division cycle 91-like 1 (S. cerevisiae) (CDC91L1), mRNA. | 6x |
| NM_017896 | chromosome 20 open reading frame 11 (C20orf11), mRNA. | 6x |
| NM_006097 | myosin regulatory light chain 2, smooth muscle isoform (MYRL2), mRNA. | 5x |
| NM_021809 | TGFB-induced factor 2 (TALE family homeobox) (TGIF2), mRNA. | 5x |
| NM_018840 | putative Rab5-interacting protein (RIP5), mRNA. | 5x |
| NM_003600 | serine/threonine kinase 6 (STK6), mRNA. | 5x |
| NM_017495 | RNA-binding region (RNP1, RRM) containing 1 (RNPC1), mRNA. | 5x |
| NM_007002 | adhesion regulating molecule 1 (ADRM1), mRNA. | 5x |
| NM_016354 | solute carrier family 21 (organic anion transporter), member 12 (SLC21A12), mRNA. | 5x |
| NM_014071 | nuclear receptor coactivator 6 (NCOA6), mRNA. | 4x |
| NM_002212 | integrin beta 4 binding protein (ITGB4BP), mRNA. | 4x |
| NM_003185 | TAF4 RNA polymerase II, TATA box binding protein (TBP)-associated factor, 135kDa (TAF4), mRNA. | 4x |
| NM_152255 | proteasome (prosome, macropain) subunit, alpha type, 7 (PSMA7), transcript variant 2, mRNA. | 4x |
| NM_022082 | chromosome 20 open reading frame 59 (C20orf59), mRNA. | 4x |
| NM_018244 | chromosome 20 open reading frame 44 (C20orf44), mRNA. | 3x |
| NM_014902 | KIAA0964 protein (KIAA0964), mRNA. | 3x |
| NM_032013 | NDRG family member 3 (NDRG3), mRNA. | 3x |
| NM_020182 | transmembrane, prostate androgen induced RNA (TMEPAI), mRNA. | 3x |
| NM_006886 | ATP synthase, H+ transporting, mitochondrial F1 complex, epsilon subunit (ATP5E), nuclear gene encoding mitochondrial protein, mRNA. | 3x |
| NM_020673 | RAB22A, member RAS oncogene family (RAB22A), mRNA. | 3x |
| BC003122 | clone IMAGE:3357127, mRNA, partial cds. | 3x |
| NM_012325 | microtubule-associated protein, RP/EB family, member 1 (MAPRE1), mRNA. | 2x |
| NM_014183 | dynein, cytoplasmic, light polypeptide 2A (DNCL2A), mRNA. | 2x |
| NM 021100 | NFS1 nitrogen fixation 1 (S. cerevisiae) (NFS1), mRNA. | 2x |
| NM_004738 | VAMP (vesicle-associated membrane protein)-associated protein B and C (VAPB), mRNA. | 2x |
| NM_016045 | chromosome 20 open reading frame 45 (C20orf45), mRNA. | 2x |
| NM_014054 | chromosome 20 open reading frame 40 (C20orf40), mRNA. | 2x |
| NM_022105 | death associated transcription factor 1 (DATF1), transcript variant 1, mRNA. | 2x |
| NM_015666 | GTP binding protein 5 (putative) (GTPBP5), mRNA. | 2x |
| NM_032527 | hypothetical protein FLJ14972 (KIAA1847), mRNA. | 2x |
| BC025345 | Similar to LOC149651, clone MGC:39393 IMAGE:4862156, mRNA, complete cds. | 2x |
| NM_033405 | peroxisomal proliferator-activated receptor A interacting complex 285 (PRIC285), mRNA. | 2x |
| NM_006892 | DNA (cytosine-5-)-methyltransferase 3 beta (DNMT3B), mRNA. | 1x |
| NM_005225 | E2F transcription factor 1 (E2F1), mRNA. | 1x |
| NM_000687 | S-adenosylhomocysteine hydrolase (AHCY), mRNA. | 1x |
| BC035639 | clone MGC:46653 IMAGE:5547220, mRNA, complete cds. | 1x |
| NM_018677 | acetyl-Coenzyme A synthetase 2 (ADP forming) (ACAS2), transcript variant 1, mRNA. | 1x |
| NM_006047 | RNA binding motif protein 12 (RBM12), transcript variant 1, mRNA. | 1x |
| NM_016436 | chromosome 20 open reading frame 104 (C20orf104), mRNA. | 1x |
| NM_015511 | chromosome 20 open reading frame 4 (C20orf4), mRNA. | 1x |
| NM_016082 | CDK5 regulatory subunit associated protein 1 (CDKSRAP1), mRNA. | 1x |
| NM_007238 | peroxisomal membrane protein 4, 24kDa (PXMP4), mRNA. | 1x |
| NM_003908 | eukaryotic translation initiation factor 2, subunit 2 beta, 38kDa (EIF2S2), mRNA. | 1x |
| NM_003610 | RAE1 RNA export 1 homolog (S. pombe) (RAE1), mRNA. | 1x |
| NM_153360 | hypothetical protein FLJ90166 (FLJ90166), mRNA. | 1x |
| NM_080425 | GNAS complex locus (GNAS), transcript variant 3, mRNA. | 1x |
| NM_000114 | endothelin 3 (EDN3), mRNA. | 1x |
| NM_001853 | collagen, type IX, alpha 3 (COL9A3), mRNA. | 1x |
| NM_144498 | oxysterol binding protein-like 2 (OSBPL2), transcript variant 2, mRNA. | 1x |
| NM_017798 | chromosome 20 open reading frame 21 (C20orf21), mRNA. | 1x |
| NM_012384 | glucocorticoid modulatory element binding protein 2 (GMEB2), mRNA. | 1x |

**Table 17: Marker genes with a modified expression between colorectal adenoma tissue and colorectal adenocarcinoma tissue with a chromosomal aberration.**

| Gene ID | Description (Table 17) | FDR | Effect |
|---|---|---|---|
| AB002314 | mRNA for KIAA0316 protein, partial cds. | 0,08747 | -0,322 |
| AB002339 | mRNA for KIAA0341 gene, partial cds. | 0,069786 | -0,26811 |
| AB006909 | mRNA for A-type microphthalmia associated transcription factor, complete cds. | 0,094327 | 0,353937 |
| AB007875 | KIAA0415 mRNA, complete cds. | 0,094327 | 0,299914 |
| AB011131 | mRNA for KIAA0559 protein, partial cds. | 0,081888 | -0,2282 |
| AB023233 | mRNA for KIAA1016 protein, partial cds. | 0,069317 | 0,146398 |
| AB033025 | mRNA for KIAA1199 protein, partial cds. | 0,094934 | -0,34034 |
| AB033070 | mRNA for KIAA1244 protein, partial cds. | 0,014074 | -1,89188 |
| AB033100 | mRNA for KIAA1274 protein, partial cds. | 0,082429 | 1,828137 |
| AB033107 | mRNA for KIAA1281 protein, partial cds. | 0,086768 | 0,231675 |
| AB037842 | mRNA for KIAA1421 protein, partial cds. | 0,08747 | -0,32792 |
| AB058758 | mRNA for KIAA1855 protein, partial cds. | 0,082581 | 0,275326 |
| AF001892 | MEN1 region clone epsilon/beta mRNA, 5' fragment. | 0,069489 | -0,58133 |
| AF070620 | clone 24694 mRNA sequence. | 0,08865 | 0,190677 |
| AF075082 | full length insert cDNA YQ80H06. | 0,022216 | -0,29092 |
| AF085835 | full length insert cDNA clone YI41B09. | 0,000966 | 3,998038 |
| AF085837 | full length insert cDNA clone YI41H11. | 0,082476 | 0,268544 |
| AF085861 | full length insert cDNA clone YN67C05. | 0,057755 | -0,37833 |
| AF085893 | full length insert cDNA clone YP95A10. | 0,051706 | 0,217617 |
| AF085900 | full length insert cDNA clone YQ28C05. | 0,065715 | -0,27278 |
| AF085955 | full length insert cDNA clone YR86G04. | 0,06872 | 0,220771 |
| AF086011 | full length insert cDNA clone YW18A11. | 0,056597 | 0,413724 |
| AF086021 | full length insert cDNA clone YW21D11. | 0,038975 | -0,40572 |
| AF086130 | full length insert cDNA clone ZA84A12. | 0,079582 | 0,117902 |
| AF086417 | full length insert cDNA clone ZD78A02. | 0,056648 | 0,538334 |
| AF087970 | full length insert cDNA clone YU75B05. | 0,086768 | -0,26453 |
| AF088051 | full length insert cDNA clone ZD63G05. | 0,02963 | -0,22944 |
| AF130090 | clone FLB9530 PR02574 mRNA, complete cds. | 0,094572 | 1,989331 |
| AF156166 | putative tumor suppressor mRNA. | 0,04221 | -0,34763 |
| AF161353 | HSPC090 mRNA, partial cds. | 0,053587 | 0,234453 |
| AF161414 | HSPC296 mRNA, partial cds. | 0,084748 | -1,0946 |
| AF191020 | E2IG5 (E2IG5) mRNA, complete cds. | 0,071441 | -0,25437 |
| AF258564 | PP3781 mRNA, complete cds. | 0,033429 | 3,63228 |
| AF304443 | B lymphocyte activation-related protein BC-2048 mRNA, complete cds. | 0,094327 | -0,7255 |
| AF339772 | clone IMAGE:128781, mRNA sequence. | 0,036825 | 0,386798 |
| AF350451 | C6orf37 mRNA, complete cds. | 0,064674 | -0,32683 |
| AF353674 | BTB domain protein (BDPL) mRNA, partial cds. | 0,05615 | -1,35116 |
| AF441770 | Tho2 mRNA, complete cds. | 0,00421 | 1,754557 |
| AF514992 | nuclear protein p30 mRNA, partial cds. | 0,090807 | -0,16537 |
| AI546979 | PN2.1_12_B02.r mynorm cDNA 5', mRNA sequence. | 0,041285 | -3,01754 |
| AI652393 | wb20f02.x1 NCI_CGAP_GC6 cDNA clone IMAGE:2306235 3', mRNA sequence. | 0,075868 | 0,223284 |
| AJ292079 | partial mRNA for MUC5AC protein (mucin gene, MUC5AC). | 0,043301 | -1,2742 |
| AJ489592 | mRNA for glycosylation-dependent cell adhesion molecule 1 (GLYCAM1 gene), isolate KH c15. | 0,050642 | 1,402198 |
| AJ489980 | mRNA for solute carrier family 13 (sodium-dependent dicarboxylate transporter), member 2 (SLC13A2 gene). | 0,076544 | -0,45616 |
| AK000165 | cDNA FLJ20158 fis, clone COL08935. | 0,077657 | -0,21885 |
| AK001130 | cDNA FLJ10268 fis, clone HEMBB1001058, weakly similar to neuronal thread protein AD7c-NTP mRNA. | 0,08327 | 0,318442 |
| AK001903 | cDNA FLJ11041 fis, clone PLACE1004405. | 0,081888 | 0,583886 |
| AK002162 | cDNA FLJ11300 fis, clone PLACE1009886. | 0,094934 | -0,269 |
| AK021691 | cDNA FLJ11629 fis, clone HEMBA1004241. | 0,055936 | 1,502259 |
| AK021812 | cDNA FLJ11750 fis, clone HEMBA1005568. | 0,045178 | -0,35545 |
| AK021887 | cDNA FLJ11825 fis, clone HEMBA1006494. | 0,072998 | -0,51913 |
| AK022101 | cDNA FLJ12039 fis, clone HEMBB1001930. | 0,080006 | -0,23547 |
| AK022220 | cDNA FLJ12158 fis, clone MAMMA1000522. | 0,061202 | -0,32507 |
| AK022263 | cDNA FLJ12201 fis, clone MAMMA1000906. | 0,084113 | -0,27479 |
| AK022295 | cDNA FLJ12233 fis, clone MAMMA1001215. | 0,093866 | 0,230759 |
| AK022424 | cDNA FLJ12362 fis, clone MAMMA1002360. | 0,099417 | -0,36672 |
| AK022426 | cDNA FLJ12364 fis, clone MAMMA1002384. | 0,082429 | -0,99196 |
| AK022479 | cDNA FLJ12417 fis, clone MAMMA1003039. | 0,074457 | 0,407503 |
| AK022745 | cDNA FLJ12683 fis, clone NT2RM4002457. | 0,08865 | 0,379521 |
| AK022947 | cDNA FLJ12885 fis, clone NT2RP2003988. | 0,085549 | 0,273694 |
| AK022976 | cDNA FLJ12914 fis, clone NT2RP2004523. | 0,085349 | 0,348863 |
| AK023018 | cDNA FLJ12956 fis, clone NT2RP2005501. | 0,040379 | 0,306279 |
| AK023616 | cDNA FLJ13554 fis, clone PLACE1007478. | 0,069317 | 0,315352 |
| AK024156 | cDNA FLJ14094 fis, clone MAMMA1000372. | 0,039111 | -0,42942 |
| AK024254 | cDNA FLJ14192 fis, clone NT2RP3000584. | 0,042174 | 0,224429 |
| AK024423 | mRNA for FLJ00012 protein, partial cds. | 0,076544 | -0,22477 |
| AK024563 | cDNA: FLJ20910 fis, clone ADSE00492. | 0,012033 | 0,349917 |
| AK024933 | cDNA: FLJ21280 fis, clone COL01884. | 0,093866 | -0,36332 |
| AK024940 | cDNA: FLJ21287 fis, clone COL01918. | 0,094327 | -0,45615 |
| AK025758 | cDNA: FLJ22105 fis, clone HEP17660. | 0,003082 | 2,552564 |
| AK026367 | cDNA: FLJ22714 fis, clone HSI13646. | 0,041668 | 0,388352 |
| AK026883 | cDNA: FLJ23230 fis, clone CAE07143. | 0,006725 | 0,426682 |
| AK027819 | cDNA FLJ14913 fis, clone PLACE1006782. | 0,080006 | -0,38327 |
| AK054799 | cDNA FLJ30237 fis, clone BRACE2002034. | 0,005971 | -0,39081 |
| AK055091 | cDNA FLJ30529 fis, clone BRAWH2001052. | 0,025102 | -0,32068 |
| AK055903 | cDNA FLJ31341 fis, clone MESAN1000050. | 0,023674 | -0,44115 |
| AK056178 | cDNA FLJ31616 fis, clone NT2RI2003019. | 0,098067 | 0,313366 |
| AK056292 | cDNA FLJ31730 fis, clone NT2RI2006838. | 0,075868 | 0,40984 |
| AK056401 | cDNA FLJ31839 fis, clone NT2RP7000086. | 0,093844 | 0,375509 |
| AK056573 | cDNA FLJ32011 fis, clone NT2RP7009507. | 0,085549 | -0,25456 |
| AK056609 | cDNA FLJ32047 fis, clone NTONG2001137. | 0,094327 | -0,26822 |
| AK056680 | cDNA FLJ32118 fis, clone PEBLM1000029. | 0,076544 | 0,189024 |
| AK057045 | cDNA FLJ32483 fis, clone SKNMC2001503, weakly similar to P-SELECTIN GLYCOPROTEIN LIGAND 1 PRECURSOR. | 0,061898 | -0,38993 |
| AK057085 | cDNA FLJ32523 fis, clone SMINT2000032. | 0,06872 | 0,393846 |
| AK057624 | cDNA FLJ33062 fis, clone TRACH2000032. | 0,047422 | 0,250799 |
| AK057664 | cDNA FLJ33102 fis, clone TRACH2000898. | 0,074457 | -0,32304 |
| AK057962 | cDNA FLJ25233 fis, clone STM01789. | 0,072458 | -0,32341 |
| AK074192 | cDNA FLJ23612 fis, clone ADKA02523. | 0,077111 | 0,255663 |
| AK074657 | cDNA FLJ90176 fis, clone MAMMA1000528. | 0,096835 | -1,13323 |
| AK075564 | cDNA PSEC0264 fis, clone NT2RP3002337. | 0,057755 | -0,77583 |
| AK090401 | mRNA for FLJ00278 protein. | 0,099712 | 0,283364 |
| AK090998 | cDNA FLJ33679 fis, clone BRAWH2002352. | 0,036982 | -0,53683 |
| AK092074 | cDNA FLJ34755 fis, clone NHNPC1000034. | 0,080739 | -0,31291 |
| AK092106 | cDNA FLJ34787 fis, clone NT2NE2004740. | 0,08969 | -0,3318 |
| AK092490 | cDNA FLJ35171 fis, clone PLACE6013220, weakly similar to TRICHOHYALIN. | 0,092847 | -0,20427 |
| AK093557 | cDNA FLJ36238 fis, clone THYMU2001422. | 0,080045 | 0,236266 |
| AK094413 | cDNA FLJ37094 fis, clone BRACE2018337. | 0,082586 | -0,32562 |
| AK094651 | cDNA FLJ37332 fis, clone BRAMY2019710. | 0,094327 | -0,20019 |
| AK094784 | cDNA FLJ37465 fis, clone BRAWH2011823, highly similar to BONE MORPHOGENETIC PROTEIN 7 PRECURSOR. | 0,024935 | 0,506101 |
| AK094894 | cDNA FLJ37575 fis, clone BRCOC2003125, moderately similar to TRIOSEPHOSPHATE ISOMERASE (EC 5.3.1.1). | 0,01059 | 1,456139 |
| AK095147 | cDNA FLJ37828 fis, clone BRSSN2006575. | 0,057233 | -0,79158 |
| AK095226 | cDNA FLJ37907 fis, clone COLON2009337. | 0,006964 | -4,31359 |
| AK095808 | cDNA FLJ38489 fis, clone FEBRA2023550, weakly similar to P3 PROTEIN. | 0,067336 | -0,25346 |
| AK095808 | cDNA FLJ38489 fis, clone FEBRA2023550, weakly similar to P3 PROTEIN. | 0,075565 | -1,19307 |
| AK096002 | cDNA FLJ38683 fis, clone KIDNE2000777. | 0,093478 | -0,89719 |
| AK096306 | cDNA FLJ38987 fis, clone NT2RI2005818. | 0,036126 | 0,295329 |
| AK096958 | cDNA FLJ39639 fis, clone SMINT2003340. | 0,099014 | 0,226423 |
| AK097101 | cDNA FLJ39782 fis, clone SPLEN2002175. | 0,093866 | 0,323348 |
| AK097488 | cDNA FLJ40169 fis, clone TESTI2016583. | 0,096059 | 0,208604 |
| AK097647 | cDNA FLJ40328 fis, clone TESTI2031356. | 0,033412 | 0,296624 |
| AK098024 | cDNA FLJ40705 fis, clone THYMU2026530. | 0,099014 | -0,20783 |
| AL080280 | mRNA full length insert cDNA clone EUROIMAGE 85905. | 0,089798 | -0,40611 |
| AL109727 | mRNA full length insert cDNA clone EUROIMAGE 200247. | 0,022702 | -0,18078 |
| AL110141 | mRNA, cDNA DKFZp564D0164 (from clone DKFZp564D0164). | 0,096125 | -0,19982 |
| AL122070 | mRNA, cDNA DKFZp434E0535 (from clone DKFZp434E0535) | 0,090912 | -1,26611 |
| AL137279 | mRNA, cDNA DKFZp434O1214 (from clone DKFZp434O1214). | 0,061202 | 0,363935 |
| AL137326 | mRNA, cDNA DKFZp434B0650 (from clone DKFZp434B0650). | 0,096591 | 0,416007 |
| AL137645 | mRNA, cDNA DKFZp586D0924 (from clone DKFZp586D0924). | 0,09471 | 0,282151 |
| AL390178 | mRNA, cDNA DKFZp547N064 (from clone DKFZp547N064). | 0,092928 | 0,259092 |
| AL713719 | mRNA, cDNA DKFZp667K1916 (from clone DKFZp667K1916). | 0,066275 | 0,488787 |
| AL713790 | mRNA, cDNA DKFZp564F1062 (from clone DKFZp564F1062). | 0,069786 | -0,25592 |
| AL831862 | mRNA, cDNA DKFZp761I2317 (from clone DKFZp761I2317). | 0,081827 | -0,16968 |
| AL831872 | mRNA, cDNA DKFZp547A1310 (from clone DKFZp547A1310). | 0,061791 | -0,46465 |
| AL831875 | mRNA, cDNA DKFZp547E1510 (from clone DKFZp547E1510). | 0,085549 | 0,206302 |
| AL832390 | mRNA, cDNA DKFZp667H2012 (from clone DKFZp667H2012). | 0,01232 | -1,72106 |
| AL833463 | mRNA, cDNA DKFZp686P07116 (from clone DKFZp686P07116). | 0,048294 | 2,360742 |
| AL833655 | mRNA, cDNA DKFZp667O0320 (from clone DKFZp667O0320). | 0,094327 | -0,34752 |
| AL833742 | mRNA, cDNA DKFZp666E186 (from clone DKFZp666E186). | 0,080045 | -0,39501 |
| AL834121 | mRNA, cDNA DKFZp761D2121 (from clone DKFZp761D2121). | 0,080045 | -0,30573 |
| AL834283 | mRNA, cDNA DKFZp547N0315 (from clone DKFZp547N0315). | 0,057809 | 1,423136 |
| AW084755 | xc57d02.x1 NCI_CGAP_Eso2 cDNA clone IMAGE:2588355 3' similar to SW:CHH2_BOMMO P05687 CHORION CLASS HIGH-CYSTEINE HCA PROTEIN 12 PRECURSOR contains element MSR1 repetitive element | 0,082476 | 0,443077 |
| AW296028 | UI-H-BW0-aiu-d-04-0-UI.s1 NCI_CGAP_Sub6 cDNA clone IMAGE:2730654 3', mRNA sequence. | 0,045452 | 0,340944 |
| AW815041 | CMO-ST0209-271099-082-c12 ST0209 cDNA, mRNA sequence. | 0,026555 | -0,29885 |
| AW972072 | EST384056 MAGE resequences, MAGL cDNA, mRNA sequence. | 0,020341 | 0,31753 |
| AW975332 | EST387440 MAGE resequences, MAGN cDNA, mRNA sequence. | 0,070066 | -0,45859 |
| AW977106 | EST389215 MAGE resequences, MAGO cDNA, mRNA sequence. | 0,031602 | 0,317498 |
| AY006312 | clone 09npa41 T cell receptor beta chain mRNA, partial cds. | 0,094934 | -0,36656 |
| AY069977 | anterior gradient protein 3 mRNA, complete cds. | 0,049992 | -0,60091 |
| AY102069 | surfactant associated protein F mRNA, partial sequence. | 0,062383 | -0,40876 |
| BC000885 | clone MGC:5619 IMAGE:3462332, mRNA, complete cds. | 0,077064 | -1,22335 |
| BC001092 | Similar to hypothetical protein FLJ20378, clone IMAGE:3506144, mRNA. | 0,056537 | -0,24494 |
| BC002782 | clone IMAGE:3621749, mRNA, partial cds. | 0,091053 | 0,314213 |
| BC002877 | Similar to hypothetical protein FLJ11585, clone MGC:11258 IMAGE:3942160, mRNA, complete cds. | 0,070596 | -0,20351 |
| BC002877 | Similar to hypothetical protein FLJ11585, clone MGC:11258 IMAGE:3942160, mRNA, complete cds. | 0,033429 | -1,00202 |
| BC003122 | clone IMAGE:3357127, mRNA, partial cds. | 0,094934 | 0,274503 |
| BC004890 | clone IMAGE:3937917, mRNA. | 0,033412 | -0,44593 |
| BC006008 | clone IMAGE:4285740, mRNA. | 0,008614 | -0,24076 |
| BC007089 | Similar to hypothetical protein FLJ20489, clone IMAGE:4248707, mRNA. | 0,09471 | -0,27466 |
| BC007399 | clone MGC:16308 IMAGE:3836116, mRNA, complete cds. | 0,078925 | 0,583373 |
| BC008502 | clone MGC:14841 IMAGE:4295121, mRNA, complete cds. | 0,071165 | -1,66179 |
| BC008975 | clone MGC:16774 IMAGE:4215274, mRNA, complete cds. | 0,066417 | 0,37827 |
| BC009198 | Similar to RNA polymerase I transcription factor RRN3, clone MGC:15321 IMAGE:3678732, mRNA, complete cds. | 0,056597 | 0,278908 |
| BC009210 | Similar to mesoderm development candiate 2, clone MGC:16185 IMAGE:3637449, mRNA, complete cds. | 0,087717 | -0,15961 |
| BC009447 | clone MGC:15887 IMAGE:3530481, mRNA, complete cds. | 0,06351 | 0,288058 |
| BC010701 | clone MGC:16795 IMAGE:3855397, mRNA, complete cds. | 0,028543 | -0,40007 |
| BC010857 | clone MGC:9168 IMAGE:3876839, mRNA, complete cds. | 0,06872 | 0,156838 |
| BC011242 | clone IMAGE:4153763, mRNA. | 0,092312 | 0,5178 |
| BC011877 | Similar to dedicator of cyto-kinesis 1, clone IMAGE:3347029, mRNA. | 0,041668 | -0,33248 |
| BC013389 | clone IMAGE:3932143, mRNA. | 0,039111 | 0,437633 |
| BC014259 | clone MGC:20791 IMAGE:4763971, mRNA, complete cds. | 0,093866 | -0,23018 |
| BC014362 | clone IMAGE:3932221, mRNA. | 0,063385 | 0,253343 |
| BC014441 | Similar to RIKEN cDNA 2810405F18 gene, clone MGC:22960 IMAGE:4865283, mRNA, complete cds. | 0,083328 | -0,23912 |
| BC015412 | clone IMAGE:4393471, mRNA. | 0,023543 | 0,397184 |
| BC016795 | clone IMAGE:4067166, mRNA. | 0,094327 | -0,46023 |
| BC016864 | clone IMAGE:3847536, mRNA. | 0,028702 | 0,335912 |
| BC018758 | Similar to RIKEN cDNA 1110032022 gene, clone MGC:31967 IMAGE:4907976, mRNA, complete cds. | 0,051706 | -0,23603 |
| BC018995 | clone MGC:20579 IMAGE:4300679, mRNA, complete cds. | 0,080045 | 0,489487 |
| BC020895 | clone IMAGE:4704474, mRNA. | 0,099308 | -0,31829 |
| BC021575 | clone IMAGE:3161966, mRNA. | 0,08865 | 0,136964 |
| BC025370 | clone IMAGE:3945331, mRNA. | 0,039111 | 0,486875 |
| BC028119 | similar to putative, clone MGC:40181 IMAGE:5172473, mRNA, complete cds. | 0,065315 | -0,31424 |
| BC028376 | clone IMAGE:4838541, mRNA. | 0,080739 | -0,22249 |
| BC029775 | hypothetical gene LOC127421, clone MGC:35394 IMAGE:5186268, mRNA, complete cds. | 0,015926 | -2,59845 |
| BC029776 | similar to CG12393 gene product, clone IMAGE:5188623, mRNA, partial cds. | 0,085475 | -1,31163 |
| BC030520 | similar to hypothetical protein F33H2.2 - Caenorhabditis elegans, clone MGC:40403 IMAGE:5180533, mRNA, complete cds. | 0,082581 | 0,189826 |
| BC030750 | clone IMAGE:4795773, mRNA. | 0,067391 | -0,31297 |
| BC031244 | dual-specificity tyrosine-(Y)-phosphorylation regulated kinase 4, clone MGC:39640 IMAGE:5266287, mRNA, complete cds. | 0,02963 | -0,31347 |
| BC031266 | clone IMAGE:5277162, mRNA. | 0,09855 | -0,14471 |
| BC031648 | KIAA1324 protein, clone MGC:35166 IMAGE:5169952, mRNA, complete cds. | 0,070585 | -3,16718 |
| BC032316 | clone IMAGE:5219499, mRNA. | 0,035205 | -0,38849 |
| BC033204 | similar to excision repair protein (clone pcDE-72), clone MGC:45855 IMAGE:4876082, mRNA, complete cds. | 0,07337 | -0,21593 |
| BC033250 | clone IMAGE:5441027, mRNA, partial cds. | 0,080045 | 0,215003 |
| BC033940 | clone IMAGE:5272723, mRNA, partial cds. | 0,004542 | 0,320051 |
| BC034374 | similar to FKSG76, clone MGC:35390 IMAGE:5185621, mRNA, complete cds. | 0,069786 | 0,23969 |
| BC034459 | clone IMAGE:5185460, mRNA. | 0,094934 | -0,2396 |
| BC034812 | Similar to HSPC182 protein, clone IMAGE:4825606, mRNA. | 0,033688 | 0,229524 |
| BC035268 | clone IMAGE:4779288, mRNA. | 0,050424 | -0,25257 |
| BC035372 | clone MGC:35266 IMAGE:5174235, mRNA, complete cds. | 0,085475 | -1,75397 |
| BC035600 | Similar to RIKEN cDNA 2810405J04 gene, clone IMAGE:4430622, mRNA. | 0,06872 | -0,21368 |
| BC036004 | clone IMAGE:4730399, mRNA. | 0,082581 | -0,28908 |
| BC036876 | clone IMAGE:5245135, mRNA. | 0,082197 | 0,275752 |
| BC038767 | clone IMAGE:5270478, mRNA. | 0,080006 | -0,51549 |
| BC038852 | clone IMAGE:5168364, mRNA. | 0,066417 | -0,70334 |
| BC039098 | Similar to desmoglein 3 (pemphigus vulgaris antigen), clone IMAGE:4822945, mRNA, partial cds. | 0,014074 | -5,58975 |
| BC039527 | clone IMAGE:5742065, mRNA. | 0,00421 | 1,937765 |
| BC039555 | clone IMAGE:4184613, mRNA. | 0,089567 | -0,20617 |
| BC040662 | clone IMAGE:4799788, mRNA. | 0,099014 | 0,217656 |
| BC040672 BC041376 | clone IMAGE:4816952, mRNA. Similar to hypothetical protein 5830442F04, clone MGC:43895 IMAGE:5274634, mRNA, complete cds. | 0,023629 0,055936 | -1,58839 -1,86045 |
| BC041626 | clone MGC:52394 IMAGE:4554923, mRNA, complete cds. | 0,064346 | -0,33008 |
| BE410139 | 601302451F1 NIH_MGC_21 cDNA clone IMAGE:3636877 5', mRNA sequence. | 0,081109 | 0,4566 |
| BE672687 | 7b71e04.x1 NCI_CGAP_Lu24 cDNA clone IMAGE:3233694 3', mRNA sequence. | 0,094934 | -0,53881 |
| BF683837 | 602140129F1 NIH_MGC_46 cDNA clone IMAGE:4301287 5', mRNA sequence. | 0,099954 | -0,26208 |
| BF967668 | 602287374T1 NIH_MGC_96 cDNA clone IMAGE:4374499 3', mRNA sequence. | 0,092551 | 0,241666 |
| BG107100 | 602291004F1 NIH_MGC_85 cDNA clone IMAGE:4385712 5', mRNA sequence. | 0,039648 | 2,110365 |
| B1520375 | 603071853F1 NIH_MGC_119 cDNA clone IMAGE:5163756 5', mRNA sequence. | 0,069786 | -0,2411 |
| BI769135 | 603053727F1 NIH_MGC_122 cDNA clone IMAGE:5203207 5', mRNA sequence. | 0,094327 | 0,284732 |
| BM476468 | AGENCOURT-6476297 NIH_MGC_85 cDNA clone IMAGE:5553395 5', mRNA sequence. | 0,030444 | 0,429324 |
| BM561501 | AGENCOURT_6567312 NIH_MGC_88 cDNA clone IMAGE:5739715 5', mRNA sequence. | 0,061202 | 0,411812 |
| BM669556 | UI-E-DX1-agw-c-13-0-UI.s1 UI-E-DX1 cDNA clone UI-E-DX1-agw-c-13-0-UI 3', mRNA sequence. | 0,032959 | 1,420023 |
| BM674956 | UI-E-EJO-ahn-c-06-0-UI.s1 UI-E-EJ0 cDNA clone UI-E-EJ0-ahn-c-06-0-UI 3', mRNA sequence. | 0,08865 | -0,26423 |
| BM921036 | AGENCOURT_6633196 NIH_MGC_115 cDNA clone IMAGE:5752355 5', mRNA sequence. | 0,002075 | 0,387272 |
| BQ055725 | AGENCOURT_6796360 NIH_MGC_99 cDNA clone IMAGE:5807946 5', mRNA sequence. | 0,057233 | -0,32696 |
| BQ072652 | AGENCOURT_6763016 NIH_MGC_118 cDNA clone IMAGE:5756116 5', mRNA sequence. | 0,080006 | 0,302702 |
| BQ189477 | UI-E-EJ1-aka-c-13-0-UI.r1 UI-E-EJ1 cDNA clone UI-E-EJ1-aka-c-13-0-UI 5', mRNA sequence. | 0,006686 | -0,57215 |
| BQ220757 | AGENCOURT_7589763 NIH_MGC_92 cDNA clone IMAGE:6067696 5', mRNA sequence. | 0,08865 | 0,288766 |
| BQ230145 | AGENCOURT_7592688 NIH_MGC_72 cDNA clone IMAGE:6050363 5', mRNA sequence. | 0,072458 | 0,310216 |
| BQ312908 | PM4-BN0143-010600-003-h03 BN0143 cDNA, mRNA sequence. | 0,089116 | 0,948883 |
| BQ646410 | AGENCOURT_8511770 NIH_MGC_100 cDNA clone IMAGE:6296949 5', mRNA sequence. | 0,080045 | 0,706676 |
| BQ719012 | AGENCOURT_8100927 Lupski_sympathetic_trunk cDNA clone IMAGE:6189773 5', mRNA sequence. | 0,099417 | 0,307114 |
| BQ921112 | AGENCOURT_8926187 NIH_MGC_101 cDNA clone IMAGE:6463013 5', mRNA sequence. | 0,039111 | 0,492923 |
| BQ932364 | AGENCOURT_8854114 NIH_MGC_71 cDNA | 0,095037 | -0,21294 |
| | clone IMAGE:6473695 5', mRNA sequence. | | |
| BU594226 | AGENCOURT_8843927 NIH_MGC_142 cDNA clone IMAGE:6449644 5', mRNA sequence. | 0,092641 | -0,20224 |
| CA314430 | UI-CF-FNO-afm-n-08-0-UI.s1 UI-CF-FN0 cDNA clone UI-CF-FN0-afm-n-08-0-UI 3', mRNA sequence. | 0,08865 | -0,23737 |
| D31887 | mRNA for KIAA0062 gene, partial cds. | 0,006725 | -0,49409 |
| H43963 | yo70a10.s1 Soares breast 3NbHBst cDNA clone IMAGE:183258 3', mRNA sequence. | 0,081086 | -0,30907 |
| K00629 | kpni repeat mrna (cdna clone pcd-kpni-4), 3' end. | 0,028949 | 0,200056 |
| L38282 | 30a mRNA fragment. | 0,028321 | 0,468719 |
| M27316 | transfer RNA-Ser. | 0,079602 | 0,78553 |
| N23022 | yx65e 12.s1 Soares melanocyte 2NbHM cDNA clone IMAGE:266638 3', mRNA sequence. | 0,092312 | -0,29388 |
| NM_000015 | N-acetyltransferase 2 (arylamine N-acetyltransferase) (NAT2), mRNA. | 0,047971 | -0,23243 |
| NM_000073 | CD3G antigen, gamma polypeptide (TiT3 complex) (CD3G), mRNA. | 0,094327 | 0,232673 |
| NM_000107 | damage-specific DNA binding protein 2, 48kDa (DDB2), mRNA. | 0,044215 | -0,23951 |
| NM_000119 | erythrocyte membrane protein band 4.2 (EPB42), mRNA. | 0,069786 | -0,24152 |
| NM_000134 | fatty acid binding protein 2, intestinal (FABP2), mRNA. | 0,095037 | -0,49638 |
| NM_000135 | Fanconi anemia, complementation group A (FANCA), mRNA. | 0,094327 | 0,192872 |
| NM_000177 | gelsolin (amyloidosis, Finnish type) (GSN), mRNA. | 0,061202 | -0,16679 |
| NM_000238 | potassium voltage-gated channel, subfamily H (eag-related), member 2 (KCNH2), transcript variant 1, mRNA. | 0,031646 | 0,440776 |
| NM_000285 | peptidase D (PEPD), mRNA. | 0,006291 | 0,293134 |
| NM_000295 | serine (or cysteine) proteinase inhibitor, clade A (alpha-1 antiproteinase, antitrypsin), member 1 (SERPINA1), mRNA. | 0,087886 | -2,34172 |
| NM_000311 | prion protein (p27-30) (Creutzfeld-Jakob disease, Gerstmann-Strausler-Scheinker syndrome, fatal familial insomnia) (PRNP), mRNA. | 0,024686 | 1,773454 |
| NM_000367 | thiopurine S-methyltransferase (TPMT), mRNA. | 0,067243 | -0,32273 |
| NM_000373 | uridine monophosphate synthetase (orotate phosphoribosyl transferase and orotidine-5'-decarboxylase) (UMPS), mRNA. | 0,082581 | 0,129645 |
| NM_000374 | uroporphyrinogen decarboxylase (UROD), mRNA. | 0,081086 | 0,244062 |
| NM_000435 | Notch H.log 3 (Drosophila) (NOTCH3), mRNA. | 0,082586 | 0,17514 |
| NM_000439 | proprotein convertase subtilisin/kexin type 1 (PCSK1), mRNA. | 0,033688 | -1,05921 |
| NM_000442 | platelet/endothelial cell adhesion molecule (CD31 antigen) (PECAM1), mRNA. | 0,057886 | -2,7086 |
| NM_000574 | decay accelerating factor for complement (CD55, Cromer blood group system) (DAF), mRNA. | 0,099737 | -0,39095 |
| NM_000637 | glutathione reductase (GSR), mRNA. | 0,006725 | -0,33537 |
| NM_000698 | arachidonate 5-lipoxygenase (ALOX5), mRNA. | 0,051215 | -2,14809 |
| NM_000835 | glutamate receptor, ionotropic, N-methyl D-aspartate 2C (GRIN2C), mRNA. | 0,033429 | 1,500558 |
| NM_000862 | hydroxy-delta-5-steroid dehydrogenase, 3 beta- and steroid delta-isomerase 1 (HSD3B1), mRNA. | 0,099331 | 0,199437 |
| NM_000901 | nuclear receptor subfamily 3, group C, member 2 (NR3C2), mRNA. | 0,067336 | -0,56338 |
| NM_001008 | ribosomal protein S4, Y-linked (RPS4Y), mRNA. | 0,065715 | -0,85366 |
| NM_001062 | transcobalamin I (vitamin B12 binding protein, R binder family) (TCN1), mRNA. | 0,052267 | -3,27926 |
| NM_001072 | UDP glycosyltransferase 1 family, polypeptide A6 (UGT1A6), mRNA. | 0,047422 | -0,37685 |
| NM_001155 | annexin A6 (ANXA6), transcript variant 1, mRNA. | 0,075565 | 1,760937 |
| NM_001249 | ectonucleoside triphosphate diphosphohydrolase 5 (ENTPD5), mRNA. | 0,091582 | -0,30505 |
| NM_001280 | cold inducible RNA binding protein (CIRBP), mRNA. | 0,079348 | -0,17706 |
| NM_001316 | CSE1 chromosome segregation 1-like (yeast) | 0,067336 | 0,212579 |
| | (CSE1L), mRNA. | | |
| NM_001324 | cleavage stimulation factor, 3' pre-RNA, subunit 1, 50kDa (CSTF1), mRNA. | 0,067154 | 0,271924 |
| NM_001432 | epiregulin (EREG), mRNA. | 0,009669 | 4,65224 |
| NM_001500 | GDP-mannose 4,6-dehydratase (GMDS), mRNA. | 0,095468 | -0,23619 |
| NM_001546 | inhibitor of DNA binding 4, dominant negative helix-loop-helix protein (ID4), mRNA. | 0,025102 | -0,70017 |
| NM_001613 | actin, alpha 2, smooth muscle, aorta (ACTA2), mRNA. | 0,093866 | 0,296416 |
| NM_001696 | ATPase, H+ transporting, lysosomal 31kDa, V1 subunit E isoform 1 (ATP6V1E1), mRNA. | 0,024446 | 0,186487 |
| NM_001800 | cyclin-dependent kinase inhibitor 2D (p19, inhibits CDK4) (CDKN2D), transcript variant 1, mRNA. | 0,072792 | 0,209575 |
| NM_001807 | carboxyl ester lipase (bile salt-stimulated lipase) (CEL), mRNA. | 0,085355 | 0,327859 |
| NM_001829 | chloride channel 3 (CLCN3), mRNA. | 0,094327 | -0,28227 |
| NM_001830 | chloride channel 4 (CLCN4), mRNA. | 0,080045 | 0,159316 |
| NM_001920 | decorin (DCN), transcript variant A1, mRNA. | 0,089651 | 0,342227 |
| NM_001933 | dihydrolipoamide S-succinyltransferase (E2 component of 2-oxo-glutarate complex) (DLST), mRNA. | 0,039111 | -0,25677 |
| NM_001951 | E2F transcription factor 5, p130-binding (E2F5), mRNA. | 0,000321 | 0,37466 |
| NM_001997 | Finkel-Biskis-Reilly murine sarcoma virus (FBR-MuSV) ubiquitously expressed (fox derived) ribosomal protein S30 (FAU), mRNA. | 0,066417 | 0,314198 |
| NM_002102 | glycophorin E (GYPE), mRNA. | 0,057233 | -0,22666 |
| NM_002156 | heat shock 60kDa protein 1 (chaperonin) (HSPD1), mRNA. | 0,03977 | 0,174064 |
| NM_002212 | integrin beta 4 binding protein (ITGB4BP), mRNA. | 0,003816 | 0,24754 |
| NM_002216 | inter-alpha (globulin) inhibitor, H2 polypeptide (ITIH2), mRNA. | 0,040599 | 2,63875 |
| NM_002225 | isovaleryl Coenzyme A dehydrogenase (IVD), nuclear gene encoding mitochondrial protein, | 0,012567 | -0,22827 |
| | mRNA. | | |
| NM_002267 | karyopherin alpha 3 (importin alpha 4) (KPNA3), mRNA. | 0,042857 | 0,213699 |
| NM_002278 | keratin, hair, acidic, 2 (KRTHA2), mRNA. | 0,08655 | 0,15975 |
| NM_002296 | lamin B receptor (LBR), mRNA. | 0,025015 | 1,545491 |
| NM_002305 | lectin, galactoside-binding, soluble, 1 (galectin 1) (LGALS1), mRNA. | 0,069165 | 0,454091 |
| NM_002345 | lumican (LUM), mRNA. | 0,057233 | 0,385281 |
| NM_002389 | membrane cofactor protein (CD46, trophoblast-lymphocyte cross-reactive antigen) (MCP), transcript variant a, mRNA. | 0,056648 | 0,241236 |
| NM_002398 | Meisl, myeloid ecotropic viral integration site 1 H.log (mouse) (MEIS1), mRNA. | 0,095862 | 0,5196 |
| NM_002431 | menage a trois 1 (CAK assembly factor) (MNAT1), mRNA. | 0,087716 | -0,1658 |
| NM_002435 | mannose phosphate isomerase (MPI), mRNA. | 0,069317 | -0,23954 |
| NM_002466 | v-myb myeloblastosis viral oncogene H.log (avian)-like 2 (MYBL2), mRNA. | 0,080739 | 0,425478 |
| NM_002525 | nardilysin (N-arginine dibasic convertase) (NRD1), mRNA. | 0,079602 | -0,32216 |
| NM_002562 | purinergic receptor P2X, ligand-gated ion channel, 7 (P2RX7), mRNA. | 0,056426 | 0,295663 |
| NM_002573 | platelet-activating factor acetylhydrolase, isoform Ib, gamma subunit 29kDa (PAFAH1B3), mRNA. | 0,082476 | 0,189294 |
| NM_002586 | pre-B-cell leukemia transcription factor 2 (PBX2), mRNA. | 0,08865 | 0,299901 |
| NM_002609 | platelet-derived growth factor receptor, beta polypeptide (PDGFRB), mRNA. | 0,020483 | 0,51344 |
| NM_002619 | platelet factor 4 (chemokine (C-X-C motif) ligand 4) (PF4), mRNA. | 0,090912 | -1,93058 |
| NM_002640 | serine (or cysteine) proteinase inhibitor, clade B (ovalbumin), member 8 (SERPINB8), mRNA. | 0,023543 | -0,40671 |
| NM_002655 | pleiomorphic adenoma gene 1 (PLAG1), mRNA. | 0,01679 | 0,575441 |
| NM_002657 | pleiomorphic adenoma gene-like 2 (PLAGL2), | 0,071755 | 0,283922 |
| | mRNA. | | |
| NM_002691 | polymerase (DNA directed), delta 1, catalytic subunit 125kDa (POLD1), mRNA. | 0,080006 | 0,221819 |
| NM_002755 | mitogen-activated protein kinase kinase 1 (MAP2K1), mRNA. | 0,042857 | -0,29003 |
| NM_002759 | protein kinase, interferon-inducible double stranded RNA dependent (PRKR), mRNA. | 0,024589 | 1,141759 |
| NM_002870 | RAB13, member RAS oncogene family (RAB13), mRNA. | 0,070066 | -0,16753 |
| NM_002885 | RAP1, GTPase activating protein 1 (RAP1GA1), mRNA. | 0,033688 | -0,17827 |
| NM_002894 | retinoblastoma binding protein 8 (RBBP8), mRNA. | 0,001559 | -0,19184 |
| NM_002899 | retinol binding protein 1, cellular (RBP1), mRNA. | 0,044713 | 2,777886 |
| NM_002905 | retinol dehydrogenase 5 (11-cisand 9-cis) (RDH5), mRNA. | 0,050001 | -0,20145 |
| NM_002915 | replication factor C (activator 1) 3, 38kDa (RFC3), mRNA. | 0,050642 | 1,281867 |
| NM_002979 | sterol carrier protein 2 (SCP2), mRNA. | 0,08865 | -0,19443 |
| NM_003014 | secreted frizzled-related protein 4 (SFRP4), mRNA. | 0,021177 | 0,842529 |
| NM_003031 | seven in absentia H.log 1 (Drosophila) (SIAH1), mRNA. | 0,070066 | 0,182138 |
| NM_003072 | SWI/SNF related, matrix associated, actin dependent regulator of chromatin, subfamily a, member 4 (SMARCA4), mRNA. | 0,056597 | -0,20215 |
| NM 003118 | secreted protein, acidic, cysteine-rich (osteonectin) (SPARC), mRNA. | 0,035205 | 0,443985 |
| NM_003121 | Spi-B transcription factor (Spi-1/PU.1 related) (SPIB), mRNA. | 0,066417 | -0,33326 |
| NM_003185 | TAF4 RNA polymerase II, TATA box binding protein (TBP)-associated factor, 135kDa (TAF4), mRNA. | 0,08747 | 0,255894 |
| NM_003186 | transgelin (TAGLN), mRNA. | 0,038899 | 0,363435 |
| NM_003212 | teratocarcinoma-derived growth factor 1 (TDGF1), mRNA. | 0,094934 | 0,213788 |
| NM_003265 | toll-like receptor 3 (TLR3), mRNA. | 0,072695 | -0,34201 |
| NM_003276 | thymopoietin (TMPO), mRNA. | 0,053471 | -0,27181 |
| NM_003298 | nuclear receptor subfamily 2, group C, member 2 (NR2C2), mRNA. | 0,085307 | -0,20389 |
| NM_003359 | UDP-glucose dehydrogenase (UGDH), mRNA. | 0,080045 | -0,28442 |
| NM_003411 | zinc finger protein, Y-linked (ZFY), mRNA. | 0,050424 | -0,82401 |
| NM_003526 | H2B histone family, member L (H2BFL), mRNA. | 0,094617 | 0,195215 |
| NM_003576 | serine/threonine kinase 24 (STE20 H.log, yeast) (STK24), mRNA. | 0,002424 | 0,304764 |
| NM_003600 | serine/threonine kinase 6 (STK6), mRNA. | 0,024589 | 2,388597 |
| NM_003615 | solute carrier family 4, sodium bicarbonate cotransporter, member 7 (SLC4A7), mRNA. | 0,080739 | 0,18244 |
| NM_003617 | regulator of G-protein signalling 5 (RGS5), mRNA. | 0,062383 | 0,377022 |
| NM_003731 | Sjogren's syndrome nuclear autoantigen 1 (SSNA1), mRNA. | 0,058099 | 0,218273 |
| NM_003747 | tankyrase, TRF1-interacting ankyrin-related ADP-ribose polymerase (TNKS), mRNA. | 0,011251 | -0,39827 |
| NM_003804 | receptor (TNFRSF)-interacting serine-threonine kinase 1 (RIPK1), mRNA. | 0,059712 | -0,30649 |
| NM_003839 | tumor necrosis factor receptor superfamily, member 11a, activator ofNFKB (TNFRSF11A), mRNA. | 0,075565 | -1,63975 |
| NM_003850 | succinate-CoA ligase, ADP-forming, beta subunit (SUCLA2), mRNA. | 5,99E-07 | 0,268062 |
| NM_003859 | dolichyl-phosphate mannosyltransferase polypeptide 1, catalytic subunit (DPM1), mRNA. | 1,29E-06 | 3,32635 |
| NM_003880 | WNT1 inducible signaling pathway protein 3 (WISP3), transcript variant 1, mRNA. | 0,099712 | -0,19109 |
| NM_003899 | Rho guanine nucleotide exchange factor (GEF) 7 (ARHGEF7), transcript variant 1, mRNA. | 0,005352 | 0,20227 |
| NM_003903 | CDC16 cell division cycle 16 H.log (S. cerevisiae) (CDC16), mRNA. | 0,069317 | 0,138743 |
| NM_004001 | Fc fragment of IgG, low affinity IIb, receptor for (CD32) (FCGR2B), mRNA. | 0,008614 | 0,295299 |
| NM_004107 | Fc fragment of IgG, receptor, transporter, alpha | 0,01799 | 0,326969 |
| | (FCGRT), mRNA. | | |
| NM_004128 | general transcription factor IIF, polypeptide 2 (30kD subunit) (GTF2F2), mRNA. | 0,06872 | 0,200892 |
| NM_004139 | lipopolysaccharide binding protein (LBP), mRNA. | 0,069786 | 0,18535 |
| NM_004232 | suppressor of cytokine signaling 4 (SOCS4), mRNA. | 0,041674 | -0,30051 |
| NM_004237 | thyroid hormone receptor interactor 13 (TRIP13), mRNA. | 0,075565 | 2,118122 |
| NM_004242 | high mobility group nucleosomal binding domain 3 (HMGN3), transcript variant 1, mRNA. | 0,09616 | -0,1732 |
| NM_004255 | cytochrome c oxidase subunit Va (COX5A), nuclear gene encoding mitochondrial protein, mRNA. | 0,063252 | -0,1947 |
| NM_004315 | N-acylsphingosine amidohydrolase (acid ceramidase) 1 (ASAH1), mRNA. | 0,08747 | -0,26787 |
| NM_004323 | BCL2-associated athanogene (BAG1), mRNA. | 0,048517 | -0,3164 |
| NM_004385 | chondroitin sulfate proteoglycan 2 (versican) (CSPG2), mRNA. | 0,099564 | 1,774975 |
| NM_004403 | deafness, autosomal dominant 5 (DFNA5), mRNA. | 0,090912 | 1,171547 |
| NM_004407 | dentin matrix acidic phosphoprotein (DMP1), mRNA. | 0,099429 | 0,268602 |
| NM_004453 | electron-transferring-flavoprotein dehydrogenase (ETFDH), nuclear gene encoding mitochondrial protein, mRNA. | 0,069033 | -0,20405 |
| NM_004539 | asparaginyl-tRNA synthetase (NARS), mRNA. | 0,000944 | -0,24154 |
| NM_004545 | NADH dehydrogenase (ubiquinone) 1 beta subcomplex, 1, 7kDa (NDUFB1), mRNA. | 0,093866 | -0,17512 |
| NM_004588 | sodium channel, voltage-gated, type II, beta polypeptide (SCN2B), mRNA. | 0,057233 | -0,19156 |
| NM_004599 | sterol regulatory element binding transcription factor 2 (SREBF2), mRNA. | 0,059015 | -0,24902 |
| NM_004603 | syntaxin 1A (brain) (STX1A), mRNA. | 0,080006 | 0,402681 |
| NM_004653 | Smcy H.log, Y chromosome (mouse) (SMCY), mRNA. | 0,042857 | -0,44454 |
| NM_004657 | serum deprivation response (phosphatidylserine | 0,099331 | 0,247845 |
| | binding protein) (SDPR), mRNA. | | |
| NM_004669 | chloride intracellular channel 3 (CLIC3), mRNA. | 0,082476 | -0,69518 |
| NM_004699 | DNA segment on chromosome X (unique) 9928 expressed sequence (DXS9928E), mRNA. | 0,084748 | 1,004674 |
| NM_004704 | U3 snoRNP-associated 55-kDa protein (U3-55K), mRNA. | 0,039111 | 0,27622 |
| NM_004715 | CTD (carboxy-terminal domain, RNA polymerase II, polypeptide A) phosphatase, subunit 1 (CTDP1), transcript variant FCPla, mRNA. | 0,000824 | -0,19524 |
| NM_004755 | ribosomal protein S6 kinase, 90kDa, polypeptide 5 (RPS6KA5), mRNA. | 0,085964 | -0,18116 |
| NM_004776 | UDP-Gal:betaGlcNAc beta 1,4-galactosyltransferase, polypeptide 5 (B4GALT5), mRNA. | 0,091532 | 0,160436 |
| NM_004786 | thioredoxin-like, 32kDa (TXNL), mRNA. | 0,036825 | -0,19712 |
| NM_004891 | mitochondrial ribosomal protein L33 (MRPL33), nuclear gene encoding mitochondrial protein, transcript variant 1, mRNA. | 0,041674 | 0,175935 |
| NM_004901 | lysosomal apyrase-like 1 (LYSAL1), mRNA. | 0,006725 | -0,23079 |
| NM_004925 | aquaporin 3 (AQP3), mRNA. | 0,057233 | -0,42326 |
| NM_004993 | Machado-Joseph disease (spinocerebellar ataxia 3, olivopontocerebellar ataxia 3, autosomal dominant, ataxin 3) (MJD), transcript variant 1, mRNA. | 0,06159 | -0,3148 |
| NM_005015 | oxidase (cytochrome c) assembly 1-like (OXA1L), mRNA. | 0,094327 | -0,15643 |
| NM_005054 | RAN binding protein 2-like 1 (RANBP2L1), transcript variant 1, mRNA. | 0,04739 | -0,283 |
| NM_005080 | X-box binding protein 1 (XBP1), mRNA. | 0,094327 | -0,24926 |
| NM_005104 | bromodomain containing 2 (BRD2), mRNA. | 0,08865 | -0,16248 |
| NM_005273 | guanine nucleotide binding protein (G protein), beta polypeptide 2 (GNB2), mRNA. | 0,070066 | 0,334504 |
| NM_005276 | glycerol-3-phosphate dehydrogenase 1 (soluble) (GPD1), mRNA. | 0,08722 | -0,23882 |
| NM_005313 | glucose regulated protein, 58kDa (GRP58), mRNA. | 0,093989 | -0,29814 |
| NM 005339 | huntingtin interacting protein 2 (HIP2), mRNA. | 0,094327 | -0,31967 |
| NM_005358 | LIM domain only 7 (LMO7), transcript variant 1, mRNA. | 0,042174 | 0,251855 |
| NM_005359 | MAD, mothers against decapentaplegic H.log 4 (Drosophila) (MADH4), mRNA. | 0,044664 | -0,33258 |
| NM_005374 | membrane protein, palmitoylated 2 (MAGUK p55 subfamily member 2) (MPP2), mRNA. | 0,063252 | -0,174 |
| NM_005415 | solute carrier family 20 (phosphate transporter), member 1 (SLC20A1), mRNA. | 0,08969 | 0,258846 |
| NM_005469 | peroxisomal acyl-CoA thioesterase (PTE1), mRNA. | 0,080556 | 0,336772 |
| NM_005531 | interferon, gamma-inducible protein 16 (IFI16), mRNA. | 0,092312 | -0,36367 |
| NM_005538 | inhibin, beta C (INHBC), mRNA. | 0,057886 | 1,147071 |
| NM_005618 | delta-like 1 (Drosophila) (DLL1), mRNA. | 0,058883 | -1,2942 |
| NM_005637 | synovial sarcoma translocation, chromosome 18 (SS18), mRNA. | 0,077078 | -0,13342 |
| NM_005870 | sin3-associated polypeptide, 18kDa (SAP18), mRNA. | 0,020691 | 0,219704 |
| NM_005877 | splicing factor 3a, subunit 1, 120kDa (SF3A1), mRNA. | 0,023629 | -1,49304 |
| NM_005944 | antigen identified by monoclonal antibody MRC OX-2 (MOX2), mRNA. | 0,094934 | -0,20733 |
| NM_005955 | metal-regulatory transcription factor 1(MTF1), mRNA. | 0,076544 | 0,192013 |
| NM_005959 | melatonin receptor 1B (MTNR1B), mRNA. | 0,069786 | -0,31553 |
| NM_006022 | transforming growth factor beta-stimulated protein TSC-22 (TSC22), mRNA. | 0,04116 | 0,203489 |
| NM_006097 | myosin regulatory light chain 2, smooth muscle isoform (MYRL2), mRNA. | 0,062383 | 0,338708 |
| NM_006117 | peroxisomal D3,D2-enoyl-CoA isomerase (PECI), mRNA. | 0,050505 | -0,36647 |
| NM_006150 | LIM domain only 6 (LMO6), mRNA. | 0,089785 | 1,102005 |
| NM_006197 | pericentriolar material 1(PCM1), mRNA. | 0,045178 | -0,27764 |
| NM_006215 | serine (or cysteine) proteinase inhibitor, clade A | 0,042174 | 0,444696 |
| | (alpha-1 antiproteinase, antitrypsin), member 4 (SERPINA4), mRNA. | | |
| NM_006251 | protein kinase, AMP-activated, alpha 1 catalytic subunit (PRKAA1), mRNA. | 0,039111 | -0,32277 |
| NM_006259 | protein kinase, cGMP-dependent, type II (PRKG2), mRNA. | 0,071967 | -1,74885 |
| NM_006416 | solute carrier family 35 (CMP-sialic acid transporter), member 1 (SLC35A1), mRNA. | 0,077064 | -1,64993 |
| NM_006492 | aristaless-like homeobox 3 (ALX3), mRNA. | 0,097652 | 0,242826 |
| NM_006493 | ceroid-lipofuscinosis, neuronal 5 (CLN5), mRNA. | 0,033965 | -0,31853 |
| NM_006602 | transcription factor-like 5 (basic helix-loop-helix) (TCFL5), mRNA. | 0,032959 | 2,252188 |
| NM_006615 | calpain 9 (nCL-4) (CAPN9), mRNA. | 0,039648 | -2,76165 |
| NM_006644 | heat shock 105kD (HSP105B), mRNA. | 0,066417 | 0,411448 |
| NM_006701 | similar to S. pombe diml+ (DIM1), mRNA. | 0,085377 | -0,14439 |
| NM_006726 | LPS-responsive vesicle trafficking, beach and anchor containing (LRBA), mRNA. | 0,04717 | -0,17132 |
| NM_006746 | sex comb on midleg-like 1 (Drosophila) (SCML1), mRNA. | 0,09878 | 0,24864 |
| NM_006752 | surfeit 5 (SURF5), transcript variant a, mRNA. | 0,08747 | 0,233567 |
| NM_006769 | LIM domain only 4 (LMO4), mRNA. | 0,093866 | -0,26606 |
| NM_006796 | AFG3 ATPase family gene 3-like 2 (yeast) (AFG3L2), nuclear gene encoding mitochondrial protein, mRNA. | 0,058226 | -0,20738 |
| NM_006809 | translocase of outer mitochondrial membrane 34 (TOMM34), mRNA. | 0,03823 | 0,466704 |
| NM_006811 | tumor differentially expressed 1 (TDE1), mRNA. | 0,093866 | 0,185639 |
| NM_006825 | cytoskeleton-associated protein 4 (CKAP4), mRNA. | 0,02765 | -0,1768 |
| NM_006833 | COP9 subunit 6 (MOV34 H.log, 34 kD) (COPS6), mRNA. | 0,080045 | 0,151558 |
| NM_006839 | inner membrane protein, mitochondrial (mitofilin) (IMMT), mRNA. | 0,036825 | -0,32053 |
| NM_006886 | ATP synthase, H+ transporting, mitochondrial F1 complex, epsilon subunit (ATP5E), nuclear gene | 0,047422 | 0,207215 |
| | encoding mitochondrial protein, mRNA. | | |
| NM_006922 | sodium channel, voltage-gated, type III, alpha polypeptide (SCN3A), mRNA. | 0,082581 | -0,207 |
| NM_007002 | adhesion regulating molecule 1 (ADRM1), mRNA. | 0,047922 | 1,658766 |
| NM_007011 | abhydrolase domain containing 2 (ABHD2), transcript variant 1, mRNA. | 0,067243 | -0,33685 |
| NM_007019 | ubiquitin-conjugating enzyme E2C (UBE2C), mRNA. | 0,009792 | 2,34477 |
| NM_007035 | keratocan (KERA), mRNA. | 0,06872 | 0,345311 |
| NM_007039 | protein tyrosine phosphatase, non-receptor type 21 (PTPN21), mRNA. | 0,061998 | -0,24194 |
| NM_007055 | polymerase (RNA) III (DNA directed) (155kD) (RPC155), mRNA. | 0,050505 | 0,14563 |
| NM_007125 | ubiquitously transcribed tetratricopeptide repeat gene, Y chromosome (UTY), mRNA. | 0,077966 | -0,61812 |
| NM_007187 | WW domain binding protein 4 (formin binding protein 21) (WBP4), mRNA. | 0,088138 | 1,148173 |
| NM_007236 | calcium binding protein P22 (CHP), mRNA. | 0,057301 | -0,20633 |
| NM_007253 | cytochrome P450, subfamily IVF, polypeptide 8 (CYP4F8), mRNA. | 0,062383 | -0,40672 |
| NM_007342 | nucleoporin-like protein 1 (NLP_1), mRNA. | 0,066671 | 0,137201 |
| NM_007345 | zinc finger protein 236 (ZNF236), mRNA. | 0,094934 | -0,17085 |
| NM_009590 | amine oxidase, copper containing 2 (retina-specific) (AOC2), transcript variant 2, mRNA. | 0,094327 | 0,458948 |
| NM_012112 | chromosome 20 open reading frame 1 (C20orf1), mRNA. | 0,033163 | 2,151017 |
| NM_012142 | cyclin D-type binding-protein 1 (CCNDBP1), transcript variant 1, mRNA. | 0,098621 | -0,30022 |
| NM_012155 | echinoderm microtubule associated protein like 2 (EML2), mRNA. | 0,033412 | -0,45142 |
| NM_012158 | F-box and leucine-rich repeat protein 3A (FBXL3A), mRNA. | 0,063252 | 0,264664 |
| NM_012180 | F-box only protein 8 (FBX08), mRNA. | 0,071755 | 0,252412 |
| NM_012190 | formyltetrahydrofolate dehydrogenase (FTHFD), | 0,023629 | -2,42935 |
| | transcript variant 1, mRNA. | | |
| NM_012331 | methionine sulfoxide reductase A (MSRA), mRNA. | 0,069786 | -0,22314 |
| NM_012392 | PEF protein with a long N-terminal hydrophobic domain (peflin) (PEF), mRNA. | 0,082476 | 0,169837 |
| NM_012449 | six transmembrane epithelial antigen of the prostate (STEAP), mRNA. | 0,020473 | -0,32771 |
| NM_012461 | TERF1 (TRF1)-interacting nuclear factor 2 (TINF2), mRNA. | 0,061202 | -0,19784 |
| NM_012469 | chromosome 20 open reading frame 14 (C20orf14), mRNA. | 0,033429 | 1,617033 |
| NM_013354 | CCR4-NOT transcription complex, subunit 7 (CNOT7), transcript variant 1, mRNA. | 0,054537 | -0,19074 |
| NM_013362 | zinc finger protein 225 (ZNF225), mRNA. | 0,056597 | -0,48787 |
| NM_013390 | transmembrane protein 2 (TMEM2), mRNA. | 0,047922 | -1,58866 |
| NM_013451 | fer-1-like 3, myoferlin (C. elegans) (FER1L3), transcript variant 1, mRNA. | 0,003816 | -0,26834 |
| NM_013974 | dimethylarginine dimethylaminohydrolase 2 (DDAH2), mRNA. | 0,08969 | 0,211115 |
| NM_014052 | GW128 protein (GW128), mRNA. | 0,000219 | 2,75826 |
| NM_014065 | HT001 protein (HT001), mRNA. | 0,082581 | 0,175696 |
| NM_014066 | HT002 protein, hypertension-related calcium-regulated gene (HT002), mRNA. | 0,043458 | 0,183686 |
| NM_014071 | nuclear receptor coactivator 6 (NCOA6), mRNA. | 0,082476 | 0,270178 |
| NM_014177 | HSPC154 protein (HSPC154), mRNA. | 0,004542 | -0,22578 |
| NM_014282 | hyaluronan binding protein 4 (HABP4), mRNA. | 0,062383 | 0,27001 |
| NM_014353 | RAB26, member RAS oncogene family (RAB26), mRNA. | 0,016631 | -0,43657 |
| NM_014444 | gamma tubulin ring complex protein (76p gene) (76P), mRNA. | 0,089116 | 0,843388 |
| NM_014471 | serine protease inhibitor, Kazal type 4 (SPINK4), mRNA. | 0,086303 | -0,41374 |
| NM_014593 | CpG binding protein (CGBP), mRNA. | 0,089567 | -0,16544 |
| NM_014622 | loss of heterozygosity, 11, chromosomal region 2, gene A (LOH11CR2A), mRNA. | 0,039648 | -1,81638 |
| NM_014636 | Ral guanine nucleotide exchange factor RalGPS1A (RALGPS1A), mRNA. | 0,062383 | -0,19961 |
| NM_014657 | KIAA0406 gene product (KIAA0406), mRNA. | 0,067336 | 0,273531 |
| NM_014729 | thymus high mobility group box protein TOX (TOX), mRNA. | 0,029713 | -0,38597 |
| NM_014747 | KIAA0237 gene product (KIAA0237), mRNA. | 0,051215 | -1,51108 |
| NM_014766 | KIAA0193 gene product (KIAA0193), mRNA. | 0,013744 | 4,253342 |
| NM_014786 | Rho-specific guanine-nucleotide exchange factor 164 kDa (P164RHOGEF), mRNA. | 0,066275 | 0,223783 |
| NM_014849 | synaptic vesicle glycoprotein 2 (SV2), mRNA. | 0,066266 | 0,151549 |
| NM_014885 | anaphase-promoting complex 10 (APC10), mRNA. | 0,066417 | 0,240729 |
| NM_014887 | hypothetical protein from BCRA2 region (CG005), mRNA. | 0,080338 | 0,233766 |
| NM_014902 | KIAA0964 protein (KIAA0964), mRNA. | 0,047922 | 1,345732 |
| NM_015070 | KIAA0853 protein (KIAA0853), mRNA. | 0,078188 | 0,196796 |
| NM_015127 | Mid-1-related chloride channel 1 (MCLC), mRNA. | 0,050505 | -0,25616 |
| NM_015338 | KIAA0978 protein (KIAA0978), mRNA. | 0,089651 | 0,250409 |
| NM_015476 | DKFZP586M1523 protein (DKFZP586M1523), mRNA. | 0,098987 | -0,1933 |
| NM_015512 | DKFZP434A236 protein (DKFZP434A236), mRNA. | 0,063004 | 0,254703 |
| NM_015540 | DKFZP727M111 protein (DKFZP727M111), mRNA. | 0,062383 | 0,167647 |
| NM_015666 | GTP binding protein 5 (putative) (GTPBP5), mRNA. | 0,094327 | 0,261554 |
| NM_015705 | hypothetical protein DJ1042K10.2 (DJ1042K10.2), mRNA. | 0,061202 | -0,39612 |
| NM_015721 | gem (nuclear organelle) associated protein 4 (GEMIN4), mRNA. | 0,070946 | 0,228449 |
| NM_015832 | methyl-CpG binding domain protein 2 (MBD2), transcript variant testis-specific, mRNA. | 0,067336 | -0,17895 |
| NM_015949 | CGI-20 protein (CGI-20), mRNA. | 0,095037 | 0,227496 |
| NM_015962 | CGI-35 protein (CGI-35), mRNA. | 0,079602 | -0,30982 |
| NM_016004 | chromosome 20 open reading frame 9 (C20orf9), | 0,094934 | 0,21615 |
| | mRNA. | | |
| NM_016014 | CGI-67 protein (CGI-67), mRNA. | 0,09289 | -0,24249 |
| NM_016127 | hypothetical protein MGC8721 (MGC8721), mRNA. | 0,061202 | -0,20445 |
| NM_016299 | likely ortholog of mouse heat shock protein, 70 kDa 4 (HSP70-4), mRNA. | 0,062237 | 0,168397 |
| NM_016305 | synovial sarcoma translocation gene on chromosome 18-like 2 (SS18L2), mRNA. | 0,082476 | 0,194934 |
| NM_016308 | UMP-CMP kinase (UMP-CMPK), mRNA. | 0,002075 | -0,25909 |
| NM_016332 | selenoprotein X, 1 (SEPX1), mRNA. | 0,09289 | 0,201764 |
| NM_016354 | solute carrier family 21 (organic anion transporter), member 12 (SLC21A12), mRNA. | 0,080013 | 1,550246 |
| NM_016397 | TH1-like (Drosophila) (TH1L), mRNA. | 0,006583 | 1,918519 |
| NM_016400 | Huntingtin interacting protein K (HYPK), mRNA. | 0,090912 | -1,04747 |
| NM_016569 | T-box 3 (ulnar mammary syndrome) (TBX3), transcript variant 2, mRNA. | 0,08865 | -0,41763 |
| NM_016594 | FK506 binding protein 11, 19 kDa (FKBP11), mRNA. | 0,066266 | -0,23327 |
| NM_016626 | hypothetical protein LOC51320 (LOC51320), mRNA. | 0,036126 | -0,22033 |
| NM_016646 | mesenchymal stem cell protein DSCD28 (LOC51336), mRNA. | 0,021802 | 1,439666 |
| NM_016938 | EGF-containing fibulin-like extracellular matrix protein 2 (EFEMP2), mRNA. | 0,059763 | 1,522942 |
| NM_017417 | UDP-N-acetyl-alpha-D-galactosamine:polypeptide N-acetylgalactosaminyltransferase 8 (GalNAc-T8) (GALNT8), mRNA. | 0,095888 | -0,40833 |
| NM_017495 | RNA-binding region (RNP1, RRM) containing 1 (RNPC1), mRNA. | 0,039255 | 1,951461 |
| NM_017540 | UDP-N-acetyl-alpha-D-galactosamine:polypeptide N-acetylgalactosaminyltransferase 10 (GalNAc-T10) (GALNT10), mRNA. | 0,094934 | -0,1997 |
| NM_017699 | hypothetical protein FLJ20174 (FLJ20174), mRNA. | 0,039648 | -2,23626 |
| NM_017707 | up-regulated in liver cancer 1 (UPLC1), mRNA. | 0,096054 | -0,32197 |
| NM_017709 | hypothetical protein FLJ20202 (FLJ20202), mRNA. | 0,079602 | -0,5167 |
| NM_017750 | hypothetical protein FLJ20296 (FLJ20296), mRNA. | 0,01679 | -0,24488 |
| NM_017762 | hypothetical protein FLJ20313 (FLJ20313), mRNA. | 0,075595 | -0,32046 |
| NM_017817 | RAB20, member RAS oncogene family (RAB20), mRNA. | 0,093866 | 0,157212 |
| NM_017872 | hypothetical protein FLJ20546 (FLJ20546), mRNA. | 0,038899 | -0,24664 |
| NM_017895 | DEAD/H (Asp-Glu-Ala-Asp/His) box polypeptide 27 (DDX27), mRNA. | 0,066417 | 0,283457 |
| NM_017896 | chromosome 20 open reading frame 11 (C20orf11), mRNA. | 0,000925 | 1,93846 |
| NM_017933 | hypothetical protein FLJ20701 (FLJ20701), mRNA. | 0,048261 | -0,35263 |
| NM_018043 | hypothetical protein FLJ10261 (FLJ10261), mRNA. | 0,070596 | -0,50301 |
| NM_018142 | hypothetical protein FLJ10569 (FLJ10569), mRNA. | 0,081888 | -0,30228 |
| NM_018147 | Fas apoptotic inhibitory molecule (FAIM), mRNA. | 0,085964 | -0,34657 |
| NM_018154 | hypothetical protein FLJ10604 (FLJ10604), mRNA. | 0,047422 | 0,229376 |
| NM_018203 | hypothetical protein FLJ10748 (FLJ10748), mRNA. | 0,061202 | -0,38393 |
| NM_018206 | vacuolar protein sorting 35 (yeast) (VPS35), mRNA. | 0,015656 | 0,147472 |
| NM_018235 | hypothetical protein FLJ10830 (FLJ10830), mRNA. | 0,095161 | -0,18303 |
| NM_018244 | chromosome 20 open reading frame 44 (C20orf44), mRNA. | 0,067511 | 1,488465 |
| NM_018246 | hypothetical protein FLJ10853 (FLJ10853), mRNA. | 0,094327 | -0,19685 |
| NM_018270 | chromosome 20 open reading frame 20 (C20orf20), mRNA. | 0,042112 | 0,977697 |
| NM_018326 | hypothetical protein FLJ11110 (FLJ11110), mRNA. | 0,069195 | 0,141442 |
| NM_018360 | chromosome X open reading frame 15 (CXorf15), mRNA. | 0,092312 | 0,341923 |
| NM_018386 | hypothetical protein FLJ11305 (FLJ11305), mRNA. | 0,022216 | 0,248688 |
| NM_018388 | CHCR (CHCR), mRNA. | 0,005045 | -0,54868 |
| NM_018414 | GalNAc alpha-2, 6-sialyltransferase I, long form (ST6GalNAcI), mRNA. | 0,024589 | -3,79484 |
| NM_018422 | hypothetical protein DKFZp761K1423 (DKFZp761K1423), mRNA. | 0,070503 | -0,44371 |
| NM_018439 | hypothetical protein IMPACT (IMPACT), mRNA. | 0,075897 | -0,35011 |
| NM_018488 | T-box 4 (TBX4), mRNA. | 0,06159 | 0,311215 |
| NM_018559 | lipopolysaccharide specific response-7 protein (LSR7), mRNA. | 0,042174 | 0,270419 |
| NM_018569 | hypothetical protein PRO0971 (PRO0971), mRNA. | 0,079602 | 0,253986 |
| NM_018662 | disrupted in schizophrenia 1 (DISC1), mRNA. | 0,047422 | -0,44603 |
| NM_018690 | apolipoprotein B48 receptor (APOB48R), mRNA. | 0,016058 | -2,61738 |
| NM_018710 | hypothetical protein DKFZp762O076 (DKFZp762O076), mRNA. | 0,039255 | 1,675976 |
| NM_018840 | putative Rab5-interacting protein (RIP5), mRNA. | 0,000614 | 2,36608 |
| NM_018962 | Down syndrome critical region gene 6 (DSCR6), mRNA. | 0,047422 | 0,320234 |
| NM_019016 | hypothetical protein FLJ20261 (FLJ20261), mRNA. | 0,081109 | -0,26741 |
| NM_019079 | hypothetical protein FLJ10884 (FLJ10884), mRNA. | 0,025102 | -0,63052 |
| NM_019846 | chemokine (C-C motif) ligand 28 (CCL28), transcript variant 1, mRNA. | 0,083402 | -0,40981 |
| NM_019853 | protein phosphatase 4, regulatory subunit 2 (PPP4R2), mRNA. | 0,090725 | 0,668805 |
| NM_020169 | latexin protein (LXN), mRNA. | 0,040379 | -0,22413 |
| NM_020182 | transmembrane, prostate androgen induced RNA (TMEPAI), mRNA. | 0,094327 | 0,390529 |
| NM_020188 | DC13 protein (DC13), mRNA. | 0,048384 | 2,401407 |
| NM_020232 | hepatocellular carcinoma susceptibility protein (HCCA3), mRNA. | 0,027924 | -0,1674 |
| NM_020233 | x 006 protein (MDS006), mRNA. | 0,09234 | -1,24087 |
| NM_020299 | aldo-keto reductase family 1, member B10 (aldose reductase) (AKR1B10), mRNA. | 0,083612 | -0,36368 |
| NM_020532 | reticulon 4 (RTN4), mRNA. | 0,010896 | 1,712038 |
| NM_020650 | hypothetical protein LOC57333 (LOC57333), mRNA. | 0,043572 | 0,474606 |
| NM_020673 | RAB22A, member RAS oncogene family (RAB22A), mRNA. | 0,08865 | 0,229513 |
| NM_020749 | AT2 receptor-interacting protein 1 (ATIP1), mRNA. | 0,021177 | -0,36043 |
| NM_020750 | exportin 5 (XP05), mRNA. | 0,091934 | 0,216215 |
| NM_020806 | gephyrin (GPHN), mRNA. | 0,069786 | -0,19149 |
| NM_020831 | megakaryoblastic leukemia (translocation) 1 | 0,075565 | -1,03031 |
| | (MKL1), mRNA. | | |
| NM_020962 | likely ortholog of mouse neighbor of Punc E11 (NOPE), mRNA. | 0,099737 | 0,320891 |
| NM_021033 | RAP2A, member of RAS oncogene family (RAP2A), mRNA. | 0,010657 | 0,314425 |
| NM_021095 | solute carrier family 5 (sodium-dependent vitamin transporter), member 6 (SLC5A6), mRNA. | 0,039111 | 0,378796 |
| NM_021158 | chromosome 20 open reading frame 97 (C20orf97), mRNA. | 0,01059 | 2,067057 |
| NM_021246 | lymphocyte antigen 6 complex, locus G6D (LY6G6D), mRNA. | 0,036825 | 0,781552 |
| NM_021258 | interleukin 22 receptor (IL22R), mRNA. | 0,081132 | 0,515985 |
| NM_021637 | hypothetical protein FLJ14084 (FLJ14084), mRNA. | 0,071297 | -0,36458 |
| NM_021724 | nuclear receptor subfamily 1, group D, member 1 (NR1D1), mRNA. | 0,061202 | 0,392836 |
| NM_021738 | supervillin (SVIL), transcript variant 2, mRNA. | 0,03532 | -1,04373 |
| NM_021809 | TGFB-induced factor 2 (TALE family homeobox) (TGIF2), mRNA. | 0,002932 | 2,265688 |
| NM_021911 | gamma-aminobutyric acid (GABA) A receptor, beta 2 (GABRB2), transcript variant 1, mRNA. | 0,019169 | -6,04422 |
| NM_021928 | hypothetical protein FLJ22649 similar to signal peptidase SPC22/23 (FLJ22649), mRNA. | 0,007549 | -0,22392 |
| NM_021945 | hypothetical protein FLJ22174 (FLJ22174), mRNA. | 0,047922 | -1,93072 |
| NM_022062 | PBX/knotted 1 homeobox 2 (PKNOX2), mRNA. | 0,03775 | 0,266646 |
| NM_022082 | chromosome 20 open reading frame 59 (C20orf59), mRNA. | 0,006964 | 3,252232 |
| NM_022130 | golgi phosphoprotein 3 (coat-protein) (GOLPH3), mRNA. | 0,041674 | -0,30666 |
| NM_022157 | Rag C protein (GTR2), mRNA. | 0,061202 | 0,180955 |
| NM_022461 | hypothetical protein FLJ21939 similar to 5-azacytidine induced gene 2 (FLJ21939), mRNA. | 0,09769 | 0,167686 |
| NM_023011 | similar to yeast Upf3, variant A (UPF3A), transcript variant 1, mRNA. | 0,099014 | 0,151456 |
| NM_024035 | hypothetical protein MGC3113 (MGC3113), | 0,095434 | 0,287896 |
| | mRNA. | | |
| NM_024075 | LENG5 protein (LENG5), mRNA. | 0,079602 | 0,149324 |
| NM_024090 | long-chain fatty-acyl elongase (LCE), mRNA. | 0,016631 | 0,199665 |
| NM_024096 | hypothetical protein MGC5627 (MGC5627), mRNA. | 0,056597 | 0,218944 |
| NM_024110 | caspase recruitment domain family, member 14 (CARD 14), transcript variant 1, mRNA. | 0,016631 | 0,235476 |
| NM_024115 | hypothetical protein MGC4309 (MGC4309), mRNA. | 0,000227 | 1,946961 |
| NM_024426 | Wilms tumor 1 (WT1), transcript variant D, mRNA. | 0,02963 | 0,688737 |
| NM_024490 | ATPase, Class V, type 10A (ATP10A), mRNA. | 0,080006 | 0,351773 |
| NM_024491 | p10-binding protein (BITE), mRNA. | 0,050505 | -0,26034 |
| NM_024513 | FYVE and coiled-coil domain containing 1 (FYCO1), mRNA. | 0,085549 | -0,21209 |
| NM_024522 | hypothetical protein FLJ12650 (FLJ12650), mRNA. | 0,000722 | 2,084454 |
| NM_024533 | hypothetical protein FLJ22167 (FLJ22167), mRNA. | 0,072191 | -1,97266 |
| NM_024537 | hypothetical protein FLJ12118 (FLJ12118), mRNA. | 0,095468 | 0,173686 |
| NM_024556 | hypothetical protein FLJ21103 (FLJ21103), mRNA. | 0,088833 | -0,18109 |
| NM_024618 | hypothetical protein FLJ21478 (FLJ21478), transcript variant 1, mRNA. | 0,093324 | -0,23144 |
| NM_024631 | hypothetical protein FLJ23342 (FLJ23342), mRNA. | 0,082581 | 0,153323 |
| NM_024725 | hypothetical protein FLJ23518 (FLJ23518), mRNA. | 0,084091 | -1,23218 |
| NM_024761 | hypothetical protein FLJ13204 (FLJ13204), mRNA. | 0,077076 | -0,18086 |
| NM_024792 | membrane protein expressed in epithelial-like lung adenocarcinoma (CT120), mRNA. | 0,09878 | -0,17414 |
| NM_024861 | hypothetical protein FLJ22671 (FLJ22671), mRNA. | 0,09616 | 0,405449 |
| NM_024868 | hypothetical protein FLJ14124 (FLJ14124), mRNA. | 0,099014 | -0,31056 |
| NM_024898 | hypothetical protein FLJ22757 (FLJ22757), mRNA. | 0,047422 | -0,17724 |
| NM_024928 | hypothetical protein FLJ22559 (FLJ22559), mRNA. | 0,044215 | -0,17755 |
| NM_025047 | hypothetical protein FLJ22595 (FLJ22595), mRNA. | 0,08865 | -0,24253 |
| NM_025067 | hypothetical protein FLJ14106 (FLJ14106), mRNA. | 0,034774 | 0,136761 |
| NM_025138 | hypothetical protein FLJ12661 (FLJ12661), transcript variant 1, mRNA. | 0,002424 | 0,273094 |
| NM_025187 | hypothetical protein FLJ12076 (FLJ12076), mRNA. | 0,028321 | 0,19899 |
| NM_025194 | inositol 1,4,5-trisphosphate 3-kinase C (ITPKC), mRNA. | 0,084748 | -1,99891 |
| NM_025209 | enhancer of polycomb H.log 1, (Drosophila) (EPC1), mRNA. | 0,08865 | 0,132535 |
| NM_030574 | START domain containing 5 (STARD5), mRNA. | 0,051215 | -1,1568 |
| NM_030579 | cytochrome b5 outer mitochondrial membrane precursor (CYB5-M), mRNA. | 0,012808 | 0,247417 |
| NM_030582 | collagen, type XVIII, alpha 1 (COL18A1), transcript variant 1, mRNA. | 0,094767 | 0,319311 |
| NM_030763 | nucleosomal binding protein 1 (NSBP1), mRNA. | 0,094327 | -0,31628 |
| NM_030789 | histocompatibility (minor) 13 (HM13), mRNA. | 0,032959 | 1,338115 |
| NM_030815 | chromosome 20 open reading frame 126 (C20orf126), mRNA. | 0,02963 | 0,252626 |
| NM_030877 | catenin, beta like 1 (CTNNBL1), mRNA. | 0,066566 | 0,248269 |
| NM 031266 | heterogeneous nuclear ribonucleoprotein A/B (HNRPAB), transcript variant 1, mRNA. | 0,085964 | -0,1542 |
| NM_031285 | hypothetical protein PP1057 (PP1057), mRNA. | 0,024446 | 0,44688 |
| NM_031299 | likely ortholog of mouse gene rich cluster, C8 gene (GRCC8), mRNA. | 0,08969 | 0,337448 |
| NM_031413 | cat eye syndrome chromosome region, candidate 2 (CECR2), mRNA. | 0,085475 | -1,83919 |
| NM_031453 | hypothetical protein MGC11034 (MGC11034), mRNA. | 0,092641 | -0,30736 |
| NM_031472 | hypothetical protein MGC11134 (MGC11134), mRNA. | 0,060975 | -0,26471 |
| NM_031920 | ARG99 protein (ARG99), mRNA. | 0,050505 | 0,208365 |
| NM_031966 | cyclin B1 (CCNB1), mRNA. | 0,047922 | 1,637206 |
| NM_032013 | NDRG family member 3 (NDRG3), mRNA. | 0,097652 | 0,159868 |
| NM_032044 | regenerating gene type IV (REG-IV), mRNA. | 0,080045 | -0,79212 |
| NM_032125 | hypothetical protein DKFZp564D0478 (DKFZP564D0478), mRNA. | 0,069786 | 0,212384 |
| NM_032229 | hypothetical protein FLJ22774 (FLJ22774), mRNA. | 0,016631 | -0,76877 |
| NM_032242 | hypothetical protein DKFZp564A176 (DKFZp564A176), mRNA. | 0,052079 | 0,249169 |
| NM_032352 | hypothetical protein MGC11296 (MGC11296), mRNA. | 0,066266 | 0,132448 |
| NM_032432 | actin binding LIM protein 2 (ABLIM2), mRNA. | 0,015656 | 0,232291 |
| NM_032527 | hypothetical protein FLJ14972 (KIAA1847), mRNA. | 0,067336 | 0,153376 |
| NM_032576 | chromosome Y open reading frame 15B (CYorf15B), mRNA. | 0,092312 | -0,51647 |
| NM_032579 | colon and small intestine-specific cysteine-rich protein precursor (HXCP2), mRNA. | 0,077064 | -4,92307 |
| NM_032611 | protein tyrosine phosphatase type IVA, member 3 (PTP4A3), transcript variant 1, mRNA. | 0,01539 | 0,370319 |
| NM_032633 | hypothetical protein MGC5457 (MGC5457), mRNA. | 0,067336 | 0,171888 |
| NM_032709 | hypothetical protein MGC13047 (MGC13047), mRNA. | 0,065196 | -0,35449 |
| NM_032744 | hypothetical protein MGC12335 (MGC12335), mRNA. | 0,057886 | -3,23807 |
| NM_032803 | solute carrier family 7 (cationic amino acid transporter, y+ system), member 3 (SLC7A3), mRNA. | 0,074519 | 0,707353 |
| NM_032813 | hypothetical protein FLJ14624 (FLJ14624), mRNA. | 0,003078 | 0,266088 |
| NM_032827 | hypothetical protein FLJ14708 (FLJ14708), mRNA. | 0,047016 | -0,48154 |
| NM_032846 | RAB2B (RAB2B), mRNA. | 0,071755 | -0,24439 |
| NM_032857 | lactamase, beta (LACTB), transcript variant 1, nuclear gene encoding mitochondrial protein, mRNA. | 0,092221 | -0,22271 |
| NM_032862 | tigger transposable element derived 5 (TIGD5), mRNA. | 0,041674 | 0,319249 |
| NM_032878 | hypothetical protein MGC15677 (MGC15677), mRNA. | 0,080006 | 0,284438 |
| NM_033111 | CG016 (LOC88523), mRNA. | 0,058945 | 0,425126 |
| NM_033120 | naked cuticle H.log 2 (Drosophila) (NKD2), mRNA. | 0,050505 | 0,693186 |
| NM_033126 | serine/threonine kinase PSKH2 (PSKH2), mRNA. | 0,080206 | 1,480433 |
| NM 033339 | caspase 7, apoptosis-related cysteine protease | 0,058672 | -1,40297 |
| | (CASP7), transcript variant gamma, mRNA. | | |
| NM_033342 | tripartite motif-containing 7 (TRIM7), mRNA. | 0,047971 | -0,35293 |
| NM_053055 | C-terminal modulator protein (CTMP), mRNA. | 0,043301 | -0,18836 |
| NM_053286 | aquaporin 6, kidney specific (AQP6), transcript variant 2, mRNA. | 0,029067 | -2,35392 |
| NM_057093 | crystallin, beta A2 (CRYBA2), transcript variant 2, mRNA. | 0,023543 | -0,41107 |
| NM_057158 | dual specificity phosphatase 4 (DUSP4), transcript variant 2, mRNA. | 0,089012 | -0,3644 |
| NM_078467 | cyclin-dependentkinase inhibitor 1A (p21, Cip1) (CDKN1A), transcript variant 2, mRNA. | 0,066266 | 0,196894 |
| NM_080429 | aquaporin 10 (AQP10), mRNA. | 0,069317 | 0,175585 |
| NM_080476 | CDC91 cell division cycle 91-like 1 (S. cerevisiae) (CDC91L1), mRNA. | 0,024686 | 1,600234 |
| NM_080752 | chromosome 20 open reading frame 164 (C20orf164), mRNA. | 0,081745 | 0,329131 |
| NM_130785 | TPTE and PTEN H.logous inositol lipid phosphatase (TPIP), mRNA. | 0,034774 | -0,20841 |
| NM_133170 | protein tyrosine phosphatase, receptor type, T (PTPRT), transcript variant 1, mRNA. | 0,067154 | -0,48547 |
| NM_133367 | chromosome 6 open reading frame 33 (C6orf33), mRNA. | 0,070585 | -1,81794 |
| NM_138284 | interleukin 17D (IL17D), mRNA. | 0,091053 | 0,509744 |
| NM_138340 | abhydrolase domain containing 3 (ABHD3), mRNA. | 0,071967 | -1,61731 |
| NM_138409 | hypothetical protein BC010003 (LOC112609), mRNA. | 0,023629 | -1,73317 |
| NM_138425 | hypothetical protein BC009925 (LOC113246), mRNA. | 0,005352 | -0,28087 |
| NM_138432 | likely ortholog of mouse serine dehydratase related sequence 1 (SDS-RS1), mRNA. | 0,094934 | -0,17005 |
| NM_138573 | neuregulin 4 (LOC145957), mRNA. | 0,075565 | -1,28984 |
| NM_138706 | beta-1,3-N-acetylglucosaminyltransferase protein (IMAGE:4907098), mRNA. | 0,074457 | -0,59194 |
| NM_138784 | hypothetical protein BC014341 (LOC116123), | 0,048697 | -0,36672 |
| | mRNA. | | |
| NM_138792 | hypothetical protein BC018147 (LOC123169), mRNA. | 0,093866 | -0,17206 |
| NM_138963 | ribosomal protein S4, Y-linked 2 (RPS4Y2), mRNA. | 0,080006 | -0,53287 |
| NM_139016 | hypothetical gene LOC128439 (LOC128439), mRNA. | 0,089422 | 0,233019 |
| NM_139167 | sarcoglycan zeta (SGCZ), mRNA. | 0,039902 | 0,228483 |
| NM_144498 | oxysterol binding protein-like 2 (OSBPL2), transcript variant 2, mRNA. | 0,079602 | 0,344545 |
| NM_144618 | hypothetical protein MGC29891 (MGC29891), mRNA. | 0,099417 | 0,189802 |
| NM_144641 | hypothetical protein FLJ32332 (FLJ32332), mRNA. | 0,095434 | 0,252008 |
| NM_144660 | hypothetical protein FLJ25082 (FLJ25082), mRNA. | 0,050505 | 0,302561 |
| NM_144664 | hypothetical protein MGC33371 (MGC33371), mRNA. | 0,082476 | -0,30557 |
| NM_144726 | hypothetical protein FLJ31951 (FLJ31951), mRNA. | 0,057755 | -0,27891 |
| NM_144736 | hypothetical protein PRO1853 (PRO1853), transcript variant 1, mRNA. | 0,06872 | 0,165416 |
| NM_144778 | muscleblind-like protein MBLL39 (MBLL39), transcript variant 1, mRNA. | 0,078188 | 0,218234 |
| NM_145235 | similar to RIKEN cDNA 1700007B22 (LOC92565), mRNA. | 0,051215 | -2,31424 |
| NM_145284 | similar to hypothetical protein MGC17347 (LOC159090), mRNA. | 0,067336 | 0,250469 |
| NM_145293 | similar to hypothetical protein FLJ20897 (LOC196549), mRNA. | 0,023161 | 0,187785 |
| NM_152312 | hypothetical protein FLJ35207 (FLJ35207), mRNA. | 0,082776 | 0,498205 |
| NM_152322 | hypothetical protein FLJ33957 (FLJ33957), mRNA. | 0,051731 | 0,373213 |
| NM_152332 | chromosome 14 open reading frame 47 (C14orf47), mRNA. | 0,030466 | -0,3124 |
| NM_152369 | hypothetical protein MGC45474 (MGC45474), mRNA. | 0,064718 | -1,60257 |
| NM_152400 | hypothetical protein FLJ39370 (FLJ39370), mRNA. | 0,058226 | -0,22856 |
| NM_152415 | hypothetical protein FLJ32642 (FLJ32642), mRNA. | 0,08865 | -0,17664 |
| NM_152429 | hypothetical protein MGC39320 (MGC39320), mRNA. | 0,082581 | -0,25357 |
| NM_152437 | hypothetical protein DKFZp761B128 (DKFZp761B128), mRNA. | 0,09769 | 0,400912 |
| NM_152586 | hypothetical protein FLJ37318 (FLJ37318), mRNA. | 0,080739 | -0,22746 |
| NM_152621 | hypothetical protein MGC26963 (MGC26963), mRNA. | 0,080739 | -0,39756 |
| NM_152707 | hypothetical protein MGC39851 (MGC39851), mRNA. | 0,053698 | 0,195109 |
| NM_152745 | neurexophilin 1 (NXPH1), mRNA. | 0,058683 | -0,30813 |
| NM_152777 | chromosome 14 open reading frame 48 (C14orf48), mRNA. | 0,00421 | 2,082743 |
| NM_153246 | hypothetical protein MGC45491 (MGC45491), mRNA. | 0,08969 | 0,520345 |
| NM_153336 | hypothetical protein MGC35392 (MGC35392), mRNA. | 0,071755 | -0,26883 |
| NM_153751 | chromosome 21 open reading frame 82 (C21ort82), mRNA. | 0,038975 | -0,31597 |
| NM_172171 | calcium/calmodulin-dependent protein kinase (CaM kinase) II gamma (CAMK2G), transcript variant 1, mRNA. | 0,082776 | 0,205556 |
| NM_172250 | methylmalonic aciduria type A (MMAA), mRNA. | 0,023543 | -0,34183 |
| NM_172366 | F-box protein 16 (FBX16), mRNA. | 0,09855 | -0,31506 |
| NM_173168 | SPCX (SPCX), mRNA. | 0,028702 | 0,290214 |
| U17623 | chromosome 21, Down syndrome critical region transcript, T7 end of clone a-2-d9. | 0,07505 | 0,255058 |
| U32248 | mRNA with TGG repeat, clone 83. | 0,093518 | -0,30275 |
| U33787 | immature B cell Ig heavy chain mRNA, third complementarity-determining region, clone MBT-20, partial cds. | 0,097621 | -1,07139 |
| U44954 | NMDA receptor glutamate-binding chain (hnrgw) mRNA, partial cds. | 0,033688 | 0,235122 |
| U50524 | BRCA2 region, mRNA sequence CG017. | 0,096218 | 0,266439 |
| U50531 | BRCA2 region, mRNA sequence CG030. | 0,06872 | 0,305838 |
| U58663 | mRNA upregulated during camptothecin-induced apoptosis of U937 cells. | 0,076544 | 0,204923 |
| W02242 | za57b04.r1 Soares fetal liver spleen 1NFLS cDNA clone IMAGE:296623 5', mRNA sequence. | 0,063252 | 0,244961 |
| Y11166 | U71b small nucleolar RNA gene. | 0,055786 | 0,484431 |
| Y16713 | mRNA from HIV associated non-Hodgkin's lymphoma (clone h11-5). | 0,007383 | 2,258568 |
| Z24725 | mitogen inducible gene mig-2, complete CDS. | 0,066417 | -0,22419 |
| Z34278 | H. (JER58) MUC5AC mRNA for mucin (partial). | 0,058672 | -4,85491 |
| Z34281 | H (MAR10) MUC5AC mRNA for mucin (partial). | 0,088138 | -5,17796 |
| Z36818 | (xs166) mRNA, 400bp. | 0,092641 | 0,202657 |

**Table 18: Marker genes with altered expression in three or more different chromosomal aberrations in colorectal adenocarcinoma cells.**

| Gene ID | Description (Table 18) | chromosomal aberration |
|---|---|---|
| | marker genes with altered expression in 5 chromosomal aberrations | |
| | | |
| NM_016397 | TH1-like (Drosophila) (TH1L), mRNA. | 8p loss |
| | | 8q gain |
| | | 15q loss |
| | | 18q loss |
| | | 20q gain |
| | | |
| | marker genes with altered expression in chromosomal aberrations | |
| | | |
| NM_006602 | transcription factor-like 5 (basic helix-loop-helix) (TCFL5), mRNA. | 8p loss |
| | | 13q gain |
| | | 18q loss |
| | | 20q gain |
| NM_016646 | mesenchymal stem cell protein DSCD28 (LOC51336), mRNA. | 13q gain |
| | | 15q loss |
| | | 18q loss |
| | | 20q gain |
| NM_018270 | chromosome20 open reading frame20 (C20orf20), mRNA. | 8p loss |
| | | 13q gain |
| | | 18q loss |
| | | 20q gain |
| NM_021945 | hypothetical protein FLJ22174 (FLJ22174), mRNA. | 13q gain |
| | | 15q loss |
| | | 18q loss |
| | | 20q gain |
| NM_024522 | hypothetical protein FLJ12650 (FLJ12650), mRNA. | 8q gain |
| | | 15q loss |
| | | 18q loss |
| | | 20q gain |
| NM_030579 | cytochrome b5 outer mitochondrial membrane precursor (CYB5-M), mRNA. | 8p loss |
| | | 13q gain |
| | | 15q loss |
| | | 18q loss |
| NM_033126 | serine/threonine kinase PSKH2 (PSKH2), mRNA. | 8p loss |
| | | 8q gain |
| | | 18q loss |
| | | 20q gain |
| AB033100 | mRNA for KIAA1274 protein, partial cds. | 8p loss |
| | | 8q gain |
| | | 18q loss |
| | | 20q gain |
| NM_002296 | lamin B receptor (LBR), mRNA. | 13q gain |
| | | 15q loss |
| | | 18q loss |
| | | 20q gain |
| NM_003839 | tumor necrosis factor receptor superfamily, member 11a, activator of NFKB (TNFRSF11A), mRNA. | 8q gain |
| | | 13q gain |
| | | 18q loss |
| | | 20q gain |
| | | |
| | marker genes with altered expression in 3 chromosomal aberrations | |
| | | |
| AB033070 | mRNA for KIAA1244 protein, partial cds. | 8p loss |
| | | 18q loss |
| | | 20q gain |
| AK057085 | cDNA FLJ32523 fis, clone SMINT2000032. | 13q gain |
| | | 15q loss |
| | | 18q loss |
| AK075564 | cDNA PSEC0264 fis, clone NT2RP3002337. | 8p loss |
| | | 15q loss |
| | | 18q loss |
| BC002877 | Similar to hypothetical protein FLJ11585, clone MGC:11258 IMAGE:3942160, mRNA, complete cds. | 8p loss |
| | | 18q loss |
| | | 20q gain |
| BC008502 | clone MGC:14841 IMAGE:4295121, mRNA, complete cds. | 13q gain |
| | | 18q loss |
| | | 20q gain |
| BC011242 | clone IMAGE:4153763, mRNA. | 13q gain |
| | | 15q loss |
| | | 18q loss |
| BC038852 | clone IMAGE:5168364, mRNA. | 8p loss |
| | | 15q loss |
| | | 18q loss |
| BM921036 | AGENCOURT_6633196 NIH_MGC_115 cDNA clone IMAGE:5752355 5', mRNA sequence. | 8p loss |
| | | 8q gain |
| | | 18q loss |
| NM_000285 | peptidase D (PEPD), mRNA. | 8p loss |
| | | 8q gain |
| | | 18q loss |
| NM_002267 | karyopherin alpha 3 (importin alpha 4) (KPNA3), mRNA. | 13q gain |
| | | 15q loss |
| | | 18q loss |
| NM_002915 | replication factor C (activator 1) 3, 38kDa (RFC3), mRNA. | 13q gain |
| | | 18q loss |
| | | 20q gain |
| NM_003014 | secreted frizzled-related protein 4 (SFRP4), mRNA. | 8p loss |
| | | 8q gain |
| | | 18q loss |
| NM_005469 | peroxisomal acyl-CoA thioesterase (PTE1), mRNA. | 8p loss |
| | | 15q loss |
| | | 18q loss |
| NM_005877 | splicing factor 3a, subunit 1, 120kDa (SF3A1), mRNA. | 8p loss |
| | | 18q loss |
| | | 20q gain |
| NM_006117 | peroxisomal D3,D2-enoyl-CoA isomerase (PECI), mRNA. | 8q gain |
| | | 13q gain |
| | | 18q loss |
| NM_006644 | heat shock 105kD (HSP105B), mRNA. | 8p loss |
| | | 15q loss |
| | | 18q loss |
| NM_006809 | translocase of outer mitochondrial membrane 34 (TOMM34), mRNA. | 8p loss |
| | | 15q loss |
| | | 18q loss |
| NM_007019 | ubiquitin-conjugating enzyme E2C (UBE2C), mRNA. | 8p loss |
| | | 18q loss |
| | | 20q gain |
| NM_007055 | polymerase (RNA) III (DNA directed) (155kD) (RPC155), mRNA. | 8q gain |
| | | 13q gain |
| | | 18q loss |
| NM_014052 | GW128 protein (GW128), mRNA. | 8p loss |
| | | 18q loss |
| | | 20q gain |
| NM_016938 | EGF-containing fibulin-like extracellular matrix protein 2 (EFEMP2), mRNA. | 8p loss |
| | | 18q loss |
| | | 20q gain |
| NM_018414 | GalNAc alpha-2, 6-sialyltransferase I, long form (ST6GalNAcI), mRNA. | 8p loss |
| | | 18q loss |
| | | 20q gain |
| NM_018710 | hypothetical protein DKFZp7620076 (DKFZp7620076), mRNA. | 8p loss |
| | | 18q loss |
| | | 20q gain |
| NM_030789 | histocompatibility (minor) 13 (HM13), mRNA. | 13q gain |
| | | 18q loss |
| | | 20q gain |
| NM_080476 | CDC91 cell division cycle 91-like 1 (S. cerevisiae) (CDC91Ll),mRNA. | 8p loss |
| | | 18q loss |
| | | 20q gain |
| NM_138425 | hypothetical protein BC009925 (LOC113246), mRNA. | 8q gain |
| | | 13 q gain |
| | | 18q loss |
| NM_144726 | hypothetical protein FLJ31951 (FLJ31951), mRNA. | 13q gain |
| | | 15q loss |
| | | 18q loss |
| AL832390 | mRNA, cDNA DKFZp667H2012 (from clone DKFZp667H2012). | 13q gain |
| | | 18q loss |
| | | 20q gain |
| NM_001951 | E2F transcription factor 5, p130-binding (E2F5), mRNA. | 8p loss |
| | | 8q gain |
| | | 18q loss |
| NM_014786 | Rho-specific guanine-nucleotide exchange factor 164 kDa (P164RHOGEF), mRNA. | 8p loss |
| | | 13q gain |
| | | 15q loss |
| NM_017896 | chromosome20 open reading frame 11 (C20orf11), mRNA. | 8p loss |
| | | 18q loss |
| | | 20q gain |
| NM_025138 | hypothetical protein FLJ12661 (FLJ12661), transcript variant 1, mRNA. | 8p loss |
| | | 13q gain |
| | | 18q loss |

## Claims

1. *An in vitro* method for detecting the presence of colorectal adenocarcinoma cells in a patient, the method comprising the steps of:
a) detecting in a test sample of said patient the expression level of at least 12 of the marker genes, selected from the group listed in Table 1, and
b) comparing the expression level of the marker genes used in step a) to that of a control sample,
wherein an elevated or decreased expression level of said at least 12 marker genes in said test sample, compared to said control sample, is indicative of the presence of colorectal adenocarcinoma cells in said patient.

2. *An in vitro* method for detecting the presence of colorectal adenocarcinoma cells in a patient, the method comprising the steps of:
a) detecting in a test sample of said patient the expression level of one or more marker genes, selected from the group listed in Table 17, and
b) comparing the expression level of said at least one marker gene used in step (a) to that of a control,
wherein an elevated or decreased expression level of said one or more marker genes in said test sample, compared to said control sample, is indicative of the presence of colorectal adenocarcinoma cells in said patient.

3. *An in vitro* method for detecting the presence of colorectal adenocarcinoma cells in a patient, the method comprising the steps of:
a) detecting in a test sample of said patient the expression level of one or more marker genes, the expression of which is altered upon the presence of a chromosomal aberration, wherein said one or marker genes comprise:
- two or more marker genes selected from the group of marker genes depicted in Table 2 of which the expression level is altered by chromosomal loss at chromosome 8p and/or,
- one or more marker genes selected from the group of marker genes depicted in Table 3 of which the expression level is altered by chromosomal gain at chromosome 8q and/or,
- three or more marker genes selected from the group of marker genes depicted in Table 4 of which the expression level is altered by chromosomal gain at chromosome 13q, and/or
- one or more marker genes selected from the group of marker genes depicted in Table 5 of which the expression level is altered by chromosomal loss at chromosome 15q,and/or
- one or more marker genes selected from the group of marker genes depicted in Table 6 of which the expression level is altered by chromosomal loss at chromosome 17p and/or,
- three or more marker genes selected from the group of marker genes depicted in Table 7 of which the expression level is altered by chromosomal loss at chromosome 18q and/or,
- nine or more marker genes selected from the group of marker genes depicted in Table 8 of which the expression level is altered by chromosomal gain at chromosome 20q, and/or
- two or more marker genes selected from the group of marker genes depicted in Table 9 of which the expression level is altered by chromosomal gain at chromosome 20q; and
b) comparing the expression level of the one or more marker genes used in step (a) to that of control sample(s);
wherein an elevated or decreased expression level of said one or more marker genes in said test sample, compared to the control sample, is indicative of the presence of adenocarcinoma cells in said patient.

4. *An in vitro* method for detecting the presence of colorectal adenocarcinoma cells in a patient, the method comprising the steps of:
a) detecting in a test sample of said patient the expression level of a plurality of marker genes the expression of which is altered upon the presence of a chromosomal aberration, said plurality of marker genes comprising:
- at least one marker gene of which the expression level is altered by chromosomal loss at chromosome 8p, selected from the group of marker genes depicted in Table 2, and
- at least one marker gene of which the expression level is altered by chromosomal gain at chromosome 8q, selected from the group of marker genes depicted in Table 3, and
- at least one marker gene of which the expression level is altered by chromosomal gain at chromosome 13, selected from the group of marker genes depicted in Table 4, and
- at least one marker gene of which the expression level is altered by chromosomal loss at chromosome 15q, selected from the group of marker genes depicted in Table 5, and
- at least one marker gene of which the expression level is altered by chromosomal loss at chromosome 17p, selected from the group of marker genes depicted in Table 6, and
- at least one marker gene of which the expression level is altered by chromosomal loss at chromosome 18q, selected from the group of marker genes depicted in Table 7, and
- at least one marker gene of which the expression level is altered by chromosomal gain at chromosome 20q, selected from the group of marker genes depicted in Table 8 or in Table 9; and
b) comparing the expression level of the marker genes used in step a) to that of a control sample,
wherein an elevated or decreased expression level of said plurality of markers in said test sample, compared to the control sample, is indicative of the presence of adenocarcinoma cells in said patient.

5. The method according to any of claims 1 to 4 wherein said marker genes have a p or FDR value below 0,05 in accordance with Tables 1 to 9.

6. The method according to any of claims 1 to 4, wherein said marker genes have a p or FDR value below 0,01, in accordance with Tables 1 to 9.

7. The method according to any of claims 2 to 6, wherein said marker genes have a difference in expression level of at least '2' between an adenoma and an adenocarcinoma cell, in accordance with Tables 2 to 9.

8. The method according to any of claims 2 to 6, wherein said marker genes have a difference in expression level of at least '4' between an adenoma and an adenocarcinoma cell in accordance with Tables 2 to 9.

9. The method according to claims 3 or 4, wherein the marker genes, the expression level of which is altered by a chromosomal loss at chromosome 8p, are selected from the group of marker genes consisting of NM_020749, NM_004315, NM_003747, NM_016353, NM_152415, NM_006197, NM_000662, NM_000015, D31887, NM_017884, NM_004462, NM_006765, NM_001715, NM_012331, NM_139167, NM_013354 and NM_005144, depicted in Table 10.

10. The method according to claims 3 or 4 wherein the marker genes, the expression level of which is altered by a chromosomal gain at chromosome 8q, are selected from the group of marker genes consisting of NM_138455, NM_032611, NM_032862, AL713790, BC030520, NM_024035, NM_017767, NM_002346, AF289596, NM_012162 and AB051475, depicted in Table 11.

11. The method according to claims 3 or 4, wherein the marker genes, the expression level of which is altered by a chromosomal gain at chromosome 13q, are selected from the group of marker genes consisting of NM_145293, NM_005358, U50531, NM_012158, NM_017817, NM_003899, NM_003903, NM_018386, BC008975, NM_023011, NM_001260, NM_006646, U50524, NM_033111, NM_024808, NM_014832, NM_006002, NM_015057, NM_024546, BC026126, NM_006493, NM_018210 and NM_017664, depicted in Table 12.

12. The method according to claims 3 or 4, wherein the marker genes the expression level of which is altered by a chromosomal loss at chromosome 15q, are selected from the group of marker genes consisting of NM_030574, NM_004255, NM_002573, AB033025, NM_033240, NM_000126, NM_015079, NM_015969 and NM_016073, depicted in Table 13.

13. The method according to claims 3 or 4, wherein the marker genes, the expression level of which is altered by a chromosomal loss at chromosome 17p, are selected from the group of marker genes consisting of NM_130766, NM_015721 and NM_031430, depicted in Table 14.

14. The method according to claims 3 or 4, wherein the marker genes, the expression level of which is altered by a chromosomal loss at chromosome 18q, are selected from the group of marker genes consisting of NM_004715, NM_006701 and NM_014913, depicted in Table 15.

15. The method according to claims 3 or 4 wherein the marker genes, the expression level of which is altered by a chromosomal gain at chromosome 20q, are selected from the group of marker genes consisting ofNM_016397, NM_018270, NM_006602, NM_080476, NM_017896, NM_006097, NM_021809, NM_018840, NM_003600, NM_017495, NM_007002, NM_016354, NM_014071, NM_002212, NM_003185, NM_152255, NM_022082, NM_018244, NM_014902, NM_032013, NM_020182, NM_006886, NM_020673, BC003122, NM_012325, NM_014183, NM_0211 00, NM_004738, NM_016045, NM_014054, NM_022105, NM_015666, NM_032527, BC025345, NM_033405, NM_006892, NM_005225, NM_000687, BC035639, NM_018677, NM_006047, NM_016436, NM_015511, NM_016082, NM_007238, NM_003908, NM_003610, NM_153360, NM_080425, NM_000114, NM_001853, NM_144498, NM_017798 and NM_012384, depicted in Table 16.

16. The method according to claims 3 or 4, wherein the marker genes the expression level of which is altered by a chromosomal gain at chromosome 20q are selected from the group of marker genes consisting of NM_016397, NM_018270, NM_006602, NM_080476, NM_017896, NM_006097, NM_021809, NM_018840, NM_003600, NM_017495, NM_007002, NM_016354, NM_014071, NM_002212, NM_003185, NM_152255, NM_022082, NM_018244, NM_014902, NM_032013, NM_020182, NM_006886, NM_020673, BC003122, NM_012325, NM_014183, NM_021100, NM_004738, NM_016045, NM_014054, NM_022105, NM_015666, NM_032527, BC025345 and NM_033405, depicted in Table 16.

17. The method according to any of claims 1 to 16, wherein the test sample is a biopsy or resection of colorectal adenoma tissue.

18. The method according to any of claims 1 to 16, wherein the test sample is selected from the group consisting of a sample of urine, blood, saliva, sweat and stool.

19. The method according to any of claims 1 to 17 wherein the control sample is a sample of the same tissue of a patient comprising colorectal adenoma cells and not colorectal carcinoma cells.

20. The method according to any of claims 1 to 19, wherein the expression level is determined at the protein level.

21. The method according to any of claims 1 to 19, wherein the expression level is determined at the RNA level.

22. The method according to any of claims 1 to 21 wherein the marker genes used are marker genes of which the expression level is elevated in adeocarcinoma cells compared to adenoma cells.

23. Use of the method according to any of claims 1 to 22 for diagnosing the progression of a colorectal adenoma into a colorectal carcinoma.

24. A kit for detecting colorectal adenocarcinoma cells comprising agents for specifically detecting the expression of at least 12 of the marker genes depicted in Table 1.

25. A kit for detecting colorectal adenocarcinoma cells comprising agents for specifically detecting the expression of at least one of the marker genes depicted in Table 17.

26. A kit for detecting colorectal adenocarcinoma cells comprising agents for specifically detecting the expression of one or more marker genes, said kit comprising:
- agents for specifically detecting three or more marker genes selected from the group depicted in Table 2 and/or,
- agents for specifically detecting one or more marker genes selected from the group depicted in Table 3 and/or,
- agents for specifically detecting two or more marker genes selected from the group depicted in Table 4 and/or,
- agents for specifically detecting one or more marker genes selected from the group depicted in Table 5 and/or,
- agents for specifically detecting one or more marker genes selected from the group depicted in Table 6 and/or,
- agents for specifically detecting three or more marker genes selected from the group depicted in Table 7 and/or,
- agents for specifically detecting seven or more marker genes selected from the group depicted in Table 8 and/or,
- agents for specifically detecting seven or more marker genes selected from the group depicted in Table 9.

27. A kit for detecting colorectal adenocarcinoma cells comprising a set of agents for specifically detecting the expression of at least one marker gene of each of the following groups: the marker genes depicted in Table 2, the marker genes depicted in Table 3, the marker genes depicted in Table 4, The marker genes depicted in Table 5, the marker genes depicted in Table 6, the marker genes depicted in Table 7, the marker genes depicted in Table 8 and the marker genes depicted in Table 9.

28. The kit according to any of claims 24 to 27, wherein said marker genes have a por FDR value below 0,05 according to Table 1 to 9.

29. The kit according to any of claims 24 to 27, wherein said marker genes have a p or FDR value below 0,01 according to Table 1 to 9.

30. The kit according to any of claims 25 to 29, wherein said marker genes have a difference in expression level between an adenoma and an adenocarcinoma cell of at least "2", according to Table 2 to 9 or 17.

31. The kit according to any of claims 25 to 29, wherein said marker genes have a difference in expression level between an adenoma and an adenocarcinoma cell of at least "4", according to Table 2 to 9 or 17.

32. The kit according to any of claims 24 to 31, wherein the agents are oligonucleotides or antibodies.

33. The kit according to claim 32, wherein the oligonucleotides are arrayed on a support.

34. The kit according to claim 32, wherein the antibodies bind to secreted proteins or proteins on the cell surface.

35. The kit according to any of claims 24 to 34, wherein one or more agents are functionalised with a label selected from a radioactive label, a magnetic label an MRI contrast label, a chromophoric label, and an ultrasound label.

36. The kit according to any one of claims 24 to 34, wherein said marker genes are genes of which the expression is elevated in adenocarcinoma cells compared to adenoma cells.

37. An *in vivo* method for detecting adenocarcinoma cells in a colorectal lesion said method comprising the steps of:
a) contacting said colorectal lesion with a labelled agent capable of specifically binding or interacting with a protein expressed by a marker gene selected from the gene depicted in Table 17, and
b) detecting the binding or interaction of the agent with said colorectal lesion,
wherein the detection of a difference in the binding or interaction of said agent within loci of said colorectal lesion is indicative for the presence of adenocarcinoma cells in said lesion.

38. The method according to claim 37, wherein said marker gene is a protein located at the cell membrane, a secreted protein or an enzyme.

39. An agent binding or interacting specifically with a marker gene depicted in Table 17 or 18, for use as an in vivo diagnostic for detecting the presence of an adenocarcinoma cell in a colorectal adenoma tissue.

40. Use of an agent binding or interacting specifically with a marker gene depicted in Table 17 or 18, in the manufacture of a diagnostic for detecting the presence of adenocarcinoma cell in a colorectal tumour tissue.
